(19) Europäisches Patentamt
European Patent Office
Office européen des brevets

(11) Numéro de publication : **0 346 239 B1**

(12) # FASCICULE DE BREVET EUROPEEN

(45) Date de publication du fascicule du brevet : **02.06.93 Bulletin 93/22**

(51) Int. Cl.$^5$ : **C07D 279/28,** C07D 417/12, C07D 417/04, A61K 31/54

(21) Numéro de dépôt : **89401602.1**

(22) Date de dépôt : **09.06.89**

(54) **Nouveau derivés de la phénothiazine, leur préparation et les compositions pharmaceutiques qui les contiennent.**

(30) Priorité : **10.06.88 FR 8807771**

(43) Date de publication de la demande : **13.12.89 Bulletin 89/50**

(45) Mention de la délivrance du brevet : **02.06.93 Bulletin 93/22**

(84) Etats contractants désignés : **AT BE CH DE ES FR GB GR IT LI LU NL SE**

(56) Documents cités : **US-A- 2 957 870** **US-A- 3 112 310**

(73) Titulaire : **RHONE-POULENC SANTE**
**20, avenue Raymond Aron**
**F-92160 Antony (FR)**

(72) Inventeur : **Garret, Claude**
**129 rue Jean Jaurès**
**F-94120 Fontenay sous Bois (FR)**
Inventeur : **Guyon, Claude**
**17 bis avenue Henri Martin**
**F-94100 Saint Maur des Fossés (FR)**
Inventeur : **Plau, Bernard**
**1 Impasse des Rouges-Gorges**
**F-94260 Fresnes (FR)**
Inventeur : **Taurand, Gérard**
**2/124 allée Jean de la Bruyère**
**F-94000 Créteil (FR)**

(74) Mandataire : **Savina, Jacques et al**
**RHONE-POULENC SANTE, Service Brevets,**
**20 Avenue Raymond Aron**
**F-92165 Antony Cédex (FR)**

EP 0 346 239 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Dans le domaine de l'analgésie, les récents progrès de la réceptorologie ont permis de mettre en évidence plusieurs types de récepteurs opiacés notamment Mu et Kappa.

Les composés de type morphinique classiques agissent au niveau des récepteurs Mu, mais présentent l'inconvénient de provoquer des effets secondaires gênants (phénomènes de dépendance physique et psychique, dépression respiratoire...) en conséquence desquels il est risqué d'utiliser de tels produits chez certains sujets.

Les produits plus spécifiques des récepteurs Kappa présentent une activité analgésique puissante sans provoquer les effets secondaires des composés de type morphinique classique.

Il a été trouvé que des dérivés de la phénothiazine ci-après désignés par la formule générale (I) présentent une activité analgésique puissante liée à une affinité préférentielle pour les récepteurs Kappa.

Des amides dérivés de la phénothiazine de formule générale :

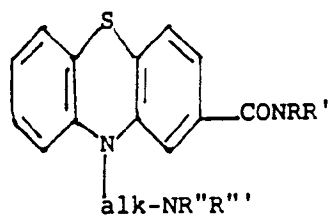

dans laquelle R est notamment un atome d'hydrogène avaient été décrits dans le brevet US 3 112 310 pour leur activité sur le système nerveux central.

Des thioamides dérivés de la phénothiazine de formule générale :

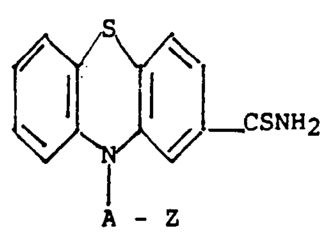

dans laquelle A est une chaîne carbonée et Z est notamment un radical dialcoylamino ou un hétérocycle azoté avaient été décrits dans le brevet belge 612 885 comme neuroleptiques, antiémétiques, adrénolytiques et antituberculeux.

La présente invention concerne les produits de formule générale :

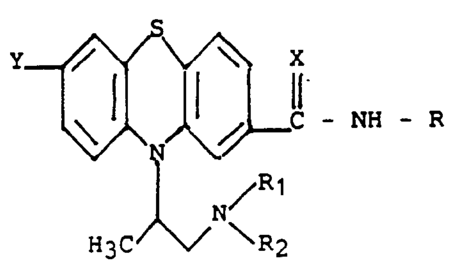

(I)

leurs sels d'addition avec les acides, leur préparation et les compositions pharmaceutiques qui les contiennent.

Dans la formule générale (I) :

le symbole Y représente un atome d'hydrogène ou d'halogène,

les symboles $R_1$ et $R_2$ identiques ou différents représentent des radicaux alcoyle, cycloalcoylalcoyle, hydroxyalcoyle, acétyloxyalcoyle ou forment ensemble avec l'atome d'azote auquel ils sont rattachés, un hétérocycle

saturé ou partiellement insaturé contenant 4 à 7 chaînons et éventuellement substitué par 1 ou 2 radicaux alcoyle, hydroxyalcoyle ou acétyloxyalcoyle, et

- soit le symbole X représente un atome d'oxygène ou de soufre ou un radical $NR_4$, et le symbole R représente un radical cycloalcoyle contenant 4 à 6 chaînons ou phényle, ou représente un radical $-CH_2R_3$ dans lequel $R_3$ est un atome d'hydrogène, un radical alcoyle contenant 1 à 5 atomes de carbone, un radical alcényle ou alcynyle contenant 2 à 4 atomes de carbone, un radical cycloalcoyle contenant 3 à 6 chaînons, un radical phényle éventuellement substitué (par 1 ou 2 atomes d'halogène ou par un radical hydroxy, alcoyle, alcoyloxy, trifluorométhyle ou nitro) ou un radical hétérocyclyle choisi parmi furyle, thiényle ou pyridyle, et le symbole $R_4$ est un atome d'hydrogène ou un radical cyano, étant entendu que le symbole X ne peut pas représenter l'atome d'oxygène lorsque simultanément $R_3$ est un atome d'hydrogène ou un radical alcoyle, $R_1$ et $R_2$ sont des radicaux alcoyle ou forment ensemble un hétérocycle azoté non substitué et Y est un atome d'hydrogène,
- soit le symbole X est un radical $N-R_4$ et le symbole R forme avec $R_4$ et les atomes auxquels ils sont rattachés, un radical imidazolinyle ou imidazolyle éventuellement substitués par 1 ou 2 radicaux alcoyle, ou un radical hexahydrobenzimidazolyle.

Il est entendu que (sauf mention spéciale) les radicaux alcoyle cités ci-dessus sont droits ou ramifiés et contiennent 1 à 4 atomes de carbone.

Lorsque le symbole Y représente un atome d'halogène ou lorsque le symbole R porte un substituant halogène, ce dernier est avantageusement choisi parmi le chlore, le fluor ou le brome.

Les produits de formule générale (I) existent sous des formes isomères, il est entendu que ces formes isomères ainsi que leurs mélanges entrent dans le cadre de la présente invention.

Selon l'invention, les dérivés de la phénothiazine de formule générale (I) pour lesquels X est défini comme précédemment, à l'exception de représenter un radical $NR_4$ dans lequel $R_4$ forme avec R et les atomes voisins un radical imidazolyle, peuvent être obtenus à partir d'un dérivé de la phénothiazine de formule générale :

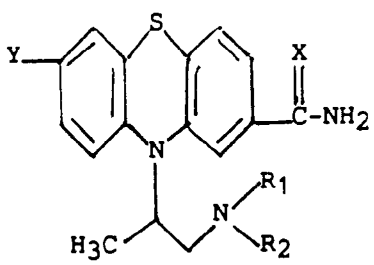

(II)

dans laquelle Y, $R_1$ et $R_2$ sont définis comme précédemment et X représente un atome de soufre ou un radical $N-R_4$ dans lequel $R_4$ est un atome d'hydrogène ou un radical cyano, par action d'une amine de formule générale :

$$R - NH_2 \qquad (III)$$

dans laquelle R est défini comme précédemment, ou par action d'une diamine si X représente un radical NH et si l'on veut obtenir une amidine dans laquelle R et $R_4$ sont liés ensemble avec les atomes voisins pour former un radical imidazolinyle éventuellement substitué ou hexahydrobenzimidazolyle, puis le cas échéant, lorsque l'on veut obtenir l'amide, si l'on a isolé le thioamide substitué de formule générale (I) pour lequel X est un atome de soufre, ce thioamide est oxydé en l'amide correspondant.

La réaction s'effectue avantageusement dans un solvant organique tel qu'un alcool (par exemple l'éthanol, le méthanol, l'isopropanol) ou sans solvant, à une température comprise entre 100 et 250°C. Lorsque l'on opère à partir du thioamide de formule générale (II), il est parfois avantageux d'opérer en présence d'acide sulfhydrique. Lorsque l'on opère à partir de l'amidine de formule générale (II), il est également possible de mettre en oeuvre le sel de ce produit.

Dans la pratique, pour préparer l'amide de formule générale (I), il n'est pas indispensable d'isoler le thioamide substitué intermédiaire.

Lorsque l'on veut isoler directement l'amide de formule générale (I), sans isoler préalablement le thioamide de formule générale (I), on opère par chromatographie ou cristallisation.

Lorsque l'on veut isoler le thioamide substitué de formule générale (I), on opère de préférence en présence d'acide sulfhydrique, puis lorsque l'on veut obtenir l'amide, on oxyde le thioamide secondaire obtenu ou son sel, par toute méthode connue pour obtenir un amide à partir du thioamide correspondant sans toucher au reste de la molécule.

L'oxydation s'effectue avantageusement au moyen d'un sel mercurique (par exemple l'acétate mercurique) ou d'un sel cuivreux, dans un solvant organique tel qu'une cétone (acétone par exemple), un alcool, un ester ou un acide carboxylique tel que par exemple l'acide acétique, à une température comprise entre 0 et 100°C.

Il est également possible d'effectuer l'oxydation par analogie avec les méthodes décrites par :

- H.J. Kim et coll., Synthesis, 11, 970 (1986),
- M.T.M. El-Wassimy, Tetrahedron 39 (10), 1729 (1983),
- K.A. Jørgensen et coll., Tetrahedron 38 (9), 1163 (1982),
- A.G. Samuelson et coll., Tetrahedron Letters, 27 (33), 3911 (1986).

Lorsque l'on veut obtenir un produit de formule générale (I) dans laquelle X est un radical N-$R_4$ et R et $R_4$ sont liés ensemble de telle manière qu'ils forment avec les atomes voisins un radical imidazolinyle éventuellement substitué ou hexahydrobenzimidazolyle, la diamine peut être mise en oeuvre indifféremment sur le thioamide de formule générale (II) ou sur l'amidine pour laquelle $R_4$ est un atome d'hydrogène.

Lorsque l'on veut obtenir un amide de formule générale (I) dont la chaîne en position -10 porte un substituant acétyloxy, ce substituant peut être mis en place dès le début de la synthèse (préparation du produit de formule générale (V) ci-après) mais il peut être également avantageux de mettre en oeuvre un dérivé de la phénothiazine de formule générale (II) portant un substituant hydroxy sur la chaîne en position -10, en isolant le thioamide secondaire de formule générale (I) puis en oxydant ce produit au moyen de l'acétate mercurique et en séparant le produit attendu par chromatographie ou cristallisation.

Selon l'invention, les amides de formule générale (I) pour lesquels X est un atome d'oxygène peuvent aussi être obtenus à partir d'un acide de formule générale :

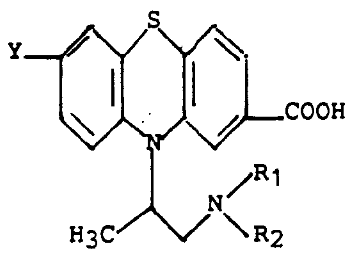

(IV)

dans laquelle Y, $R_1$ et $R_2$ sont définis comme précédemment, par toute méthode connue pour obtenir un amide substitué à partir d'un acide sans toucher au reste de la molécule.

On opère notamment par action d'une amine de formule générale (III) sur un dérivé réactif de l'acide (éventuellement préparé in situ), par exemple le chlorure d'acide, un ester activé ou un anhydride mixte, dans un solvant organique tel qu'un éther, un solvant chloré (chlorure de méthylène, chloroforme, dichloréthane, par exemple) ou dans un amide (diméthylformamide), en présence d'un accepteur d'acide tel qu'une base organique azotée comme par exemple une trialcoylamine (triéthylamine notamment), à une température comprise entre -40 et +40°C.

Il est également possible de faire réagir l'amine de formule générale (III) directement sur l'acide en opérant en présence d'un agent de condensation tel qu'un carbodiimide (dicyclohexylcarbodiimide), le NN'-carbonyldiimidazole ou le N-hydroxybenzotriazole dans un solvant organique tel que cité ci-dessus, et à une température telle que définie ci-dessus.

Il est entendu que dans les cas où le radical R de l'amine de formule générale (III) contient des fonctions pouvant interférer avec la réaction, ce dernier doit être préalablement protégé. Le radical protecteur mis en place est éliminé postérieurement à la réaction. Notamment lorsque le radical R contient un radical hydroxy, il est préférable de protéger ce radical. La protection s'effectue par exemple sous forme d'un radical méthoxy ou benzyloxy qui peuvent être respectivement éliminés par traitement par l'acide bromhydrique ou le tribromure de bore, ou par hydrogénolyse dans le cas du radical benzyloxy.

Selon l'invention, les produits de formule générale (I) pour lesquels X est un atome d'oxygène peuvent également être obtenus à partir d'un nitrile de formule générale :

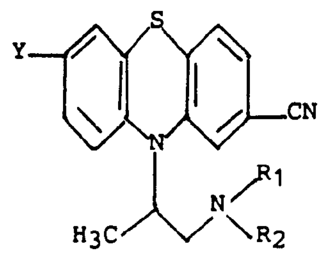

(V)

dans laquelle Y, $R_1$ et $R_2$ sont définis comme précédemment, par toute méthode connue pour obtenir un amide substitué à partir d'un nitrile sans toucher au reste de la molécule.

Notamment on prépare in situ un imidate intermédiaire sur lequel on fait agir un dérivé halogéné de formule générale :

R - Hal (VI)

dans laquelle R est défini comme précédemment et Hal représente un atome d'iode ou de brome.

De préférence la réaction s'effectue dans un mélange alcool-alcoolate ou alcool-potasse par exemple tertiobutanol - tertiobutylate de potassium, tertiobutanol-potasse ou isobutanol - isobutylate de potassium, à une température comprise entre 50 et 150°C.

Il est également possible d'opérer en présence d'un fort excès d'une amine de formule générale (III), avec ou sans solvant, à une température comprise entre 150 et 250°C.

Le cas échéant les solvants sont avantageusement choisis parmi les alcools (éthanol, méthanol par exemple), les éthers à haut point d'ébullition, les polyéthers, les hydrocarbures aromatiques (par exemple le toluène, le xylène ou le chlorobenzène).

Il est également possible d'opérer selon la méthode décrite par S. LINKE, Synthesis, 4, 303 (1978) ou selon la méthode décrite par G.W. CANNON et coll., J. Org. Chem., 18, 516 (1953).

Selon l'invention les carboxamidines de formule générale (I) dans laquelle X est un radical N-$R_4$ pour lequel $R_4$ est un atome d'hydrogène et R est défini comme précédemment ou $R_4$ forme avec R et les atomes auquels il sont rattachés un radical imidazolinyle éventuellement substitué ou un radical hexahydrobenzimidazolyle, peuvent aussi être préparés à partir d'un imidate de formule générale :

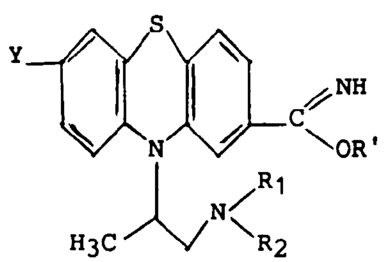

(VII)

dans laquelle Y, $R_1$ et $R_2$ sont définis comme précédemment et R' est un radical alcoyle (contenant 1 à 10 atomes de carbone) ou de son sel par action d'une amine de formule générale (III) ou une diamine telles que $R_4$ et R soient définis comme ci-dessus.

La réaction s'effectue généralement dans un solvant organique tel qu'un alcool (méthanol, éthanol par exemple) à une température comprise entre -10 et 60°C.

Selon l'invention, les dérivés de la phénothiazine de formule générale (I) pour lesquels X est un radical N-$R_4$ et R forme avec $R_4$ un radical imidazolyle éventuellement substitué par un radical alcoyle, peuvent également être obtenus à partir d'un dérivé de la phénothiazine de formule générale (II) dans laquelle X est un radical NH et Y, $R_1$ et $R_2$ sont définis comme précédement, ou de son sel par action d'une α-halogénocétone ou d'un α-halogénoaldéhyde, de formule générale :

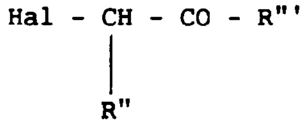

(VIII)

dans laquelle Hal est un atome d'halogène et, R” et R‴ identiques ou différents représentent un atome d'hydrogène ou un radical alcoyle.

La réaction s'effectue avantageusement dans un solvant organique tel qu'un alcool (méthanol, éthanol par exemple) un éther (tétrahydrofuranne, diglyme ou dioxanne par exemple) ou un solvant halogéné (chlorure de méthylène, chloroforme, dichloro-1,2 éthane par exemple) à une température comprise entre 10°C et la température de reflux du mélange réactionnel.

De préférence l'atome d'halogène est choisi parmi le chlore et le brome.

Le dérivé de la phénothiazine de formule générale (II) pour lequel X est un atome de soufre peut être obtenu à partir d'un nitrile de formule générale (V) par toute méthode connue pour obtenir un thioamide à partir d'un nitrile, sans toucher au reste de la molécule.

On opère généralement en milieu basique anhydre, en présence d'acide sulfhydrique, à une température comprise entre 0 et 100°C. La réaction s'effectue avantageusement en présence d'une base organique azotée comme la triéthylamine, dans un solvant organique tel que la pyridine.

Les dérivés de la phénothiazine de formule générale (II) pour lesquels X est un radical $-NR_4$ peuvent être obtenus à partir de l'imidate de formule générale (VII), respectivement par action de l'ammoniac lorsque l'on veut obtenir une amidine pour laquelle $R_4$ est un atome d'hydrogène, ou par action de la cyanamide lorsque l'on veut obtenir une amidine pour laquelle $R_4$ est un radical cyano.

La réaction s'effectue généralement dans un solvant organique tel qu'un alcool (méthanol, éthanol par exemple) ou dans un éther (dioxanne, glyme, diglyme par exemple), à une température comprise entre 0 et 50°C lorsque l'on fait agir l'ammoniac, ou à une température comprise entre 70°C et la température de reflux du mélange réactionnel lorsque l'on fait agir la cyanamide.

L'acide de formule générale (IV) peut être obtenu à partir d'un nitrile de formule générale (V) par toute méthode connue pour obtenir un acide à partir d'un nitrile sans toucher au reste de la molécule. On opère notamment par hydrolyse en milieu acide ou basique dans un solvant organique à une température comprise entre 50°C et la température de reflux du mélange réactionnel. La réaction s'effectue avantageusement dans le glycol en présence de potasse.

Le nitrile de formule générale (V) peut être obtenu selon le schéma suivant :

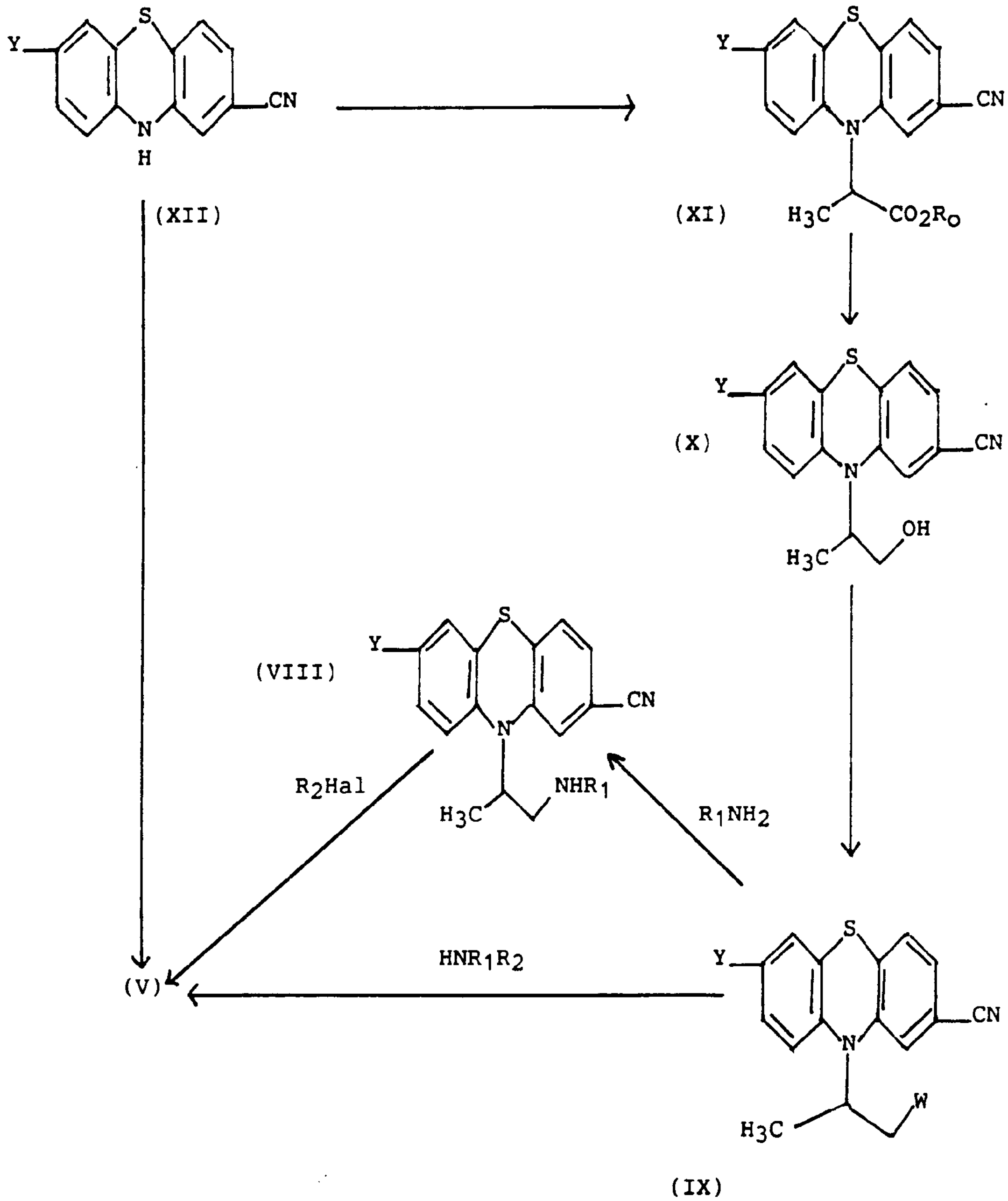


dans lequel W est un reste p.toluènesulfonyloxy, méthylsulfonyloxy ou diaryloxyphosphoryloxy et $R_o$ est un radical alcoyle contenant 1 à 4 atomes de carbone (éthyle par exemple) et dont les conditions opératoires sont définies plus en détail ci-après dans les exemples 2, 8 à 12, 14, 16, 17, 18, 21, 34, 35, 37, 39, 47, 52, 58, 88 à 90.

Le nitrile de formule générale (XII) pour lequel Y est un atome d'hydrogène peut être obtenu comme décrit dans le brevet américain 2 877 224.

Le nitrile de formule générale (XII) pour lequel Y est un atome de chlore ou de brome peut être obtenu par action d'un agent d'halogénation sur le dérivé de la phénothiazine de formule générale (XII) pour lequel Y est un atome d'hydrogène.

L'halogénation s'effectue de préférence par action de l'halogène (chlore, brome) ou du chlorure de sulfuryle, en présence de trichlorure d'aluminium, dans un solvant organique tel qu'un hydrocarbure halogéné (par exemple le dichloroéthane) ou un éther, à une température comprise entre -50°C et la température de reflux du mélange réactionnel.

Lorsque l'on veut obtenir un produit de formule générale (XII) pour lequel Y est un atome de fluor, on opère

selon la méthode décrite dans la demande de brevet GB 2 132 194.

L'iminoéther de formule générale (VII) peut être obtenu par traitement en milieu acide d'un nitrile de formule générale (V), par un alcool de formule générale :

R'OH (XIII)

dans laquelle R' est défini comme précédemment.

Généralement, on opère en présence d'acide chlorhydrique, à une température comprise entre -10 et 60°C.

Les isomères des produits de formule générale (I) peuvent être obtenus selon les méthodes connues.

On opère notamment par préparation de l'isomère du dérivé de la phénothiazine de formule générale (X) qui est ensuite transformé en un dérivé de la phénothiazine de formule générale (I) par les méthodes décrites précédemment.

Le dérivé optiquement actif du produit de formule générale (X) est notamment obtenu par préparation de l'ester d'un diacide, formation d'un sel optiquement actif, séparation des isomères par cristallisation et saponification de l'isomère obtenu.

Plus particulièrement l'ester est obtenu au moyen de l'anhydride d'un diacide comme par exemple l'anhydride phtalique, l'anhydride maléique ou succinique. Le sel est formé par addition d'une amine optiquement active, par exemple la (+) phényl-1 éthylamine, ou la (-) phényl-1 éthylamine.

Dans les exemples qui suivent, on appelle série D les dérivés de la phénothiazine préparés à partir de l'alcool de formule générale (X) pour lequel le pouvoir rotatoire en solution dans le chloroforme est positif; on appelle série L les dérivés de la phénothiazine préparés à partir de l'alcool de formule générale (X) pour lequel le pouvoir rotatoire en solution dans le chloroforme est négatif.

Les produits de formule générale (I) peuvent être purifiés par chromatographie ou cristallisation.

Les dérivés de la phénothiazine de formule générale (I) peuvent être transformés en sels d'addition avec les acides, par action d'un acide dans un solvant organique tel qu'un alcool, une cétone, un ester, un éther ou un solvant chloré. Le sel précipite, éventuellement après concentration de sa solution, il est séparé par filtration ou décantation.

Comme sels pharmaceutiquement acceptables, on peut citer les sels d'addition avec les acides minéraux tels que chlorhydrates, bromhydrates, sulfates, nitrates, phosphates, ou organiques tels que acétates, propionates, succinates, maléates, fumarates, méthanesulfonates, p.toluènesulfonates, iséthionates ou des dérivés de substitution de ces composés.

Les dérivés de la phénothiazine de formule générale (I) présentent une activité analgésique et diurétique particulièrement intéressante du fait de leur affinité préférentielle pour les récepteurs Kappa et de leur faible toxicité.

Ils se sont en effet montrés actifs à des concentrations comprises entre 1 et 500nM dans la méthode de binding à l'éthylkétocyclazocine tritiée dans des homogénats de cervelet de cobaye, inspirée de la technique de L.E. Robson et coll., Opioid binding sites of the Kappa type in ginea pig cerebellum , Neuroscience, 12, 621 (1984).

Ils se sont également montrés actifs dans la technique de l'inhibition des contractions induites par stimulation électrique sur iléon isolé de cobaye (inspirée de W.D.M. Paton, Brit. J. Pharmacol., 11, 119 (1957) à des concentrations comprises entre 1 et 300nM.

Les produits ayant une affinité pour les récepteurs Kappa manifestent un effet diurétique [J.D. Leander, The Journal of Pharmacology and Experimental Therapeutics, 224 (1), 89 (1983) et G.R. Slizgi et coll., The Journal of Pharmacology and Experimental Therapeutics, 230 (3), 641 (1984)]. Il a été également mis en évidence par l'étude de plusieurs produits, que les produits de formule générale (I) manifestent un effet diurétique significatif chez le rat, à des doses comprises entre 1 et 20 mg/kg par voie sous-cutanée, dans la technique décrite par J.D. Leander (référence ci-dessus).

Par ailleurs la toxicité aigue ($DL_{50}$) des produits de formule générale (I) chez la souris est peu élevée aux doses d'utilisation. Leur $DL_{50}$ est généralement comprise entre 30 à 100 mg/kg p.o. et des doses nettement supérieures à 100 mg/kg p.o.

D'un intérêt particulier sont les produits de formule générale (I) dans laquelle :
le symbole Y est un atome d'hydrogène ou d'halogène,
les symboles $R_1$ et $R_2$ identiques ou différents représentent des radicaux alcoyle contenant 1 à 3 atomes de carbone en chaîne droite ou ramifiée, alcoyle (contenant 1 ou 2 atomes de carbone) substitué par cycloalcoyle, hydroxy ou acétyloxy, ou forment ensemble avec l'atome d'azote auquel ils sont rattachés, un hétérocyle saturé ou partiellement insaturé contenant 4 à 7 chaînons et éventuellement substitué par 1 ou 2 radicaux méthyle ou alcoyle (contenant 1 ou 2 atomes de carbone) substitué par hydroxy ou acétyloxy,

- soit le symbole X représente un atome d'oxygène ou de soufre ou un radical $NR_4$, et le symbole R représente un radical cycloalcoyle contenant 4 à 6 chaînons ou phényle, ou représente un radical $-CH_2R_3$

dans lequel $R_3$ est un atome d'hydrogène, un radical alcoyle droit ou ramifié contenant 1 à 5 atomes de carbone, un radical alcényle ou alcynyle contenant 2 à 4 atomes de carbone, un radical cycloalcoyle contenant 3 à 6 chaînons, un radical phényle éventuellement, substitué (par 1 ou 2 atomes d'halogène ou par un radical hydroxy, méthyle, méthoxy trifluorométhyle ou nitro) ou un radical hétérocyclyle choisi parmi furyle, thiényle ou pyridyle, et le symbole $R_4$ est un atome d'hydrogène ou un radical cyano, étant entendu que le symbole X ne peut pas représenter l'atome d'oxygène lorsque simultanément $R_3$ est un atome d'hydrogène ou un radical alcoyle, $R_1$ et $R_2$ sont des radicaux alcoyle ou forment ensemble un hétérocyle azoté non substitué et Y est un atome d'hydrogène,

- soit le symbole X est un radical N-$R_4$ et le symbole R forme avec $R_4$ et les atomes auxquels ils sont rattachés, un radical imidazolyle éventuellement substitué par 1 ou 2 radicaux méthyle ou éthyle ou un radical hexahydrobenzimidazolyle.

Parmi ces produits, plus spécialement actifs sont les dérivés de phénothiazine de formule générale (I) pour lesquels :

le symbole Y est un atome d'hydrogène ou d'halogène,

les symboles $R_1$ et $R_2$ identiques ou différents représentent des radicaux alcoyle contenant 1 ou 2 atomes de carbone à l'exception de représenter simultanément des radicaux méthyle, acétyloxyalcoyle dont la partie alcoyle contient 1 ou 2 atomes de carbone ou forment ensemble avec l'atome d'azote auquel ils sont rattachés, un hétérocycle saturé ou partiellement insaturé contenant 4 à 7 chaînons et éventuellement substitué par 1 ou 2 radicaux méthyle, hydroxyméthyle ou acétyloxyméthyle et,

- soit le symbole X représente un atome d'oxygène ou de soufre ou un radical N-$R_4$, et le symbole R représente un radical cycloalcoyle contenant 4 à 6 chaînons ou représente un radical -$CH_2R_3$ dans lequel $R_3$ est un radical alcoyle droit ou ramifié conenant 1 à 5 atomes de carbone, un radical alcényle ou alcynyle contenant 2 à 4 atomes de carbone, un radical cycloalcoyle contenant 3 à 6 chaînons, ou un radical phényle éventuellement substitué (par 1 ou 2 atomes d'halogène ou par un radical hydroxy, méthyle, méthoxy, trifluro- méthyle ou nitro), ou un radical hétérocyclyle choisi parmi furyle, thiényle ou pyridyle, et le symbole $R_4$ est un atome d'hydrogène ou un radical cyano, étant entendu que le symbole X ne peut pas représenter l'atome d'oxygène lorsque simultanément $R_3$ est un atome d'hydrogène ou un radical alcoyle, $R_1$ et $R_2$ sont des radicaux alcoyle ou forment ensemble un hétérocycle azoté non substitué et Y est un atome d'hydrogène,
- soit le symbole X est un radical N-$R_4$ et le symbole R forme avec $R_4$ et les atomes auxquels ils sont rattachés, un radical imidazolyle éventuellement substitué par 1 radical alcoyle contenant 1 ou 2 atomes de carbone, ou un radical hexahydrobenzimidazolyle, sous forme d'un mélange d'isomères ou sous forme de leurs isomères de série L ;

et notamment les produits suivants :

- N-cyclobutylméthyl [(pyrrolidinyl-1-)-1 propyl-2]-10 phénothiazinecarboxamide-2 ;
- N-(chloro-3 benzyl) [(pyrrolidinyl-1)-1 propyl-2]-10 phénothiazinecarboxamide-2 ;
- N-(chloro-2 benzyl) [(pyrrolidinyl-1)-1 propyl-2]-10 phénothiazinecarboxamide-2 ;
- N-(fluoro-2 benzyl) [(pyrrolidinyl-1)-1 propyl-2]-10 phénothiazinecarboxamide-2 ;
- N-benzyl [(pyrrolidinyl-1)-1 propyl-2]-10 phénothiazinecarboxamide-2.

Les exemples suivants, donnés à titre non limitatif, illustrent la présente invention.

## EXEMPLE 1

Une solution de 1,3 g de [(N-méthyl N-éthyl-amino)-1 propyl-2-(2RS)]-10 phénothiazinecarbothioamide-2 et de 52 cm3 d'une solution à 33% de méthylamine dans l'éthanol dans 26 cm3 d'éthanol absolu est chauffée pendant 16 heures à une température voisine de 100°C. Après refroidissement, on concentre à sec sous pression réduite (30 mm de Hg; 4 kPa) à 40°C. Le résidu est dissous dans 100 cm3 d'acétate d'éthyle et la solution est lavée par 2 fois 50 cm3 d'eau distillée, séchée sur sulfate de magnésium, filtrée et concentrée à sec sous pression réduite (30 mm de Hg; 4 kPa) à 40°C. Le résidu gommeux jaune (1,1 g) est purifié par chromatographie sur une colonne (hauteur : 20 cm; diamètre : 3 cm) de gel de silice (0,04-0,063 mm) avec une légère surpression d'azote (40 kPa) en éluant successivement avec 300 cm3 d'un mélange de chlorure de méthylène et de méthanol (97,5-2,5 en volumes) puis 500 cm3 d'un mélange de chlorure de méthylène et de méthanol (95-5 en volumes) et en recueillant des fractions de 25 cm3. Les fractions 13 à 28 sont réunies et concentrées à sec sous pression réduite (30 mm-de Hg; 4 kPa) à 40°C. L'huile jaune résiduelle (0,55 g) est dissoute dans 100 cm3 d'oxyde d'isopropyle et 10 cm3 d'acétate d'éthyle et la solution est additionnée sous agitation, de 0,5 cm3 d'une solution d'acide chlorhydrique 3N dans l'éther éthylique. Le solide formé est essoré, lavé par 5 cm3 d'oxyde d'isopropyle et séché sous pression réduite (5 mm de Hg; 0,68 kPa) à 40°C. On obtient ainsi 0,58 g de chlorhydrate de N-méthyl [N'-méthyl N'-éthyl-amino)-1 propyl-2-(2RS)]-10 phénothiazinecarbothioamide-2 sous forme de

cristaux jaunes fondant à 140-145°C (fusion pâteuse).

RMN du proton (250 MHz, DMSO, δ en ppm, J en Hz).

En solution dans le DMSO, on observe deux formes dues à la salification de l'azote.

1,07 et 1,21 (2T, J = 7, 3H, $-NCH_2C\underline{H}_3$); 1,85 (D, J = 7, 3H, $-CH_3$); 2,8 (Mf, 3H, $>N-CH_3$); 3,19 (D, J = 5, 3H, $-CSNH-C\underline{H}_3$); environ 3,20 (Mt, 2H, $>N-C\underline{H}_2-CH_3$); 3,51 et 3,78 (2Mt, de 1H chacun, $>N-CH_2-$); 4,86 (Mf, 1H, $>N-CH<$); 7,05 à 7,4 (Mt, 5H, aromatiques); 7,52 (D large, J = 8, 1H, -H en 3); 7,65 (S large, 1H, -H en 1); 10,37 et 10,63 (2Mf, de 1H chacun, $-NH^+$ et -CSNH-).

Spectre infra-rouge (KBr), bandes caractéristiques en $cm^{-1}$ : 3200, 2970, 2650, 2480, 1590, 1535, 1460, 880, 820, 755.

EXEMPLE 2

Un mélange de 10 cm3 d'une solution à 33% de méthylamine dans l'éthanol absolu et de 2 g de [diéthylamino-1 propyl-2-(2RS)]-10 phénothiazinecarbothioamide-2, dans 15 cm3 d'éthanol absolu est porté pendant 24 heures à une température voisine de 100°C. Après refroidissement, on concentre à sec sous pression réduite (30 mm de Hg; 4 kPa) à 40°C. Le résidu est repris par 100 cm3 d'acétate d'éthyle, lavé par 2 fois 50 cm3 d'eau distillée, séché sur sulfate de magnésium en présence de noir 3S, filtré et concentré à sec sous pression réduite (30 mm de Hg; 4 kPa) à 40°C. L'huile orangée résiduelle (1,7 g) est purifiée par chromatographie sur une colonne (hauteur : 25 cm; diamètre : 2,5 cm) de gel de silice (0,04-0,063 mm) avec une légère surpression d'azote (40 kPa) en éluant successivement avec 750 cm3 d'acétate d'éthyle et avec 500 cm3 d'un mélange d'acétate d'éthyle et d'éthanol (90-10 en volumes) et en recueillant des fractions de 50 cm3. Les fractions 2 à 7 sont réunies et concentrées à sec sous pression réduite (30 mm de Hg; 4 kPa) à 40°C. L'huile brune résiduelle (1,3 g) est dissoute à 50°C dans 3 cm3 d'éthanol et la solution obtenue est versée dans une solution de 0,39 g d'acide fumarique dans 3 cm3 d'éthanol, sous agitation et à une température voisine de 60°C. Après 1 heure sous agitation à une température voisine de 5°C, le solide formé est essoré, lavé par 3 fois 1 cm3 d'éthanol et séché à l'air. On redissout ce solide dans 20 cm3 d'éthanol au reflux puis on conserve 1 heure à une température voisine de 5°C. Le solide formé est essoré, lavé par 3 fois 1 cm3 d'éthanol et séché sous pression réduite (5 mm de Hg; 0,68 kPa) à 40°C. On obtient ainsi 1,15 g de fumarate acide de [diéthylamino-1 propyl-2-(2RS)]-10 N-méthyl phénothiazinecarbothioamide-2 sous forme d'un solide jaune fondant à 224°C.

RMN du proton (250 MHz, DMSO, δ en ppm, J en Hz).

0,9 (T, J = 7, 6H, $-N(CH_2C\underline{H}_3)_2$); 1,65 (D, J = 7, 3H, $-CH_3$); 2,6 (Mt, 4H, $-N(C\underline{H}_2CH_3)_2$); 2,85 (DD, J = 14 et 6, 1H, 1H du $>N-CH_2-$); 3,14 (DD, J = 14 et 7,5, 1H, 1H du $>NCH_2-$); 3,12 (D, J = 4,5, 3H, $>N-CH_3$); 4,27 (Mt, J = 7,5 - 7 et 4,5, 1H, $>N-CH<$); 6,58 (S, 2H, -CH=CH- du fumarate); 6,9 à 7,25 (Mt, 5H, aromatiques); 7,3 (DD, J = 8 et 1, 1H, -H en 3); 7,65 (D, J = 1, 1H, -H en 1); 10,28 (Q, J = 4,5, 1H, -CSNH-).

Spectre infra-rouge (KBr), bandes caractéristiques en $cm^{-1}$ : 3230, 2980, 2650, 2490, 1690, 1615, 1590, 1540, 1465, 980, 750, 645.

Une solution de 5,66 g de [diéthylamino-1 propyl-2-(2RS)]-10 phénothiazinecarbonitrile-2 et de 2,35 cm3 de triéthylamine dans 113 cm3 de pyridine est additionnée d'acide sulfhydrique pendant 1 heure puis agitée pendant 3 jours à une température voisine de 20°C. La solution verdâtre obtenue est purgée à l'azote, versée dans 500 cm3 d'eau distillée et extraite par 2 fois 500 cm3 d'acétate d'éthyle. Les phases organiques réunies sont lavées successivement par 2 fois 200 cm3 d'eau distillée et par 200 cm3 d'une solution aqueuse saturée de chlorure de sodium, séchées sur sulfate de magnésium, filtrées et concentrées à sec sous pression réduite (30 mm de Hg; 4 kPa) à 40°C. L'huile orangée résiduelle (9,1 g) est purifiée par chromatographie sur une colonne (hauteur : 25 cm; diamètre : 3 cm) de gel de silice (0,04-0,063 mm) avec une légère surpression d'azote (40 kPa) en éluant avec 750 cm3 d'acétate d'éthyle et en recueillant des fractions de 50 cm3. Les fractions 4 à 10 réunies sont concentrées à sec sous pression réduite (30 mm de Hg; 4 kPa) à 40°C. On obtient ainsi 6,2 g de [diéthylamino-1 propyl-2-(2RS)]-10 phénothiazinecarbothioamide-2 sous forme d'une huile orangée.

Le [diéthylamino-1 propyl-2-(2RS)]-10 phénothiazinecarbonitrile-2 peut être préparé de la manière suivante :

A une solution de 114,4 g de p-toluènesulfochlorure dans 600 cm3 de N,N-diméthylformamide, on ajoute goutte à goutte une solution de 78,7 g de diéthylamino-1 propanol-2 dans 60 cm3 de N,N-diméthylformamide en 55 minutes. Le mélange est agité à 25°C pendant 12 heures.

A une solution de 44,86 g de phénothiazinecarbonitrile-2 dans 600 cm3 de N,N-diméthylformamide, on ajoute 19,2 g d'hydrure de sodium à 50% en dispersion dans la vaseline en 20 minutes. Le mélange obtenu est ensuite chauffé à 110°C puis on ajoute en 35 minutes la solution de chlorhydrate de diéthylamino-1 p-toluènesulfonate de propyle-2 précédemment préparée. Le mélange réactionnel est agité à 110°C pendant 7 heures puis dilué, après refroidissement, par 2 litres d'acétate d'éthyle. Le mélange est lavé par 5 fois 1 litre d'eau distillée. La phase organique est extraite par 240 cm3 d'acide chlorhydrique 4N et la phase aqueuse acide est lavée par 750 cm3 d'acétate d'éthyle, puis elle est alcalinisée par 250 cm3 de lessive de soude 5N. Le milieu

alcalin est alors extrait par 1250 cm3 d'acétate d'éthyle. La phase organique est lavée par 3 fois 300 cm3 d'eau distillée, séchée sur sulfate de magnésium, filtrée et concentrée à sec sous pression réduite (30 mm de Hg; 4 kPa) à 40°C. Le résidu est agité en présence de 100 cm3 d'éther éthylique; il y a formation d'un précipité qui est filtré et le filtrat est purifié par chromatographie sur colonne de gel de silice (0,2 - 0,063 mm) (hauteur : 82 cm; diamètre : 4,5 cm) en éluant par un mélange de cyclohexane et d'acétate d'éthyle 80-20 (7 litres) (en volumes) et en recueillant des fractions de 100 cm3. Après concentration à sec sous pression réduite (30 mm de Hg; 4 kPa) à 50°C, on obtient à partir des fractions 4 à 9, 6,76 g de [diéthylamino-1 propyl-2-(2RS)]-10 phénothiazinecarbonitrile-2, et à partir des fractions 10 à 22, 8,5 g d'un mélange de [diéthylamino-1 propyl-2-(2RS)]-10 phénothiazinecarbonitrile-2 et de [diéthylamino-2 propyl-1-(1RS)]-10 phénothiazinecarbonitrile-2. Le mélange est purifié par chromatographie sur colonne de gel de silice (0,2 - 0,063 mm) (hauteur : 67 cm; diamètre : 3,0 cm) en éluant par un mélange de cyclohexane et d'acétate d'éthyle 85-15 (3 litres) (en volumes) et en recueillant des fractions de 50 cm3. Les fractions 22 à 29 sont réunies et concentrées à sec à 50°C sous pression réduite (30 mm de Hg; 4 kPa) pour donner 2,76 g de [diéthylamino-1 propyl-2-(2RS)]-10 phénothiazinecarbonitrile-2.

Spectre RMN du proton (250 MHz, DMSO, δ en ppm, J en Hz) :

0,85 (T, J = 7,5, 6H, $-N(CH_2C\underline{H}_3)_2$; 1,57 (D, J = 7, 3H, $-CH_3$; 2,25 à 2,57 (Mt, environ 4H, $-N(C\underline{H}_2CH_3)_2$); 2,63 (DD, J = 13,5 et 6, 1H, 1H du $>NCH_2$-); 2,98 (DD, J = 13,5 et 6,5, 1H, 1H du $>N-CH_2$-); 4,08 (Mt, J = 7- 6,5 et 6, 1H $>N-CH<$); 6,9 à 7,25 (Mt, 5H, aromatiques); 7,31 (DD, J = 8 et 1, 1H, -H en 3), 7,59 (D, J = 1, 1H, -H en 1).

## EXEMPLE 3

Une solution de 2,9 g de [diéthylamino-1 propyl-2-(2RS)]-10 phénothiazinecarbothioamide-2 et de 7,8 cm3 d'éthylamine dans 40 cm3 d'éthanol absolu est saturée avec de l'acide sulfhydrique puis chauffée pendant 16 heures à une température voisine de 100°C. Après refroidissement, la solution jaune obtenue est concentrée à sec sous pression réduite (30 mm de Hg; 4 kPa) à 40°C. Le résidu huileux orangé est purifié par chromatographie sur une colonne (hauteur : 25 cm; diamètre : 3 cm) de gel de silice (0,04-0,063 mm) avec une légère surpression d'azote (40 kPa) en éluant par 500 cm3 d'un mélange d'acétate d'éthyle et d'éthanol (90-10 en volumes) et en recueillant des fractions de 60 cm3. Les fractions 3 à 5 sont réunies et concentrées à sec sous pression réduite (30 mm de Hg; 4 kPa) à 40°C. L'huile orangée résiduelle (2,38 g) est dissoute à 50°C dans 6 cm3 d'éthanol et versée sur une solution à 60°C de 0,69 g d'acide fumarique dans 6 cm3 d'éthanol, sous agitation. Après 1 heure d'agitation à une température voisine de 5°C, le solide formé est essoré, lavé successivement par 3 fois 2 cm3 d'éthanol et par 25 cm3, puis 5 cm3 de sulfure de carbone et versé sous pression réduite (5 mm de Hg; 0,68 kPa).

On obtient ainsi 2,2 g de fumarate acide de [diéthylamino-1 propyl-2-(2RS)]-10 N-éthyl phénothiazinecarbothioamide-2 sous forme d'un solide jaune fondant à 194°C.

RMN du proton (250 MHz, DMSO, δ en ppm, J en Hz).

0,90 (T, J = 7, 6H, $-N(CH_2C\underline{H}_3)_2$); 1,23 (T, J = 7, 3H, $-NH-CH_2C\underline{H}_3$); 1,65 (D, J = 7, 3H, $-CH_3$); 2,6 (Mt, 4H, $-N(C\underline{H}_2CH_3)_2$); 2,84 (DD, J = 13,5 et 5,5, 1H, 1H du $>N-CH_2$-); 3,13 (DD, J = 13,5 et 7,5, 1H, 1H du $>N-CH_2$-); 3,7 (Mt, $-CSNH-C\underline{H}_2$-); 4,26 (Mt, J = 7,5 - 7 et 5,5, 1H, $>N-CH<$); 6,6 (S, 2H, -CH=CH- du fumarate); 6,9 à 7,25 (Mt, 5H, aromatiques); 7,28 (DD, J = 8 et 1, 1H, -H en 3); 7,63 (D, J = 1, 1H, -H en 1); 9,25 (T, J = 5, 1H, -CSNH-).

Spectre infra-rouge (KBr), bandes caractéristiques en $cm^{-1}$ : 3240, 2980, 2940, 2500, 1690, 1590, 1535, 1465, 975, 870, 800, 750, 640.

## EXEMPLE 4

Un mélange de 4,4 g de méthanesulfonate de [diméthylamino-1 propyl-2-(2RS)]-10 phénothiazinecarbothioamide-2 et de 12,5 cm3 de propylamine dans 50 cm3 d'éthanol absolu est chauffé pendant 22 heures à une température voisine de 100°C. Après refroidissement, on concentre à sec sous pression réduite (30 mm de Hg; 4 kPa) à 40°C. Le résidu est repris par 200 cm3 de chlorure de méthylène et la solution obtenue est lavée par 2 fois 50 cm3 d'eau distillée, séchée sur sulfate de magnésium, filtrée et concentrée à sec sous pression réduite (30 mm de Hg; 4 kPa) à 40°C. La gomme orangée résiduelle est purifiée par chromatographie sur colonne (hauteur : 25 cm; diamètre : 4 cm) de gel de silice (0,04-0,063 mm) avec une légère surpression d'azote (40 kPa) en éluant par 1 litre d'un mélange d'acétate d'éthyle et d'éthanol (75-25 en volumes) et en recueillant des fractions de 60 cm3. Les fractions 5 à 10 sont réunies et concentrées à sec sous pression réduite (30 mm de Hg; 4 kPa) à 40°C. Le résidu gommeux orangé (3,3 g) est dissous dans 100 cm3 d'éther éthylique et additionné, sous agitation à une température voisine de 5°C, de 2,4 cm3 d'une solution 3,3N d'acide chlorhydrique dans l'éther éthylique. Le précipité formé est essoré, lavé par 3 fois 10 cm3 d'éther éthylique et séché sous pression réduite (5 mm de Hg; 0,68 kPa) à 40°C. On obtient ainsi 2,95 g de chlorhydrate de [diméthylamino-1 propyl-2-(2RS)]-10 N-propyl phénothiazinecarbothioamide-2 sous forme d'un solide jaune fondant à 130°C (fusion pâteuse).

RMN du proton (250 MHz, DMSO, δ en ppm, J en Hz).

0,94 (T, J = 7,5, 3H, $-(CH_2)_2C\underline{H}_3$); 1,71 (Mt, 2H, $-C\underline{H}_2-CH_3$ du propyl); 1,76 (D, J = 7, 3H, $-CH_3$); 2,8 (S, 6H, $-N(CH_3)_2$); 3,53 (DD, J = 14 et 4,5, 1H, 1H du $>N-CH_2-$); 3,66 (Mt, 2H, $-CSNH-C\underline{H}_2-$); 3,75 (DD, J = 14 et 8, 1H, 1H du $>N-CH_2-$); 4,78 (Mt, J = 8 - 7 et 4,5, 1H, $>N-CH<$); 7 à 7,35 (Mt, 5H, aromatiques); 7,43 (DD, J = 8 et 1, 1H, -H en 3); 7,55 (D, J = 1, 1H, -H en 1); 10,5 (T, J = 5, 1H, -CSNH-); 10,53 (Mf, 1H, $-NH^+$).

Spectre infra-rouge (KBr), bandes caractéristiques en $cm^{-1}$ :
3200, 2960, 2930, 2870, 2660, 2510, 2470, 1590, 1530, 1460, 880, 825, 750.

## EXEMPLE 5

Un mélange de 2,4 g de [(N-méthyl N-éthyl-amino)-1 propyl-2-(2RS)]-10 phénothiazinecarbothioamide-2 et de 8,27 cm3 de propylamine dans 48 cm3 d'éthanol est chauffé pendant 16 heures à une température voisine de 100°C. Après refroidissement, on concentre à sec sous pression réduite (30 mm de Hg; 4 kPa) à 40°C. L'huile brune résiduelle (3 g) est purifiée par chromatographie sur une colonne (hauteur : 25 cm; diamètre : 4 cm) de gel de silice (0,04-0,063 mm) avec une légère surpression d'azote (40 kPa) en éluant par 2 litres d'un mélange de chlorure de méthylène et de méthanol (95-5 en volumes) et en recueillant des fractions de 60 cm3. Les fractions 8 à 20 sont réunies et concentrées à sec sous pression réduite (30 mm de Hg; 4 kPa) à 40°C. L'huile résiduelle (1,68 g) est dissoute dans 50 cm3 d'éther éthylique en présence de noir 3S. On filtre et le filtrat jaune est additionné, sous agitation de 1,3 cm3 d'une solution d'acide chlorhydrique 3,3N dans l'éther éthylique et on poursuit l'agitation pendant 2 heures à une température voisine de 20°C. Le précipité formé est essoré, lavé par 5 cm3 d'éther éthylique et séché sous pression réduite (5 mm de Hg; 0,68 kPa) à 35°C. On obtient ainsi 1,35 g de chlorhydrate de [(N-méthyl N-éthyl-amino)-1 propyl-2-(2RS)]-10 N-propyl phénothiazinecarbothioamide-2 sous forme d'un solide jaune fondant à 110 -115°C (fusion pâteuse).

RMN du proton (250 MHz, DMSO, δ en ppm, J en Hz).

En solution dans le DMSO, on observe deux formes dues à la salification de l'azote, ce phénomène disparaît par ajout de $CD_3COOD$.

0,97 (T, J = 7,5, 3H, $-(CH_2)_2C\underline{H}_3$); 1,06 et 1,20 (2T, J = 7,5, 3H, $>NCH_2C\underline{H}_3$); 1,74 (Mt, 2H, $-CH_2-C\underline{H}_2-CH_3$); 1,82 (Mt, 3H, $-CH_3$); 2,8 (Mt, 3H, $>N-CH_3$); 3,18 (Mt, 2H, $>N-C\underline{H}_2CH_3$); 3,54 et 3,80 (2Mt, 1H chacun, $>N-CH_2-$); 3,7 (Mt, 2H, $-CSNH-C\underline{H}_2-$); 7,05 à 7,4 (Mt, 5H, aromatiques); 7,45 (D large, J = 8, 1H, H en 3); 7,6 (S large, 1H, -H en 1); 10,31 et 10,52 (2Mt, 1H chacun, $-NH^+$ et -CSNH-).

Spectre infra-rouge (KBr), bandes caractéristiques en $cm^{-1}$ :
3210, 2970, 2940, 2880, 2650, 2480, 1590, 1535, 1465, 880, 820, 750.

## EXEMPLE 6

Une solution de 2,1 g de [diéthylamino-1 propyl-2-(2RS)]-10 phénothiazinecarbothioamide-2 et de 7 cm3 de propylamine dans 30 cm3 d'éthanol absolu est saturée avec de l'acide sulfhydrique puis chauffée pendant 16 heures à une température voisine de 100°C. Après refroidissement, la solution orangée obtenue est concentrée à sec sous pression réduite (30 mm de Hg; 4 kPa) à 40°C. Le résidu est repris par 100 cm3 d'eau distillée et on extrait successivement par 200 cm3 puis par 100 cm3 d'acétate d'éthyle. Les phases organiques réunies sont lavées par 100 cm3 d'une solution aqueuse saturée de chlorure de sodium, séchées sur sulfate de magnésium, filtrées et concentrées à sec sous pression réduite (30 mm de Hg; 4 kPa) à 40°C. Le résidu huileux orangé (2,2 g) est purifié par chromatographie sur une colonne (hauteur : 25 cm, diamètre : 3 cm) de gel de silice (0,04-0,063 mm) avec une légère surpression d'azote (40 kPa) en éluant par 500 cm3 d'acétate d'éthyle et en recueillant des fractions de 50 cm3. Les fractions 2 à 6 sont réunies et concentrées à sec sous pression réduite (30 mm de Hg; 4 kPa) à 40°C. L'huile jaune résiduelle (2 g) est dissoute dans 100 cm3 d'oxyde d'isopropyle et additionnée sous agitation de 1,45 cm3 d'une solution 3,3N d'acide chlorhydrique dans l'oxyde d'isopropyle. Après 15 minutes d'agitation à une température voisine de 20°C, le solide formé est essoré, lavé par 3 fois 20 cm3 d'oxyde d'isopropyle et séché sous pression réduite (5 mm de Hg; 0,68 kPa) à 40°C. On obtient ainsi 1,7 g de chlorhydrate de [diéthylamino-1 propyl-2-(2RS)]-10 N-propyl phénothiazinecarbothioamide-2 sous forme d'un solide jaune fondant à 120°C (fusion pâteuse).

RMN du proton (250 MHz, DMSO, δ en ppm, J en Hz).

0,95 (T, J = 7, 3H, $-(CH_2)_2C\underline{H}_3$); 1,02 et 1,15 (2T, J = 7, 6H, $-N(CH_2C\underline{H}_3)_2$); 1,71 (Mt, 2H, $-CH_2C\underline{H}_2-CH_3$); 1,83 (D, J = 7, 3H, $-CH_3$); 3,17 (Mt, 4H, $-N(C\underline{H}_2CH_3)_2$); 3,41 (D large, J = 14, 1H, 1H du $N-CH_2-$); 3,66 (Mt, 2H,

-CSNH-C$\underline{H_2}$-); 3,78 (DD, J = 14 et 7,5, 1H, 1H du >N-$CH_2$-); 4,88 (Mt, 1H, >N-CH<); 7 à 7,35 (Mt, 5H, aromatiques); 7,41 (D, J = 8, 1H, -H en 3); 7,57 (S, 1H, -H en 1); 10,43 (Mf, 1H, -$NH^+$); 10,52 (T, J = 5, 1H, -CSNH-).

Spectre infra-rouge (KBr), bandes caractéristiques en $cm^{-1}$ :
3200, 2970, 2940, 2880, 2580, 2480, 1590, 1535, 1465, 885, 825, 750.

EXEMPLE 7

Une solution de 0,56 g de fumarate acide de (diéthylamino-1 propyl-2)-10 phénothiazinecarbothioamide-2, série L, et de 1,4 cm3 de propylamine dans 10 cm3 d'éthanol absolu est saturée avec de l'acide sulfhydrique et chauffée pendant 16 heures à une température voisine de 100°C. Après refroidissement, on concentre à sec sous pression réduite (30 mm de Hg; 4 kPa) à 40°C. L'huile orangée résiduelle est purifiée par chromatographie sur une colonne (hauteur : 25 cm; diamètre : 1 cm) de gel de silice (0,04-0,063 mm) avec une légère surpression d'azote (40 kPa) en éluant avec 150 cm3 d'un mélange d'acétate d'éthyle et de cyclohexane (80-20 en volumes) et en recueillant des fractions de 15 cm3. Les fractions 4 à 7 réunies sont concentrées à sec sous pression réduite (30 mm de Hg; 4 kPa) à 40°C. On obtient ainsi 0,5 g de (diéthylamino-1 propyl-2)-10 N-propyl phénothiazinecarbothioamide-2, série L, sous forme d'une huile jaune.
On dissout 0,25 g de ce produit dans 25 cm3 d'oxyde d'isoproppyle et on ajoute, goutte à goutte, sous agitation et à une température voisine de 5°C, 2,2 cm3 d'une solution 0,3N d'acide chlorhydrique dans l'oxyde d'isopropyle. On maintient l'agitation pendant 1 heure à une température voisine de 5°C. Le précipité est essoré, lavé par 3 fois 5 cm3 d'oxyde d'isopropyle et séché sous pression réduite (5 mm de Hg; 4 kPa) à 40°C. On obtient ainsi 0,24 g de chlorhydrate de (diéthylamino-1 propyl-2)-10 N-propyl phénothiazinecarbothioamide-2, série L, sous forme d'un solide jaune fondant à 95-100°C (fusion pâteuse) dont les caractéristiques RMN sont identiques à celles du produit décrit à l'exemple 6.

Spectre infra-rouge (KBr), bandes caractéristiques en $cm^{-1}$ :
3180, 2960, 2930, 2870, 2620, 2580, 2480, 1590, 1530, 1460, 1415, 880, 825, 750.

EXEMPLE 8

A une solution de 0,6 g de chloro-7 [diéthylamino-1 propyl-2-(2RS)]-10 phénothiazinecarbothioamide-2 dans 5 cm3 d'éthanol absolu, on ajoute 0,75 cm3 de propylamine. Le mélange est porté à 200°C pendant 4 heures puis dilué par 50 cm3 d'acétate d'éthyle, lavé par 2 fois 100 cm3 d'eau distillée, séché sur sulfate de magnésium puis concentré à sec sous pression réduite (30 mm de Hg; 4 kPa) à 40°C. Le solide résiduel est purifié par chromatographie sur une colonne (hauteur : 8,5 cm; diamètre : 2 cm) de gel de silice (0,6-0,02 mm) en éluant avec un mélange d'acétate d'éthyle et de cyclohexane 20-80 en volumes (1,5 litre) en recueillant des fractions de 30 cm3. Les fractions 25 à 36 sont réunies et concentrées sous pression réduite (30 mm de Hg; 4 kPa) pour donner une huile marron qui est mise en solution dans 10 cm3 d'éther éthylique. A cette solution est ajoutée 2 cm3 d'éther chlorhydrique 2,2 N. La suspension obtenue est filtrée pour donner 0,06 g de chloro-7 [diéthylamino-1 propyl-2-(2RS)]-10 N-propyl phénothiazinecarbothioamide-2 fondant à 130°C.

Spectre de RMN (250 MHz, DMSO, $\delta$ en ppm, J en Hz).

0,92 (T, J = 7,5, 3H, -$CH_3$ propyle); 0,97 et 1,16 (2T, J = 7,5, 6H, $CH_3$ du diéthyle en 10); 1,7 (Mt, 2H, -$CH_2$-C$\underline{H_2}$-$CH_3$); 1,79 (D, J = 7,5, 3H, -$CH_3$); 3,14 (Mf, 4H, -N(C$\underline{H_2}$$CH_3$)$_2$); 3,35 (Mf, 1H, 1H du >N-$CH_2$-); 3,64 (Mt, J = 7 et 5, 2H, -CSNH-C$\underline{H_2}$-); 3,75 (Mf, 1H, 1H du >N-$CH_2$-); 4,77 (Mf, 1H, N-CH); 7,1 à 7,45 (Mt, 5H, aromatiques); 7,56 (D, J = 1, 1H, -H en 1); 9,97 (Mf, 1H, -$NH^+$); 10,44 (T, J = 5, 1H, -CSNH-).

Spectre infra-rouge (KBr), bandes caractéristiques en $cm^{-1}$ :
3200, 2960, 2930, 2870, 2640, 2580, 2480, 1590, 1530, 1460, 1420, 870, 805.

Le chloro-7 [diéthylamino-1 propyl-2-(2RS)]-10 phénothiazinecarbothioamide-2 peut être obtenu de la manière suivante :

Un mélange de 55,1 g de chloro-7 [diéthylamino-1 propyl-2-(2RS)]-10 phénothiazinecarbonitrile-2 et de 10,4 cm3 de triéthylamine dans 350 cm3 de pyridine anhydre est saturé par bullage d'acide sulfhydrique pendant 5 heures à 25°C. La solution limpide obtenue est maintenue sous agitation pendant 12 heures à 25°C, puis le mélange est dégazé par bullage d'azote pendant 90 minutes. Le mélange réactionnel est alors dilué par 1000 cm3 d'acétate d'éthyle et lavé par 5 fois 1000 cm3 d'eau distillée. La phase organique est séchée sur sulfate de magnésium, filtrée et le filtrat est concentré à sec sous pression réduite (30 mm de Hg; 4 kPa). Le résidu est purifié par chromatographie sur colonne de gel de silice 0,02-0,6 mm (hauteur : 31 cm ; diamètre : 7,5 cm) en éluant par un mélange de cyclohexane et d'acétate d'éthyle 80-20 en volumes (30 litres) et en recueillant des fractions de 1000 cm3. Les fractions 22 à 28 sont réunies et concentrées sous pression réduite (30 mm de Hg; 4 kPa) pour donner une huile contenant le produit attendu. L'huile obtenue est purifiée par chromatographie sur une colonne de gel de silice 0,04-0,06 mm avec une légère surpression d'azote (40 kPa) (hauteur : 30 cm; diamètre : 4,5 cm) en éluant avec de l'acétate d'éthyle pur (1,5 litre) et en recueillant des fractions de 100 cm3. Les fractions 8 à 12 sont réunies et concentrées à sec sous pression réduite (30 mm de Hg; 4 kPa) pour donner 1,7 g de chloro-7 [diéthylamino-1 propyl-2-(2RS)]-10 phénothiazinecarbothioamide-2.

On charge dans un autoclave 36 g de méthanesulfonate de (chloro-7 cyano-2 phénothiazinyl-10)-2-(2RS) propyle avec 200 cm3 d'éthylamine. Le mélange est porté à 100°C pendant 17 heures. Après refroidissement, le mélange réactionnel est versé dans 1 litre d'eau. La solution est extraite par 500 cm3 d'acétate d'éthyle et la phase organique est lavée par 2 fois 500 cm3 d'eau distillée. Après décantation, elle est séchée sur sulfate de magnésium, filtrée et concentrée à sec à 40°C pour donner une gomme marron qui est purifiée par chromatographie sur colonne (hauteur : 50 cm; diamètre : 6 cm) de silice (0,06-0,3 mm) en éluant par de l'acétate d'éthyle pur (8 litres) puis par un mélange d'acétate d'éthyle et de méthanol (mélange d'acétate d'éthyle et de triéthylamine (97-3 en volumes) (2 litres) et enfin par un mélange de diéthylamine et de méthanol (10-90 en volumes) (2 litres) et en recueillant des fractions de 500 cm3. Les fractions 32 à 37 sont réunies et concentrées à sec à 40°C sous pression réduite (30 mm de Hg; 4 kPa) pour donner 20 g de chloro-7 [éthylamino-1 propyl-2-(2RS)]-10 phénothiazinecarbonitrile-2 sous la forme d'une meringue marron.

On charge un mélange de 5 g de chloro-7 [éthylamino-1 propyl-2-(2RS)]-10 phénothiazinecarbonitrile-2, 2,2 cm3 d'iodoéthane, 2 g de carbonate de sodium dans 50 cm3 de diméthylformamide et on porte le mélange 9 heures à 160°C. Après refroidissement, le mélange réactionnel est versé dans un mélange de 500 cm3 d'eau distillée et de 500 cm3 d'acétate d'éthyle. La phase organique est décantée, lavée par 4 fois 300 cm3 d'eau distillée, séchée sur sulfate de magnésium, filtrée et concentrée à sec à 40°C sous pression réduite (30 mm de Hg; 4 kPa) pour donner 5 g de chloro-7 [diéthylamino-1 propyl-2-(2RS)]-10 phénothiazinecarbonitrile-2.

EXEMPLE 9

Une solution de 1,6 g de fumarate acide de [(N-méthyl N-propyl-amino)-1 propyl-2-(2RS)]-10 phénothiazinecarbothioamide-2 et de 4 cm3 de propylamine dans 32 cm3 d'éthanol est chauffée pendant 16 heures à une température voisine de 100°C. Après refroidissement, on concentre à sec sous pression réduite (30 mm de Hg; 4 kPa) à 50°C. Le résidu est repris par 200 cm3 d'éther éthylique et la solution obtenue est lavée avec 10 cm3 d'une solution aqueuse de soude N, puis lavée avec 50 cm3 d'une solution aqueuse saturée de chlorure de sodium, séchée sur sulfate de magnésium, filtrée et concentrée à sec sous pression réduite (30 mm de Hg; 4 kPa) à 40°C. L'huile jaune résiduelle (1,6 g) est purifiée par chromatographie sur une colonne (hauteur : 30 cm; diamètre : 3 cm) de gel de silice (0,04-0,063 mm) avec une légère surpression d'azote (40 kPa) en éluant avec 1 litre d'un mélange de chlorure de méthylène et de méthanol (97-3 en volumes) et en recueillant des fractions de 60 cm3. Les fractions 4 à 9 réunies sont concentrées à sec sous pression réduite (30 mm de Hg; 4 kPa) à 40°C. L'huile jaune résiduelle (1 g) est dissoute dans 100 cm3 d'éther éthylique, on ajoute 1 cm3 d'une solution 3N d'acide chlorhydrique dans l'éther éthylique et on agite 1 heure à une température voisine de 20°C. Le solide formé est essoré, lavé avec 10 cm3 d'éther éthylique et séché sous pression réduite (5 mm de Hg; 0,68 kPa) à 40°C. On obtient ainsi 0,64 g de chlorhydrate de [(N-méthyl N-propyl-amino)-1 propyl-2-(2RS)]-10 N'-propyl phénothiazinecarbothioamide-2 sous forme d'un solide jaune fondant à 137-139°C (fusion pâteuse).

RMN du proton (250 MHz, DMSO, $\delta$ en ppm, J en Hz).

En solution dans le DMSO, on observe deux formes dues à la salification de l'azote.

0,78 et 0,83 (2T, J = 7,5, 3H, $-N(CH_2)_2C\underline{H}_3$); 0,95 (T, J = 7,5, 3H, $-(CH_2)_2C\underline{H}_3$); 1,35 à 1,80 (Mt, 2H, $-NCH_2C\underline{H}_2CH_3$); 1,75 (Mt, 2H, $-CH_2C\underline{H}_2CH_3)_2$); 1,84 (2D, J = 7, 3H, 1H $-CH_3$-); 2,8 (2D, J = 5, 3H, $N-CH_3$); 2,90 à 3,25 (Mt, 2H, $>N-C\underline{H}_2-CH_2-CH_3$); 3,53 (D large, 1H, 1H du $>N-CH_2$-); 3,70 (Mt, 2H, $-CSNH-C\underline{H}_2$-); 3,8 (Mt, 1H, autre H du

$N-CH_2$-); 4,88 (Mt, 1H, N-CH ); 7 à 7,35 (Mt, 5H, aromatiques); 7,44 (D large, J = 8, 1H, -H en 3); 7,58 (S large, 1H, -H en 1); 10,3 et 10,4 (2Mf, 1H, $-NH^+$); 10,5 (Mt, 1H, -CSNH-).

Spectre infra-rouge (KBr), bandes caractéristiques en $cm^{-1}$ : 3190, 2960, 2940, 2880, 2580, 2490, 1590, 1535, 1465, 880, 820, 750.

Une solution de 4,3 g de [(N-méthyl N-propyl-amino)-1 propyl-2-(2RS)]-10 phénothiazinecarbonitrile-2 et de 1,77 cm3 de triéthylamine dans 86 cm3 de pyridine est additionnée d'acide sulfhydrique et on laisse agiter 16 heures à une température voisine de 20°C. La solution verte obtenue est diluée avec 200 cm3 d'acétate d'éthyle, lavée avec 5 fois 40 cm3 d'eau distillée, séchée sur sulfate de magnésium, filtrée et concentrée à sec sous pression réduite (30 mm de Hg; 4 kPa) à 40°C. L'huile résiduelle (5,4 g) est purifiée par chromatographie sur une colonne (hauteur : 30 cm; diamètre : 5,5 cm) de gel de silice (0,04-0,063 mm) avec une légère surpression d'azote (40 kPa) en éluant avec 5 litres d'un mélange de chlorure de méthylène et de méthanol (97,5-2,5 en volumes) et en recueillant des fractions de 100 cm3. Les fractions 13 à 44 réunies sont concentrées à sec sous pression réduite (30 mm de Hg; 4 kPa) à 40°C. L'huile jaune résiduelle (4,37 g) est dissoute

dans 12 cm3 d'éthanol au reflux et additionnée, sous agitation, d'une solution de 1,4 g d'acide fumarique dans 15 cm3 d'éthanol au reflux. Après 2 heures d'agitation à une température voisine de 5°C, le solide formé est essoré, lavé par 5 cm3 d'éthanol et séché sous pression réduite (5 mm de Hg; 0,68 kPa) à 40°C. On obtient ainsi 4,35 g de fumarate acide de [(N-méthyl N-propyl-amino)-1 propyl-2-(2RS)]-10 phénothiazinecarbothoamide-2 sous forme de cristaux jaunes fondant à 204°C.

Le [(N-méthyl N-propyl-amino)-1 propyl-2-(2RS)]-10 phénothiazinecarbonitrile-2 peut être obtenu de la manière suivante :

Une suspension de 4,7 g de [méthylamino-1 propyl-2-(2RS)]-10 phénothiazinecarbonitrile-2 de 1,55 cm3 d'iodo-1 propane et de 2 g d'hydrogénocarbonate de sodium dans 47 cm3 de diméthylformamide est chauffée pendant 6 heures à une température voisine de 140°C. Après refroidissement, le mélange réactionnel est dilué avec 200 cm3 d'acétate d'éthyle, lavé avec 4 fois 30 cm3 d'eau distillée, séché sur sulfate de magnésium, filtré et concentré à sec sous pression réduite (30 mm de Hg; 4 kPa) à 40°C. L'huile résiduelle (5,2 g) est purifiée par chromatographie sur une colonne (hauteur : 25 cm; diamètre : 4 cm) de gel de silice (0,04-0,063 mm) avec une légère surpression d'azote (40 kPa) en éluant avec 1 litre d'un mélange de cyclohexane et d'acétate d'éthyle (70-30 en volumes) et en recueillant des fractions de 60 cm3. Les fractions 6 à 14 réunies sont concentrées à sec sous pression réduite (30 mm de Hg; 4 kPa) à 40°C. On obtient ainsi 4,4 g de [(N-méthyl N-propyl-amino)-1 propyl-2-(2RS)]-10 phénothiazinecarbonitrile-2 sous forme d'une huile jaune.

Le [méthylamino-1 propyl-2-(2RS)]-10 phénothiazinecarbothioamide-2 peut être préparé de la manière suivante :

On refroidit à une température voisine de -20°C une solution de 25 g de méthanesulfonate de (cyano-2 phénothiazinyl-10)-2 propyle-1-(2RS) dans 375 cm3 de toluène et on ajoute 50 cm3 de méthylamine (condensée à une température voisine de -60°C) puis on porte ce mélange à une température voisine de 100°C pendant 14 heures. Après refroidissement, on concentre à sec sous pression réduite (30 mm de Hg; 4 kPa) à 40°C. Le résidu est repris par 750 cm3 d'acétate d'éthyle et on extrait par 2 fois 500 cm3 d'une solution aqueuse d'acide chlorhydrique N. Les phases aqueuses réunies sont lavées par 200 cm3 d'acétate d'éthyle, alcalinisées par de la lessive de soude (d = 1,33) jusqu'à pH 13 à une température voisine de 5°C et extraites par 2 fois 500 cm3 d'acétate d'éthyle. Les phases organiques réunies sont lavées successivement par 2 fois 250 cm3 d'eau distillée et par 250 cm3 d'une solution aqueuse saturée de chlorure de sodium, séchées sur sulfate de magnésium, filtrées et concentrées à sec sous pression réduite (30 mm de Hg; 4 kPa) à 40°C. On obtient ainsi 19,3 g de [méthylamino-1 propyl-2-(2RS)]-10 phénothiazinecarbothioamide-2 sous forme d'une huile jaune.

## EXEMPLE 10

Un mélange de 1,6 g de chlorhydrate de [N-méthyl N-(méthyl-1 éthyl)-amino-1 propyl-2-(2RS)]-10 phénothiazinecarbothioamide-2 et de 4,8 cm3 de propylamine dans 32 cm3 d'éthanol absolu est chauffé pendant 16 heures à une température voisine de 100°C. Après refroidissement, on concentre à sec sous pression réduite (30 mm de Hg; 4 kPa) à 40°C. Le résidu est dissous dans 100 cm3 d'éther éthylique et la solution obtenue est lavée par 4 cm3 d'une solution aqueuse de soude N puis par 2 fois 10 cm3 d'une solution aqueuse saturée de chlorure de sodium, séchée sur sulfate de magnésium, filtrée et concentrée à sec sous pression réduite (30 mm de Hg; 4 kPa) à 40°C. La gomme brun-rouge résiduelle est purifiée par chromatographie sur une colonne (hauteur : 25 cm; diamètre : 3 cm) de gel de silice (0,04-0,063 mm) avec une légère surpression d'azote (40 kPa) en éluant avec 1 litre d'un mélange de chlorure de méthylène et de méthanol (97-3 en volumes) et en recueillant des fractions de 60 cm3. Les fractions 6 à 12 sont réunies et concentrées à sec sous pression réduite (30 mm de Hg; 4 kPa) à 40°C. L'huile jaune résiduelle (0,85 g) est dissoute dans 50 cm3 d'éther éthylique en présence de noir 3S. On filtre et on ajoute au filtrat jaune 1 cm3 d'une solution d'acide chlorhydrique 3N dans l'éther éthylique et on agite pendant 1 heure à une température voisine de 20°C. Le solide formé est essoré, lavé avec 5 cm3 d'éther éthylique et séché sous pression réduite (5 mm de Hg; 0,68 kPa) à 40°C. On obtient ainsi 0,58 g de chlorhydrate de [N-méthyl N-(méthyl-1 -éthyl)-amino-1 propyl-2-(2RS)]-10 N-propyl phénothiazinecarbothioamide-2 sous forme d'un solide jaune fondant vers 140°C (fusion pâteuse).

RMN du proton (250 MHz, DMSO, δ en ppm, J en Hz).

En solution dans le DMSO, on observe deux formes dues à la salification de l'azote, ce phénomène disparaît par ajout de $CD_3COOD$).

0,96 et 1,24 (Mt, environ 9H, $-(C\underline{H}_2)_2C\underline{H}_3$ et $-CH(C\underline{H}_3)_2$); 1,75 (Mt, 2H, $-CH_2C\underline{H}_2CH_3$); 1,85 (D, J = 7, 3H, $-CH_3$); 2,72 et 2,77 (2D, J = 5, 3H en totalité, $>NCH_3$); 3,45 à 4 (Mt, 5H, $N-CH_2-$, $-C\underline{H}(CH_3)_2-$, $-CSNH-C\underline{H}_2-$); 4,92 (Mt, 1H, $>N-CH<$); 7,05 à 7,68 (Mt, 7H, aromatiques); 9,85 - 10,10 - 10,5 et 10,6 (4Mf, 2H en totalité, $-NH^+$ et -CSNH-).

Spectre infra-rouge (KBr), bandes caractéristiques en $cm^{-1}$ :
3200, 2960, 2930, 2870, 2640, 2480, 1590, 1530, 1460, 880, 820, 750.

Le chlorhydrate de {[N-méthyl N-(méthyl-1 éthyl)-amino)-1] propyl-2-(2RS)}-10 phénothiazinecarbothioamide-2 peut être obtenu de la manière suivante :

Une solution de 4,2 g de {[N-méthyl N-(méthyl-1 éthyl)-amino]-1 propyl-2-(2RS)}-10 phénothiazinecarbonitrile-2 et de 1,75 cm3 de triéthylamine dans 84 cm3 de pyridine est additionnée d'acide sulfhydrique pendant 1 heure puis on agite pendant 16 heures à une température voisine de 20°C. Le mélange réaction el est dilué avec 500 cm3 d'acétate d'éthyle, lavé avec 5 fois 50 cm3 d'eau distillée, séché sur sulfate de magnésium, filtré et concentré à sec sous pression réduite (30 mm de Hg; 4 kPa) à 40°C. L'huile brune résiduelle (4,9 g) est purifiée par chromatographie sur une colonne (hauteur : 25 cm; diamètre : 6 cm) de gel de silice (0,04-0,063 mm) avec une légère surpression d'azote (40 kPa) en éluant successivement avec 1 litre d'un mélange de chlorure de méthylène et de méthanol (97,5-2,5 en volumes) puis 1,5 litre d'un mélange de chlorure de méthylène et de méthanol (95-5 en volumes) et 1 litre d'un mélange de chlorure de méthylène et de méthanol (92,5-7,5 en volumes) et en recueillant des fractions de 100 cm3. Les fractions 18 à 32 sont réunies et concentrées à sec sous pression réduite (30 mm de Hg; 4 kPa) à 40°C. Le résidu est dissous dans 100 cm3 d'acétate d'éthyle en présence de noir 3S. On filtre et le filtrat jaune est concentré à sec sous pression réduite (30 mm de Hg; 4 kPa) à 40°C. Le résidu huileux jaune (2,6 g) est dissous dans 200 cm3 d'éther éthylique et on ajoute sous agitation, 2,35 cm3 d'une solution d'acide chlorhydrique 3N dans l'éther éthylique puis on poursuit l'agitation pendant 1 heure à une température voisine de 20°C. Le précipité formé est essoré, lavé par 2 fois 10 cm3 d'éther éthylique et séché sous pression réduite (5 mm de Hg; 0,68 kPa) à 35°C. On obtient ainsi 2,3 g de chlorhydrate de {[N-méthyl N-(méthyl-1 éthyl)-amino)-1 propyl-2-(2RS)}-10 phénothiazinecarbothioamide-2 sous forme d'un solide jaune fondant à 168-172°C.

Le {[N-méthyl N-(méthyl-1 éthyl)-amino]-1 propyl-2-(2RS)]-10 phénothiazinecarbonitrile-2 peut être obtenu de la manière suivante :

Un mélange de 4,55 g de [méthylamino-1 propyl-2-(2RS)]-10 phénothiazinecarbonitrile-2, de 2 cm3 d'iodo-2 propane et de 1,9 g d'hydrogènocarbonate de sodium dans 45,5 cm3 de diméthylformamide sec est chauffé pendant 6 heures à une température voisine de 140°C. Après refroidissement, le mélange réactionnel est dilué avec 200 cm3 d'acétate d'éthyle, lavé avec 4 fois 30 cm3 d'eau distillée, séché sur sulfate de magnésium, filtré et concentré à sec sous pression réduite (30 mm de Hg; 4 kPa) à 40°C. Le résidu huileux jaune est purifié par chromatographie sur une colonne (hauteur : 25 cm; diamètre : 4 cm) de gel de silice (0,04-0,063 mm) avec une légère surpression d'azote (40 kPa) et par 1 litre d'un mélange de chlorure de méthylène et de méthanol (97,5-2,5 en volumes) et en recueillant des fractions de 60 cm3. Les fractions 4 à 12 sont réunies et concentrées à sec sous pression réduite (30 mm de Hg; 4 kPa) à 40°C. Le résidu huileux jaune (2,9 g) est purifié à nouveau par chromatographie sur une colonne (hauteur : 25 cm; diamètre : 4 cm) de gel de silice (0,2-0,063 mm) en éluant par 1 litre d'un mélange de cyclohexane et d'acétate d'éthyle (80-20 en volumes) et en recueillant des fractions de 60 cm3. Les fractions 4 à 12 sont réunies et concentrées à sec sous pression réduite (30 mm de Hg; 4 kPa) à 40°C. On obtient ainsi 2,2 g de {[N-méthyl N-(méthyl-1 éthyl)-amino]-1 propyl-2-(2RS)}-10 phénothiazinecarbonitrile-2 sous forme d'une huile jaune.

## EXEMPLE 11

Une solution de 2,1 g de {[N-éthyl N-(méthyl-1 éthyl)-amino]-1 propyl-2-(2RS)}-10 phénothiazinecarbothioamide-2 et de 6,7 cm3 de propylamine dans 27 cm3 d'éthanol absolu est saturée d'acide sulfhydrique et chauffée pendant 16 heures à une température voisine de 100°C. Après refroidissement, on concentre à sec sous pression réduite (30 mm de Hg; 4 kPa) à 40°C. L'huile orangée résiduelle est purifiée par chromatographie sur une colonne (hauteur : 25 cm; diamètre : 4 cm) de gel de silice (0,04-0,063 mm) avec une légère surpression d'azote (40 kPa) en éluant par 1 litre d'un mélange d'acétate d'éthyle et de cyclohexane (50-50 en volumes) et en recueillant des fractions de 60 cm3. Les fractions 7 à 14 réunies sont concentrées à sec sous pression réduite (30 mm de Hg; 4 kPa) à 40°C. L'huile orangée résiduelle (0,7 g) est dissoute dans 100 cm3 d'oxyde d'isopropyle puis additionnée, goutte à goutte, sous agitation et à une température voisine de 5°C, de 10,48 cm3 d'une solution d'acide chlorhydrique 0,156N dans l'oxyde d'isopropyle. Après 1 heure d'agitation à une température voisine de 5°C, le solide formé est essoré, lavé par 3 fois 10 cm3 d'oxyde d'isopropyle et séché sous pression réduite (5 mm de Hg; 0,68 kPa) à 40°C. On obtient ainsi 0,7 g de chlorhydrate de {[N-éthyl N-(méthyl-1 éthyl)-amino]-1 propyl-2-(2RS)}-10 N'-propyl phénothiazinecarbothioamide-2 sous la forme d'un solide jaune fondant à 109°C.

RMN du proton (250 MHz, DMSO, $\delta$ en ppm, J en Hz).

En solution dans le DMSO, on observe deux formes dues à la salification de l'azote.

0,89 et 1,33 (2D, J = 7,5, 3H, 1 $CH_3$ de l'isopropyle); 0,97 (T, J = 7,5, 3H, $-(CH_2)_2C\underline{H}_3$); 1,10 à 1,29 (2T, J = 7,5, 3H, $-NCH_2C\underline{H}_3$); 1,21 (Mt, 3H, 1 $CH_3$ de l'isopropyle); 1,73 (Mt, 2H, $-CH_2C\underline{H}_2CH_3$); 1,89 (D, J = 7, 3H, $-CH_3$); 3,05 à 3,35 (2Mt, 2H, $>N-C\underline{H}_2CH_3$); 3,5 à 3,9 (Mt, 3H, $>N-CH<$ et $>N-CH_2-$); 3,68 (Mt, 2H, $-CSNH-C\underline{H}_2-$);

4,9 (Mt, 1H, >N-CH<); 7,05 à 7,35 (Mt, 5H, aromatiques); 7,42 (D large, J = 8, 1H, -H en 3); 7,6 (S large, 1H, -H en 1); 9,68 et 9,89 (2Mf, 1H, $-NH^+$); 10,5 (Mt, 1H, -CSNH-).

Le {[N-éthyl N-(méthyl-1 éthyl)-amino]-1 propyl-2-(2RS)}-10 phénothiazinecarbothioamide-2 peut être préparé de la manière suivante :

Une solution de 2,3 g de {[N-éthyl N-(méthyl-1 éthyl)-ami-no]-1 propyl-2-(2RS)}-10 phénothiazinecarbonitrile-2 et de 0,92 cm3 de triéthylamine dans 46 cm3 de pyridine anhydre est additionnée d'acide sulfhydrique et agitée pendant 16 heures à une température voisine de 20°C. La solution verdâtre obtenue est purgée à l'azote, versée dans 200 cm3 d'eau distillée et extraite par 200 cm3 puis 100 cm3 d'acétate d'éthyle. Les phases organiques réunies sont lavées par 3 fois 100 cm3 d'eau distillée, séchées sur sulfate de magnésium, filtrées et concentrées à sec sous pression réduite (30 mm de Hg; 4 kPa) à 40°C. L'huile jaune résiduelle (4 g) est purifiée par chromatographie sur une colonne (hauteur : 25 cm; diamètre : 4 cm) de gel de silice (0,04-0,063 mm) avec une légère surpression d'azote (40 kPa) en éluant avec 750 cm3 d'un mélange d'acétate d'éthyle et de cyclohexane (75-25 en volumes) et en recueillant des fractions de 60 cm3. Les fractions 6 à 10 réunies sont concentrées à sous pression réduite (30 mm de Hg; 4 kPa) à 40°C. On obtient ainsi 2,5 g de {[N-éthyl N-(méthyl-1 éthyl)-amino]-1 propyl-2-(2RS)}-10 phénothiazinecarbothioamide-2 sous forme d'une huile jaune.

Le {[N-éthyl N-(méthyl-1 éthyl)-amino]-1 propyl-2-(2RS)}-10 phénothiazinecarbonitrile-2 peut être préparé de la manière suivante :

Une suspension de 4,5 g d'[éthylamino-1 propyl-2-(2RS)]-10 phénothiazinecarbonitrile-2, de 7,2 cm3 d'iodo-2 propane et de 4,6 g de carbonate de sodium dans 60 cm3 de diméthylformamide sec est chauffée 34 heures à une température voisine de 150°C. Après refroidissement, on concentre à sec sous pression réduite (5 mm de Hg; 0,68 kPa) à 40°C. Le résidu est repris par 200 cm3 d'acétate d'éthyle, lavé par 3 fois 100 cm3 d'eau distillée, séché sur sulfate de magnésium, filtré et concentré à sec sous pression réduite (30 mm de Hg; 4 kPa) à 40°C. L'huile brune résiduelle est purifiée par chromatographie sur une colonne (hauteur : 25 cm; diamètre : 4 cm) de gel de silice (0,04-0,063 mm) sous une légère surpression d'azote (40 kPa) en éluant par 500 cm3 d'un mélange d'acétate d'éthyle et de cyclohexane (75-25 en volumes) et en recueillant des fractions de 60 cm3. Les fractions 3 à 7 réunies sont concentrées à sec sous pression réduite (30 mm de Hg; 4 kPa) à 40°C. On obtient ainsi 2,37 g de {[N-éthyl N-(méthyl-1 éthyl)-amino]-1 propyl-2-(2RS)}-10 phénothiazinecarbonitrile-2 sous forme d'une huile orangée.

L'[éthylamino-1 propyl-2-(2RS)]-10 phénothiazinecarbonitrile-2 peut être préparé de la manière suivante :

Une solution de 50 g de méthanesulfonate de (cyano-2 phénothiazinyl-10)-2 propyle-(2RS) et de 100 cm3 d'éthylamine dans 600 cm3 de toluène est chauffée pendant 24 heures à une température voisine de 105°C. Après refroidissement, on concentre à sec sous pression réduite (30 mm de Hg; 4 kPa) à 40°C. Le résidu est repris par 250 cm3 d'eau distillée et on extrait successivement par 500 cm3 et 250 cm3 d'acétate d'éthyle. Les phases organiques réunies sont extraites par 2 fois 500 cm3 d'une solution aqueuse d'acide chlorhydrique N. Les phases aqueuses sont alcalinisées avec de la lessive de soude (d = 1,33) jusqu'à pH 13 et extraites successivement par 500 cm3 et par 250 cm3 d'acétate d'éthyle. Les phases organiques réunies sont lavées par 250 cm3 d'une solution aqueuse saturée de chlorure de sodium, séchées sur sulfate de magnésium, filtrées et concentrées à sec sous pression réduite (30 mm de Hg; 4 kPa) à 40°C. On obtient ainsi 30,4 g d'[éthylamino-1 propyl-2-(2RS)]-10 phénothiazinecarbonitrile-2 sous forme d'une huile orangée.

## EXEMPLE 12

Une solution de 1,5 g de [N-méthyl N-(hydroxy-2 éthyl)-amino-1 propyl-2-(2RS)]-10 phénothiazinecarbothioamide-2 et de 5 cm3 de propylamine dans 20 cm3 d'éthanol absolu est chauffée pendant 16 heures à une température voisine de 100°C. Après refroidissement, on concentre à sec sous pression réduite (30 mm de Hg; 4 kPa) à 40°C. L'huile orangée résiduelle est purifiée par chromatographie sur une colonne (hauteur : 35cm; diamètre : 2,8 cm) de gel de silice (0,2-0,063 mm) en éluant avec 1 litre d'un mélange d'acétate d'éthyle et de cyclohexane (75-25 en volumes) et en recueillant des fractions de 50 cm3. Les fractions 9 à 16 sont réunies et concentrées à sec sous pression réduite (30 mm de Hg; 4 kPa) à 40°C. L'huile jaune résiduelle (1 g) est dissoute dans 100 cm3 d'oxyde d'isopropyle et on ajoute, sous agitation et à une température voisine de 5°C, 0,7 cm3 d'une solution 3,3N d'acide chlorhydrique dans l'oxyde d'isopropyle. L'agitation est poursuivie pendant 1 heure à une température voisine de 5°C. Le précipité formé est essoré, lavé par 3 fois 10 cm3 d'oxyde d'isopropyle et séché sous pression réduite (5 mm de Hg; 0,68 kPa) à 40°C. On obtient ainsi 0,7 g de chlorhydrate de [N-méthyl N-(hydroxy-2 éthyl)-amino-1 propyl-2-(2RS)]-10 N'-propyl phénothiazinecarbothioamide-2 sous forme d'un solide jaune fondant à 126°C (fusion pâteuse vers 90°C).

RMN du proton (250 MHz, DMSO, δ en ppm, J en Hz).

En solution dans le DMSO, on observe deux formes dues à la salification de l'azote, ce phénomène disparaît par ajout de $CD_3COOD$.

0,96 (T, J = 7,5, 3H, $-(CH_2)_2CH_3$); 1,73 (Mt, 2H, $-CH_2-CH_2CH_3$); 1,81 (D large, 3H, $-CH_3$); 2,85 (S large, 3H, >$NCH_3$); 3 à 4 (Mt, 8H, $-CH_2-N-CH_2CH_2O-$ et $-CSNH-CH_2-$); 4,82 (Mt, 1H, N-CH ); 5,36 (Mf, 1H, -OH ); 7,05 à 7,4 (Mt, 5H, aromatiques); 7,44 (D large, J = 8, 1H, -H en 3); 7,58 (S large, 1H, -H en 1); 10,01 (Mf, 1H, $-NH^+$); 10,50 (Mt, 1H, -CSNH-).

Spectre infra-rouge (KBr), bandes caractéristiques en $cm^{-1}$ :
3250, 2960, 2930, 2875, 2640, 1590, 1530, 1460, 880, 820, 750.

Le [N-méthyl N-(hydroxy-2 éthyl)-amino-1 propyl-2-(2RS)]-10 phénothiazinecarbothioamide-2 peut être obtenu de la manière suivante :

Une solution de 3,1 g de [N-méthyl (N-hydroxy-2 éthyl)-amino-1 propyl-2-(2RS)]-10 phénothiazinecarbonitrile-2 et de 1,3 cm3 de triéthylamine dans 62 cm3 de pyridine anhydre est additionnée d'acide sulfhydrique. La solution verdâtre obtenue est alors agitée pendant 16 heures à une température voisine de 20°C, purgée à l'azote, versée dans 500 cm3 d'eau distillée et extraite par 2 fois 250 cm3 d'acétate d'éthyle. Les phases organiques réunies sont lavées successivement par 2 fois 100 cm3 d'eau distillée et par 100 cm3 d'une solution aqueuse saturée de chlorure de sodium, séchées sur sulfate de magnésium, filtrées et concentrées à sec sous pression réduite (30 mm de Hg; 4 kPa) à 40°C. L'huile orangée résiduelle (4,8 g) est purifiée par chromatographie sur une colonne (hauteur : 30 cm;diamètre : 2,8 cm) de gel de silice (0,2-0,063 mm) en éluant avec 3 litres d'un mélange d'acétate d'éthyle et de cyclohexane (75-25 en volumes) et en recueillant des fractions de 50 cm3. Les fractions 19 à 50 réunies sont concentrées à sec sous pression réduite (30 mm de Hg; 4kPa) à 40°C. On obtient ainsi 2,2 g de [N-méthyl N-(hydroxy-2 éthyl)-amino-1 propyl-2-(2RS)]-10 phénothiazinecarbothioamide-2 sous forme d'une meringue jaune.

Le [N-méthyl N-(hydroxy-2 éthyl)-amino-1 propyl-2-(2RS)]-10 phénothiazinecarbonitrile-2 peut être obtenu de la manière suivante :

Une suspension de 3,7 g de [méthylamino-1 propyl-2-(2RS)]-10 phénothiazinecarbonitrile-2, de 1,6 g d'hydrogénocarbonate de sodium et de 0,9 cm3 de bromo-2 éthanol dans 60 cm3 de diméthylformamide est chauffée au reflux pendant 6 heures 15 minutes. Après refroidissement, on concentre à sec sous pression réduite (5 mm de Hg; 0,68 kPa) à) 40°C. Le résidu est repris par 100 cm3 d'acétate d'éthyle, lavé successivement par 2 fois 50 cm3 d'eau distillée et par 50 cm3 d'une solution aqueuse saturée de chlorure de sodium, séché sur sulfate de magnésium, filtré et concentré à sec sous pression réduite (30 mm de Hg; 4 kPa) à 40°C. L'huile orangée résiduelle (4,4 g) est purifiée par chromatographie sur une colonne (hauteur : 35 cm; diamètre : 2,6 cm) de gel de silice (0,2-0,063 mm) en éluant par 1,25 litre d'un mélange d'acétate d'éthyle et de cyclohexane (70-30 en volumes) et en recueillant des fractions de 50 cm3. Les fractions 9 à 22 réunies réunies sont concentrées à sec sous pression réduite (30 mm de Hg; 4 kPa) à 40°C. On obtient ainsi 3,1 g de [N-méthyl N-(hydroxy-2 éthyl)-amino-1 propyl-2-(2RS)]-10 phénothiazinecarbonitrile-2 sous forme d'une huile jaune.

## EXEMPLE 13

On sature d'acide sulfhydrique une solution de 0,9 g de [(pyrrolidinyl-1)-1 propyl-2-(2RS)]-10 phénothiazinecarbothioamide-2 et de 3 cm3 de propylamine dans 18 cm3 d'éthanol absolu et on porte le mélange à une température voisine de 100°C pendant 16 heures. Après refroidissement, on concentre à sec sous pression réduite (30 mm de Hg; 4 kPa) à 40°C pour obtenir une huile jaune. Cette huile est purifiée par chromatographie sur une colonne (hauteur : 25 cm; diamètre : 2,5 cm) de gel de silice (0,04-0,063 mm) avec une légère surpression d'azote (40 kPa) en éluant successivement avec 100 cm3 de chlorure de méthylène puis 300 cm3 d'un mélange de chlorure de méthylène et de méthanol (95-5 en volumes) et en recueillant des fractions de 50 cm3. Les fractions 3 à 5 sont réunies et concentrées à sec sous pression réduite (30 mm de Hg; 4 kPa) à 40°C. L'huile jaune résiduelle (1 g) est dissoute dans 9 cm3 d'éthanol au reflux et additionnée d'une solution bouillante de 0,29 g d'acide fumarique dans 5 cm3 d'éthanol. On laisse refroidir et on maintient pendant 4 heures à une température voisine de 5°C. Les cristaux formés sont essorés, lavés avec 2 cm3 d'éthanol glacé et séchés sous pression réduite (5 mm de Hg; 0,7 kPa) à 35°C. On obtient ainsi 1,12 g de fumarate neutre de N-propyl [(pyrrolidinyl-1)-1 propyl-2-(2RS)]-10 phénothiazinecarbothioamide-2 sous forme de cristaux jaunes fondant à 150-152°C.

RMN du proton (250 MHz, DMSO, $\delta$ en ppm, J en Hz).

0,92 (T, J = 7,5, 3H, $-CH_3$ du propyle); 1,58 (D, J = 7, 3H, $-CH_3$); 1,6 à 1,8 (Mt, 6H, $-CH_2-$ de la pyrrolidine et $-CH_2CH_3$ du propyle); 2,5 à 2,7 (Mt, 4H, >$N-CH_2-$ de la pyrrolidine); 2,99 (DD, J = 13 et 7,5, 1H, 1H du >$N-CH_2-$); 3,11 (DD, J = 13 et 6, 1H, 1H du >$NCH_2-$); 3,63 (Mt, 2H, $-CSNH-CH_2-$); 4,26 (Mt, J = 7,5 - 7 et 6, 1H, >N-CH<); 6,59 (S, 1H, -CH=CH- de l'hemifumarate); 6,95 à 7,30 (Mt,5H, aromatiques); 7,31 (DD, J = 8 et 1, 1H, -H en 3); 7,6 (D, J = 1, 1H, -H en 1); 10,28 (T, J = 5, 1H, -CSNH-).

Spectre infra-rouge (KBr), bandes caractéristiques en $cm^{-1}$ : 3210, 2960, 2930, 2880, 1700, 1590, 1535,

1460, 880, 820, 760, 670.

EXEMPLE 14

Un mélange de 10,3 g de (pyrrolidinyl-1)-1 propyl-2]-10 phénothiazinecarbothioamide-2, série L, et de 32 cm3 de propylamine dans 150 cm3 d'éthanol est saturé avec de l'acide sulfhydrique puis chauffé 16 heures à 105°C en autoclave. Après refroidissement, la solution est concentrée à sec sous pression réduite (30 mm de Hg; 4 kPa) à 40°C. On obtient 10,2 g d'une huile orangée qui est purifiée par chromatographie sur colonne de silice (0,2 - 0,063 mm) diamètre : 4 cm, hauteur : 25 cm) en éluant par un mélange de chlorure de méthylène et de méthanol 95-5 (2 litres) (en volumes) et en recueillant des fractions de 100 cm3. Les fractions 13 à 17 sont réunies et concentrées sous pression réduite (30 mm de Hg ; 4 kPa) à 40°C. On obtient 9,5 g de N-propyl [(pyrrolidinyl-1)-1 propyl-2]-10 phénothiazinecarbothioamide-2, série L, sous forme d'une huile jaune.
$[\alpha]_D^{20}$ = +30,4 ±0,6° (1%; méthanol).

On ajoute une solution de 2,9 g de [(pyrolidinyl-1)-1 propyl-2]-10 N-propyl phénothiazinecarbothioamide-2, série L, dans 14 cm3 d'éthanol à une température voisine de 70°C dans une solution de 0,82 g d'acide fumarique dans 14 cm3 d'éthanol au reflux. On laisse refroidir et conserve 16 heures à une température voisine de 5°C. On essore le précipité formé, le lave avec 2 cm3 d'éthanol et le sèche sous pression réduite (5 mm de Hg; 0,68 kPa) à 40°C. On obtient ainsi 0,73 g de fumarate neutre de [(pyrrolidinyl-1)-1 propyl-2]-10 N-propyl phénothiazinecarbothioamide-2, série L, sous forme d'un solide jaune fondant à 118-123°C (fusion pâteuse) dont les caractéristiques RMN sont identiques à celles du produit de l'exemple 13.

Spectre infra-rouge (KBr), bandes caractéristiques en $cm^{-1}$ : 3210, 2960, 2920, 2870, 2580, 2470, 1700, 1590, 1530, 1460, 980, 880, 815, 750, 670.

Le [(pyrrolidinyl-1)-1 propyl-2]-10 phénothiazinecarbothioamide-2, série L, peut être préparé de la manière suivante :

Un mélange de 11,2 g de [(pyrrolidinyl-1)-1 propyl-2]-10 phénothiazinecarbonitrile-2, série L et de 4,7 cm3 de triéthylamine dans 225 cm3 de pyridine anhydre est saturé par bullage d'acide sulfhydrique pendant une heure à 25°C. On agite pendant 20 heures à 25°C. Le mélange réactionnel est dégazé par barbottage d'azote puis dilué par 500 cm3 d'acétate d'éthyle et lavé par 500 cm3 d'eau distillée. La phase aqueuse est extraite à nouveau par 250 cm3 d'acétate d'éthyle. Les phases organiques réunies sont lavées par 2 fois 200 cm3 d'eau et 200 cm3 de solution aqueuse saturée de chlorure de sodium, séchées sur sulfate de magnésium et concentrées sous pression réduite (30 mm de Hg; 4 kPa) à 40°C. On obtient 14,4 g d'une huile orangée qui est purifiée par chromatographie sur colonne de silice (0,2 - 0,063 mm) (diamètre : 4 cm; hauteur : 30 cm) en éluant par un mélange de chlorure de méthylène et de méthanol 95-5 (3 litres) (en volumes) et en recueillant des fractions de 120 cm3. Les fractions 12 à 27 sont réunies et concentrées sous pression réduite (30 mm de Hg; 4 kPa) à 40°C. On obtient 10,3 g de [(pyrrolidinyl-1)-1 propyl-2]-10 phénothiazinecarbothioamide-2, série L, sous forme d'une meringue orangée.
$[\alpha]_D^{20}$ = -43,5 ±0,6° (1,3%; chloroforme).

Le [(pyrrolidinyl-1)-1 propyl-2]-10 phénothiazinecarbonitrile-2, série L, peut être préparé de la manière suivante :

Un mélange de 25 g de méthanesulfonate de (cyano-2 phénothiazinyl-10)-2 propyle-1, série L, et de 26,6 cm3 de pyrrolidine dans 250 cm3 de toluène est chauffé pendant 55 heures à une température voisine de 90°C. Le mélange réactionnel est concentré à sec sous pression réduite (30 mm de Hg; 4 kPa) à 40°C. Le résidu est repris par 500 cm3 d'éther éthylique et extrait par 2 fois 100 cm3 par une solution aqueuse d'acide méthanesulfonique 2N. La phase aqueuse est alcalinisée par de la lessive de soude à une température voisine de 5°C et extraite par 2 fois 250 cm3 d'éther éthylique. Les phases organiques réunies sont lavées successivement par 100 cm3 d'éther éthylique. Les phases organiques réunies sont lavées successivement par 100 cm3 d'eau distillée et par 100 cm3 d'une solution aqueuse saturée de chlorure de sodium, séchées sur sulfate de magnésium, filtrées et le filtrat jaune est concentré à sec sous pression réduite (30 mm de Hg; 4 kPa) à 40°C. Les 17,1 g de l'huile orangée ainsi obtenue sont chromatographiés sur une colonne (hauteur : 45 cm; diamètre : 4 cm) de gel de silice (0,063-0,2 mm) en éluant par 1 litre d'un mélange de chlorure de méthylène et de méthanol (95-5 en volumes) et en recueillant des fractions de 100 cm3. Les fractions 3 à 7 sont réunies et concentrées à sec sous pression réduite (30 mm de Hg; 4 kPa) à 40°C. On obtient ainsi 11,2 g de [(pyrrolidinyl-1)-1 propyl-2]-10 phénothiazinecarbonitrile-2, série L, sous forme d'une huile jaune.
$[\alpha]_D^{20}$ = +9,7 ±0,3° (1,2%; chloroforme).

Le méthanesulfonate de (cyano-2 phénothiazinyl-10)-2 propyle-1, série L, peut être préparé de la manière suivante :

A une solution de 12,6 g de (hydroxy-1 propyl-2)-10 phénothiazinecarbonitrile-2, série L, dans 126 cm3

de chlorure de méthylène refroidie à une température voisine de 5°C, on ajoute, sous agitation, 10 cm3 de triéthylamine puis on verse, goutte à goutte pendant 25 minutes, une solution de 5,6 cm3 de chlorure de méthanesulfonyle dans 56 cm3 de chlorure de méthylène et on poursuit l'agitation pendant 1 heure 15 minutes à une température voisine de 10-15°C. Le mélange réactionnel est lavé successivement par deux fois 100 cm3 d'eau distillée et par 100 cm3 d'une solution aqueuse saturée de chlorure de sodium, séché sur sulfate de magnésium, filtré et concentré à sec sous pression réduite (30 mm de Hg; 4 kPa) à 40°C. On obtient ainsi 16,2 g de méthanesulfonate de (cyano-2 phénothiazinyl-10)-2 propyle-1, série L, sous forme d'une huile orangée qui est utilisée sans autre purification pour la suite des synthèses.

$[\alpha]_D^{20}$ = +29,9, ±0,3° (2,4%; chloroforme).

L'(hydroxy-1 propyl-2)-10 phénothiazinecarbonitrile, série L, peut être préparé de la manière suivante :

A une solution de 42 g de phtalate de (+)-(cyano-2 phénothiazinyl-10)-2 propyle et de phényl-1 éthylammonium-(R) dans 420 cm3 d'éthanol au reflux, on ajoute 84,9 cm3 d'une solution de potasse alcoolique 1,97M et on poursuit le reflux sous agitation pendant 15 minutes. Le mélange réactionnel est alors versé sur 500 g de glace pilée et extrait par 500 cm3 puis deux fois 250 cm3 d'acétate d'éthyle. Les phases organiques sont réunies, lavées successivement par 200 cm3 d'une solution aqueuse d'acide chlorhydrique 0,5N, par 100 cm3 d'une solution aqueuse d'acide chlorhydrique 0,1N, par deux fois 250 cm3 d'une solution aqueuse saturée d'hydrogénocarbonate de sodium et par 100 cm3 d'une solution aqueuse saturée de chlorure de sodium, séchées sur sulfate de magnésium, filtrées et concentrées à sec sous pression réduite (30 mm de Hg; 4 kPa) à 40°C. Le solide jaune résiduel est repris par 100 cm3 d'oxyde d'isopropyle, trituré, essoré, lavé par 10 cm3 d'oxyde d'isopropyle et séché sous pression réduite (5 mm de Hg; 0,7 kPa) à 40°C. On obtient ainsi 17,8 g de (hydroxy-1 propyl-2)-10 phénothiazinecarbonitrile-2, série L, sous forme de cristaux jaunes fondant à 136°C.

$[\alpha]_D^{20}$ = -13 ±0,4° (1,2%; chloroforme).

Le phtalate de (+)-(cyano-2 phénothiazinyl-10)-2 propyle, et de phényl-1 éthylammonium-(1R) peut être préparé de la manière suivante :

On porte à reflux pendant 6 heures, sous agitation, une suspension de 56,5 g d'[(hydroxy-1 propyl-2-(2RS)]-10 phénothiazinecarbonitrile-2 et de 32,6 g d'anhydride phtalique dans 100 cm3 de pyridine anhydre. Après refroidissement, le mélange réactionnel est dilué par 500 cm3 de chlorure de méthylène, lavé par 4 fois 100 cm3 d'eau distillée, séché sur sulfate de magnésium, filtré et concentré à sec sous pression réduite (30 mm de Hg; 4 kPa) à 40°C. Le résidu est agité avec 500 cm3 d'une solution aqueuse N d'acide chlorhydrique puis décanté et dissous dans 500 cm3 d'acétate d'éthyle. La solution est lavée par 2 fois 100 cm3 d'une solution aqueuse N d'acide chlorhydrique puis par 100 cm3 d'une solution aqueuse de chlorure de sodium. La phase organique est séchée sur sulfate de magnésium, filtrée et concentrée à sec sous pression réduite (30 mm de Hg; 4 kPa) à 50°C. On obtient ainsi 102 g d'une huile épaisse contenant de l'acide [cyano-2 phénothiazinyl-10)-2 propyl-1-] oxycarbonyl-2 benzènecarboxylique et utilisés tels quels ultérieurement.

On dissout 102 g de l'huile précédemment obtenue et contenant l'acide [(cyano-2 phénothiazinyl-10)-2 propyl-1-(2RS)]oxycarbonyl-2 benzènecarboxylique dans 500 cm3 d'acétate d'éthyle et on ajoute, sous agitation, à une température voisine de 20°C, une solution de 24,2 g de (-)-phényl-1 éthylamine-(1S) dans 360 cm3 d'acétate d'éthyle. Après 2 jours d'agitation à une température voisine de 20°C, le solide formé est filtré et conservé.

Le filtrat est concentré à sec sous pression réduite (30 mm de Hg; 4 kPa) à 40°C. Le résidu est repris par 500 cm3 d'une solution aqueuse N d'acide chlorhydrique et extrait par 2 fois 250 cm3 d'acétate d'éthyle. Les phases organiques réunies sont concentrées à sec sous pression réduite (30 mm de Hg; 4 kPa) à 40°C. Le résidu (50 g) est dissous dans 500 cm3 d'acétate d'éthyle et on ajoute 14 g de (+)-phényl-1 éthylamine-(1R). Après 16 heures d'agitation à une température voisine de 20°C, le solide formé est essoré et dissous dans 450 cm3 d'acétate d'éthyle au reflux. Après refroidissement, le solide formé est essoré, lavé par 40 cm3 d'acétate d'éthyle et séché sous pression réduite (50 mm de Hg; 4 kPa) à 40°C. On obtient ainsi 44,3 g de phtalate de (+)-(cyano-2 phénothiazinyl-10)-2 propyle et de phényl-1 éthylammonium-(1R) sous forme de cristaux jaunes clairs fondant à 154-155°C.

$[\alpha]_D^{20}$ = +20,8 ±0,5° (1,1%; chloroforme).

L'[hydroxy-1 propyl-2-(2RS)]-10 phénothiazinecarbonirile-2 peut être préparé de la manière suivante :

Dans une suspension de 52 g de borohydrure de sodium dans 1,4 litre de tétrahydrofuranne, on verse, sous agitation en 15 minutes et à une température voisine de 20°C, 113 cm3 d'éthanedithiol-1,2 puis, en 15 minutes dans les mêmes conditions, une solution de 296 g de (cyano-2 phénothiazinyl-10)-2 propionate d'éthyle-(2RS) dans 1,4 litre de tétrahydrofuranne. A la fin de l'addition, on chauffe le mélange réactionnel pendant 20 heures à une température voisine de 60°C. Après refroidissement à une température de 5°C, on verse 1 litre d'une solution aqueuse de soude 4N pendant 1 heure : un vif dégagement gazeux est observé. Le mélange réactionnel est ensuite versé dans un mélange de 1 litre d'une solution aqueuse de soude 4N et de 3 litres de chlorure de méthylène sous agitation. On isole la phase organique et on réextrait la phase aqueuse avec 1

litre de chlorure de méthylène. Les phases organiques réunies sont lavées par 2 fois 1 litre d'une solution aqueuse saturée de chlorure de sodium, séchées sur sulfate de magnésium, filtrées et concentrées à sec sous pression réduite (30 mm de Hg; 4 kPa) à 30°C. L'huile visqueuse orangée (290 g) est purifiée sur une colonne (hauteur : 50 cm; diamètre : 8,5 cm) de gel de silice (0,2-0,063 mm) en éluant successivement par 3 litres de chlorure de méthylène, puis par 4 litres d'un mélange de chlorure de méthylène et de méthanol (97,5-2,5 en volumes) et par 10 litres un mélange de chlorure de méthylène et de méthanol (95-5 en volumes) et en recueillant des fractions de 1 litre. Les fractions 3 à 15 sont réunies et concentrées à sec sous pression réduite (30 mm de Hg; 4 kPa) à 30°C. On obtient ainsi 169,7 g d'[hydroxy-1 propyl-2-(2RS)]-10 phénothiazinecarbonitrile-2 sous forme d'un solide jaune fondant à 123°C.

Le (cyano-2 phénothiazinyl-10)-2 propionate d'éthyle-(2RS) peut être préparé de la manière suivante :

Dans une suspension de 24 g d'hydrure de sodium dans 1 litre de diméthylformamide à une température voisine de 25°C, on verse, sous agitation et en 2 heures 30, une solution de phénothiazinecarbonitrile-2 (224,5 g) dans 1 litre de diméthylformamide puis on laisse encore agiter 1 heure 15 minutes jusqu'à fin de dégagement gazeux. La suspension fine obtenue est versée, sous agitation et à une température voisine de 25°C, en 4 heures 30 minutes dans une solution de 255 cm3 de chloro-2 propionate d'éthyle dans 1 litre de diméthylformamide et on poursuit l'agitation pendant 16 heures. On verse alors, dans le mélange réactionnel, 100 cm3 d'éthanol, puis on verse le tout sur un mélange de 2 kg de glace dans 4 litres d'eau distillée : une gomme précipite puis cristallise. On essore le solide formé, le lave successivement avec 6 fois 500 cm3 d'eau distillée et 2 fois 500 cm3 d'éther de pétrole et le sèche à l'air. On obtient ainsi 296,5 g de (cyano-2 phénothiazinyl-10)-2 propionate d'éthyle-2(2RS) sous la forme de cristaux kaki fondant à 117-8°C, utilisé tel quel à l'étape suivante.

## EXEMPLE 15

Une solution de 2,5 g de [(pyrrolidinyl-1)-1 propyl-2]-10 phénothiazinecarbothioamide-2, série D, et de 8,3 cm3 de propylamine dans 50 cm3 d'éthanol absolu est saturée avec de l'acide sulfhydrique et chauffée pendant 16 heures à une température voisine de 100°C. Après refroidissement, la solution orangée obtenu est concentrée à sec sous pression réduite (30 mm de Hg; 4 kPa) à 40°C. L'huile brune résiduelle est purifiée par chromatographie sur une colonne (hauteur : 25 cm; diamètre : 2 cm) de gel de silice (0,04-0,063 mm) avec une légère surpression d'azote (40 kPa) en éluant avec 1 litre d'un mélange d'acétate d'éthyle et de cyclohexane (80-20 en volumes) et en recueillant des fractions de 40 cm3. Les fractions 8 à 18 réunies sont concentrées à sec sous pression réduite (30 mm de Hg; 4 kPa) à 40°C. On obtient ainsi 2,4 g de N-propyl [(pyrrolidinyl-1)-1 propyl-2]-10 phénothiazinecarbothioamide-2, série D, sous forme d'une huile jaune {$[\alpha]_D^{20}$ = -22 ±0,5° (,06%; méthanol)}. On dissout cette huile dans 7,5 cm3 de propanol-2 à une température voisine de 50°C et on verse cette solution, sous agitation, dans une solution de 0,68 g d'acide fumarique dans 7,5 cm3 de propanol-2 à une température voisine de 60°C. On poursuit l'agitation pendant 2 heures à une température voisine de 5°C. Le solide formé est essoré, lavé par 2 fois 2 cm3 de propanol-2 et séché sous pression réduite (5 mm de Hg; 0,68 kPa) à 40°C. On obtient ainsi 2 g de fumarate acide de N-propyl [(pyrrolidinyl-1)-1 propyl-2]-10 phénothiazinecarbothioamide-2, série D, sous forme d'un solide jaune fondant à 206°C dont les caractéristiques RMN sont identiques à celles décrites à l'exemple 13.

$[\alpha]_D^{20}$ = + 5,9 ±0,5° (0,9%; diméthylformamide).

Spectre infra-rouge (KBr), bandes caractéristiques en $cm^{-1}$ : 3240, 2960, 2935, 2880, 2580, 2480, 1700, 1590, 1530, 1460, 980, 820, 755, 645.

## EXEMPLE 16

Une solution de 0,9 g de chlorhydrate de {[diméthyl-2,5 pyrrolidinyl-1-(2RS,5RS)]-1 propyl-2-(2RS)}-10 phénothiazinecarbothioamide-2 et de 2,9 cm3 de propylamine dans 20 cm3 d'éthanol absolu est saturée avec de l'acide sulfhydrique et chauffée pendant 12 heures à une température voisine de 120°C. Après refroidissement, la solution obtenue est purgée à l'azote et concentrée à sec sous pression réduite (30 mm de Hg; 4 kPa) à 40°C. Le résidu est dissous dans 100 cm3 d'acétate d'éthyle et la solution est lavée avec 2 fois 20 cm3 d'eau distillée, séchée sur sulfate de magnésium, filtrée et concentrée à sec sous pression réduite (30 mm de Hg; 4 kPa) à 40°C. L'huile jaune résiduelle (1,09 g) est purifiée par chromatographie sur une colonne (hauteur : 30 cm; diamètre : 1 cm) de gel de silice (0,04-0,063 mm) avec une légère surpression d'azote (40 kPa) en éluant avec 200 cm3 de chlorure de méthylène puis avec 250 cm3 d'un mélange de chlorure de méthylène et de méthanol (95-5 en volumes) et en recueillant des fractions de 50 cm3. Les fractions 3 à 7 réunies sont concentrées à sec sous pression réduite (30 mm de Hg; 4 kPa) à 40°C. L'huile jaune résiduelle (0,75 g) est dissoute

dans 20 cm3 d'acétate d'éthyle et additionnée, sous agitation de 0,6 cm3 d'une solution d'acide chlorhydrique 3N dans l'éther éthylique. La gomme formée est séparée par décantation du solvant, reprise en solution dans 2 cm3 de propanol-2 et additionnée de 10 cm3 d'éther éthylique. Le solide formé est essoré, lavé avec 2 fois 5 cm3 d'éther éthylique et séché sous pression réduite (5 mm de Hg; 0,68 kPa) à 40°C. On obtient ainsi 0,63 g de chlorhydrate de {[diméthyl-2,5 pyrrolidinyl-1-(2RS,5RS)]-1 propyl-2-(2RS)}-10 N-propyl phénothiazine-carbothioamide-2 sous forme d'un solide jaune fondant à 138-140°C (fusion pâteuse).

RMN du proton (200 MHz, DMSO, δ en ppm, J en Hz).

0,94 (T, J = 7,5, 3H, $(-CH_2)_2C\underline{H}_3$); 1,17 et 1,27 (2D, J = 6,5, 6H, $-CH_3$ du diméthyl-2,5 pyrrolidinyle); 1,38 - 1,60 et 2,05 (3Mt, 1H - 1H et 2H respectivement, $-CH_2-$ du pyrrolidinyle); 1,72 (Mt, 2H, $-C\underline{H}_2-C\underline{H}_2CH_3$); 1,88 (D, J = 7, 3H, $-CH_3$); 3,3 à 3,9 (Mt, 4H,>N-$CH_2$- et >N-CH< du diméthyl-2,5 pyrrolidinyle); 3,65 (Mt, 2H, -CSNH-$C\underline{H}_2$-); 4,83 (Mt, 1H, CH-N ); 7 à 7,4 (Mt, 5H, aromatiques); 7,43 (D, J = 8, 1H, -H en 3); 7,61 (S, 1H, -H en 1); 10 (Mt, 1H, $-NH^+$); 10,58 (T, J = 5, 1H, -CSNH-).

Spectre infra-rouge (KBr), bandes caractéristiques en $cm^{-1}$ : 3180, 2960, 2930, 2880, 2590, 2500, 1600, 1535, 1560, 1415, 880, 820, 750.

Le chlorhydrate de {[diméthyl-2,5 pyrrolidinyl-1-(2RS, 5RS)] -1 propyl-2-(2RS)}-10 phénothiazinecarbothioamide-2 peut être préparé de la manière suivante :

Une solution de 0,85 g de {[diméthyl-2,5 pyrrolidinyl-1-(2RS,5RS)]-1 propyl-2-(2RS)}-10 phénothiazine-carbonitrile-2 et de 0,325 cm3 de triéthylamine dans 17 cm3 de pyridine anhydre est additionnée d'acide sulfhydrique et on agite pendant 16 heures à une température voisine de 20°C. Le mélange réactionnel est dilué par 50 cm3 d'acétate d'éthyle, lavé avec 3 fois 20 cm3 d'eau distillée, séché sur sulfate de magnésium, filtré et concentré à sec sous pression réduite (30 mm de Hg; 4 kPa) à 40°C. La gomme résiduelle jaune est purifiée par chromatographie sur une colonne (hauteur : 20 cm, diamètre : 1 cm) de gel de silice (0,04-0,063 mm) avec une légère surpression d'azote (40 kPa) en éluant par 500 cm3 d'un mélange de chlorure de méthylène et de méthanol (95-5 en volumes) et en recueillant des fractions de 40 cm3. Les fractions 5 à 9 réunies sont concentrées à sec sous pression réduite (30 mm de Hg; 4 kPa) à 40°C. La gomme jaune résiduelle (1 g) est dissoute dans 20 cm3 d'acétate d'éthyle et additionnée sous agitation, de 0,75 cm3 d'une solution d'acide chlorhydrique 3,4N dans l'éther éthylique. Le solide cristallisé formé est essoré, lavé par 5 cm3 d'éther éthylique et séché sous pression réduite (5 mm de Hg; 0,68 kPa) à 40°C. Le produit (1 g) est dissous dans 50 cm3 d'éthanol au reflux en présence de noir 3S et la solution est filtrée à une température voisine de 70°C. Après refroidissement, on maintient pendant 4 heures à une température voisine de 5°C et les cristaux formés sont essorés, lavés par 2 fois 5 cm3 d'éthanol et séchés sous pression réduite (5 mm de Hg; 0,68 kPa) à 40°C. On obtient ainsi 0,7 g de chlorhydrate de {[diméthyl-2,5 pyrrolidinyl-1-(2RS, 5RS)]-1 propyl-2-(2RS)}-10 phénothiazinecarbothioamide-2 sous forme de cristaux jaune vif fondant à 210-215°C (décomposition).

RMN du proton (250 MHz, DMSO, δ en ppm, J en Hz).

En solution dans le DMSO, on observe deux formes dues à la salification de l'azote, ce phénomène disparaît par addition de $CD_3COOD$.

1,59 et 2,05 (2Mt, 2H chacun, $-CH_2-$ du pyrrolidinyle); 1,9 (D, J = 7, 3H, $-CH_3$); 4,7 (Mt, 1H, >N-CH<); 7 à 7,35 (Mt, 5H, aromatiques); 7,56 (DD, J = 8 et 1, 1H, 1H du >N-$CH_2$-); 7,68 (D, J = 1, 1H, 1H du >N-$CH_2$-); 9,7 et 9,95 (2Mf, 1H et 2H respectivement, $-CSNH_2$ et $-NH^+$).

Le {[diméthyl-2,5 pyrrolidinyl-1-(2RS,5RS)]-1 propyl-2-(2RS)}-10 phénothiazinecarbonitrile-2 peut être préparé de la manière suivante :

Une solution de 5 g de méthanesulfonate de (cyano-2 phénothiazinyl-10)-2 propyle-1 et de 5 g de diméthyl-2,5 pyrrolidine-(2RS,5RS) dans 50 cm3 de toluène est chauffée pendant 4 jours à une température voisine de 100°C. Après refroidissement, le mélange réactionnel est dilué avec 200 cm3 d'acétate d'éthyle et extrait successivement par 50 cm3 et 2 fois 25 cm3 d'une solution aqueuse d'acide chlorhydrique N. Les phases aqueuses réunies sont alcalinisées avec une solution aqueuse de soude 10N jusqu'à pH 13 et extraites successivement avec 200 cm3 et 50 cm3 d'éther éthylique. Les phases organiques réunies sont séchées sur sulfate de magnésium, filtrées et concentrées à sec sous pression réduite (30 mm de Hg; 4 kPa) à 30°C. La gomme jaune résiduelle (2,8 g) est purifiée par chromatographie sur une colonne (hauteur : 30 cm; diamètre : 2 cm) de gel de silice (0,2-0,063 mm) en éluant avec 500 cm3 d'un mélange de cyclohexane et d'acétate d'éthyle (80-20 en volumes) et en recueillant des fractions de 30 cm3. Les fractions 5 à 11 réunies sont concentrées à sec sous pression réduite (30 mm de Hg; 4 kPa) à 40°C. Le résidu visqueux jaune (2,4 g) est dissous dans 15 cm3 d'oxyde d'isopropyle au reflux. La solution est refroidie puis conservée pendant 2 heures à une température voisine de 5°C. Les cristaux formés sont essorés, lavés par 2 cm3 d'oxyde d'isopropyle et séchés sous pression réduite (5 mm de Hg; 0,68 kPa) à 40°C. On obtient ainsi 1 g de {[diméthyl-2,5 pyrrolidinyl-1-(2RS,5RS)]-1 propyl-2-(2RS)}-10 phénothiazinecarbonitrile-2 sous forme de cristaux jaune clair fondant à 105°C.

EXEMPLE 17

Un mélange de 1 g de fumarate acide de {[méthyl-3 pipéridinyl-1-(3RS)]-1 propyl-2-(2RS)}-10 phénothiazinecarbothioamide-2 et de 2,5 cm3 de propylamine dans 20 cm3 d'éthanol absolu est saturé d'acide sulfhydrique et chauffé pendant 16 heures à une température voisine de 100°C. Après refroidissement, on concentre à sec sous pression réduite (30 mm de Hg; 4 kPa) à 40°C. L'huile orangée résiduelle (1,5 g) est purifiée par chromatographie sur une colonne (hauteur : 25 cm, diamètre : 2 cm) de gel de silice (0,04-0,063 mm) avec une légère surpression d'azote (40 kPa) en éluant avec 250 cm3 d'un mélange d'acétate d'éthyle et de cyclohexane (80-20 en volumes) et en recueillant des fractions de 40 cm3. Les fractions 3 et 4 réunies sont concentrées à sec sous pression réduite (30 mm de Hg; 4 kPa) à 40°C. L'huile jaune résiduelle (0,9 g) est dissoute dans 3 cm3 d'éthanol à 60°C et on verse cette solution chaude, sous agitation, dans une solution de 0,23 g d'acide fumarique dans 3,5 cm3 d'éthanol au reflux. Après refroidissement, on poursuit l'agitation pendant 2 heures à une température voisine de 5°C. Le solide formé est essoré, lavé par 2 fois 1 cm3 d'éthanol et séché sous pression réduite (5 mm de Hg; 0,68 kPa) à 40°C. On obtient ainsi 0,53 g de fumarate neutre de {[méthyl-3 pipéridinyl-1-(3RS)]-1 propyl-2-(2RS)}-10 N-propyl phénothiazinecarbothioamide-2 sous forme d'un solide jaune fondant à 126°C.

RMN du proton (250 MHz, DMSO, $\delta$ en ppm, J en Hz).

0,76 (D, J = 7, 3H, $-CH_3$ du méthyl-3 pipéridinyle); 0,93 (T, J = 7,5, 3H, $-(CH_2)_2C\underline{H}_3$); 0,8, de 1,35 à 1,80, 1,98 et 2,80 (4Mt, intégrant respectivement pour 1H, 5H, 1H et 2H, $-CH_2-$, $\searrow$CH-et$>$N-$C\underline{H}_2$-); 1,56 (D, J = 7, 3H, $-CH_3$); 1,7 (Mt, 2H, $-CH_2C\underline{H}_2CH_3$); 2,68 (DD, J = 13 et 6, 1H, 1H du $>$N-$CH_2$-); 2,93 (DD, J = 13 et 6,5, 1H, 1H du $>$N-$CH_2$-); 3,64 (Mt, 2H, -CSNH-$C\underline{H}_2$-); 4,23 (Mt, J = 7 -6,5 et 6, 1H, $>$N-CH$<$); 6,6 (S, 1H, -CH=CH- de l'hemifumarate); 6,9 à 7,2 (Mt, 5H, aromatiques); 7,25 (DD, J = 8 et 1, 1H, -H en 3); 7,61 (D, J = 1, 1H, -H en 1); 10,25 (T, J = 5, 1H -CSNH-).

Spectre infra-rouge (KBr), bandes caractéristiques en $cm^{-1}$ : 3200, 2960, 2930, 2875, 1700, 1590, 1570, 1530, 1460, 980, 820, 755, 665.

Le fumarate acide de {[méthyl-3 pipéridinyl-1-(3RS)]-1 propyl-2-(2RS)}-10 phénothiazinecarbothioamide-2 peut être préparé de la manière suivante :

Une solution de 7,2 g de {[méthyl-3 pipéridinyl-1-(3RS))]-1 propyl-2-(2RS)}-10 phénothiazinecarbonitrile-2 et de 2,8 cm3 de triéthylamine dans 148 cm3 de pyridine anhydre est additionnée d'acide sulfhydrique et agitée pendant 16 heures à une température voisine de 20°C. La solution obtenue est purgée à l'azote pendant 40 minutes, diluée par 1 litre d'eau distillée et extraite par 2 fois 500 cm3 d'acétate d'éthyle. Les phases organiques réunies sont lavées successivement par 2 fois 250 cm3 d'eau distillée et par 250 cm3 d'une solution aqueuse saturée de chlorure de sodium, séchées sur sulfate de magnésium, filtrées et concentrées à sec sous pression réduite (30 mm de Hg; 4 kPa) à 40°C. L'huile orangée résiduelle (9,8 g) est purifiée par chromatographie sur une colonne (hauteur : 40 cm; diamètre : 3 cm) de gel de silice (0,2-0,063 mm) en éluant par 1,5 litre d'un mélange d'acétate d'éthyle et de cyclohexane (60-40 en volumes) et en recueillant des fractions de 100 cm3. Les fractions 6 à 10 réunies sont concentrées à sec sous pression réduite (30 mm de Hg; 4 kPa) à 40°C. On obtient ainsi 6,7 g d'une meringue jaune. On dissout 2,3 g de ce produit dans 10 cm3 d'éthanol à 60°C et on verse la solution chaude obtenue, sous agitation, dans une solution de 0,67 g d'acide fumarique dans 10 cm3 d'éthanol à 60°C. Après refroidissement, on poursuit l'agitation pendant 3 heures à une température voisine de 5°C. Le solide formé est essoré, lavé par 3 fois 5 cm3 d'éthanol et séché sous pression réduite (5 mm de Hg; 0,68 kPa) à 40°C. On obtient ainsi 2,45 g de fumarate acide de {[méthyl-3 pipéridinyl-1-(3RS)]-1 propyl-2-(2RS)}-10 phénothiazinecarbothioamide-2 sous forme d'un solide jaune fondant à 236°C.

RMN du proton (250 MHz, DMSO, $\delta$ en ppm, J en Hz).

6,9 à 7,25 (Mt, 5H, aromatiques); 7,42 (DD, J = 8 et 1, 1H, -H en 3); 7,75 (S large, 1H, -H en 1); 9,48 et 9,85 (2S, 1H chacun, $-CSNH_2$).

Le {[méthyl-3 pipéridinyl-1-(3RS)]-1 propyl-2-(2RS)}-10 phénothiazinecarbonitrile-2 peut être préparé de la manière suivante :

Une solution de 14,4 g de méthanesulfonate de (cyano-2 phénothiazinyl-10)-2 propyle-1-(2RS) et de 9,4 cm3 de méthyl-3 pipéridine-(3RS) dans 75 cm3 de toluène est agitée au reflux pendant 20 heures. Après refroidissement, on extrait par 2 fois 100 cm3 d'une solution aqueous d'acide chlorhydrique N. Les phases aqueuses réunies sont alcalinisées par de la lessive de soude (d = 1,33) jusqu'à pH 13 et extraites par 2 fois 200 cm3 d'acétate d'éthyle. Les phases organiques réunies sont lavées successivement par 2 fois 50 cm3 d'eau distillée et par 50 cm3 d'une solution aqueuse saturée de chlorure de sodium, séchées sur sulfate de magnésium, filtrées et concentrées à sec sous pression réduite (30 mm de Hg; 4 kPa) à 40°C. L'huile jaune résiduelle (14,4 g) est purifiée par chromatographie sur une colonne (hauteur : 40 cm; diamètre : 4 cm) de gel de silice (0,2-0,063 mm) an éluant par 2,5 litres d'un mélange de cyclohexane et d'acétate d'éthyle (80-20 en volumes)

et en recueillant des fractions de 100 cm3. Les fractions 10 à 12 réunies sont concentrées à sec sous pression réduite (30 mm de Hg; 4 kPa) à 40°C. On obtient ainsi 7,2 g de {[méthyl-3 pipéridinyl-1-(3RS)]-1 propyl-2-(2RS)}-10 phénothiazinecarbonitrile-2 sous forme d'une huile jaune utilisée telle quelle ultérieurement.

EXEMPLE 18

Une solution de 5,1 g de [(perhydroazépinyl-1)-1 propyl-2-(2RS)]-10 phénothiazinecarbothioamide-2 et de 3,78 g de propylamine dans 100 cm3 d'éthanol est chauffée pendant 16 heures à une température voisine de 150°C. Après refroidissement, le mélange réactionnel est concentré à sec sous pression réduite ( 30 mm de Hg; 4 kPa) à 40°C. Le résidu est dissous dans 250 cm3 d'acétate d'éthyle, lavé successivement par 3 fois 100 cm3 d'eau distillée puis par 100 cm3 d'une solution aqueuse demi-saturée de chlorure de sodium, séché sur sulfate de magnésium, filtré et concentré à sec sous pression réduite (30 mm de Hg; 4kPa) à 40°C. Le résidu (4,74 g) est purifié par chromatographie sur colonne (hauteur: 30 cm; diamètre: 4,6 cm) de gel de silice (0,04-0,063 mm) sous légère surpression d'azote (40kPa) en éluant avec 1,5 litre d'un mélange de cyclohexane et d'acétate d'éthyle (70-30 en volumes) et en recueillant des fractions de 60 cm3. Les fractions 11 à 20 sont réunies et concentrées à sec sous pression réduite (30 mm de Hg; 4 kPa) à 40°C pour donner 0,77 g d'un résidu huileux. Ce résidu est dissous dans 15 cm3 d'éthanol et on ajoute 0,45 cm3 d'une solution 4N d'acide chlorhydrique dans l'éthanol. La solution obtenue est concentrée à sec sous pression réduite (30 mm de Hg ; 4kPa) à 40°C. Le résidu est concrété dans 50 cm3 d'oxyde de diéthyle sous agitation pendant 16 heures et le solide est filtré, lavé par deux fois 10 cm3 d'oxyde de diéthyle et séché sous pression réduite (5 mm de Hg ; 0,67 kPa) à 45°C. On obtient ainsi 0,62 g de chlorhydrate de [(perhydroazépinyl-1)-1 propyl-2-(2RS)]-10 N-propyl-phénothiazinecarbothioamide-2 sous forme d'une poudre beige fondant à 187-188°C.

RMN du proton (250 MHz, DMSO D6, δ en ppm, J en Hz).

0,95 (T, J = 7, 3H, $-(CH_2)_2C\underline{H_3}$ ; 1,4 à 1,9 (Mt, 8H, $-CH_2-$ du perhydroazépinyle) ; 1,73 (Mt, 2 H, $-CH_2C\underline{H_2}CH_3$) ; 1,81 (D, J = 7, 3H, $-CH_3$) ; 3,15 à 3,45 (Mt, 4H, $>N-CH_2-$ du perhydroazépinyle) ; 3,50 et 3,8 (2 Mt, 1H chacun, $>N-CH_2-$) ; 3,70 (Mt, J = 7 et 5, 1H, $-CONH-C\underline{H_2}-$) ; 4,85 (Mt, 1H, $>N-CH<$) ; 7,05 à 7,4 (Mt, 5 H, aromatiques) ; 7,41 (D large, J = 8, 1H, -H en 3) , 7,59 (D, J = 1, 1H, -H en 1) ; 10,15 (Mf, 1H, $-NH^+Cl^-$) ; 10,44 (T, J = 5, 1H, -CONH-).

Spectre infra-rouge (KBr), bandes caractéristiques en $cm^{-1}$ : 3175, 2960, 2930, 2870, 2620, 2530, 1590, 1465, 1530, 885, 825, 750.

Le [(perhydroazépinyl-1)-1 propyl-2-(2RS)]-10 phénothiazinecarbothioamide-2 peut être préparé de la manière suivante :

Un mélange de 5,11 g de [(perhydroazépinyl-1)-1 propyl-2-(2RS)]-10 phénothiazinecarbonitrile-2 et de 1,96 cm3 de triéthylamine dans 50 cm3 de pyridine anhydre est saturé par bullage d'acide sulfhydrique pendant 3 heures à 25°C. La solution limpide obtenue est maintenue sous agitation pendant 16 heures à 25°C, puis le mélange est dégazé par bullage d'azote pendant 2 heures. Le mélange réactionnel est dilué par 300 cm3 d'acétate d'éthyle et lavé par 10 fois 100 cm3 d'eau distillée. La phase organique est séchée sur sulfate de magnésium, filtrée et le filtrat est concentré à sec sous pression réduite (30 mm de Hg ; 4 kPa) à 40°C. Le résidu est purifié par chromatographie sur colonne de gel de silice 0,06-0,2 mm (hauteur : 38 cm ; diamètre : 3 cm) en éluant par 1,5 l d'un mélange de cyclohexane et d'acétate d'éthyle (50-50 proportions en volumes) et en recueillant des fractions de 125 cm3. Les fractions 3 à 9 sont réunies et concentrées à sec sous pression réduite (30 mm de Hg ; 4 kPa) pour donner 5,15 g de [(perhydroazépinyl-1)-1 propyl-2-(2RS)] -10 phénothiazinecarbothioamide-2 sous forme d'une huile orangée.

Le [(perhydroazépinyl-1)-1 propyl-2-(2RS)]-10 phénothiazinecarbonitrile-2 peut être préparé de la manière suivante :

Une solution de 9,05 g de mésylate de (cyano-2 phénothiazinyl-10)-2 propyle dans 28,2 cm3 d'hexaméthylèneimine est portée à 90°C pendant 16 heures. Après refroidissement, le mélange est dilué par 200 cm3 d'acétate d'éthyle, lavé par 4 fois 150 cm3 d'eau distillée. La phase organique est séchée sur sulfate de magnésium, filtrée et concentrée à sec sous pression réduite (30 mm de Hg ; 4 kPa) à 40°C. Le résidu est purifié par chromatographie sur colonne de gel de silice 0,06-0,2 mm (hauteur : 44 cm ; diamètre: 3,8 cm) en éluant par des mélanges de cyclohexane et d'acétate d'éthyle 80-20 (500 cm3) et 70-30 (1500 cm3) (proportions en volumes) et en recueillant des fractions de 125 cm3. Les fractions 7 à 15 sont réunies et concentrées à sec sous pression réduite (30 mm de Hg ; 4 kPa) à 40°C pour donner 5,27 g de [(perhydroazépinyl-1)-1 propyl-2-(2RS)]-10 phénothiazinecarbonitrile-2 sous forme d'un miel jaune.

EXAMPLE 19

Une solution de 5 g de [(dihydro-2,5 pyrrolyl-1)-1 propyl-2]-10 phénothiazinecarbothioamide-2, série L,

et de 4,02 g de propylamine dans 35 cm3 d'éthanol est saturée par barbottage pendant 25 minutes avec de l'acide sulfhydrique puis chauffée pendant 16 heures à une température voisine de 150°C. Après refroidissement, le mélange réactionnel est concentré à sec sous pression réduite (30 mm de Hg; 4 kPa) à 40°C. Le résidu est dissous dans 50 cm3 d'acétate d'éthyle, lavé successivement par 5 fois 50 cm3 d'eau distillée puis par 50 cm3 d'une solution aqueuse demi-saturée de chlorure de sodium, séché sur sulfate de magnésium, filtré et concentré à sec sous pression réduite (30 mm de Hg; 4kPa) à 40°C. Le résidu (5,34 g) est purifié par chromatographie sur colonne (hauteur: 23 cm; diamètre: 4,4 cm) de gel de silice (0,04-0,063 mm) sous légère surpression d'azote (40kPa) en éluant avec 2,5 litre d'un mélange de cyclohexane et d'acétate d'éthyle (70-30 en volumes) et en recueillant des fractions de 125 cm3. Les fractions 5 à 16 sont réunies et concentrées à sec sous pression réduite (30 mm de Hg; 4kPa) à 40°C pour donner 3 g d'une meringue jaunâtre. 1 g de ce résidu est dissout dans 5 cm3 de propanol-2 au reflux et additionné d'une solution tiède de 0,28 g d'acide fumarique dans 5,6 cm3 de propanol-2. Après refroidissement et cristallisation le solide formé est filtré, lavé successivement par 2 cm3 de propanol-2 et par 10 cm3 d'oxyde de diéthyle puis séché sous pression réduite (5 mm de Hg; 0,67 kPa). On obtient ainsi 0,77 g de fumarate de N-propyl [(dihydro-2,5 pyrrolyl-1)-1 propyl-2]-10 phénothiazinecarbothioamide-2, série L, sous forme d'une poudre jaune fondant vers 206°C.

$[\alpha]_D^{20}$ = + 39,5 ± 0,8° (0,8 % ; méthanol).

RMN du proton (250 MHz, DMSO D6, δ en ppm, J en Hz).

0,95 (T, J = 7,5, 3H, $-(CH_2)_2C\underline{H}_3$) ; 1,62 (D, J = 7, 3H, $-CH_3$) ; 1,7 (Mt, 2H, $-CH_2C\underline{H}_2CH_3$) ; 3,12 (DD, J = 12,5 et 6,5, 1H, 1 H du $>N-CH_2-$); 3,32 (DD, J = 12,6 et 6, 1H, 1 H du $>N-CH_2-$) ; 3,57 (AB, 6H, $>N-CH_2-$ de la pyrroline) ; 3,65 (Mt, 2 H, $-CS-NH-CH_2-$) ; 4,22 (Mt, J = 7, 6,5 et 6, 1H, $>N-CH<$) ; 5,79 (Mf, 2H, -CH=CH-) ; 6,63 (S, 2H, -CH=CH- du fumarate) ; 6,90 à 7,30 (Mt, 5H, aromatiques) ; 7,31 (DD, J = 8 et 1, 1H, -H en 3) ; 7,59 (D, J = 1, 1H, -H en 1) ; 10,26 (T, J = 5,5, 1H, -CS-NH-).

Spectre infra-rouge (KBr), bandes caractéristiques en $cm^{-1}$ : 2965, 2930, 2870, 2750, 2250, 2250, 1850, 1680, 1640, 1590, 1555, 1460, 1530, 980, 880, 810, 800, 755, 645.

Le [(dihydro-2,5 pyrrolyl-1)-1 propyl-2]-10 phénothiazine-carbothioamide-2, série L peut être préparé de la manière suivante :

Une solution de 12,57 g de [(dihydro-2,5 pyrrolyl-1)-1 propyl-2]-10 phénothiazinecarbonitrile-2, série L et de 5,5 cm3 de triéthylamine dans 115 cm3 de pyridine est additionnée d'acide sulfhydrique par barbottage pendant 2 heures. Après 16 heures d'agitation à une température voisine de 25°C, la solution brun-clair obtenue est purgée à l'azote pendant 2 heures, diluée par 300 cm3 d'acétate d'éthyle, lavée par 10 fois 100 cm3 d'eau distillée, séchée sur sulfate de magnésium, filtrée et concentrée à sec sous pression réduite (30 mm de Hg; 4 kPa) à 40°C. Le résidu meringué jaune (13,3 g) est purifié par chromatographie sur colonne (hauteur: 47 cm; diamètre: 3,8 cm) de gel de silice (0,04-0,063 mm) sous une légère surpression d'azote (40kPa) en éluant avec 2 litres d'acétate d'éthyle et en recueillant des fractions de 125 cm3. Les fractions 4 à 10 sont réunies et concentrées à sec sous pression réduite (30 mm de Hg; 4 kPa) à 40°C pour donner 10,6 g de [(dihydro-2,5 pyrrolyl-1)-1 propyl-2]-10 phénothiazinecarbothioamide-2, série L, sous forme d'une meringue jaune.

$[\alpha]_D^{20}$ = -41,9 ± 0,7° (1% ; chloroforme).

Le [(dihydro-2,5 pyrrolyl-1)-1 propyl-2]-10 phénothiazine-carbonitrile-2, série L peut être préparé de la manière suivante :

Une solution de 44 g de methanesulfonate de (cyano-2 phénothiazinyl-10)-2 propyl-1, série L, et de 42,2 g de dihydro-2,5 pyrrole dans 250 cm3 de toluène anhydre est chauffée à 90°C sous agitation pendant 18 heures. Après refroidissement, le mélange réactionnel est extrait par 2 fois 100 cm3 d'eau distillée puis par 250 cm3 d'une solution aqueuse d'acide chlorhydrique N. Les phases aqueuses acides réunies sont alcalinisées par une solution aqueuse de soude N et extraites par 3 fois 100 cm3 d'acétate d'éthyle. Les phases organiques sont réunies, lavées successivement par 100 cm3 d'eau distillée puis par 100 cm3 d'une solution aqueuse saturée de chlorure de sodium, séchées sur sulfate de magnésium, filtrées et concentrées à sec sous pression réduite (30 mm de Hg; 4 kPa) à 50°C. Le résidu huileux jaunâtre (30,4 g) est purifié par chromatographie sur colonne (hauteur: 38 cm; diamètre: 3,8 cm) de gel de silice (0,04-0,063 mm) sous une légère surpression d'azote (40kPa) en éluant avec 15 litres d'un mélange de cyclohexane et d'acétate d'éthyle (75-25 en volumes) et en recueillant des fractions de 1 litre. Les fractions 6 à 11 sont réunies et concentrées à sec sous pression réduite (30 mm de Hg; 4 kPa) à 40°C pour donner 11,15 g de [(dihydro-2,5 pyrrolyl-1)-1 propyl-2]-10 phénothiazinecarbonitrile-2, série L, sous forme d'une huile jaune.

$[\alpha]_D^{20}$ = + 32,6 ± 0,6° (1 % ; méthanol).

EXEMPLE 20

A une solution de 20 g de [diméthylamino-1 propyl-2-(2RS)]-10 phénothiazinecarbothioamide-2 dans 5

cm3 d'éthanol absolu, on ajoute 0,35 cm3 de n-butylamine. Le mélange est porté à 200°C pendant 5 heures puis dilué par 500 cm3 d'acétate d'éthyle, lavé par 3 fois 500 cm3 d'eau distillée, séché sur sulfate de magnésium puis concentré à sec sous pression réduite (30 mm de Hg; 4 kPa). Le solide résiduel est purifié par chromatographie sur une colonne (hauteur : 18 cm; diamètre : 5 cm) de gel de silice (0,6-0,02 mm) en éluant avec des mélanges d'acétate d'éthyle et de cyclohexane 20-80 (1,5 litre), 30-70 (11,5 litres) (en volumes) en recueillant des fractions de 250 cm3. Les fractions 28 à 45 sont réunies et concentrées sous pression réduite (30 mm de Hg; 4 kPa) pour donner 11,5 g d'une huile marron dont une partie aliquote (1,5 g) est mise en solution dans 30 cm3 d' acétate d'éthyle. A cette solution est ajoutée 1,01 g d'acide oxalique en solution dans 30 cm3 d'acétate d'éthyle. Un solide se développe et la suspension obtenue est filtrée pour donner 0,42 g d'oxalate acide de N-butyl [diméthylamino-1 propyl-2-(2RS)]-10 phénothiazinecarbothioamide-2 fondant à 154°C.

Spectre de RMN (250 MHz, DMSO, δ en ppm, J en Hz).

0,93 (T, J = 7,5,3H, $-CH_3$ butyle); 1,36 (Mt, 2H, $-C\underline{H_2}-CH_3$ butyle); 1,67 (Mt, 2H, $-C\underline{H_2}CH_2CH_3$ butyle); 1,72 (D, J = 7,5, 3H, $-CH_3$); 2,7 (S, 6H, $-N(CH_3)_2$); 3,36 (DD, J = 14 et 4,5, 1H, 1H du $>NCH_2-$); 3,56 (DD, J = 14 et 7,5, 1H, 1H du $>NCH_2-$); 3,70 (Q, J = 7, 2H, $=CSNH-C\underline{H_2}-$); 4,63 (Mt, $>NH-CH<$); 7 à 7,35 (Mt, 5H, aromatiques); 7,4 (DD, J = 8,5 et 1, 1H, -H en 3); 7,53 (D, J = 1, 1H, -H en 1).

Spectre infra-rouge (KBr), bandes caractéristiques en $cm^{-1}$ : 3230, 2960, 2930, 2270, 2690, 1720, 1640, 1590, 1530, 1460, 880, 830, 750.

EXEMPLE 21

Un mélange de 2,1 g de [N-éthyl N-méthyl-amino-1 propyl-2-(2RS)]-10 phénothiazinecarbothioamide-2 et de 8,5 cm3 de butylamine dans 30 cm3 d'éthanol absolu est saturé d'acide sulfhydrique et chauffé pendant 16 heures à une température voisine de 100°C. Après refroidissement, la solution orangée obtenue est concentrée à sec sous pression réduite (30 mm de Hg; 4 kPa) à 40°C. L'huile brune résiduelle est purifiée par chromatographie sur une colonne (hauteur : 4 cm; diamètre : 3 cm) de gel de silice (0,2-0,063 mm) en éluant successivement par 2 litres de chlorure de méthylène et par 1 litre d'un mélange de chlorure de méthylène et de méthanol (90-10 en volumes) et en recueillant des fractions de 60 cm3. Les fractions 36 à 39 sont réunies et concentrées à sec sous pression réduite (30 mm de Hg; 4kPa) à 40°C. L'huile résiduelle orangée (1,8 g) est à nouveau purifiée par chromatographie sur une colonne (hauteur : 25 cm; diamètre : 4 cm) de gel de silice (0,04-0,063 cm) sous une légère surpression d'azote (40 kPa) en éluant par 1 litre d'un mélange d'acétate d'éthyle et de cyclohexane (75-25 en volumes) et en recueillant des fractions de 60 cm3. Les fractions 5 à 12 sont réunies et concentrées à sec sous pression réduite (30 mm de Hg; 4 kPa) à 40°C. On obtient ainsi 1,25 g de N-butyl [N'-éthyl N'-méthyl-amino-1 propyl-2-(2RS)]-10 phénothiazinecarbothioamide-2 sous forme d'une huile jaune. Ce produit est dissous dans 100 cm3 d'oxyde d'isopropyle et additionné, goutte à goutte sous agitation de 10,9 cm3 d'une solution 0,28N d'acide chlorhydrique dans l'oxyde d'isopropyle. Après 1 heure sous agitation à une température voisine de 5°C, le précipité formé est essoré, lavé par 3 fois 10 cm3 d'oxyde d'isopropyle et séché sous pression réduite (5 mm de Hg; 0,68 kPa) à 40°C. On obtient ainsi 1,1 g de chlorhydrate de N-butyl [(N'-méthyl N'-éthyl-amino)-1 propyl-2-(2RS)]-10 phénothiazinecarbothioamide-2 sous forme d'un solide jaune fondant à 102°C (fusion pâteuse).

RMN du proton (250 MHz, DMSO, δ en ppm, J en Hz).

En solution dans le DMSO, on observe deux formes dues à la salification de l'azote.

0,96 (T, J = 7,5, 3H, $-(CH_2)_3C\underline{H_3}$); 1,07 et 1,21 (2T, J = 7,5, 3H, $-NCH_2C\underline{H_3}$); 1,4 (Mt, 2H, $-(CH_2)_2C\underline{H_2}CH_3$); 1,72 (Mt, 2H, $-C\underline{H_2}-CH_2CH_3$); 1,83 (Mt, 3H, $-CH_3$); 2,8 (Mt, 3H, $>N-CH_3$); 3 à 3,35 (Mt, 2H, $>NC\underline{H_2}CH_3$); 3,5 à 3,90 (Mt, 2H, $>N-CH_2-$); 3,75 (Mt, 2H, $-CSNH-C\underline{H_2}-$); 4,8 (Mt, 1H, $>N-CH<$); 7 à 7,4 (Mt, 5H, aromatiques); 7,45 (D, J = 8, 1H, -H en 3); 7,6 (S,1H, -H en 1); 10,12 (Mf, 1H, $-NH^+$); 10,5 (Mt, 1H, -CSNH-).

Spectre infra-rouge (KBr), bandes caractéristiques en $cm^{-1}$ : 3200, 2960, 2930, 2870, 2660, 2600, 2480, 1590, 1530, 1460, 870, 820, 755.

Le [(N-méthyl N-éthyl-amino)-1-propyl-2-(2RS)]-10 phénothiazinecarbothioamide-2 peut être préparé de la manière suivante :

Une solution de 3 g de [(N-éthyl N-méthyl-amino-1 propyl-2-(2RS)]-10 phénothiazinecarbonitrile-2 et de 1,3 cm3 de triéthylamine dans 60 cm3 de pyridine anhydre est additionnée d'acide sulfhydrique, en excès, puis on poursuit l'agitation pendant 16 heures à une température voisine de 20°C. La solution obtenue est purgée avec un courant d'azote pendant 1 heure, versée dans 100 cm3 d'acétate d'éthyle, lavée par 3 fois 200 cm3 d'eau distillée, séchée sur sulfate de magnésium, filtrée et concentrée à sec sous pression réduite (30 mm de Hg; 4 kPa) à 40°C. L'huile brune résiduelle est purifiée par chromatographie sur une colonne (hauteur : 40 cm; diamètre : 3 cm) de gel de silice (0,2-0,06 mm) en éluant successivement par 1 litre de chlorure de méthylène, 1 litre d'un mélange de chlorure de méthylène et de méthanol (95-5 en volumes) et 3 litres d'un mélange de chlorure de méthylène et de méthanol (80-20 en volumes) et en recueillant des fractions de 150

cm3. Les fractions 26 à 30 sont réunies et concentrées à sec sous pression réduite (30 mm de Hg; 4 kPa) à 40°C. On obtient ainsi 2.15 g de [N-éthyl N-méthyl-amino-1 propyl-2-(2RS)]-10 phénothiazinecarbothioamide-2 sous forme d'une huile orangée.

Le [N-éthyl N-méthyl-amino-1 propyl-2-(2RS)]-10 phénothiazinecarbonitrile-2 peut être préparé de la manière suivante :

Un mélange de 4,4 g d'[éthylamino-1 propyl-2-(2RS)]-10 phénothiazinecarbonitrile-2, de 2,25 g de carbonate de sodium et de 0,9 cm3 d'iodométhane dans 60 cm3 de diméthylformamide est chauffé pendant 6 heures à une température voisine de 150°C. Le mélange réactionnel est ensuite concentré à sec sous pression réduite (5 mm de Hg; 0,7 kPa) à 40°C et le résidu est repris par 100 cm3 d'eau distillée et extrait par 2 fois 100 cm3 d'acétate d'éthyle. Les phases organiques réunies sont extraites par 2 fois 50 cm3 d'une solution aqueuse d'acide chlorhydrique N. Les phases aqueuses réunies sont alcalinisées avec de la soude (d = 1,33) jusqu'à pH 13 et extraites par 2 fois 100 cm3 d'acétate d'éthyle. Les phases organiques réunies sont lavées successivement avec 50 cm3 d'eau distillée et avec 50 cm3 d'une solution aqueuse saturée de chlorure de sodium, séchées sur sulfate de magnésium, filtrées et concentrées à sec sous pression réduite (30 mm de Hg; 4 kPa) à 40°C. On obtient ainsi 3 g de [N-éthyl N-méthyl-amino-1 propyl-2-(2RS)]-10 phénothiazinecarbonitrile-2 sous forme d'une huile orangée.

EXEMPLE 22

Une solution de 2,1 g de [diéthylamino-1 propyl-2-(2RS)]-10 phénothiazinecarbothioamide-2 et de 8,2 cm3 de butylamine dans 30 cm3 d'éthanol absolu est saturée avec de l'acide sulfhydrique puis chauffée pendant 16 heures à une température voisine de 100°C. Après refroidissement, la solution jaune obtenue est concentrée à sec sous pression réduite (30 mm de Hg; 4 kPa) à 40°C. Le résidu est repris par 50 cm3 d'eau distillée et on extrait successivement par 100 cm3 puis par 50 cm3 d'acétate d'éthyle. Les phases organiques réunies sont lavées par 50 cm3 d'une solution aqueuse saturée de chlorure de sodium, séchées sur sulfate de magnésium, filtrées et concentrées à sec sous pression réduite (30 mm de Hg; 4 kPa) à 40°C. Le résidu huileux orangé (2,25 g) est purifié par chromatographie sur une colonne (hauteur : 25 cm; diamètre : 3 cm) de gel de silice (0,04-0,063 mm) avec une légère surpression d'azote (40 kPa) en éluant par 500 cm3 d'un mélange d'acétate d'éthyle et de cyclohexane (80-20 en volumes) et en recueillant des fractions de 50 cm3. Les fractions 6 à 8 sont réunies et concentrées à sec sous pression réduite (30 mm de Hg; 4 kPa) à 40°C. L'huile jaune résiduelle (1,8 g) est dissoute dans 100 cm3 d'oxyde d'isopropyle et additionnée sous agitation de 1 cm3 d'une solution 3,3N d'acide chlorhydrique dans l'oxyde d'isopropyle. Après 15 minutes d'agitation à une température voisine de 20°C, le solide formé est essoré, lavé par 3 fois 20 cm3 d'oxyde d'isopropyle et séché sous pression réduite (5 mm de Hg; 0,68 kPa) à 40°C. On obtient ainsi 1,5 g de chlorhydrate de N-butyl [diéthylamino-1 propyl-2-(2RS)]-10 phénothiazinecarbothioamide-2 sous forme d'un solide jaune fondant à 116°C (fusion pâteuse).

RMN du proton (250 MHz, DMSO, δ en ppm, J en Hz).

0,93 (T, J = 7, 3H, $-(CH_2)_3C\underline{H_3}$); 1 et 1,15 (2T, J = 7, 6H, $-N(CH_2C\underline{H_3})_2$); 1,37 (Mt, 2H, $-(CH_2)_2-C\underline{H_2}-CH_3$); 1,69 (Mt, 2H, $-CH_2-C\underline{H_2}CH_2CH_3$); 1,83 (D, J = 7, 3H, $-CH_3$); 3,17 (Mt, 4H, $-N(C\underline{H_2}CH_3)_2$); 3,41 (D large, J = 14, 1H, 1H du $\rangle N-CH_2-$); 3,70 (Mt, 2H, $-CSNH-C\underline{H_2}-$); 3,77 (DD, J = 14 et 7,5, 1H, 1H du $\rangle N-CH_2-$); 4,88 (Mt, 1H, $\rangle N-CH\langle$); 7 à 7,35 (Mt, 5H, aromatiques); 7,41 (D, J = 8, 1H, -H en 3); 7,56 (S, 1H, -H en 1); 10,4 (Mf, 1H, $-NH^+$); 10,5 (T, J = 5, 1H, -CSNH-).

Spectre infra-rouge (KBr), bandes caractéristiques en $cm^{-1}$ :

3200, 2960, 2930, 2870, 2580, 2480, 1590, 1535, 1465, 870, 825, 750.

EXEMPLE 23

Une solution de 1,91 g de [N-méthyl N-(méthyl-1 éthyl)-amino-1 propyl-2-(2RS)]-10 phénothiazinecarbothioamide-2 et de 3 cm3 de butylamine dans 20 cm3 d'éthanol absolu est additionnée d'acide sulfhydrique pendant 10 minutes puis chauffée à une température voisine de 125°C pendant 24 heures. Après refroidissement, on concentre à sec sous pression réduite (30 mm de Hg; 4 kPa) à 50°C. La pâte jaune résiduelle est reprise par 50 cm3 d'éther éthylique et 25 cm3 d'eau distillée. La phase organique est séparée, lavée par 25 cm3 d'eau distillée, séchée sur du carbonate de potassium, filtrée et concentrée à sec sous pression réduite (30 mm de Hg; 4 kPa) à 40°C. L'huile jaune résiduelle (1,9 g) est purifiée par chromatographie sur une colonne (hauteur : 40 cm; diamètre : 3,2 cm) de gel de silice (0,04-0,063 mm) avec une légère surpression d'azote (40 kPa) en éluant par 750 cm3 d'acétate d'éthyle et en recueillant des fractions de 25 cm3. Les fractions 11 à 20 réunies sont concentrées à sec sous pression réduite (30 mm de Hg; 4 kPa) à 50°C. L'huile jaune visqueuse résiduelle (1,6 g) est dissoute dans 50 cm3 d'oxyde d'isopropyle et additionnée, sous agitation, de 2 cm3 d'une solution d'acide chlorhydrique 3N dans l'oxyde d'isopropyle. Le précipité floconneux jaune formé est essoré,

lavé par 3 fois 15 cm3 d'oxyde d'isopropyle et séché sous pression réduite (5 mm de Hg; 0,68 kPa) à 20°C en présence d'acide sulfurique. On obtient ainsi 1,2 g de chlorhydrate de N'-butyl [N-méthyl N-(méthyl-1 éthyl)-amino-1 propyl-2-(2RS))]-10 phénothiazinecarbothioamide-2 sous forme d'une poudre jaune fondant à 130-140°C (fusion pâteuse).

RMN du proton (250 MHz, $CDCl_3$, δ en ppm, J en Hz).

En solution dans le $CDCl_3$, on observe deux formes dues à la salification de l'azote.

0,98 (T, J = 7,5, 3H, $-(CH_2)_3CH_3$); 1,02, 1,33 et 1,39 (3D, J = 7, 6H, $-CH(CH_3)_2$); 1,47 (Mt, 2H, $-(CH_2)_2-CH_2-CH_3$); 1,84 (D, J = 7, 3H, $-CH_3$); 1,85 (Mt, 2H, $-CH_2-CH_2-CH_2-CH_3$); 2,65 et 2,85 (2D, J = 5, 3H, $>N-CH_3$); 3,08 et 3,16 (2D large, 1H, 1H du $>N-CH_2-$); 3,51 et 4,08 (2Mt, 1H, $-CH(CH_3)_2$); 3,55 à 4 (Mt, 4H, $>NCH_2-$ et $-CSNH-CH_2-$); 5,65 et 5,90 (2Mt, 1H, N-CH ); 6,90 à 7,55 (Mt, 7H, aromatiques); 9,08 et 9,15 (2T, 1H, -CSNH-); 11,7 (Mf, 1H, $-NH^+$).

Spectre infra-rouge (KBr), bandes caractéristiques en $cm^{-1}$ : 3210, 2960, 2930, 2865, 2660, 2500, 1590, 1530, 1460, 1415, 820, 750.

EXEMPLE 24

A une solution de 3,7 g de [(pyrrolidinyl-1)-1 propyl-2]-10 phénothiazinecarbothioamide-2, série L, dans 55 cm3 d'éthanol absolu, on ajoute 9,6 cm3 de butylamine et on sature cette solution avec de l'acide sulfhydrique. Le mélange est ensuite porté à une température voisine de 105°C pendant 16 heures. Après refroidissement, la solution est concentrée à sec sous pression réduite (30 mm de Hg; 4 kPa) à 40°C. On obtient une huile orangée qui est purifiée par chromatographie sous une légère surpression d'azote (40 kPa) sur une colonne (hauteur : 25 cm; diamètre : 4 cm) de gel de silice (0,2-0,063 mm) en éluant par un litre d'un mélange d'acétate d'éthyle et de cyclohexane (70-30 en volumes) et en recueillant des fractions de 60 cm3. Les fractions 7 à 12 sont réunies et concentrées à sec sous pression réduite (30 mm de Hg; 4 kPa) à 40°C. On obtient ainsi 3,4 g d'une huile orangée. Ce produit est dissous dans 150 cm3 d'oxyde d'isopropyle et on ajoute 2,4 cm3 d'une solution d'acide chlorhydrique 3,3N dans l'oxyde d'isopropyle. Le précipité formé est essoré, lavé par 3 fois 10 cm3 d'oxyde d'isopropyle et séché à 40°C sous pression réduite (5 mm -de Hg; 0,7 kPa). On obtient 3,16 g de chlorhydrate de N-butyl [(pyrrolidinyl-1)-1 propyl-2]-10 phénothiazinecarbothioamide-2, série L, sous forme d'un solide jaune fondant à 125-130°C (fusion pâteuse).

$[\alpha]_D^{20}$ = +27,5 ±0,6° (1%; diméthylformamide).

RMN du proton (250 MHz, $CDCl_3$, δ en ppm, J en Hz).

1 (T, J = 7,5, 3H, $-CH_3$ du butyle); 1,49 (Mt, 2H, $-CH_2CH_3$); 1,85 (D, J = 7, 3H, $-CH_3$); 1,86 (Mt, 2H, $-CH_2-CH_2CH_3$); 1,9 à 2,25 (Mt, 4H, $-CH_2-CH_2-$ de la pyrrolidine); 2,82 - 2,98 - 3,85 et 4,08 (4Mf, de 1H chacun, - $CH_2-N-CH_2-$ de la pyrrolidine); 3,45 (D large, J = 13, 1H, 1H du $>N-CH_2-$); 3,65 à 3,95 (Mt, 3H, autre H du $>N-CH_2-$ et $-CSNH-CH_2-$); 5,6 (Mt, 1H, CH-N ); 6,9 à 7,25 (Mt, 5H, aromatiques); 7,33 (D, J = 1, 1H, -H en 1); 7,5 (DD, J = 8 et 1, 1H, -H en 3); 8,93 (Mf, 1H, -CSNH-); 12,25 (Mf, 1H, $-NH^+$).

EXEMPLE 25

Une solution de 3,7 g de [(pyrrolidinyl-1)-1 propyl-2]-10 phénothiazinecarbothioamide-2, série D, et de 9,6 cm3 de butylamine dans 55 cm3 d'éthanol absolu est saturée avec de l'acide sulfhydrique et chauffée pendant 16 heures à une température voisine de 105°C. Après refroidissement, on concentre à sec sous pression réduite (30 mm de Hg; 4 kPa) à 40°C. L'huile orangée résiduelle est purifiée par chromatographie sur une colonne (hauteur : 35 cm; diamètre : 3 cm) de gel de silice (0,2-0,063 mm) en éluant avec 1 litre d'un mélange de chlorure de méthylène et de méthanol (97,5-2,5 en volumes) et en recueillant des fractions de 40 cm3. Les fractions 16 à 19 réunies sont concentrées à sec sous pression réduite (30 mm de Hg; 4 kPa) à 40°C. L'huile orangée résiduelle (2,43 g) est dissoute dans 100 cm3 d'oxyde d'isopropyle et additionnée, sous agitation, de 6,7 cm3 d'une solution 0,84N d'acide chlorhydrique dans l'oxyde d'isopropyle. Après 30 minutes d'agitation à une température voisine de 5°C, le précipité formé est essoré, lavé par 3 fois 10 cm3 d'oxyde d'isopropyle et séché sous pression réduite (5 mm de Hg; 0,68 kPa) à 40°C. On obtient ainsi 2,1 g de chlorhydrate de N-butyl [(pyrrolidinyl-1)-1 propyl-2]-10 phénothiazinecarbothioamide-2, série D, sous forme d'un solide jaune fondant à 105-110°C (fusion pâteuse) dont les caractéristiques RMN sont identiques à celles du produit décrit à l'exemple 24.

$[\alpha]_D^{20}$ = -25,6 ±0,8° (0,6%; diméthylformamide).

Spectre infra-rouge (KBr), bandes caractéristiques en $cm^{-1}$ : 3200, 2955, 2925, 2870, 2580, 2470, 1590, 1530, 1460, 1415, 870, 820, 750.

EXEMPLE 26

A une solution de 2,16 g de [diméthylamino-1 propyl-2-(2RS)]-10 phénothiazinecarbothioamide-2 dans 4 cm3 d'éthanol absolu, on ajoute 3,8 cm3 d' isobutylamine. Le mélange est porté à 200°C pendant 5 heures puis dilué par 75 cm3 d'acétate d'éthyle, lavé par 3 fois 75 cm3 d'eau distillée, séché sur sulfate de magnésium puis concentré à sec sous pression réduite (30 mm de Hg; 4 kPa) à 40°C. Le solide résiduel est purifié par chromatographie sur une colonne (hauteur : 25 cm; diamètre : 4 cm) de gel de silice (0,6-0,02 mm) en éluant avec un mélange d'acétate d'éthyle et de cyclohexane 50-50 en volumes (1 litre) en recueillant des fractions de 30 cm3. Les fractions 3 à 13 sont réunies et concentrées sous pression réduite (30 mm de Hg; 4 kPa) à 50°C pour donner 1,79 g d'une huile marron dont une partie aliquote (1,5 g) est mise en solution dans 7 cm3 d'acétonitrile. A cette solution est ajoutée 0,52 g d'acide fumarique en solution dans 5 cm3 d'éthanol. La solution obtenue est concentrée sous pression réduite (30 mm de Hg; 4 kPa) jusqu'à un volume résiduel de 2 cm3 puis additionnée de 8 cm3 d'acétonitrile. Après grattage, une cristallisation se développe et la suspension obtenue est filtrée pour donner 1,7 g de fumarate acide de N-(méthyl-2 propyl) [diméthylamino-1 propyl-2-(2RS)]-10 phénothiazinecarbothioamide-2 fondant à 200°C.

Spectre de RMN (200 MHz, DMSO, $\delta$ en ppm, J en Hz).

0,95 (D, J = 7, 6H, $-CH_3$ du méthyl-2 propyle); 1,65 (D, J = 7, 3H, $-CH_3$); 2,18 (Mt, 1H, -CH< du méthyl-2 propyle); 2,35 (S, 6H, $-N(CH_3)_2$); 2,86 (DD, J = 13 et 6, 1H, 1H du >N-$CH_2$-); 3,1 (DD, J = 13 et 6,5, 1H, 1H du >N-$CH_2$-); 3,57 (Mt, 2H, -CSNH-$C\underline{H}_2$-); 4,35 (Mt, J = 7 - 6,5 et 6, 1H, N-CH ); 6,63 (S, 2H, -CH=CH- du fumarate); 6,95 à 7,3 (Mt, 5H, aromatiques); 7,35 (D large, J = 8, 1H, -H en 3); 7,57 (S large, 1H, -H en 1).

Spectre infra-rouge (KBr), bandes caractéristiques en $cm^{-1}$ : 3250, 2960, 2930, 2880, 2460, 1690, 1590, 1570, 1560, 1525, 1465, 980, 885, 815, 760, 645.

EXEMPLE 27

Une solution de 2,3 g de [diéthylamino-1 propyl-2-(2RS)]-10 phénothiazinecarbothioamide-2 et de 9,4 cm3 de méthyl-2 propylamine dans 30 cm3 d'éthanol absolu est saturée avec de l'acide sulfhydrique et chauffée pendant 16 heures à une température voisine de 100°C. La solution jaune obtenue est concentrée à sec sous pression réduite (30 mm de Hg; 4 kPa) à 40°C. L'huile orangée résiduelle est purifiée par chromatographie sur une colonne (hauteur : 25 cm; diamètre : 4 cm) de gel de silice (0,04-0,063 mm) avec une légère surpression d'azote (40 kPa) en éluant par 750 cm3 d'un mélange d'acétate d'éthyle et de cyclohexane (75-25 en volumes) et en recueillant des fractions de 60 cm3. Les fractions 5 à 10 réunies sont concentrées à sec sous pression réduite (30 mm de Hg; 4 kPa) à 40°C. L'huile orangée résiduelle (2,4 g) est dissoute dans 100 cm3 d'oxyde d'isopropyle et additionnée sous agitation goutte à goutte à une température voisine de 5°C, d'une solution de 0,29 cm3 d'acide méthanesulfonique dans un mélange de 30 cm3 d'oxyde d'isopropyle et d'acétate d'éthyle (66-33 en volumes). On maintient l'agitation pendant 30 minutes à une température voisine de 5°C. Le solide formé est essoré, lavé avec 5 fois 10 cm3 d'oxyde d'isopropyle et séché sous pression réduite (5 mm de Hg; 0,68 kPa) à 40°C. On obtient ainsi 2,1 g de méthanesulfonate de [diéthylamino-1 propyl-2-(2RS)]-10 N-(méthyl-2 propyl) phénothiazinecarbothioamide-2 sous forme d'un solide jaune fondant à 143°C.

RMN du proton (400 MHz, DMSO, $\delta$ en ppm, J en Hz).

En solution dans le DMSO, on observe deux formes dues à la salification de l'azote.

0,92 et 1,17 (2T, J = 7, 3H chacun, $-N(CH_2C\underline{H}_3)_2$); 0,95 (D, J = 7, 6H, $-CH(C\underline{H}_3)_2$); 1,83 (D, J = 7, 3H, $-CH_3$); 2,17 (Mt, 1H, $-C\underline{H}(CH_3)_2$); 2,33 (S, 3H, $-CH_3$ du méthanesulfonate); 3,17 (Mt, 4H, $-N(C\underline{H}_2CH_3)_2$); 3,41 (Mt, 1H, 1H du >$NCH_2$-); 3,55 (Mt, 2H, -CSNH-$C\underline{H}_2$-); 3,77 (Mt, 1H, 1H du >N-$CH_2$-); 4,71 (Mt, 1H, N-CH ); 7,05 à 7,35 (Mt, 5H, aromatiques); 7,40 (D large, J = 8, 1H, -H en 3); 7,55 (S large, 1H, -H en 1); 9,22 (Mf, 1H, $-NH^+$); 10,37 (T, J = 5,5, 1H, -CSNH-).

Spectre infra-rouge (KBr), bandes caractéristiques en $cm^{-1}$ : 3240, 2960, 2930, 2870, 2660, 1590, 1535, 1465, 1210, 1040, 885, 820, 755.

EXEMPLE 28

Un mélange de 3 g de (diéthylamino-1 propyl-2)-10 phénothiazinecarbothioamide-2, série L, et de 3 cm3 de méthyl-2 propylamine dans 60 cm3 d'éthanol est saturé d'acide sulfhydrique et porté à une température voisine de 100°C pendant 16 heures. Après refroidissement, le mélange réactionnel est concentré à sec sous pression réduite (30 mm de Hg; 4 kPa) à 40°C pour donner 3,5 g d'une huile orangée. Ce produit est purifié par chromatographie sur colonne (hauteur : 25 cm; diamètre : 3 cm) de gel de silice (0,063-0,2 mm) en éluant avec un litre d'un mélange de chlorure de méthylène et de méthanol (97,5-2,5 en volumes) et en recueillant des fractions de 50 cm3. Les fractions 12 à 18 sont réunies et concentrées à sec sous pression réduite (30

mm de Hg; 4 kPa) à 40°C. On obtient ainsi 2,42 g d'une huile orangée que l'on dissout dans 260 cm3 d'un mélange d'oxyde d'isopropyle et d'acétate d'éthyle (89-11 en volumes) puis additionne, goutte à goutte, sous agitation et à une température voisins de 5°C, 28 cm3 d'une solution 0,22 N d'acide chlorhydrique dans l'oxyde d'isopropyle. Le précipité formé est essoré, lavé par 3 fois 28 cm3 d'oxyde d'isopropyle et séché sous pression réduite (5 mm de Hg; 0,68 kPa) à 40°C. On obtient ainsi 1,9 g de chlorhydrate de (diéthylamino-1 propyl-2)-10 N-(méthyl-2 propyl) phénothiazinecarbothioamide-2, série L, sous forme d'un solide jaune fondant à 110-115°C (fusion pâteuse) et dont les caractéristiques RMN sont identiques à celles du produit obtenu à l'exemple 27.

$[\alpha]_D^{20}$ = +29,8 ±1,2° (0,4%; diméthylformamide).

Spectre infra-rouge (KBr), bandes caractéristiques en $cm^{-1}$ : 3210, 2960, 2930, 2870, 2640, 2480, 1590, 1570, 1530, 1460, 865, 820, 755.

EXEMPLE 29

Une suspension de 2 g de [(pyrrolidinyl-1)-1 propyl-2-(2RS)]-10 phénothiazinecarbothioamide-2 et de 3 cm3 de méthyl-2 propylamine dans 20 cm3 d'éthanol absolu est saturée pendant 15 minutes avec de l'acide sulfhydrique puis chauffée pendant 23 heures à une température voisine de 115°C. Après refroidissement, on concentre à sec sous pression réduite (30 mm de Hg; 4 kPa) à 30°C. La pâte résiduelle est reprise par 30 cm3 d'acétate d'éthyle et 20 cm3 d'eau distillée. La phase organique est séparée, lavée par 20 cm3 d'une solution aqueuse saturée de chlorure de sodium, séchée sur carbonate de potassium, filtrée et concentrée à sec sous pression réduite (30 mm de Hg; 4 kPa) à 50°C. L'huile visqueuse jaune résiduelle (2,5 g) est purifiée par chromatographie sur une colonne (hauteur : 35 cm; diamètre : 2,6 cm) de gel de silice (0,2-0,063 mm) en éluant par 200 cm3 d'un mélange d'acétate d'éthyle et de méthanol (92-8 en volumes) et en recueillant les fractions de 15 cm3. Les fractions 5 à 11 sont réunies et concentrées à sec sous pression réduite (30 mm de Hg; 4 kPa) à 40°C. On obtient ainsi 2,04 g de N-(méthyl-2 propyl) [(pyrrolidinyl-1)-1 propyl-2-(2RS)]-10 phénothiazinecarbothioamide-2 sous forme d'une huile visqueuse jaune. On dissout 0,51 g de cette huile dans 20 cm3 d'oxyde d'isopropyle et on ajoute 0,5 cm3 d'une solution 3,3N d'acide chlorhydrique dans l'oxyde d'isopropyle. Le précipité formé est essoré, lavé par 3 fois 5 cm3 d'oxyde d'isopropyle et séché sous pression réduite (5 mm de Hg; 0,68 kPa) à 40°C. On obtient ainsi 0,5 g de chlorhydrate de N-(méthyl-2 propyl) [(pyrrolidinyl-1)-1 propyl-2-(2RS)]-10 phénothiazinecarbothioamide-2 sous forme d'une poudre jaune fondant à 150-155°C (fusion pâteuse).

RMN du proton (250 MHz, DMSO, δ en ppm, J en Hz).

0,95 (D, J = 7, 6H, $-CH(C\underline{H}_3)_2$); 1,77 (D, J = 7, 3H, $-CH_3$); 1,75 à 2 (Mt, 4H, $-CH_2-$ de la pyrrolidine); 2,20 (Mt, 1H, $-C\underline{H}(CH_3)_2$); 2,83 - 3,12 et de 3,50 à 3,95 (2Mf de 1H chacun et Mt, $N-CH_2-$ de la pyrrolidine); 3,56 (Mt, 2H, $-CSNH-C\underline{H}_2-$); 3,5 à 3,95 (Mt, 2H, $>N-CH_2-$); 4,77 (Mt, 1H, $>N-CH<$); 7 à 7,35 (Mt, 5H, aromatiques); 7,42 (DD, J = 8 et 1, 1H, -H en 3); 7,55 (D, J = 1, 1H, -H en 1); 10,5 (T, J = 5 et Mf, 2H, -CSNH- et $-NH^+$).

Spectre infra-rouge (KBr), bandes caractéristiques en $cm^{-1}$ : 3190, 2950, 2920, 2860, 2580, 2470, 1590, 1530, 1460, 1410, 820, 750.

EXEMPLE 30

Une suspension de 2 g de [(pyrrolidinyl-1)-1 propyl-2-(2RS)]-10 phénothiazinecarbothioamide-2 et de 3 cm3 de pentylamine dans 30 cm3 d'éthanol absolu est saturée avec de l'acide sulfhydrique puis chauffée pendant 2 heures à une température voisine de 100°C. Après refroidissement, on concentre à sec sous pression réduite (30 mm de Hg; 4 kPa) à 40°C. Le résidu pâteux est repris par 50 cm3 d'éther éthylique et 15 cm3 d'eau distillée. La phase organique est séparée, séchée sur carbonate de potassium et concentrée à sec sous pression réduite (30 mm de Hg; 4 kPa) à 40°C. L'huile jaune visqueuse résiduelle est purifiée par chromatographie sur une colonne (hauteur : 35 cm; diamètre : 2,6 cm) de gel de silice (0,2-0,063 mm) en éluant par 300 cm3 d'acétate d'éthyle et en recueillant des fractions de 25 cm3. Les fractions 3 à 9 sont réunies et concentrées à sec sous pression réduite (30 mm de Hg; 4 kPa) à 40°C. On obtient ainsi 2,25 g de N-pentyl [(pyrrolidinyl-1)-1 propyl-2-(2RS)]-10 phénothiazinecarbothioamide-2 sous forme d'une huile jaune. On dissout 0,55 g de ce produit dans 20 cm3 d'oxyde d'isopropyle et on verse, goutte à goutte sous agitation, 0,6 cm3 d'une solution 3,3N d'acide chlorhydrique dans l'oxyde d'isopropyle. Le précipité formé est essoré, lavé par 3 fois 5 cm3 d'oxyde d'isopropyle et séché sous pression réduite (5 mm de Hg; 0,68 kPa) à 40°C. On obtient ainsi 0,56 g de chlorhydrate de N-pentyl [(pyrrolidinyl-1)-1 propyl-2-(2RS)]-10 phénothiazinecarbothioamide-2 sous forme d'une poudre jaune pâle fondant à 155-160°C (fusion pâteuse).

RMN du proton (250 MHz, DMSO, δ en ppm, J en Hz).

0,92 (T, J = 7, 3H, $-CH_3$ du pentyle); 1,35 (Mt, 4H, $-C\underline{H}_2-C\underline{H}_2-CH_3$); 1,7 (Mt, 2H, $-C\underline{H}_2-(CH_2)_2CH_3$); 3,55 à 3,90 (Mt, 4H, >$N-CH_2$- et -CSNH-$C\underline{H}_2$-); 7 à 7,35 (Mt, 5H, aromatiques); 7,43 (DD, J = 8 et 1, 1H, -H en 3); 7,57 (D, J = 1, 1H, -H en 1).

Spectre infra-rouge (KBr), bandes caractéristiques en $cm^{-1}$ : 3200, 2960, 2930, 2860, 2580, 2470, 1590, 1530, 1460, 1410, 870, 820, 750.

EXEMPLE 31

Un mélange de 1 g de [diméthylamino-1 propyl-2-(2RS)]-10 phénothiazinecarbothioamide-2 et de 5,2 cm3 de méthyl-3 butylamine dans 15 cm3 d'éthanol absolu est saturé avec de l'acide sulfhydrique et chauffé pendant 16 heures à une température voisine de 100°C. Après refroidissement, on concentre à sec sous pression réduite (30 mm de Hg; 4 kPa) à 40°C. Le résidu huileux orangé est purifié par chromatographie sur une colonne (hauteur : 25 cm; diamètre : 4 cm) de gel de silice (0,04-0,063 mm) avec une légère surpression d'azote (40 kPa) en éluant avec un litre d'acétate d'éthyle et en recueillant des fractions de 60 cm3. Les fractions 6 à 11 sont réunies et concentrées à sec sous pression réduite (30 mm de Hg; 4 kPa) à 40°C. L'huile jaune résiduelle (1,2 g) est dissoute dans 100 cm3 d'oxyde d'isopropyle et additionnée, goutte à goutte, sous agitation, à une température voisine de 5°C, de 10,85 cm3 d'une solution 0,267N d'acide chlorhydrique dans l'oxyde d'isopropyle. Après 1 heure, le solide formé est essoré, lavé avec 5 fois 10 cm3 d'oxyde d'isopropyle et séché sous pression réduite (5 mm de Hg; 0,68 kPa) à 40°C. On obtient ainsi 0,9 g de chlorhydrate de [diméthylamino-1 propyl-2-(2RS)]-10 N-(méthyl-3 butyl) phénothiazinecarbothioamide-2 sous forme d'un solide jaune fondant à 112°C (fusion pâteuse).

RMN du proton (250 MHz, DMSO, δ en ppm, J en Hz).

0,96 (D, J = 7, 6H, $-CH(C\underline{H}_3)_2$); 1,62 (Mt, 2H, $-C\underline{H}_2-CH$ ); 1,7 (Mt, 1H, $-C\underline{H}(CH_3)_2$); 1,8 (D, J = 7, 3H, $-CH_3$); 2,82 (S, 6H, $-N(CH_3)_2$); 3,53 (DD, J = 14 et 4,5, 1H du >$NCH_2$-); 3,74 (Mt, 3H, -CSNHC$\underline{H}_2$- et 1H du $NCH_2$-); 4,76 (Mt, 1H, >N-CH<); 7 à 7,4 (Mt, 5H, aromatiques); 7,45 (DD, J = 8 et 1, 1H, -H en 3); 7,55 (D, J = 1, 1H, -H en 1); 10,4 (Mf, 1H, $-NH^+$); 10,5 (T, J = 5,5, 1H, -CSNH-).

Spectre infra-rouge (KBr), bandes caractéristiques en $cm^{-1}$ : 3250, 2960, 2930, 2870, 2680, 2500, 2470, 1590, 1530, 1460, 870, 820, 750.

EXEMPLE 32

Une solution de 1 g de [N-éthyl N-méthyl-amino-1 propyl-2-(2RS)]-10 phénothiazinecarbothioamide-2 et de 4,9 cm3 de méthyl-3 butylamine dans 15 cm3 d'éthanol absolu est saturée avec de l'acide sulfhydrique et chauffée pendant 16 heures à une température voisine de 100°C. Après refroidissement, la solution orangée obtenue est concentrée à sec sous pression réduite (30 mm de Hg; 4 kPa) à 40°C. L'huile orangée résiduelle est purifiée par chromatographie sur une colonne (hauteur : 25 cm, diamètre : 2 cm) de gel de silice (0,04-0,063 mm) sous une légère surpression d'azote (40 kPa) en éluant par 500 cm3 d'un mélange d'acétate d'éthyle et de cyclohexane (80-20 en volumes) et en recueillant des fractions de 30 cm3. Les fractions 4 à 8 sont réunies et concentrées à sec sous pression réduite (30 mm de Hg; 4 kPa) à 40°C. On obtient ainsi 1,3 g de N-(méthyl-3 butyl) [N'-éthyl N'-méthyl-amino-1 propyl-2-(2RS)]-10 phénothiazinecarbothioamide-2 sous forme d'une huile jaune orangée. On dissout 1 g de ce produit dans 5 cm3 d'acétate d'éthyle, ajoute 40 cm3 d'oxyde d'isopropyle et verse, sous agitation et à une température voisine de 5°C, 5,68 cm3 d'une solution 0,41N d'acide chlorhydrique dans l'oxyde d'isopropyle. Après 30 minutes d'agitation à une température voisine de 5°C, le précipité formé est essoré, lavé par 3 fois 5 cm3 d'oxyde d'isopropyle et séché sous pression réduite (5 mm de Hg; 0,68 kPa) à 40°C. On obtient ainsi 0,9 g de chlorhydrate de N-(méthyl-3 butyl) [(N'-éthyl N'-méthyl-amino)-1 propyl-2-(2RS)]-10 phénothiazinecarbothioamide-2 sous forme d'un solide jaune fondant à 105-110°C (fusion pâteuse).

RMN du proton (250 MHz, DMSO, δ en ppm, J en Hz).

En solution dans le DMSO, on observe deux formes dues à la salification de l'azote.

0,93 (D, J = 7, 6H, $-CH(C\underline{H}_3)_2$); 1,05 et 1,18 (2T, J = 7, 3H, >$NCH_2C\underline{H}_3$); 1,6 (Mt, 2H, $-C\underline{H}_2$-CH<); 1,66 (Mt, 1H, >CH- du méthyl-3 butyle); 1,8 (Mt, 3H,$-CH_3$); 2,77 (Mf, 3H, >$N-CH_3$); 3 à 3,35 (Mt, 2H,>$N-C\underline{H}_2CH_3$); 3,50 et 3,65 à 3,90 (2Mt, 2H, >$N-CH_2$-); 3,73 (Mt, 2H, -CSNHC$\underline{H}_2$-); 4,84 (Mt, 1H, N-CH ); 7 à 7,35 (Mt, 5H, aromatiques); 7,41 (D large, J = 8, 1H, -H en 3); 7,54 (S large, 1H,-H en 1); 10,45 et 10,55 (Mf et Mt, 2H, $-NH^+$ et -CSNH-).

Spectre infra-rouge (KBr), bandes caractéristiques en $cm^{-1}$ : 3180, 2960, 2930, 2870, 2590, 2480, 1590, 1535, 1465, 870, 820, 750.

EXEMPLE 33

A une solution de 1,86 g de [diéthylamino-1 propyl-2-(2RS)]-10 phénothiazinecarbothioamide-2 dans 25 cm3 d'éthanol absolu, on ajoute 2,9 cm3 de méthyl-3 butylamine. Le mélange est porté à 150°C pendant 16 heures. Le mélange réactionnel est dilué par 100 cm3 d'acétate d'éthyle puis lavé par 3 fois 50 cm3 d'eau distillée. La phase organique est séchée sur sulfate de magnésium, filtrée puis concentrée à sec sous pression réduite (30 mm de Hg; 4 kPa) à 50°C. Le résidu est purifié par chromatographie sur une colonne (hauteur : 18,5 cm; diamètre : 2,6 cm) de gel de silice (0,04-0,06 mm) avec une légère surpression d'azote (40 kPa) en éluant avec un mélange de cyclohexane et d'acétate d'éthyle 50-50 en volumes (1 litre) puis par de l'acétate d'éthyle pur (500 cm3) en recueillant des fractions de 60 cm3. Les fractions 2 à 4 sont réunies et concentrées à sec sous pression réduite (30 mm de Hg; 4 kPa) à 50°C pour donner 1,10 g d'une meringue orangée. Cette dernière est reprise dans 50 cm3 d'oxyde d'isopropyle pour donner une suspension qui est filtrée, lavée par 3 fois 1 cm3 d'éther éthylique et séchée à 50°C sous pression réduite (5 mm de Hg; 0,7 kPa) pour donner 0,95 g de [diéthylamino-1 propyl-2-(2RS))]-10 N-(méthyl-3 butyl) phénothiazinecarbothioamide-2 fondant à 80°C.

Spectre de RMN (250 MHz, DMSO, $\delta$ en ppm, J en Hz).

On observe deux formes qui disparaissent lors de l'addition de $CD_3COOD$.

0,93 (D, J = 6,5, 6H, $-CH(C\underline{H_3})_2$); 1,02 (T, J = 7, 6H, $-C\underline{H}(CH_3)_2$); 1,5 à 1,75 (Mt, 3H, $-C\underline{H_2}-CH(CH_3)_2$); 1,77 (D, J = 7, 3H, $-CH_3$); 3,03 (Mt, 4H, $-N(C\underline{H_2}CH_3)_2$); 3,3 (Mt, 1H, 1H du >N-$CH_2$-); 3,45 à 3,8 (Mt, autre H du >N-$CH_2$ - et -CSNH-$CH_2$-); 4,45 à 4,75 (Mt, 1H, >NCH<); 7 à 7,6 (Mt, 7H, aromatiques); 10,34 (T, J = 5, 1H, -CSNH-).

Spectre infra-rouge (KBr), bandes caractéristiques en $cm^{-1}$ : 3280, 2960, 2660, 1725, 1630, 1590, 1535, 1460, 865, 825.

EXEMPLE 34

A une solution de 3 g de fumarate acide de (diéthylamino-1 propyl-2)-10 phénothiazinecarbothioamide-2, série L, dans 45 cm3 d'éthanol absolu, on ajoute 10,7 cm3 de méthyl-3 butylamine et on sature cette solution avec de l'acide sulfhydrique. Le mélange est ensuite porté à une température voisine de 105°C pendant 16 heures et concentré à sec sous pression réduite (30 mm de Hg; 4 kPa). L'huile orangée résiduelle est purifiée par chromatographie sur une colonne (hauteur : 35 cm; diamètre : 3 cm) de gel de silice (0,2-0,063 mm) en éluant avec 400 m3 d'un mélange d'acétate d'éthyle et de cyclohexane (70-30 en volumes) en recueillant des fractions de 30 cm3. Les fractions 7 à 11 sont réunies et concentrées à sec sous pression réduite (30 mm de Hg; 4 kPa) à 50°C pour donner 3,1 g d'huile orangée. 1 g de ce produit est dissous dans dans 100 cm3 d'oxyde d'isopropyle et on ajoute à 5°C sous agitation 10,7 cm3 d'une solution 0,158M d'acide chlorhydrique dans l'oxyde d'isopropyle. Après 30 minutes, le précipité formé est essoré, lavé par 3 fois 10 cm3 d'oxyde d'isopropyle et séché à 40°C sous pression réduite (5 mm de Hg; 0,68 kPa) pour donner 0,85 g de chlorhydrate de (diéthylamino-1 propyl-2)-10 N-(méthyl-3 butyl-1) phénothiazinecarbothioamide-2, série L, solide fondant à 110-116°C (fusion pâteuse) dont les caractéristiques RMN sont identiques à celles de l'exemple 33.

$[\alpha]_D^{20}$ = +24,2° (0,7%; diméthylformamide).

Spectre infra-rouge (KBr), bandes caractéristiques en $cm^{-1}$ : 3180, 2950, 2920, 2860, 2580, 2480, 1590, 1530, 1460, 1415, 820, 750.

Le fumarate acide de (diéthylamino-1 propyl-2)-10 phénothiazinecarbothioamide-2, série L, peut être préparé de la manière suivante :

Une solution de 5,2 g de (diéthylamino-1 propyl-2)-10 phénothiazinecarbonitrile-2, série L, et de 2,2 cm3 de triéthylamine dans 104 cm3 de pyridine anhydre est saturée d'acide sulfhydrique à 20°C pendant 1 heure sous agitation puis agitée à 20°C pendant 17 heures. Le mélange réactionnel est purgé à l'azote pendant 1 heure, versé dans 500 cm3 d'eau distillée et extrait par 2 fois 250 cm3 d'acétate d'éthyle. Les phases organiques réunies sont lavées successivement par 3 fois 100 cm3 d'eau distillée et par 100 cm3 d'une solution aqueuse saturée de chlorure de sodium, séchées sur sulfate de magnésium et filtrées. Le filtrat jaune est concentré à sec sous pression réduite (30 mm de Hg; 4 kPa) à 40°C pour donner 6,5 g d'une huile orangée. Ce produit est purifié par chromatographie sur une colonne (hauteur : 44 cm; diamètre : 3,4 cm) de gel de silice (0,2-0,063 mm) en éluant par 3 litres d'un mélange de cyclohexane et d'acétate d'éthyle (30-70 en volumes) et en recueillant des fractions de 150 cm3. Les fractions 8 à 17 sont réunies et concentrées à sec sous pression réduite (30 mm de Hg; 4 kPa) à 40°C. On obtient 5,08 g d'une huile orangée ($[\alpha]_D^{20}$ = -33,7° ±0,6°; 1,006%; chloroforme). Ce produit est dissous à une température voisine de 60°C dans 20 cm3 d'éthanol et cette solution est versée dans une solution de 1,56 g d'acide fumarique dans 20 cm3 d'éthanol à une température voisine de 60°C puis laissée au repos pendant 16 heures à une température voisine de 5°C. Les cristaux formés sont essorés, lavés avec 2 fois 2 cm3 d'éthanol et séchés à 40°C sous pression réduite (5 mm de Hg; 0,7 kPa). On

obtient ainsi 5,5 g de fumarate acide de (diéthylamino-1 propyl-2)-10 phénothiazinecarbothioamide-2, série L, sous forme de cristaux jaunes fondant à 186°C.

$[\alpha]_D^{20}$ = +29,1 ±0,6° (1%; diméthylformamide).

Le [(diéthylamino-1 propyl-2)-10 phénothiazinecarbonitrile-2], série L, peut être préparé de la manière suivante :

A une solution de 7 g d'(éthylamino-1 propyl-2)-10 phénothiazinecarbonitrile-2, série L, dans 86 cm3 de diméthylformamide, on ajoute 3,2 g de carbonate de sodium et 2,3 cm3 d'iodoéthane puis on porte ce mélange à une température voisine de 150°C pendant 6 heures. Après refroidissement, le mélange réactionnel est concentré à sec sous pression réduite (30 mm de Hg; 4 kPa) à 40°C et le résidu est repris par 250 cm3 d'acétate d'éthyle. La solution obtenue est lavée successivement par 2 fois 100 cm3 d'eau distillée et par 100 cm3 d'une solution aqueuse saturée de chlorure de sodium, séchée sur sulfate de magnésium en présence de noir 3S et filtrée. Le filtrat jaune est concentré à sec sous pression réduite (30 mm de Hg; 4 kPa) à 40°C pour donner 6,65 g d'une huile orangée qui cristallise lentement. Ce résidu est dissous dans le minimum d'oxyde d'isopropyle au reflux, le léger insoluble est filtré à chaud et le filtrat est conservé pendant 3 jours à une température voisine de 5°C. Les cristaux formés sont essorés, lavés avec 2 fois 2 cm3 d'oxyde d'isopropyle et séchés à 40°C sous pression réduite (5 mm de Hg; 0,7 kPa). On obtient ainsi 2,9 g de (diéthylamino-1 propyl-2)-10 phénothiazinecarbonitrile-2, série L, sous forme de cristaux beiges fondant à 83°C ($[\delta]_D^{20}$ = +9 ±0,3°; 0,978%; chloroforme). Le filtrat est alors concentré à sec sous pression réduite (30 mm de Hg; 4 kPa) à 40°C pour donner encore 2,3 g de (diéthylamino-1 propyl-2)-10 phénothiazinecarbonitrile-2, série L, sous forme d'un solide beige fondant à 81-82°C.

$[\alpha]_D^{20}$ = +8,7 ±0,3° (1,2%; chloroforme).

L'(éthylamino-1 propyl-2)-10 phénothiazinecarbonitrile-2, série L, peut être préparé de la manière suivante :

A une solution de 16 g de méthanesulfonate de (cyano-2 phénothiazinyl-10)-2 propyle-1, série L, dans 160 cm3 de toluène, on ajoute 30 cm3 d'éthylamine et on porte ce mélange à une température voisine de 105°C pendant 24 heures. Après refroidissement, le mélange réactionnel est concentré à sec sous pression réduite (30 mm de Hg; 4 kPa) à 40°C et le résidu est repris par 250 cm3 d'éther éthylique et 50 cm3 d'une solution aqueuse de soude N. Après agitation, la phase organique est séparée, lavée successivement par 50 cm3 d'eau distillée et par 50 cm3 d'une solution aqueuse saturée de chlorure de sodium, séchée sur sulfate de magnésium, filtrée et concentrée à sec sous pression réduite (30 mm de Hg; 4 kPa) pour donner 13,8 g d'une huile jaune. Ce résidu est dissous à une température voisine de 60°C dans 46 cm3 d'éthanol et cette solution est versée dans une solution à 60°C de 5,2 g d'acide fumarique dans 46 cm3 d'éthanol puis laissée pendant 16 heures à une température voisine de 5°C. Le précipité jaune formé est essoré, lavé par 2 fois 5 cm3 d'éthanol et séché à 40°C sous pression réduite (5 mm de Hg; 0,7 kPa). On obtient ainsi 9,7 g de fumarate d'(éthylamino-1 propyl-2)-10 phénothiazinecarbonitrile-2, série L, ($[\alpha]_D^{20}$ = +6,2 ±0,4°; 1,008%; diméthylformamide). Ce produit est mis en suspension dans 200 cm3 d'éther éthylique et on ajoute 100 cm3 d'une solution aqueuse de soude N. Après agitation, la phase organique est séparée et la phase aqueuse est extraite avec 50 cm3 d'éther éthylique. Les phases organiques sont réunies, lavées par 100 cm3 d'une solution aqueuse saturée de chlorure de sodium, séchées sur sulfate de magnésium, filtrées et concentrées à sec sous pression réduite (300 mm de Hg; 40 kPa puis 30 mm de Hg; 4 kPa) à 40°C. On obtient ainsi 7 g d'(éthylamino-1 propyl-2)-10 phénothiazinecarbonitrile-2, série L, sous forme d'une huile jaune.

$[\alpha]_D^{20}$ = +12 ±0,3° (2%; chloroforme).

EXEMPLE 35

A une solution de 3 g de fumarate acide de (diéthylamino-1 propyl-2)-10 phénothiazinecarbothioamide-2, série D, dans 45 cm3 d'éthanol absolu, on ajoute 10,7 cm3 de méthyl-3 butylamine et on sature cette solution avec de l'acide sulfhydrique. Le mélange est ensuite porté à une température voisine de 105°C pendant 16 heures et concentré à sec sous pression réduite (30 mm de Hg; 4 kPa) à 50°C. L'huile orangée résiduelle est purifiée par chromatographie sur une colonne (hauteur : 35 cm; diamètre : 3 cm) de gel de silice (0,2-0,063 mm) en éluant avec 400 cm3 d'un mélange d'acétate d'éthyle et de cyclohexane (75-25 en volumes) et en recueillant des fractions de 40 cm3. Les fractions 4 à 6 sont réunies et concentrées à sec sous pression réduite (30 mm de Hg; 4 kPa) à 50°C pour donner 3,1 g d'huile orangée. 1 g de ce produit est dissous dans 100 cm3 d'oxyde d'isopropyle et additionné à 5°C sous agitation de 10,7 cm3 d'une solution 0,158M d'acide chlorhydrique dans l'oxyde d'isopropyle. Après 30 minutes, le précipité formé est essoré, lavé par 3 fois 10 cm3 d'oxyde d'isopropyle et séché à 40°C sous pression réduite (5 mm de Hg; 0,68 kPa) pour donner 0,8 g de chlorhydrate de (diéthylamino-1 propyl-2)-10 N-(méthyl-3 butyl-1) phénothiazinecarbothioamide-2, série D, sous forme

d'un solide jaune fondant à 115-120°C (fusion pâteuse) dont les caractéristiques RMN sont identiques à celles du produit décrit à l'exemple 33.

Spectre infra-rouge (KBr), bandes caractéristiques en $cm^{-1}$ : 3190, 2960, 2870, 2580, 2480, 1590, 1530, 1460, 1415, 865, 825, 750.

Le fumarate acide de (diéthylamino-1 propyl-2)-10 phénothiazinecarbothioamide-2, série D, peut être obtenu de la manière suivante :

En opérant comme décrit à l'exemple 34 mais à partir de 3,4 g de (diéthylamino-1 propyl-2)-10 phénothiazinecarbonitrile-2, série D, et de 1,4 cm3 de triéthylamine dans 68 cm3 de pyridine anhydre, on obtient 4 g d'une huile orangée. Ce produit est dissous à une température voisine de 60°C dans 13 cm3 d'éthanol et cette solution est versée dans une solution de 1,16 g d'acide fumarique dans 13 cm3 d'éthanol à une température voisine de 5°C. Les cristaux formés sont essorés, lavés avec 2 fois 2 cm3 d'éthanol et séchés à 40°C sous pression réduite (5 mm de Hg; 0,7 kPa). On obtient ainsi 3,47 g de fumarate acide de (diéthylamino-1 propyl-2)-10 phénothiazinecarbothioamide-2, série D, sous forme d'un solide jaune fondant à 180°C.

$[\alpha]_D^{20}$ = -32,8 ±0,6° (0,9%; diméthylformamide).

Le (diéthylamino-1 propyl-2)-10 phénothiazinecarbonitrile-2, série D, peut être obtenu de la manière suivante :

On chauffe à une température voisine de 150°C pendant 6 heures un mélange de 5 g de fumarate acide d'(éthylamino-1 propyl-2)-10 phénothiazinecarbonitrile-2, série D, de 1,32 g de carbonate de sodium et de 1 cm3 d'iodoéthane dans 50 cm3 de diméthylformamide. Après refroidissement, on concentre à sec (30 mm de Hg; 4 kPa) à 50°C le mélange réactionnel et le résidu est repris par 100 cm3 d'acétate d'éthyle et lavé par successivement 50 cm3 d'eau distillée et par 50 cm3 d'une solution aqueuse saturée de chlorure de sodium. La phase organique est séchée sur sulfate de magnésium, filtrée et concentrée à sec sous pression réduite (30 mm de Hg; 4 kPa) à 40°C. On obtient ainsi 3,4 g de (diéthylamino-1 propyl-2)-10 phénothiazinecarbonitrile-2, série D, sous forme d'une huile jaune.

$[\alpha]_D^{20}$ = -6,8 ±0,4° (1,3%; chloroforme).

Le fumarate acide d'(éthylamino-1 propyl-2)-10 phénothiazinecarbonitrile-2, série D, peut être obtenu de la manière suivante :

A une solution de 10 g de méthanesulfonate de (cyano-2 phénothiazinyl-10)-2 propyle-1, série D, dans 100 cm3 de toluène, on ajoute 18,75 cm3 d'éthylamine et on porte ce mélange à une température voisine de 105°C pendant 24 heures. Après refroidissement, le mélange réactionnel est concentré à sec sous pression réduite (30 mm de Hg; 4 kPa) à 40°C. Le résidu est repris par 250 cm3 d'éther éthylique et extrait successivement par 2 fois 100 cm3 d'une solution aqueuse d'acide chlorhydrique N. Les phases aqueuses réunies sont alcalinisées par de la lessive de soude (d = 1,33) jusqu'à pH 13 et extraites par 2 fois 200 cm3 d'éther éthylique. Les phases organiques réunies sont lavées successivement par 50 cm3 d'eau distillée et par 50 cm3 d'une solution aqueuse saturée de chlorure de sodium, séchées sur sulfate de magnésium, filtrées et concentrés à sec sous pression réduite (30 mm de Hg; 4 kPa) à 40°C. On obtient ainsi 5 g d'(éthylamino-1 propyl-2)-10 phénothiazinecarbonitrile-2, série D, sous forme d'une huile orangée. Ce produit est dissous à une température voisine de 60°C dans 17 cm3 d'éthanol et cette solution est versée dans une solution de 1,9 g d'acide fumarique dans 17 cm3 d'éthanol et cette solution est versée dans une solution de 1,9 g d'acide fumarique dans 17 cm3 d'éthanol à la même température puis on amorce la cristallisation et laisse pendant 16 heures à une température voisine de 20°C. Le solide est essoré, lavé par 2 fois 2 cm3 d'éthanol et séché sous pression réduite (5 mm de Hg; 0,7 kPa) à 40°C. On obtient ainsi 4 g de fumarate acide d'(éthylamino-1 propyl-2)-10 phénothiazinecarbonitrile-2, série D, sous forme d'un solide jaune fondant à 208°C.

$[\alpha]_D^{20}$ = -5,2 ±0,5° (0,9%; diméthylformamide).

EXEMPLE 36

On sature d'acide sulfhydrique une solution de 1,89 g de [(pyrrolidinyl-1)-1 propyl-2-(2RS)]-10 phénothiazinecarbothioamide-2 et de 8,9 cm3 de méthyl-3 butylamine dans 28 cm3 d'éthanol absolu et on chauffe ce mélange pendant 16 heures à une température voisine de 110°C. Après refroidissement, le mélange réactionnel est concentré à sec sous pression réduite (30 mm de Hg; 4 kPa) à 40°C et le résidu huileux jaune (2,8 g) est purifié par chromatographie sur une colonne (hauteur : 30 cm; diamètre : 2 cm) de gel de silice (0,04-0,063 mm) sous une légère surpression d'azote (40 kPa) en éluant successivement avec 100 cm3 de chlorure de méthylène puis 300 cm3 d'un mélange de chlorure de méthylène et de méthanol (95-5 en volumes) et en recueillant des fractions de 30 cm3. Les fractions 4 à 8 sont réunies et concentrées à sec sous pression réduite (30 mm de Hg; 4 kPa) à 40°C. Le résidu huileux jaune (2,1 g) est dissous dans 15 cm3 d'acétate d'éthyle et on ajoute 2 cm3 d'une solution 3N d'acide chlorhydrique dans l'éther éthylique. On maintient sous agitation

pendant 1 heure à une température voisine de 5°C. Le précipité formé est essoré, lavé par 2 cm3 d'acétate d'éthyle et séché sous pression réduite (5 mm de Hg; 0,7 kPa) à 35°C. On obtient ainsi 2,1 g de chlorhydrate N-(méthyl-3 butyl) [(pyrrolidinyl-1)-1 propyl-2-(2RS)]-10 phénothiazinecarbothioamide-2 sous forme de cristaux jaunes fondant à 190°C et dont le spectre de RMN est identique à celui du carbothioamide décrit ci-après à l'exemple 37.

Spectre infra-rouge (KBr), bandes caractéristiques en $cm^{-1}$ : 3210, 2950, 2920, 2860, 2680, 2610, 2480, 1590, 1535, 1460, 1410, 820, 745.

EXEMPLE 37

A une solution de 4,1 g de [(pyrrolidinyl-1)-1 propyl-2]-10 phénothiazinecarbothiamide-2, série D, dans 61,5 cm3 d'éthanol, on ajoute 12,9 cm3 de méthyl-3 butylamine puis on sature d'acide sulfhydrique et on chauffe pendant 16 heures à une température voisine de 105°C. Après refroidissement, la solution est concentrée à sec sous pression réduite (30 mm de Hg; 4 kPa) à 40°C. On obtient une huile jaune qui est purifiée par chromatographie sur une colonne (hauteur : 30 cm; diamètre : 4 cm) de gel de silice (0,2-0,063 mm), en éluant par 1,5 litre d'un mélange de chlorure de méthylène et de méthanol (95-5 en volumes) et en recueillant des fractions de 80 cm3. Les fractions 14 à 17 sont réunies et concentrées à sec sous pression réduite (30 mm de Hg; 4 kPa) à 40°C. On obtient 4,7 g de N-(méthyl-3 butyl) [(pyrrolidinyl-1)-1 propyl-2]-10 phénothiazinecarbothioamide-2, série D, sous forme d'une huile orangée. ($[\delta]_D^{20}$ = -3,2 ±0.2°; 1,92%; chloroforme). 2,7 g de ce produit sont dissous dans 20 cm3 d'acétate d'éthyle, puis on ajoute 1,85 cm3 d'une solution 3,3N d'acide chlorhydrique dans l'oxyde d'isopropyle. Le précipité formé est essoré, lavé par 3 fois 5 cm3 d'oxyde d'isopropyle et séché sous pression réduite (5 mm de Hg; 0,68 kPa). On obtient ainsi 1,7 g de chlorhydrate de N-(méthyl-3 butyl) [(pyrrolidinyl-1)-1 propyl-2]-10 phénothiazinecarbothioamide-2, série D, sous la forme d'un solide jaune fondant à 189°C.

$[\alpha]_D^{20}$ = -26,3 ±0,5° (1,2%; diméthylformamide).

RMN du proton (250 MHz, DMSO, δ en ppm, J en Hz).

0,94 (D, J = 7, 6H, $-CH(C\underline{H}_3)_2$); 1,6 (Mt, 2H, $-NHCH_2C\underline{H}_2-$); 1,64 (Mt, 1H, >CH-); 1,77 (D, J = 7, 3H, $-CH_3$); 1,7 à 2 (Mt, 4H, $-C\underline{H}_2-C\underline{H}_2-$ de la pyrrolidine); 2,85 - 3,10 et de 3,5 à 3,9 (2Mf de 1H chacun et Mt, $-CH_2-N-CH_2-$ de la pyrrolidine); 3,5 à 3,9 (Mt, 4H, $>N-CH_2-$ et $-CSNH-C\underline{H}_2-$); 4,75 (Mt, 1H, >CH-N<); 7 à 7,4 (Mt, 5H, aromatiques); 7,42 (DD, J = 8 et 1, 1H, -H en 3); 7,55 (D, J = 1, 1H, -H en 1); 10,48 (T, J = 5, 1H, -CSNH-); 10,68 (Mf, 1H, $-NH^+$).

Spectre infra-rouge (KBr), bandes caractéristiques en $cm^{-1}$ : 3200, 2955, 2930, 2870, 2600, 2480, 1590, 1530, 1460, 1415, 870, 820, 750.

Le [(pyrrolidinyl-1)-1 propyl-2]-10 phénothiazinecarbothioamide-2, série D, peut être préparé de la manière suivante :

Un mélange de 11,7 g de [(pyrrolidinyl-1)-1 propyl-2]-10 phénothiazinecarbonitrile-2, série D, et de 4,9 cm3 de triéthylamine dans 234 cm3 de pyridine anhydre est saturé d'acide sulfhydrique pendant 1 heure à 25°C. On agite pendant 20 heures à 25°C puis le mélange réactionnel est dégazé par barbottage d'azote, dilué par 100 cm3 d'acétate d'éthyle, versé sur 1 litre d'eau distillée et extrait par 2 fois 500 cm3 d'acétate d'éthyle. Les phases organiques réunies sont lavées successivement par 2 fois 250 cm3 d'eau distillée et 250 cm3 d'une solution aqueuse saturée de chlorure de sodium, séchées sur sulfate de magnésium, filtrées et le filtrat jaune est concentré à sec sous pression réduite (30 mm de Hg; 4 kPa) à 40°C. On obtient 18 g d'une huile jaune qui est purifiée par chromatographie sur une colonne (hauteur : 45 cm; diamètre : 4 cm) de gel de silice (0,2-0,063 mm) en éluant par 1 litre de chlorure de méthylène puis par 5 litres d'un mélange de chlorure de méthylène et de méthanol (95-5 en volumes) et en recueillant des fractions de 100 cm3. Les fractions 23 à 50 sont réunies et concentrées à sec sous pression réduite (30 mm de Hg; 4 kPa) à 40°C. On obtient ainsi 10,7 g de [(pyrrolidinyl-1)-1 propyl-2)-10 phénothiazinecarbothioamide-2, série D, sous forme d'une meringue jaune.

$[\alpha]_D^{20}$ = +40,7 ±0,6° (1,1%; chloroforme).

Le [(pyrrolidinyl-1)-1 propyl-2)-10 phénothiazinecarbonitrile-2, série D, peut être préparé de la manière suivante :

Un mélange de 25,5 g de méthanesulfonate de (cyano-2 phénothiazinyl-10)-2 propyle-1, série D, et de 29,6 cm3 de pyrrolidine dans 260 cm3 de toluène est chauffé pendant 52 heures à une température voisine de 90°C. Le mélange réactionnel est concentré à sec sous pression réduite (30 mm de Hg; 4 kPa) à 40°C. Le résidu est repris par 500 cm3 d'éther éthylique et extrait par 2 fois 100 cm3 d'une solution aqueuse 2N d'acide méthanesulfonique. La phase aqueuse est alcalinisée par de la lessive de soude (d = 1,33) jusqu'à pH 13 à une température voisine de 5°C et extraite successivement par 100 cm3 d'eau distillée et par 100 cm3 d'une

solution aqueuse saturée de chlorure de sodium, séchées sur sulfate de magnésium en présence de noir 3S, filtrées et le filtrat jaune est concentré à sec sous pression réduite (30 mm de Hg; 4 kPa) à 40°C. Les 18,2 g d'huile orangée ainsi obtenus sont chromatographiés sur une colonne hauteur : 45 cm; diamètre : 4 cm) de gel de silice (0,063-0,2 mm) en éluant par 2,5 litres d'un mélange de chlorure de méthylène et de méthanol (97,5-2,5 en volumes) et en recueillant des fractions de 150 cm3. Les fractions 8 à 16 sont réunies et concentrés à sec sous pression réduite (30 mm de Hg; 4 kPa) à 40°C. On obtient ainsi 11,7 g de [(pyrrolidinyl-1)-1 propyl-2]-10 phénothiazinecarbonitrile-2, série D, sous forme d'une huile jaune.

$[\alpha]_D^{20}$ = -9,8 ±0,4° (1,1%; chloroforme).

Le méthanesulfonate de (cyano-2 phénothiazinyl)-10)-2 propyle-1, série D, peut être préparé de la manière suivante :

A une solution de 20 g d'(hydroxy-1 propyl-2)-10 phénothiazinecarbonitrile, série D, dans 200 cm3 de chlorure de méthylène refroidie à une température voisine de 5°C, on ajoute, sous agitation, 16,2 cm3 de triéthylamine puis on verse, goutte à goutte pendant 25 minutes, une solution de 8,9 cm3 de chlorure de méthanesulfonyle dans 89 cm3 de chlorure de méthylène et poursuit l'agitation pendant 50 minutes à une température voisine de 10°C. Le mélange réactionnel est lavé successivement par 2 fois 100 cm3 d'eau distillée et par 100 cm3 d'une solution aqueuse saturée de chlorure de sodium, séché sur sulfate de magnésium, filtré et concentré à sec sous pression réduite (30 mm de Hg; 4 kPa) à 40°C. On obtient ainsi 25,8 g de méthanesulfonate de (cyano-2 phénothiazinyl-10)-2 propyle-1, série D, sous forme d'une gomme orangée qui est utilisée sans autre purifications pour la suite des synthèses.

$[\alpha]_D^{20}$ = -23,1 ±0,4° (1,4%; chloroforme).

L'(hydroxy-1 propyl-2)-10 phénothiazinecarbonitrile-2, série D, peut être préparé de la manière suivante :

A une solution de 23,5 g de potasse dans 1150 cm3 d'éthanol au reflux, on ajoute 95,4 g de phtalate de (-)-(cyano-2 phénothiazinyl-10)-2 propyle, et de phényl-1 éthylammonium-(S) et poursuit le reflux sous agitation pendant 10 minutes. Le mélange réactionnel est alors jeté sur 1 litre d'eau glacée et extrait par 2 litres puis 500 cm3 d'acétate d'éthyle. Les phases organiques réunies sont lavées successivement par deux fois 500 cm3 d'une solution aqueuse d'acide chlorhydrique 0,1N, par 2 fois 500 cm3 d'une solution aqueuse saturée d'hydrogénocarbonate de sodium et par 500 cm3 d'une solution aqueuse saturée de chlorure de sodium, séchées sur sulfate de magnésium, filtrées et concentrées à sec sous pression réduite (30 mm de Hg; 4 kPa) à 40°C. Le solide jaune résiduel (44 g) est repris par 200 cm3 d'oxyde d'isopropyle au reflux et le produit cristallise à chaud. On laisse revenir à une température voisine de 20°C et on essore le solide, le lave par 20 cm3 d'oxyde d'isopropyle et le sèche sous pression réduite (5 mm de Hg; 0,7 kPa) à 20°C. On obtient ainsi 36,5 g d'(hydroxy-1 propyl-2)-10 phénothiazinecarbonitrile-2, série D, sous forme de cristaux jaunes fondant à 135°C.

$[\alpha]_D^{20}$ = +13,1 ±0,5° (1,0%; chloroforme).

Le phtalate de (-)-(cyano-2 phénothiazinyl-10)-2 propyle et de phényl-1 éthylammonium-(S) peut être préparé de la manière décrite à l'exemple 14 pour l'obtention de son isomère. Le solide précédemment conservé à l'exemple 14 est dissous dans 600 cm3 d'acétate d'éthyle au reflux. Après refroidissement, le solide formé est essoré, lavé par 50 cm3 d'acétate d'éthyle et séché sous pression réduite (30 mm de Hg; 4 kPa) à 40°C. On obtient ainsi 44,2 g de phtalate de (-)-(cyano-2 phénothiazinyl-10)-2 propyle et de phényl-1 éthylammonium-(1S) sous forme de cristaux jaunes clairs fondant à 154°C.

$[\alpha]_D^{20}$ = -21,5° ±0,6° (1%; chloroforme).

## EXEMPLE 38

Une solution de 6 g de [(pyrrolidinyl-1)-1 propyl-2]-10 phénothiazinecarbothioamide-2, série L, et de 18,9 cm3 de méthyl-3 butylamine dans 90 cm3 d'éthanol absolu est saturée d'acide sulfhydrique, et portée pendant 16 heures à une température voisine de 105°C. Après refroidissement, le mélange réactionnel est concentré à sec sous pression réduite (30 mm de Hg; 4 kPa) à 40°C. Le résidu huileux brun est purifié par chromatographie sur une colonne (hauteur : 40 cm; diamètre : 4 cm) de gel de silice (0,02-0,063 mm) en éluant par 1 litre de chlorure de méthylène puis par 1,5 litre d'un mélange de chlorure de méthylène et de méthanol (95-5 en volumes) et en recueillant des fractions de 80 cm3. Les fractions 23 à 36 sont réunies et concentrées à sec sous pression réduite (30 mm de Hg; 4 kPa) à 40°C. On obtient ainsi 6,76 g de N-(méthyl-3 butyl) [(pyrrolidinyl-1)-1 propyl-2]-10 phénothiazinecarbothiamide-2, série L, sous forme d'une huile orangée. On dissout 1 g de cette huile dans 10 cm3 d'acétate d'éthyle et on verse sous agitation 0,7 cm3 d'une solution 3,3N d'acide chlorhydrique dans l'oxyde d'isopropyle. Après 30 minutes d'agitation à une température voisine de 5°C, on essore les cristaux formés, lave par 3 fois 10 cm3 d'oxyde de diéthyle et sèche sous pression réduite (5 mm de Hg; 0,68 kPa) à 40°C. On obtient ainsi 0,75 g de chlorhydrate de N-(méthyl-3 butyl) [(pyrrolidinyl-1)-1 propyl-2]-10 phénothiazinecarbothioamide-2, série L, sous forme de cristaux jaunes fondant à 186°C dont le spectre

de RMN est identique à celui du produit obtenu à l'exemple 35.

$[\alpha]_D^{20}$ = +22,2 ±0,6° (0,8%; diméthylformamide).

Spectre infra-rouge (KBr), bandes caractéristiques en $cm^{-1}$ : 3200, 2955, 2870, 2600, 2480, 1590, 1530, 1460, 1415, 865. 820, 750.

EXEMPLE 39

A une solution de 1,20 g d'{[hydroxyméthyl-2-(2S) pyrrolidinyl-1]-1 propyl-2}-10 phénothiazinecarbothioamide-2, série L, dans 20 cm3 d'éthanol absolu, on ajoute 1,74 cm3 de méthyl-3 butylamine. Le mélange est porté à 150°C pendant 16 heures. Le mélange réactionnel est concentré à sec sous pression réduite (30 mm de Hg; 4 kPa) à 40°C. Le résidu est dilué par 100 cm3 d'acétate d'éthyle, lavé par 2 fois 50 cm3 d'eau distillée, séché sur sulfate de magnésium puis concentré à sec sous pression réduite (30 mm de Hg; 4 kPa) avant d'être purifié par chromatographie sur une colonne (hauteur : 14,5 cm; diamètre : 2,8 cm) de gel de silice (0,04-0,06 mm) avec une légère surpression d'azote (40 kPa) en éluant avec des mélanges de cyclohexane et d'acétate d'éthyle 65-35 (1,2 litre), 35-65 (1,2 litre) (en volumes) en recueillant des fractions de 60 cm3. Les fractions 5 à 13 sont réunies et évaporées sous pression réduite (30 mm de Hg; 4 kPa) à 40°C pour donner 0,64 g d'{hydroxyméthyl-2-(2S) pyrrolidinyl-1]-1 propyl-2}-10 N-(méthyl-3 butyl) phénothiazinecarbothioamide-2, série L, sous la forme d'une laque jaune.

A une solution de 0,42 g de {[hydroxyméthyl-2-(2S) pyrrolidinyl-1]-1 propyl-2}-10 N-(méthyl-3 butyl) phénothiazinecarbothioamide-2, série L, dans 40 cm3 d'éther éthylique, on ajoute 0,41 cm3 d'une solution d'éther chlorhydrique 2,2N. Un précipité gommeux se dépose sur les parois du ballon. Le mélange est concentré à sec sous pression réduite (30 mm de Hg; 4 kPa) à 40°C. La suspension est agitée pendant 12 heures à 25°C dans 50 cm3 d'éther éthylique. Le précipité est séparé par filtration, lavé par 2 fois 10 cm3 d'éther éthylique et séché à 40°C sous pression réduite (5 mm de Hg; 0,068 kPa) pour donner 0,39 g de chlorhydrate de {[hydroxyméthyl-2-(2S) pyrrolidinyl-1]-1 propyl-2}-10 N-(méthyl-3 butyl) phénothiazinecarbothioamide-2, série L, fondant à 131°C.

RMN du proton (250 MHz, DMSO, δ en ppm, J en Hz).

0,95 (D, J = 6,5, 6H, $-C\underline{H}(CH_3)_2$); 1,62 (Mt, 2H, $-NHCH_2C\underline{H_2}-$); 1,72 (D, J = 7, 3H - $CH_3$); 1,6 à 2,2 (Mt, 5H, $-C\underline{H}(CH_3)_2$ et $-CH_2-$ de la pyrrolidine); 3,25 - 3,7 et 4,05 (3Mt, 9H, $>N-CH_2-$, $-CSNH-C\underline{H_2}-$ $NCH_2-$ et NCH de la pyrrolidine, $-CH_2O-$); 4,08 (Mt, 1H, >N-CH<); 5,55 (Mf, 1H, -OH); 7 à 7,4 (Mt, 5H, aromatiques); 7,46 (DD, J = 8 et 1, 1H, -H en 3); 7,56 (D, J = 1, 1H, -H en 1); 9,96 (Mf, 1H, $-NH^+$); 10,51 (T, J = 5,5, 1H, -CSNH-).

Spectre infra-rouge (KBr), bandes caractéristiques en $cm^{-1}$ : 3260, 2960, 2870, 2800 à 2300, 1590, 1535, 1460, 1415, 1060, 825, 755.

L'{[hydroxyméthyl-2-(2S) pyrrolidinyl-1]-1 propyl-2}-10 phénothiazinecarbothioamide-2, série L, peut être préparé de la manière suivante :

Un mélange de 3,66 g d'{[hydroxyméthyl-2-(2S) pyrrolidinyl-1]-1 propyl-2}-10 phénothiazinecarbonitrile-2, série L, et de 1,4 cm3 de triéthylamine dans 30 cm3 de pyridine anhydre est saturé par bullage d'acide sulfhydrique pendant 4 heures à 25°C. La solution limpide obtenue est maintenue sous agitation pendant 12 heures à 25°C, puis le mélange est dégazé par bullage d'azote pendant 2 heures, puis dilué par 500 cm3 d'actétate d'éthyle et lavé par 6 fois 100 cm3 d'eau distillée. La phase organique est séchée sur sulfate de magnésium, filtrée et le filtrat est concentré à sec sous pression réduite (30 mm de Hg; 4 kPa) à 50°C. Le résidu est purifié par chromatographie sur colonne (hauteur : 48 cm; diamètre : 2,5 cm) de gel de silice (0,06-0,2 mm) en éluant par un mélange de cyclohexane et d'acétate d'éthyle 50-50 (1 litre) (en volumes) puis par de l'acétate d'éthyle pur (2 litres) et en recueillant des fractions de 50 cm3. Les fractions 22 à 35 sont réunies et concentrées à sec sous pression réduite (30 mm de Hg; 4 kPa) à 50°C pour donner 2,12 g d'{(hydroxyméthyl-2-(2S) pyrrolidinyl-1]-1 propyl-2}-10 phénothiazinecarbothioamide-2, série L.

L'{[hydroxyméthyl-2-(2S) pyrrolidinyl-1]-1 propyl-2}-10 phénothiazinecarbonitrile-2, série L, peut être préparé de la manière suivante :

A une suspension de 72,1 g de méthanesulfonate de (cyano-2 phénothiazinyl-10)-2-propyle-(2RS) dans 500 cm3 de toluène, on ajoute 39,5 cm3 de (S) -(+)-pyrrolidinyl-2 méthanol. Le mélange est porté à 90°C pendant 160 heures. Après refroidissement, le mélange est lavé par 4 fois 200 cm3 d'eau distillée. La phase organique est séchée sur sulfate de magnésium, filtrée et concentrée à sec sous pression réduite (30 mm de Hg; 4 kPa). Le résidu est purifié par chromatographie sur une colonne d'alumine neutre (hauteur : 47 cm; diamètre : 5 cm) en éluant par des mélanges d'oxyde d'isopropyle et d'acétone_ 97-3 (22 litres) 95-5 (12 litres) et 93-7 (12 litres) ( en volumes) en recueillant des fractions de 500 cm3. Les fractions 4 à 88 sont réunies et concentrées à sec sous pression réduite (30 mm de Hg; 4 kPa) à 50°C pour donner 56,9 g d'une meringue jaune contenant le produit attendu. Ce résidu est à nouveau purifié par chromatographie sur colonne d'alumine

neutre (hauteur : 42 cm; diamètre : 6 cm) en éluant par des mélanges d'oxyde d'isopropyle et d'acétone 98-2 (10 litres) 96-4 (10 litres) et 94-6 (60 litres) (en volumes) en recueillant des fractions de 500 cm3. Les fractions 51 à 70 sont réunies et concentrées à sec sous pression réduite (30 mm de Hg; 4 kPa) à 50°C pour donner 13,7 g d'{[hydroxyméthyl-2-(2S) pyrrolidinyl-1]-1 propyl-2}-10 phénothiazinecarbonitrile-2, série L.

EXEMPLE 40

A une solution de 5,86 g d'{[hydroxyméthyl-2-(2R) pyrrolidinyl-1]-1 propyl-2}-10 phénothiazinecarbothioamide-2, série L, dans 135 cm3 d'éthanol absolu, on ajoute 8,5 cm3 de méthyl-3 butylamine. Le mélange est saturé en acide sulfhydrique puis porté à 150°C pendant 16 heures. Le mélange réactionnel est concentré à sec sous pression réduite (30 mm de Hg; 4 kPa) à 50°C. Le résidu est dilué par 150 cm3 d'actétate d'éthyle, lavé par 3 fois 100 cm3 d'eau distillée et par 100 cm3 de solution saturée de chlorure de sodium, séché sur sulfate de magnésium puis concentré à sec sous pression réduite (30 mm de Hg; 4 kPa) à 50°C avant d'être purifié par chromatographie sur une colonne (hauteur : 32 cm; diamètre : 4 cm) de gel de silice (0,06-0,2 mm) en éluant avec de l'acétate d'éthyle pur (1000 cm3) en recueillant des fractions de 60 cm3. Les fractions 6 à 12 sont réunies et concentrées à sec sous pression réduite (30 mm de Hg; 4 kPa) pour donner un résidu qui est dissous dans 80 cm3 d'oxyde d'isopropyle. La cristallisation est amorcée par grattage et le mélange est agité pendant 12 heures à 25°C. Les cristaux sont filtrés pour donner 4,3 g d'{[hydroxyméthyl-2-(2R) pyrrolidinyl-1]-1 propyl-2}-10 N-(méthyl-3 butyl) phénothiazinecarbothioamide-2, série L, sous la forme de cristaux jaunes fondant à 140°C.

RMN du proton (250 MHz, DMSO, δ en ppm et J en Hz).

0,95 (D, J = 7, 6H, -CH($C\underline{H}_3$)$_2$); 1,4 à 1,85 (Mt, 7H, >CH-$CH_2$- et -$CH_2$- de la pyrrolidine); 1,6 (D, J = 7, 3H, -$CH_3$); 2,22 (Mt, 1H, du >N-$CH_2$- de la pyrrolidine); 2,55 (Mt, environ 1H, >N-CH< de la pyrrolidine); 2,92 (DD, J = 12,5 et 5,5, 1H, du >N-$CH_2$-); 3,2 (Mt, environ 1H, 1H du $NCH_2$- de la pyrrolidine); 3,25 à 3,55 (Mt,>N-$CH_2$- et -$CH_2$O-); 3,74 (Mt, 2H, -CSNH-$C\underline{H}_2$-); 4,18 (Mt, 1H, >N-CH<); 4,42 (Mf, 1H, -OH); 6,9 à 7,25 (Mt, 5H, aromatiques); 7,29 (D large, J = 8, 1H, -H en 3); 7,63 (S large, 1H, -H en 1); 10,21 (T, J = 5, 1H, -CSNH-).

Spectre infra-rouge ($CHCl_3$), bandes caractéristiques en $cm^{-1}$ : 3390, 3280, 2960, 2930, 2875, 2820, 1600, 1570, 1550, 1510, 1460, 1415, 810.

EXEMPLE 41

A une solution de 1,86 g de [diéthylamino-1 propyl-2-(2RS)]-10 phénothiazinecarbothioamide-2 dans 25 cm3 d'éthanol absolu, on ajoute 2,9 cm3 de méthyl-2-(2RS) butylamine. Le mélange est porté à 150°C pendant 16 heures. Le mélange réactionnel est dilué par 150 cm3 d'acétate d'éthyle puis lavé par 3 fois 70 cm3 d'eau distillée. La phase organique est séchée sur sulfate de magnésium, filtrée puis concentrée à sec sous pression réduite (30 mm de Hg; 4 kPa) à 50°C. Le résidu est purifié par chromatographie sur une colonne (hauteur : 16,5 cm; diamètre : 2,8 cm) de gel de silice (0,04-0,06 mm) avec une légère surpression d'azote (40 kPa) en éluant avec un mélange de cyclohexane et d'acétate d'éthyle 75-25 (en volumes) (0,5 litre) en recueillant des fractions de 25 cm3. Les fractions 5 à 15 sont réunies et concentrées à sec sous pression réduite (30 mm de Hg; 4 kPa)à 50°C pour donner 1,12 g d'une meringue orangée. Cette dernière est à nouveau chromatographiée, sur une colonne (hauteur : 14 cm; diamètre : 3,0 cm) de gel de silice (0,04-0,06 mm) avec une légère surpression d'azote (40 kPa) en éluant avec un mélange de cyclohexane et d'acétate d'éthyle 75-25 en volumes (300 cm3) en recueillant des fractions de 20 cm3. Les fractions 4 à 15 sont réunies et concentrées à sec sous pression réduite (30 mm de Hg; 4 kPa) à 50°C pour donner un produit jaune qui est chromatographié une dernière fois sur une colonne (hauteur : 14 cm; diamètre : 3,0 cm) de gel de silice (0,04-0,06 mm) avec une légère surpression d'azote (40 kPa) en éluant avec des mélanges de cyclohexane et d'acétate d'éthyle 90-10 (300 cm3) et 75-25 (350 cm3) (en volumes) en recueillant des fractions de 20 cm3. Les fractions 19 à 32 sont réunies et concentrées à sec sous pression réduite (30 mm de Hg; 4 kPa) à 50°C pour donner 0,53 g d'un produit jaune qui est dissous dans 5 cm3 de propanol-2 bouillant et on ajoute à la solution obtenue 0,14 g d'acide fumarique dissous dans 3 cm3 de propanol-2. La cristallisation est amorcée par grattage. Le mélange est laissé sous agitation pendant 24 heures à 5°C puis les cristaux sont filtrés, séchés à 50°C sous pression réduite (5 mm de Hg; 0,7 kPa) pour donner 0,48 g de fumarate de [diéthylamino-1 propyl-2-(2RS)]-10 N-[méthyl-2-(2RS) butyl] phénothiazinecarbothioamide-2 fondant à 168°C.

Spectre infra-rouge (KBr), bandes caractéristiques en $cm^{-1}$ : 3240, 2960, 2930, 2880, 2650, 2490, 1700, 1590, 1535, 1460, 1420, 980, 885, 820.

Spectre de RMN (250 MHz, DMSO, δ en ppm, J en Hz).

0,9 (Mt, 12H, -$CH_2$CH($C\underline{H}_3$)-$CH_2$$C\underline{H}_3$ et -N($CH_2$$C\underline{H}_3$)$_2$); 1,20 et 1,43 (2Mt, de 1H chacun, -CH($CH_3$)$C\underline{H}_2$-$CH_3$); 1,64 (D, J = 7,5, 3H, -$CH_3$); 1,95 (Mt, 1H, -CH< du méthyl-2 butyle); 2,57 (Mt, 4H, -N($C\underline{H}_2$$CH_3$)$_2$); 2,80

(DD, J = 14 et 5,5, 1H, 1H du >N-$CH_2$-); 3,10 (DD, J = 14 et 7, 1H, 1H du >N-$CH_2$-); 3,50 et 3,64 (2Mt, de 1H chacun, -CSNH-C<u>H</u>$_2$-); 4,25 (Mt, J = 7,5 - 7 et 5,5, 1H, >N-CH<); 6,6 (S, 2H, -CH=CH- du fumarate); 6,9 à 7,3 (Mt, 6H, aromatiques); 7,56 (D, J = 1, 1H, -H en 1); 10,25 (T, J = 5, 1H, -CSNH-).

EXEMPLE 42

Une solution de 1 g de [N-éthyl N-méthyl-amino-1 propyl-2-(2RS)]-10 phénothiazinecarbothioamide-2 et de 4,9 cm3 de méthyl-2 butylamine-(2RS) dans 15 cm3 d'éthanol absolu est saturée avec de l'acide sulfhydrique et chauffée pendant 16 heures à une température voisine de 100°C. Après refroidissement, on concentre à sec sous pression réduite (30 mm de Hg; 4 kPa) à 40°C. Le résidu huileux orangé est purifié par chromatographie sur une colonne (hauteur : 25 cm, diamètre : 2 cm) de gel de silice (0,04-0,063 mm) sous une légère surpression d'azote (40 kPa) en éluant par 500 cm3 d'un mélange d'acétate d'éthyle et de cyclohexane (80-20 en volumes) et en recueillant des fractions de 40 cm3. Les fractions 4 à 8 sont réunies et concentrées à sec sous pression réduite (30 mm de Hg; 4 kPa) à 40°C. On obtient 1,88 g d'une huile jaune. On dissout 0,8 g de cette huile dans 5 cm3 d'acétate d'éthyle et on ajoute 50 cm3 d'oxyde d'isopropyle puis, goutte à goutte sous agitation et à une température voisine de 5°C, 5,55 cm3 d'une solution 0,335N d'acide chlorhydrique dans l'oxyde d'isopropyle et on poursuit l'agitation pendant 30 minutes à la même température. Le précipité formé est essoré, lavé avec 2 cm3 d'oxyde d'isopropyle et séché sous pression réduite (5 mm de Hg; 0,68 kPa) à 40°C. On obtient ainsi 0,73 g de chlorhydrate de [(N-méthyl N-éthyl-amino-1 propyl-2-(2RS)]-10 N'-[méthyl-2 butyl-(2RS)] phénothiazinecarbothioamide-2 sous forme d'un solide jaune fondant à 105-110°C (fusion pâteuse).

RMN du proton (250 MHz, DMSO, δ en ppm, J en Hz).

En solution dans le DMSO, on observe deux formes dues à la salification de l'azote.

0,91 (T, J = 7,5, -$CH_2$-C<u>H</u>$_3$); 0,93 (D, J = 7,5, >CH-C<u>H</u>$_3$ du méthyl-2 butyle); 1,05 et 1,16 (2T, J = 7,5, 3H, -N$CH_2$C<u>H</u>$_3$); 1,20 et 1,39 (2Mt, 2H, -C<u>H</u>$_2$$CH_3$); 1,78 (D large, 3H, -$CH_3$); 1,99 (Mt, 1H, >CH-du méthyl-2 butyle); 2,77 (S large, 3H, >N-$CH_3$); 3,12 (Mf, 2H, >N-C<u>H</u>$_2$-$CH_3$); 3,35 à 3,90 (Mt, 4H, >N-$CH_2$ et -CSNH-C<u>H</u>$_2$-); 4,82 (Mt, 1H, >N-CH<); 7 à 7,35 (Mt, 5H, aromatiques); 7,41 (D, J = 8, 1H, -H en 3); 7,55 (S, 1H, -H en 1); 10,30 (Mf, 1H, -$NH^+$); 10,50 (Mt, 1H, -CSNH-).

Spectre infra-rouge (KBr), bandes caractéristiques en $cm^{-1}$ : 3200, 2960, 2920, 2875, 2580, 1590, 1530, 1460, 880, 820, 750.

EXEMPLE 43

On sature d'acide sulfhydrique une suspension agitée de 2 g de [(pyrrolidinyl-1)-1 propyl-2-(2RS)]-10 phénothiazinecarbo thioamide-2 et de 3 cm3 de méthyl-2 butylamine dans 15 cm3 d'éthanol anhydre et on chauffe pendant 1 heure à une température voisine de 115°C. Après refroidissement, on concentre à sec sous pression réduite (30 mm de Hg; 4kPa) à 50°C. La pâte jaune résiduelle est reprise par 30 cm3 d'acétate d'éthyle et 20 cm3 d'eau distillée. La phase organique est séparée, lavée par 20 cm3 d'une solution aqueuse saturée de chlorure de sodium, séchée sur sulfate de magnésium, filtrée et concentrée à sec sous pression réduite (30 mm de Hg; 4 kPa) à 40°C. L'huile jaune visqueuse résiduelle (2,2 g) est purifiée par chromatographie sur une colonne (hauteur : 40 cm, diamètre : 3,2 cm) de gel de silice (0,04-0,063 mm) en éluant avec 300 cm3 d'un mélange d'acétate d'éthyle et de méthanol (92-8 en volumes) et en recueillant des fractions de 25 cm3. Les fractions 6 à 11 sont réunies et concentrées à sec sous pression réduite (30 mm de Hg; 4 kPa) à 40°C. On obtient ainsi 1,8 g de N-[méthyl-2 butyl-(2RS)] [(pyrrolidinyl-1)-1 propyl-2-(2RS)]-10 phénothiazinecarbothioamide-2 sous forme d'une huile jaune visqueuse. On dissout 0,4 g de cette huile dans 15 cm3 d'oxyde d'isopropyle et on ajoute 0,4 cm3 d'une solution 3,3N d'acide chlorhydrique dans l'oxyde d'isopropyle. Le précipité formé est essoré, lavé par 3 fois 5 cm3 d'oxyde d'isopropyle et séché sous pression réduite (5 mm de Hg; 0,68 kPa) à 40°C. On obtient ainsi 0,4 g de chlorhydrate de N-[méthyl-2 butyl-(2RS)] [(pyrrolidinyl-1)-1 propyl-2-(2RS)]-10 phénothiazinecarbothioamide-2 sous forme d'une poudre jaune fondant à 155-160°C (fusion pâteuse).

RMN du proton (250 MHz, DMSO, δ en ppm, J en Hz).

0,93 (T, J = 7,5, 3H, -$CH_2$C<u>H</u>$_3$); 0,95 (D, J = 7,5, 3H, >CH-C<u>H</u>$_3$ du méthyl-2 butyle); 1,20 et 1,45 (2Mt, de 1H chacun, -C<u>H</u>$_2$$CH_3$); 1,78 (D, J = 7, 3H, -$CH_3$); 1,7 à 2 (Mt, 4H -C<u>H</u>$_2$C<u>H</u>$_2$- de la pyrrolidine); 2 (Mt, 1H, >CH-du méthyl-2 butyle); 2,85 - 3,13 et de 3,4 à 3,95 (2Mf de 1H chacun et Mt, -$CH_2$N$CH_2$- de la pyrrolidine); 3,4 à 3,95 (Mt, 4H, N-$CH_2$- et -CSNH-C<u>H</u>$_2$)-; 4,78 (Mt, 1H, >N-CH<); 7 à 7,35 (Mt, 5H, aromatiques); 7,43 (D large, J = 8, 1H, -H en 3); 7,55 (S large, 1H, -H en 1); 10,48 (T, J = 5, 1H, -CSNH-); 10,57 (Mf, 1H, -$NH^+$).

Spectre infra-rouge (KBr), bandes caractéristiques en $cm^{-1}$ : 3210, 2960, 2920, 2870, 2580, 2470, 1590,

1530, 1460, 1415, 820, 750.

EXEMPLE 44

Un mélange de 2 g de [(pyrrolidinyl-1)-1 propyl-2]-10 phénothiazinecarbothioamide-2, série L, et de 9,5 cm3 de méthyl-2 butylamine-(2RS) dans 30 cm3 d'éthanol est saturé d'acide sulfhydrique puis chauffé pendant 16 heures à 100°C. Après refroidissement, la solution est concentrée à sec sous pression réduite (30 mm de Hg, 4 kPa) à 40°C. On obtient 3,8 g d'une huile orangée que l'on purifie par chromatographie sur colonne (hauteur : 40 cm; diamètre : 3 cm) de gel de silice (0,063-0,2 mm) en éluant par 1,5 litre d'un mélange de chlorure de méthylène et de méthanol 97,5-2,5 en volumes) et en recueillant des fractions de 30 cm3. Les fractions 35 à 46 sont réunies et concentrées à sec sous pression réduite (30 mm de Hg; 4 kPa) à 40°C. On obtient 1,8 g d'une huile orangée. Ce produit est dissous dans 60 cm3 d'un mélange d'oxyde d'isopropyle et d'acétate d'éthyle (80-20 en volumes) puis on ajoute, sous agitation et à une température voisine de 5°C, 11 cm3 d'une solution 0,37N d'acide chlorhydrique dans l'oxyde d'isopropyle. Le précipité formé est essoré, lavé par 3 fois 10 cm3 d'oxyde d'isopropyle et séché sous pression réduite (5 mm de Hg; 0,7 kPa) à 40°C. On obtient ainsi, 1,4 g de chlorhydrate de N-[méthyl-2 butyl-(2RS)] [(pyrrolidinyl-1)-1 propyl-2]-10 phénothiazinecarbothioamide-2, série L, sous forme d'un solide jaune fondant à 120-125°C (fusion pâteuse) et dont le spectre de RMN est identique à celui du thioamide décrit à l'exemple 43.

$[\alpha]_D^{20}$ = +24,3 ±0,8° (0,7%; diméthylformamide).

Spectre infra-rouge (KBr), bandes caractéristiques en $cm^{-1}$ : 3210, 2960, 2930, 2880, 2580, 2480, 1590, 1530, 1460, 870 820, 755.

EXEMPLE 45

Un mélange de 4 g de [(pyrrolidinyl-1)-1 propyl-2]-10 phénothiazinecarbothioamide-2, série L, et de 5 g de méthyl-2 butylamine-(2S) dans 60 cm3 d'éthanol est saturé d'acide sulfhydrique puis chauffé pendant 16 heures à 100°C. Après refroidissement, la solution est concentrée à sec sous pression réduite (30 mm de Hg; 4 kPa) à 40°C. On obtient 5,3 g d'une huile orangée que l'on purifie par chromatographie sur colonne (hauteur : 48 cm; diamètre : 4 cm) de gel de silice (0,063-0,2 mm) en éluant par 2 litres d'un mélange de chlorure de méthylène et de méthanol (97,5-2,5 en volumes) et en recueillant des fractions de 60 cm3. Les fractions 23 à 29 sont réunies et concentrées à sec sous pression réduite (30 mm de Hg; 4 kPa) à 40°C. On obtient 4,1 g d'une huile orangée. Ce produit est dissous dans 200 cm3 d'un mélange d'oxyde d'isopropyle et d'acétate d'éthyle (75-25 en volumes) puis on ajoute, sous agitation et à une température voisine de 5°C, 12,8 cm3 d'une solution 0,72 N d'acide chlorhydrique dans l'oxyde d'isopropyle. Le précipité formé est essoré, lavé par 3 fois 20 cm3 d'oxyde d'isopropyle et séché sous pression réduite (5 mm de Hg; 0,7 kPa) à 40°C. On obtient ainsi 3,2 g de chlorhydrate de N-[méthyl-2 butyl-(2S)] phénothiazinecarbothioamide-2, série L, sous forme d'un solide jaune fondant à 135-140°C (fusion pâteuse) et dont le spectre de RMN est identique à celui du thioamide décrit à l'exemple 43.

$[\alpha]_D^{20}$ = +28,3 ±0,6° (1%; diméthylformamide).

Spectre infra-rouge (KBr), bandes caractéristiques en $cm^{-1}$ : 3220, 2950, 2925, 2870, 2680, 2600, 2475, 1590, 1530, 1460, 865, 820, 750.

EXEMPLE 46

Une solution de 2 g de [(pyrrolidinyl-1)-1 propyl-2-(2RS)]-10 phénothiazinecarbothioamide-2 et de 7,4 g de chlorhydrate de méthyl-4 pentylamine dans 30 cm3 d'éthanol absolu est saturée d'acide sulfhydrique. Le mélange réactionnel est ensuite porté pendant 16 heures à une température voisine de 105°C. Après refroidissement, on concentre à sec sous pression réduit (30 mm de Hg; 4 kPa) à 40°C. Le résidu pâteux (5 g) est purifié par chromatographie sur une colonne (hauteur : 35 cm; diamètre : 3 cm) de gel de silice (0,2-0,063 mm) en éluant successivement par 500 cm3 de chlorure de méthylène puis par 500 cm3 d'un mélange de chlorure de méthylène et de méthanol (95-5 en volumes) et en recueillant des fractions de 30 cm3. Les fractions 25 à 28 sont réunies et concentrées à sec sous pression réduite (30 mm de Hg; 4 kPa) à 40°C. L'huile orangée résiduelle (2,16 g) est dissoute dans 10 cm3 d'acétate d'éthyle et on ajoute sous agitation 1,5 cm3 d'une solution 3,3N d'acide chlorhydrique dans l'oxyde d'isopropyle puis de l'éther éthylique jusqu'à formation d'un trouble persistant. Après 1 heure à une température voisine de 5°C, le solide formé est essoré, lavé par 3 fois 10 cm3 d'éther éthylique et séché sous pression réduite (5 mm de Hg; 0,7 kPa) à 40°C. On obtient ainsi 1,7 g de chlorhydrate N-(méthyl-4 pentyl) [(pyrrolidinyl-1)-1 propyl-2-(2RS)]-10 phénothiazinecarbothioamide-2 sous forme

d'un solide jaune fondant à 186°C.

RMN du proton (250 MHz, DMSO, δ en ppm, J en Hz).

0,90 (D, J = 7, 6H, -CH(C$\underline{H}_3$)$_2$); 1,26 (Mt, 2H, -C$\underline{H}_2$-CH(CH$_3$)$_2$); 1,58 (Mt, 1H, >CH-du méthyl-4 pentyle); 1,7 (Mt, 2H, -NHCH$_2$-C$\underline{H}_2$-); 3,72 (Mt, 2H, -CSNHC$\underline{H}_2$-); 7 à 7,35 (Mt, 5H, aromatiques); 7,45 (D large, J = 8, 1H, -H en 3); 7,55 (S large, 1H, -H en 1); 10,52 (T, J = 5, 1H, -CSNH-).

Spectre infra-rouge (KBr), bandes caractéristiques en cm$^{-1}$ : 3200, 2950, 2920, 2860, 2680, 2600, 2470, 1590, 1530, 1460, 1410, 815, 745.

EXEMPLE 47

Un mélange de 3,7 g de [(pyrrolidinyl-1)-1 propyl-2-(2RS)]-10 phénothiazinecarbothioamide-2, de 3,3 g de chlorhydrate de [méthyl-3 pentyl-1-(3RS)] amine et de 3,4 cm3 de triéthylamine dans 55 cm3 d'éthanol est saturé d'acide sulfhydrique puis chauffé pendant 16 heures à 105°C. Après refroidissement, la solution est concentrée à sec sous pression réduite (30 mm de Hg; 4 kPa) à 40°C. On obtient 5,6 g d'une huile orangée qui est purifiée par chromatographie sous surpression d'azote (40 kPa) sur colonne (hauteur : 25 cm; diamètre : 4 cm) de gel de silice (0,063-0,2 mm) en éluant par 1 litre d'un mélange de chlorure de méthylène et de méthanol (95-5 en volumes) et en recueillant des fractions de 60 cm3. Les fractions 8 à 9 sont réunies et concentrées à sec sous pression réduite (30 mm de Hg; 4 kPa) à 40°C. On obtient 2,3 g d'une huile orangée. Ce produit est dissous dans un mélange de 10 cm3 d'acétate d'éthyle et de 100 cm3 d'oxyde d'isopropyle puis on ajoute 1,5 cm3 d'une solution 3,3N d'acide chlorhydrique dans l'oxyde d'isopropyle. Le précipité formé est essoré, lavé par 3 fois 10 cm3 d'oxyde d'isopropyle et séché sous pression réduite (5 mm de Hg; 0,7 kPa) à 40°C. On obtient ainsi 1,7 g de chlorhydrate de N-[méthyl-3 pentyl-1-(3RS) [(pyrrolidinyl-1)-1 propyl-2-(2RS)-10 phénothiazinecarbothioamide-2 sous forme d'un solide jaune fondant à 135-140°C (fusion pâteuse).

RMN du proton (250 MHz, DMSO, δ en ppm, J en Hz).

0,88 (T, J = 7, 3H, -CH$_2$-C$\underline{H}_3$); 0,93 (D, J = 7, 3H, >CH-C$\underline{H}_3$ du méthyl-3 pentyle); 1,2 et 1,4 (2Mt, 1H chacun, -C$\underline{H}_2$CH$_3$); 1,49 (Mt, 2H, -NHCH$_2$C$\underline{H}_2$-); 1,72 (Mt, 1H, -CH- du méthyl-3 pentyle); 3,50 à 3,90 (Mt, 4H, N-CH$_2$ et -CSNH-C$\underline{H}_2$); 7 à 7,35 (Mt, 5H, aromatiques); 7,41 (D large, J = 8, 1H, -H en 3); 7,56 (S large, 1H, -H en 1); 10,4 (T, J = 5, 1H, -CSNH-).

Spectre infra-rouge (KBr), bandes caractéristiques en cm$^{-1}$ : 3220, 2960, 2930, 2880, 2680, 2600, 2480, 1590, 1535, 1460, 1415, 870, 825, 755.

Le [(pyrrolidinyl-1)-1 propyl-2-(2RS)]-10 phénothiazinecarbothioamide-2 peut être obtenu de la manière suivante :

Une solution de 5,3 g de [(pyrrolidinyl-1)-1 propyl-2-(2RS)]-10 phénothiazinecarbonitrile-2 et 2,2 cm3 de triéthylamine dans 106 cm3 de pyridine anhydre est saturée d'acide sulfhydrique et laissée sous agitation pendant 16 heures à une température voisine de 20°C. Le mélange réactionnel est versé sur 500 cm3 d'eau distillée et extrait successivement par 500 cm3 puis deux fois 250 cm3 d'acétate d'éthyle. Les phases organiques réunies sont lavées par 3 fois 300 cm3 d'eau distillée, séchées sur sulfate de magnésium, filtrées et concentrées à sec sous pression réduite (30 mm de Hg; 4 kPa) à 40°C. Les 6,5 g de l'huile orangée obtenue sont chromatographiés sur une colonne (hauteur : 30 cm; diamètre : 4 cm) de gel de silice (0,2-0,063 mm) sous surpression d'azote (40 kPa) en éluant par 1 litre d'un mélange de chlorure de méthylène et de méthanol (95-5 en volumes) puis par 1 litre d'un mélange de chlorure de méthylène et de méthanol (90-10 en volumes) et en recueillant des fractions de 100 cm3. Les fractions 8 à 16 sont réunies et concentrées à sec sous pression réduite (30 mm de Hg; 4 kPa) à 40°C. On obtient ainsi 5,1 g de [(pyrrolidinyl-1)-1 propyl-2-(2RS)]-10 phénothiazinecarbothioamide-2 sous forme d'un solide jaune. On dissout, à une température voisine de 60°C, 1,4 g de ce produit dans 25 cm3 d'isopropanol. Après refroidissement, on essore les cristaux formés, les lave par 5 cm3 d'isopropanol froid et sèche sous pression réduite (5 mm de Hg; 0,7 kPa) à 30°C. On obtient ainsi 1,1 g de [(pyrrolidinyl-1)-1 propyl-2-(2RS)]-10 phénothiazinecarbothioamide-2 sous forme de cristaux jaunes fondant à 150°C.

Le [(pyrrolidinyl-1)-1 propyl-2-(2RS)]-10 phénothiazinecarbonitrile-2 peut être obtenu de la manière suivante :

Un mélange de 10 g de méthanesulfonate de (cyano-2 phénothiazinyl-10)-2 propyle-1-(2RS) et de 11,6 cm3 de pyrrolidine dans 50 cm3 de toluène est porté à 90°C sous agitation pendant 24 heures. Après refroidissement, on concentre à sec sous pression réduite (30 mm de Hg; 4 kPa) à 40°C et on reprend le résidu avec 200 cm3 d'éther éthylique et 15 cm3 d'une solution aqueuse de soude 4N. Après avoir agité pendant 10 minutes, on décante et lave la phase organique avec 3 fois 25 cm3 d'une solution aqueuse saturée de chlorure de sodium, sèche sur sulfate de magnésium, filtre et concentre à sec sous pression réduite (30 mm de Hg; 4 kPa) à 20°C. Les 11,3 g d'huile résiduelle sont dissous dans 60 cm3 d'une solution aqueuse d'acide chlorhydrique 0,5N. Cette solution est lavée avec 100 cm3 d'éther éthylique puis alcalinisée avec un excès d'une solution aqueuse de soude N et extraite par 100 cm3 d'éther éthylique. La phase organique est lavée avec 50 cm3 d'une solution aqueuse saturée de chlorure de sodium, séchée sur sulfate de magnésium, filtrée et concentrée à sec sous pression réduite (30 mm de Hg; 4 kPa) à 20°C. L'huile jaune résiduelle (9,5 g) est purifiée sur une colonne (hauteur : 30 cm; diamètre : 5,8 cm) de gel de silice (0,04-0,063 mm) sous légère surpression d'azote (40 kPa) en éluant avec un litre d'un mélange de chlorure de méthylène et de méthanol (95-5 en volumes) et en recueillant des fractions de 100 cm3. Les fractions 3 à 10 sont réunies et concentrées à sec sous pression réduite (30 mm de Hg; 4 kPa) à 40°C. On obtient ainsi 5,45 g de [(pyrrolidinyl-1)-1 propyl-2-(2RS)]-10 phénothiazinecarbonitrile-2 sous forme d'une huile jaune.

Le méthanesulfonate de (cyano-2 phénothiazinyl-10)-2 propyle-1-(2RS) peut être obtenu de la manière suivante :

Sur une solution de 120,5 g d'[hydroxy-1 propyl-2-(2RS)]-10 phénothiazinecarbonitrile-2 dans 1280 cm3 de chlorure de méthylène refroidie à une température voisine de 5°C, on verse, sous agitation, 100 cm3 de triéthylamine puis, en 30 minutes, 55,9 cm3 de chlorure de méthanesulfonyle et on poursuit l'agitation pendant 15 minutes en maintenant la température vers 10-15°C. Le mélange réactionnel est dilué avec 500 cm3 d'eau distillée à 5°C et la phase organique est séparée, lavée avec 500 cm3 d'une solution aqueuse saturée de chlorure de sodium, séchée sur sulfate de sodium, filtrée et concentrée à sec sous pression réduite (30 mm de Hg; 4 kPa) à 40°C. L'huile résiduelle (164 g) est purifiée par chromatographie sur une colonne (hauteur : 54 cm; diamètre : 8,5 cm) de gel de silice (0,2-0,063 mm) en éluant par 4,4 litres de chlorure de méthylène puis par 7 litres d'un mélange de chlorure de méthylène et de méthanol (99-1 en volumes) et en recueillant des fractions de 1 litre. Les fractions 3 à 11 sont réunies et concentrées à sec sous pression réduite (30 mm de Hg; 4 kPa) à 40°C. On obtient ainsi 153,5 g d'une huile jaune que l'on reprend par 400 cm3 d'oxyde d'isopropyle au reflux. Par refroidissement, un produit cristallise et on poursuit l'agitation pendant 1 heure à une température voisine de 5°C. Le solide formé est essoré, lavé par 2 fois 50 cm3 d'oxyde d'isopropyle glacé et séché à 30°C sous pression réduite (30 mm de Hg; 0,4 kPa). On obtient ainsi 131,6 g de méthanesulfonate de (cyano-2 phénothiazinyl-10)-2 propyle-1-(2RS) sous forme de cristaux jaune clair fondant à 124°C.

EXEMPLE 48

Un mélange de 3 g de [(pyrrolidinyl-1)-1 propyl-2-(2RS)]-10 phénothiazinecarbothioamide-2 et de 5,7 g de diméthyl-3,3 butylamine dans 30 cm3 d'éthanol absolu est porté à une température voisine de 100°C pendant 16 heures. Après refroidissement on concentre à sec sous pression réduite (30 mm de Hg; 4 kPa) à 40°C. Le résidu est dissous dans 100 cm3 d'acétate d'éthyle et cette solution est lavée par 3 fois 20 cm3 d'eau distillée, séchée sur sulfate de magnésium en présence de noir 3S et concentrée à sec sous pression réduite (30 mm de Hg; 4 kPa) à 40°C. Le résidu (5 g) est purifié par chromatographie sur une colonne (hauteur : 30 cm; diamètre : 5 cm) de gel de silice (0,04-0,063 mm) en éluant par 2,5 litres d'un mélange de chlorure de méthylène et de méthanol (97-3 en volumes) et en recueillant des fractions de 100 cm3. Les fractions 18 à 24 sont réunies et concentrées à sec sous pression réduite (30 mm de Hg; 4 kPa) à 40°C. Le résidu (3,68 g) est dissous dans 35 cm3 d'acétate d'éthyle. Cette solution est filtrée et on ajoute 3,5 cm3 d'une solution 3N d'acide chlorhydrique dans l'éther éthylique. On laisse reposer 2 heures à une température voisine de5°C et le solide formé est essoré, lavé par 5 cm3 d'éther éthylique et séché sous pression réduite (5 mm de Hg; 0,7 kPa) à 35°C. On obtient ainsi 3,2 g de chlorhydrate de N-(diméthyl-3,3 butyl) [(pyrrolidinyl-1)-1 propyl-2-(2RS)]-10 phénothiazinecarbothioamide-2 sous forme de cristaux jaunes fondant à 199-200°C.

RMN du proton (250 MHz, DMSO, δ en ppm, J en Hz).

0,97 (s, 9H, $-C(CH_3)_3$); 1,61 (Mt, 2H, $-C\underline{H_2}-C(CH_3)_3$); 1,78 (D, J = 7, 3H, $-CH_3$); 3,5 à 3,9 (Mt, 4H, >N-$CH_2$ et -CSNHC$\underline{H_2}$-); 7 à 7,35 (Mt, 5H, aromatiques); 7,42 (D, J = 8, 1H, -H en 3); 7,53 (S, 1H, -H en 1); 10,45 (T, J = 5, 1H, -CSNH-).

Spectre infra-rouge (KBr), bandes caractéristiques en $cm^{-1}$ : 3190, 2950, 2860, 2680, 2600, 2470, 1590, 1530, 1460, 1410, 865, 820, 745.

EXEMPLE 49

Une solution de 7,4 g de [(pyrrolidinyl-1)-1 propyl-2]-10 phénothiazinecarbothioamide-2, série L, dans 40 cm3 d'éthanol absolu est saturé par barbottage pendant 20 minutes avec de l'acide sulfhydrique puis on ajoute une solution de 8,5 g de cyclobutylméthylamine dans 50 cm3 d'éthanol et le mélange est porté à une température de 130°C pendant 20 heures. Après refroidissement, le mélange réactionnel est concentré à sec sous pression réduite (30 mm de Hg; 4 kPa) à 40°C. Le résidu est purifié par chromatographie sur une colonne (hauteur: 38 cm; diamètre: 4,5 cm) de gel de silice (0,04-0,06 mm) avec une légère surpression d'azote (40,5 kPa) en éluant par 2 litres d'acétate d'éthyle et en recueillant des fractions de 125 cm3. Les fractions 1 à 6 sont

réunies et concentrées à sec sous pression réduite ( 30 mm de Hg ; 4 kPa) à 40°C pour donner 8,53 g de N-cyclobutylméthyl [(pyrrolidinyl-1)-1 propyl-2]-10 phénothiazinecarbothioamide-2, série L.

$[\alpha]_D^{20}$ = + 24,4 ± 0,5° (c = 1 % ; Méthanol)

A une solution de 2,19 g de N-cyclobutylméthyl [(pyrrolidinyl-1)-1 propyl-2]-10 phénothiazinecarbothioamide-2, série L, dans 16 cm3 de propanol-2 au reflux, on ajoute une solution de 0,58 g d'acide fumarique dans 14 cm3 de propanol-2 au reflux. Après refroidissement, le mélange est concentré à sec sous pression réduite ( 30 mm de Hg; 4 kPa) à 40°C et le résidu est repris sous agitation pendant 16 heures par 70 cm3 d'oxyde d'isopropyle. La suspension obtenue est filtrée et le solide est séché sous pression réduite (5 mm de Hg, 4 kPa) à 50°C, dissous dans 50 cm3 de propanol-2 au reflux. Après refroidissement, le produit cristallise et on poursuit l'agitation pendant 16 heures à 20°C. Les cristaux sont filtrés et séchés sous pression réduite (5 mm de Hg, 4 kPa) à 50°C. On obtient ainsi 1,52 g de fumarate de N-cyclobutylméthyl [(pyrrolidinyl-1)-1 propyl-2]-10 phénothiazinecarbothioamide-2, série L, sous forme de cristaux jaunes fondant à 194°C.

$[\alpha]_D^{20}$ = + 29,9 ± 0,6° (c = 1 % ; Méthanol)

RMN du proton (250 MHz, DMSO D6, δ en ppm, J en Hz).

1,62 (D, J = 7, 3H, $-CH_3$) ; 1,74 (Mt, 4H, $-CH_2-$ de la pyrrolidine) ; 1,7 à 1,95 et 2,05 (2 Mt, respectivement 4H et 2H, $-CH_2-$ du cyclobutylméthyle) ; 2,6 à 2,9 (Mt, 5H, >CH- du cyclobutylméthyle et >N-$CH_2$- de la pyrrolidine) ; 3,11 (DD, J = 12,5 et 6, 1 H, 1 H du >N-$CH_2$-) ; 3,23 (DD, J = 12,5 et 6,5, 1H, 1 H du >N-$CH_2$-) ; 3,77 (DD, J = 7 et 5,5, 2H, -CSNHC$\underline{H_2}$-) ; 4,37 (Mt, J = 7, 6,5 et 6, 1H, >N-CH<) ; 6,59 (S, 2 H, -CH=CH- du fumarate) ; 6,95 à 7,35 (Mt, 6H, aromatiques) ; 7,54 (S large, 1H, -H en 1) ; 10,3 (T, J = 5,5, 1H, -CSNH-).

Spectre infra-rouge (KBr), bandes caractéristiques en $cm^{-1}$ : 3220, 2970, 2860, 2800, 2060, 2000, 1800, 1700, 1590, 1540, 1485, 1460, 980, 870, 815, 755, 640.

## EXEMPLE 50

Un mélange de 2 g de [diméthylamino-1 propyl-2-(2RS)]-10 phénothiazinecarbothioamide-2 et de 11,4 cm3 de cyclohexylméthylamine dans 30 cm3 d'éthanol absolu est saturé avec de l'acide sulfhydrique et chauffé pendant 16 heures à une température voisine de 125°C. Après refroidissement, la solution orangée obtenue est concentrée à sec sous pression réduite (30 mm de Hg; 4 kPa) à 40°C. L'huile orangée résiduelle est purifiée par chromatographie sur une colonne (hauteur : 25 cm; diamètre : 4 cm) de gel de silice (0,06-0,04 mm) avec une légère surpression d'azote (40 kPa) en éluant par 1 litre d'un mélange d'acétate d'éthyle et de cyclohexane 60-40 (en volumes) et en recueillant des fractions de 60 cm3. Les fractions 6 à 13 réunies sont concentrées à sec sous pression réduite (30 mm de Hg; 4 kPa) à 40°C. La gomme orangée résiduelle (2,5 g) est dissoute dans 8 cm3 d'isopropanol au reflux et additionnée sous agitation d'une solution à 50°C de 0,66 g d'acide fumarique dans 8 cm3 d'isopropanol. On laisse refroidir et on poursuit l'agitation pendant 1 heure à une température voisine de 5°C. Le solide formé est essoré, lavé par 2 fois 1 cm3 d'isopropanol et séché sous pression réduite (5 mm de Hg; 0,68 kPa) à 40°C. On obtient ainsi 2,53 g de fumarate neutre de N-cyclohexylméthyl [diméthylamino-1 propyl-2-(2RS)]-10 phénothiazinecarbothioamide-2 sous forme d'un solide jaune fondant à 167°C.

RMN du proton (250 MHz, DMSO, δ en ppm, J en Hz).

0,9 à 1,35 et 1,5 à 1,95 (Mt, 11H, $-C_6H_{11}$); 1,58 (D, J = 7, 3H, $-CH_3$); 2,28 (S, 6H, $-N(CH_3)_2$); 2,75 (DD, J = 13 et 6,5, 1H, 1H du>N-$CH_2$-); 2,99 (DD, J = 13 et 6, 1H, 1H du >N-$CH_2$-); 3,55 (Mt, 2H, -CSNH-C$\underline{H_2}$-); 4,24 (Mt, J = 7 - 6,5 et 6, 1H, >N-CH<); 6,58 (S, 1H, -CH=CH- de l'hemifumarate); 6,9 à 7,25 (Mt, 5H, aromatiques); 7,27 (DD, J = 8 et 1, 1H, -H en 3); 7,50 (D, J = 1, 1H, -H en 1); 10,23 (T, J = 5,5, 1H, -CSNH-).

Spectre infra-rouge (KBr), bandes caractéristiques en $cm^{-1}$ : 3180, 2980, 2920, 2860, 1700, 1590, 1535, 1460, 760, 670.

## EXEMPLE 51

Une solution de 0,74 g de [(dihydro-2,5 pyrrolyl-1)-1 propyl-2]-10 phénothiazinecarbothioamide-2, série L, dans 7 cm3 d'allylamine est saturée par barbottage pendant 25 minutes avec de l'acide sulfhydrique puis chauffée pendant 5 heures à une température voisine de 55°C. Après refroidissement, le mélange réactionnel est concentré à sec sous pression réduite ( 30 mm de Hg; 4 kPa) à 40°C. Le résidu est dissous dans 30 cm3 d'acétate d'éthyle, lavé successivement par 5 fois 30 cm3 d'eau distillée, séché sur sulfate de magnésium, filtré et concentré à sec sous pression réduite (30 mm de Hg; 4kPa) à 40°C. Le résidu (0,89 g) est purifié par chromatographie sur colonne (hauteur:18 cm; diamètre: 2,4 cm) de gel de silice (0,04-0,063 mm) sous une légère surpression d'azote (40kPa) en éluant avec 0,5 litre d'un mélange de cyclohexane et d'acétate d'éthyle (50-50 en volumes) et en recueillant des fractions de 25 cm3. Les fractions 4 à 8 sont réunies et concentrées

à sec sous pression réduite (30 mm de Hg; 4 kPa) à 40°C pour donner 0,42 g d'une huile jaunâtre. Ce résidu est dissous dans 2 cm3 de propanol-2 au reflux et additionné d'une solution tiède de 0,11 g d'acide fumarique dans 2,3 cm3 de propanol-2. Après refroidissement, le solide cristallisé formé est filtré, lavé par 10 cm3 d'oxyde de diéthyle et séché sous pression réduite (5 mm de Hg; 0,67 kPa). On obtient ainsi 0,30 g de fumarate de N-allyl [(dihydro-2,5 pyrrolyl-1)-1 propyl-2]-10 phénothiazinecarbothioamide-2, série L, sous forme d'une poudre jaune fondant vers 197°C.

$[\alpha]_D^{20}$ = + 36,4 ± 0,7° (1 % ; méthanol)

RMN du proton (250 MHz, DMSO D6, δ en ppm, J en Hz).

1,63 (D, J= 7, 3H, $-CH_3$) ; 3,1 (DD, J = 12,5 et 7,5, 1H, 1 H du $>N-CH_2-$) ; 3,28 (DD, J = 12,5 et 6,5, 1H, 1 H du $>N-CH_2-$) ; 3,54 (AB, 4H, $>N-CH_2-$ de la pyrroline) ; 4,20 (Mt, J = 7,5, 7 et 6,5, 1H, $>N-CH<$) ; 4,37 (DD, J = 6,5 et 5, 2H, $-CSNHC\underline{H_2}-$) ; 5,23 (Mt, 2H, $-CH_2CH=C\underline{H_2}$) ; 5,80 (Mf, 2H, -CH=CH- de la pyrroline) ; 5,97 (Mt, 1H, $-CH=CH_2$) ; 6,64 (S, 2 H, -CH=CH- du fumarate) ; 6,9 à 7,3 (Mt, 5 H, aromatiques) ; 7,34 (DD, J = 8 et 1, 1H, -H en 3) ; 7,62 (D, J = 1, 1H, -H en 1) ; 10,45 (T, J = 5, 1H, -CSNH-).

Spectre infra-rouge (KBr), bandes caractéristiques en $cm^{-1}$ : 3240, 2980, 2930, 2720, 2100, 1800, 1705, 1640, 1590, 1560, 1460, 1530, 985, 925, 885, 820, 755, 645.

EXEMPLE 52

Un mélange de 2,5 g de [N-éthyl N-(hydroxy-2 éthyl)-amino-1 propyl-2-(2RS)]-10 phénothiazinecarbothioamide-2 et de 7 cm3 de benzylamine dans 50 cm3 d'éthanol est saturé avec de l'acide sulfhydrique gaz puis chauffé 13 heures à 110°C en autoclave. Après refroidissement, la solution est concentrée à sec sous pression réduite (30 mm de Hg; 4 kPa) à 40°C. Le résidu est dilué par 100 cm3 d'acétate d'éthyle et la phase organique est lavée par 4 fois 50 cm3 d'eau distillée, séchée sur sulfate de magnésium et filtrée. Le filtrat est concentré à sec à 40°C sous pression réduite (30 mm de Hg; 4 kPa) pour donner une huile orangée qui est purifiée par chromatographie sur colonne (hauteur : 30 cm; diamètre : 3 cm) de silice (0,06-0,2 mm) en éluant par un mélange de cyclohexane et d'acétate d'éthyle 50-50 (en volumes) (600cm3) et en recueillant des fractions de 50 cm3. Les fractions 5 à 10 sont réunies et concentrées à sec sous pression réduite (30 mm de Hg ; 4 kPa) à 40°C. On obtient ainsi 2,6 g d'une huile jaune.

A une solution de 2,6 g de N-benzyl [N'-éthyl N'(hydroxy-2 éthyl)amino-1 propyl-2-(2RS)]-10 phénothiazinecarbothioamide-2 dans 30 cm3 d'éthanol à 50°C, on ajoute 0,6 g d'acide fumarique en solution dans 10 cm3 d'éthanol à 50°C. La cristallisation est amorcée par grattage et le mélange est agité pendant 1 heure à 20°C. Le solide est séparé par filtration, lavé par 2 fois 4 cm3 d'éthanol et séché à 40°C sous pression réduite (1 mm de Hg; 0,13 kPa) pour donner 2,1 g de fumarate neutre de N'-benzyl [N-éthyl N-(hydroxy-2 éthyl)-amino-1 propyl-2-(2RS)]-10 phénothiazinecarbothioamide-2 sous la forme de cristaux jaunes fondant à 154°C.

RMN du proton (250 MHz, DMSO, δ en ppm, J en Hz).

0,85 (T, J = 7, 3H, $>NCH_2C\underline{H_3}$); 1,63 (D, J = 7, 3H, $-CH_3$); 2,50 (Mt masqué, 4H, $HOCH_2C\underline{H_2}-N-C\underline{H_2}\,CH_3$); 2,8 (DD, J = 13 et 6, 1H, 1H du $>N-CH_2-$); 3,10 (DD, J = 13 et 6,5, 1H, 1H du $>N-CH_2-$); 3,37 (Mt en partie masqué, $-C\underline{H_2}OH$); 4,20 (Mt, J = 7 - 6,5 et 6, 1H, N-CH ); 5 (D, J = 6, 2H, $-CSNH-C\underline{H_2}-$); 6,64 (S, 1H, -CH=CH- de l'hémifumarate); 6,90 à 7,45 (Mt, 11H, aromatiques); 7,72 (D, J = 1, 1H -H en 1); 10,77 (T, J = 6, 1H, -CSNH-).

Spectre infra-rouge (KBr), bandes caractéristiques en $cm^{-1}$ : 3200, 2970, 2930, 2870, 1590, 1530, 1495, 1485, 1460, 985, 750, 700, 660, 650.

Le [N-éthyl N-(hydroxy-2 éthyl)-amino-1 propyl-2-(2RS)]-10 phénothiazinecarbothioamide-2 peut être préparé de la manière suivante :

A un mélange de 3,1 g de [N-éthyl N-(hydroxy-2 éthyl)-amino-1 propyl-2-(2RS)]-10 phénothiazinecarbonitrile-2 dans 62 cm3 de pyridine, on ajoute 1,2 cm3 de triéthylamine puis le mélange est saturé d'hydrogène sulfuré par barbottage pendant 1 heure. La solution limpide obtenue est maintenue sous agitation pendant 12 heures à 25°C, puis dégazée par bullage d'azote pendant 1 heure. Le mélange réactionnel est alors dilué par 150 cm3 d'acétate d'éthyle et lavé par 6 fois 100 cm3 d'eau distillée, et par 3 fois 120 cm3 de solution saturée de chlorure de sodium. La phase organique est séchée sur sulfate de magnésium, filtrée et le filtrat est concentré à sec à 40°C sous pression réduite (30 mm de Hg; 4 kPa). Le résidu est purifié par chromatographie sur colonne (hauteur : 29 cm; diamètre : 2,5 cm) de gel de silice (0,06-0,2 mm) en éluant par un mélange de cyclohexane et d'acétate d'éthyle 30-70 en volumes (1 litre) et en recueillant des fractions de 50 cm3. Les fractions 5 à 15 sont réunies et concentrées à sec à 50°C sous pression réduite (30 mm de Hg; 4 kPa) pour donner 2,5 g de [N-éthyl N-(hydroxy-2 éthyl)-amino-1 propyl-2-(2RS)]-10 phénothiazinecarbothioamide-2 sous la forme d'une meringue orangée.

Le [N-éthyl N-(hydroxy-2 éthyl)-amino-1 propyl-2-(2RS)]-10 phénothiazinecarbonitrile-2 peut être préparé de la manière suivante :

A une solution de 5 g d'[éthylamino-1 propyl-2-(2RS)]-10 phénothiazinecarbonitrile-2 dans 50 cm3 de diméthylformamide, on ajoute 1,2 g de bromo-2 éthanol et 2,4 g d'hydrogénocarbonate de potassium. Le mélange est chauffé à 130°C pendant 6 heures. Après refroidissement, la solution est concentrées à sec à 80°C sous pression réduite (10 mm de Hg; 1,33 kPa). Le résidu est repris par 100 cm3 d'acétate d'éthyle puis lavé par 6 fois 100 cm3 d'eau distillée et par 100 cm3 de saumure. La phase organique est séchée sur sulfate de magnésium, filtrée et le filtrat est concentré à sec à 40°C sous pression réduite (30 mm de Hg; 4 kPa) pour donner 5,0 g d'une huile jaune pâle qui est purifiée par chromatographie sur colonne (hauteur : 35 cm; diamètre : 3 cm) de gel de silice (0,06-0,2 mm) en éluant par un mélange de cyclohexane et d'acétate d'éthyle 30-70 (en volumes) (600cm3) et en recueillant des fractions de 60 cm3. Les fractions 5 à 7 sont réunies et concentrées à sec à 50°C sous pression réduite (30 mm de Hg; 4 kPa) pour donner 3,2 g de [N-éthyl N-(hydroxy-2 éthyl)-amino-1 propyl-2-(2RS)]-10 phénothiazinecarbonitrile-2 sous la forme d'une huile jaune.

## EXEMPLE 53

En opérant d'une manière analogue à celle décrite ci-après à l'exemple 80 mais à partir de 2 g de N-(chloro-3 benzyl) [(pyrroli- dinyl-1)-1 propyl-2]-10 phénothiazinecarbothioamide-2, série L, dans 60 cm3 d'acide acétique et de 1,3 g d'acétate mercurique, on obtient 1,2 g de N-(chloro-3 benzyl) [(pyrrolidinyl-1)-1 propyl-2]-10 phénothiazinecarboxamide-2,série L, sous la forme d'une meringue jaune. Cette meringue est dissoute dans 20 cm3 d'acétonitrile puis on ajoute 0,8 cm3 d'une solution 2,4 N d'acide chlorhydrique dans l'éther éthylique. Les cristaux sont séparés par filtration, séchés sous pression réduite (5 mm de Hg; 0,67 kPa) à 40°C. On obtient 1,0 g de chlorhydrate de N-(chloro-3 benzyl) [(pyrrolidinyl-1)-1 propyl-2] -10 phénothiazinecarboxamide-2,série L, sous la forme de cristaux blancs fondant à 208°C.

$[\alpha]_D^{20}$ = + 15,7 (c = 1 % ; Méthanol)

RMN du proton (250 MHz, DMSO D6, δ en ppm, J en Hz).

1,77 (D, J = 7, 3H, $-CH_3$) ; 1,7 à 2 (Mt, 4 H, $-CH_2-$ de la pyrrolidine) ; 2,80, 3,08 et 3,45 à 3,9 (3 Mt, respectivement 1H, 1H et 2H, $>N-CH_2-$ de la pyrrolidine) ; 3,75 (Mt, 2H, $>N-CH_2-$) ; 4,48 (D, J = 6, 2H, $-CONHC\underline{H_2}-$) ; 4,7 (Mt, 1H, $>N-CH<$) ; 7 à 7,45 (Mt, 9H, aromatiques) ; 7,57 (S, 1H, -H en 1) ; 7,59 (D, J = 8, 1 H, -H en 3) ; 9,28 (T, J = 6, 1H, -CONH-) ; 10,35 (Mf, 1H, $-NH^+Cl^-$).

Spectre infra-rouge (KBr), bandes caractéristiques en $cm^{-1}$ : 3280, 2970, 2940, 2680, 2480, 1660, 1590, 1570, 1530, 1460, 865, 855, 830, 790, 755, 710.

## EXEMPLE 54

A une solution de 3,70 g de [(pyrrolidinyl-1)-1 propyl-2-(2RS)]-10 phénothiazinecarbothioamide-2 dans 50 cm3 d'éthanol absolu, on ajoute 2,3 cm3 de fluoro-3 benzylamine. Le mélange est saturé d'acide sulfhydrique et porté à 110°C pendant 20 heures. Après refroidissement à 20°C, le mélange est concentré à sec sous pression réduite (30 mm de Hg; 4 kPa) à 40°C et le résidu est purifié par chromatographie sur une colonne (hauteur : 22 cm; diamètre : 4,2 cm) de gel de silice (0,04-0,06 mm) avec une légère surpression d'azote (40 kPa) en éluant par de l'acétate d'éthyle pur (400 cm3) puis par un mélange d'acétate d'éthyle et de méthanol 90-10 (en volumes) (400 cm3) et en recueillant des fractions de 80 cm3. Les fractions 3 à 5 sont réunies et concentrées à sec sous pression réduite (30 mm de Hg; 4 kPa) à 40°C pour donner 2,3 g de N-(fluoro-3 benzyl) [(pyrrolidinyl-1)-1 propyl-2-(2RS)]-10 phénothiazinecarbothioamide-2 sous le forme d'une meringue jaune.

A une solution de 2,3 g de N-(fluoro-3 benzyl) [(pyrrolidinyl-1)-1 propyl-2-(2RS)]-10 phénothiazinecarbothioamide-2 dans 30 cm3 d'éthanol bouillant, on ajoute 0,6 g d'acide fumarique en solution dans 5 cm3 d'éthanol. Après 4 heures de refroidissement à 4°C, le solide cristallisé est séparé par filtration, lavé par 2 fois 5 cm3 d'éthanol froid puis séché à 50°C sous pression réduite (5 mm de Hg; 0,7 kPa) pour donner 2,2 g de fumarate neutre de N-(fluoro-3 benzyl) [(pyrrolidinyl-1)-1 propyl-2-(2RS)]-10 phénothiazinecarbothioamide-2 fondant à 205°C.

RMN du proton (250 MHz, DMSO, δ en ppm, J en Hz).

1,61 (D, J = 7, 3H $-CH_3$); 1,7 (Mt, 4H, $-CH_2-CH_2-$ de la pyrrolidine); 2,65 (Mt, 4H, $-CH_2-\overset{N}{|}-CH_2-$de la pyrrolidine); 3 (DD, J = 12,5 et 6,5, 1H, 1H du $>N-CH_2-$); 3,14 (DD, J = 12,5 et 7, 1H, 1H du $>N-CH_2-$); 4,28 (Mt, J = 7 et 6,5, 1H, $>N-CH<$); 5,02 (D, J = 5, 2H, $-CSNH-C\underline{H_2}-$); 6,6 (S, 1H, -CH=CH- de l'hémifumarate); 6,95 à7,45 (Mt, 9H, aromatiques) ; 7,4 (DD, J = 8 et 1, 1H, -H en 3); 7,67 (D, J = 1, 1H, -H en 1); 10,8 (T, J = 5, 1H,-CSNH-).

Spectre infra-rouge (KBr), bandes caractéristiques en $cm^{-1}$ : 3200, 1610, 1590, 1530, 1485, 1460, 965, 755, 670.

EXEMPLE 55

A une solution de 3,70 g de [(pyrrolidinyl-1)-1 propyl-2-(2RS)]-10 phénothiazinecarbothioamide-2 dans 50 cm3 d'éthanol absolu, on ajoute 6,0 cm3 de méthyl-2 benzylamine. Le mélange est saturé d'acide sulfhydrique et porté à 110°C pendant 20 heures. Après refroidissement à 20°C, le mélange est concentré à sec sous pression réduite (30 mm de Hg; 4 kPa) à 40°C et le résidu est purifié par chromatographie sur une colonne (hauteur : 22 cm; diamètre : 4,2 cm) de gel de silice (0,04-0,06 mm) avec une légère surpression d'azote (40 kPa) en éluant par de l'acétate d'éthyle pur (400 cm3) puis par un mélange d'acétate d'éthyle et de méthanol 90-10 (en volumes) (400 cm3) et en recueillant des fractions de 80 cm3. Les fractions 3 à 5 sont réunies et concentrées à sec sous pression réduite (30 mm de Hg; 4 kPa) pour donner 2,6 g de N-(méthyl-2 benzyl) [(pyrrolidinyl-1)-1 propyl-2-(2RS)]-10 phénothiazinecarbothioamide-2 sous la forme d'une meringue jaune.

A une solution de 2,6 g de N-(méthyl-2 benzyl) [(pyrrolidinyl-1)-1 propyl-2-(2RS)]-10 phénothiazinecarbothioamide-2 dans 30 cm3 d'éthanol bouillant, on ajoute 1,0 g d'acide fumarique en solution dans 20 cm3 d'éthanol. Le mélange est concentré à sec sous pression réduite (30 mm de Hg; 4 kPa) à 40°C et le résidu est repris dans 60 cm3 d'oxyde d'isopropyle. La suspension est agitée pendant 2 heures. Le solide est séparé par filtration, lavé par 2 fois 5 cm3 d'oxyde d'isopropyle puis séché à 35°C sous pression réduite (5 mm de Hg ; 0,7 kPa) pour donner 3,1 g de fumarate acide de N-(méthyl-2 benzyl) [(pyrrolidinyl-1)-1 propyl-2-(2RS)]-10 phénothiazinecarbothioamide-2 fondant à 110°C.

RMN du proton (250 MHz, DMSO, $\delta$ en ppm, J en Hz).

1,67 (D, J = 7,5, 3H, $-CH_3$); 1,77 (Mt, 4H, $-CH_2-CH_2-$ de la pyrrolidine); 2,35 (S, 3H, $-CH_3$ du méthyl-2 benzyle); 2,75 à 3 (Mt, 4H, $-CH_2-\overset{|}{N}-CH_2-$de la pyrrolidine); 3,26 (DD, J = 13,5 et 5,5, 1H, 1H du $>N-CH_2-$); 3,4 (DD, J = 13,5 et 7, 1H, 1H du $>N-CH_2-$); 4,48 (Mt, J = 7,5 - 7 et 5,5, 1H, $>N-CH<$); 4,93 (AB limite, 2H, $-CSNH-C\underline{H_2}-$); 6,63 (S, 2H, -CH=CH- du fumarate); 7 à 7,3 (Mt, 9H, aromatiques) ; 7,43 (DD, J = 8 et 1, 1H, -H en 3); 7,62 (D, J = 1, 1H, -H en 1); 10,75 (T, J = 5,5, 1H, -CSNH-).

Spectre infra-rouge (KBr), bandes caractéristiques en $cm^{-1}$ : 3220, 3100 à 2100, 1685, 1630, 1590, 1530, 1460, 970, 870, 820, 750, 645.

EXEMPLE 56

A une solution de 1,8 g de N-propyl [(dihydro-2,5 pyrrolyl -1)-1 propyl-2]-10 phénothiazinecarbothioamide-2, série L, dans 30 cm3 d'acide acétique, on ajoute goutte à goutte, sous agitation et à une température voisine de 45°C, une solution de 1,46 g d'acétate mercurique dans 30 cm3 d'acide acétique. Après 1 heure, la suspension noire obtenue est filtrée et le filtrat est concentré à sec sous pression réduite (30 mm de Hg; 4 kPa) à 60°C. Le résidu est repris par 60 cm3 d'acétate d'éthyle, lavé successivement par 2 fois 20 cm3 d'une solution aqueuse de soude 2N, puis par 3 fois 20 cm3 d'eau distillée, séché sur sulfate de magnésium, filtré et concentré à sec sous pression réduite (30 mm de Hg; 4 kPa) à 40°C. Le résidu huileux jaune est purifié par chromatographie sur colonne (hauteur: 18 cm; diamètre: 2,8 cm) de gel de silice (0,04-0,063 mm) sous légère surpression d'azote (40kPa) en éluant avec 1 litre d'acétate d'éthyle et en recueillant des fractions de 25 cm3. Les fractions 7 à 25 sont réunies et concentrées à sec sous pression réduite (30 mm de Hg; 4 kPa) à 40°C pour donner 11,09 g d'une laque jaune. Ce produit est dissous dans 5,25 cm3 de propanol-2 à une température voisine de 50°C, et additionné sous agitation d'une solution de 0,31 g d'acide fumarique dans 6,2 cm3 de propanol-2 au reflux. Après refroidissement la solution jaune est concentrée à sec sous pression réduite (30 mm de Hg; 4 kPa) à 40°C et le résidu est repris par 10 cm3 d'acétonitrile. Après 16 heures à une température voisine de 5°C, les cristaux formés sont filtrés, lavés par 2 cm3 d'acétonitrile puis par 3 cm3 d'oxyde de diéthyle et séchés sous pression réduite (5 mm de Hg; 0,67 kPa) à 40°C. On obtient ainsi 0,95 g de fumarate de N-propyl [(dihydro-2,5 pyrrolyl-1)-1 propyl-2]-10 phénothiazinecarboxamide-2, série L, sous forme d'un solide blanc fondant à 160°C.

$[\alpha]_D^{20} = + 21,1 \pm 0,5°$ (1 % ; méthanol)

RMN du proton (250 MHz, DMSO D6, $\delta$ en ppm et J en Hz).

0,9 (T, J = 7,5, 3H, $-(CH_2)_2C\underline{H_3}$) ; 1,54 (Mt, 2H, $-CH_2C\underline{H_2}CH_3$) ; 1,63 (D, J = 7, 3H, $-CH_3$) ; 3,11 (DD, J = 12,5 et 7,5, 1H, 1 H du $>N-CH_2-$) ; 3,23 (Mt, 2H, $-CONHC\underline{H_2}-$) ; 3,29 (DD, J = 12,5 et 6,5, 1H, 1 H du $>N-CH_2-$) ; 3,56 (AB, 4H, $N-CH_2-$ de la pyrroline) ; 4,21 (Mt, J = 7,5, 7 et 6,5, 1H, $>N-CH<$) ; 5,79 (Mf, 2H, -CH=CH- du fumarate) ; 6,9 à 7,3 (Mt, 5H, aromatiques) ; 7,43 (D large, J = 8, 1H, -H en 3) ; 7,54 (S large, 1H, -H en 1) ; 8,45 (T, J = 5,5, 1H, -CONH-).

Spectre infra-rouge (KBr), bandes caractéristiques en $cm^{-1}$ : 2960, 2930, 2870, 2700, 2100, 2100, 1800, 1700, 1635, 1590, 1460, 1550, 980, 890, 830, 755, 645.

EXEMPLE 57

A une solution de 1,20 g d'{[hydroxyméthyl-2-(2S) pyrrolidinyl-1]-1 propyl-2}-10 phénothiazinecarbothioamide-2, série L, dans 20 cm3 d'éthanol absolu, on ajoute 1,74 cm3 de méthyl-3 butylamine. Le mélange est porté à 150°C pendant 16 heures puis concentré à sec sous pression réduite (30 mm de Hg; 4 kPa) à 50°C. Le résidu est dilué par 100 cm3 d'acétate d'éthyle, lavé par 2 fois 50 cm3 d'eau distillée, séché sur sulfate de magnésium puis concentré à sec sous pression réduite (30 mm de Hg; 4 kPa) à 50°C avant d'être purifié par chromatographie sur une colonne (hauteur : 14,5 cm; diamètre : 2,8 cm) de gel de silice (0,04-0,06 mm) avec une légère surpression d'azote (40 kPa) en éluant avec des mélanges de cyclohexane et d'acétate d'éthyle 65-35 (1200 cm3), 35-65 (1,2 litre) (en volumes) en recueillant des fractions de 60 cm3. Les fractions 16 à 33 sont réunies et concentrées à sec sous pression réduite (30 mm de Hg; 4 kPa) à 50°C pour donner 0,34 g d'{[hydroxyméthyl-2-(2S) pyrrolidinyl-1]-1 propyl-2}-10 N-(méthyl-3 butyl) phénothiazinecarboxamide-2, série L, sous la forme d'une laque jaune.

A une solution de 0,25 g d'{[hydroxyméthyl-2-(2S) pyrrolidinyl-1]-1 propyl-2}-10 N-(méthyl-3 butyl) phénothiazinecarboxamide-2, série L, dans 25 cm3 d'éther éthylique, on ajoute 0,25 cm3 d'une solution d'éther chlorhydrique 2,2 N. Un précipité gommeux se dépose sur les parois du ballon. Le mélange est concentré à sec sous pression réduite (30 mm de Hg; 4 kPa) à 50°C. La suspension est agitée pendant 12 heures à 25°C dans 50 cm3 d'éther éthylique. Le précipité est filtré sur verre fritté, lavé par 2 fois 10 cm3 d'éther éthylique et séché à 40°C sous pression réduite (5 mm de Hg; 0,068 kPa) pour donner 0,39 g de chlorhydrate de l'{[hydroxyméthyl-2-(2S) pyrrolidinyl-1]-1 propyl-2}-10 N-(méthyl-3 butyl) phénothiazinecarboxamide-2, série L, fondant à 145-147°C.

RMN du proton (250 MHz, DMSO, δ en ppm, J en Hz).

0,92 (D, J = 6,5, 6H, $-CH(C\underline{H}_3)_2$); 1,45 (Mt, 2H, $-C\underline{H}_2-CH(CH_3)_2$); 1,65 (Mt, 1H, $-C\underline{H}(CH_3)_2$); 1,72 (D, J = 7, 3H, $-CH_3$); 1,7 à 2,2 _Mt, 4H, $-CH_2-CH_2-$ de la pyrrolidine); 3,3 - 3,7 et 4,05 (3Mt, 3H - 5H et 1H respectivement, $-CONH-C\underline{H}_2-$, $>N-CH_2-$, $>NCH_2-$ et $>N-CH<$ de la pyrrolidine, $-CH_2O-$); 4,75 (Mt, 1H, N-CH ); 5,55 (Mf étalé, 1H, -OH); 7 à 7,4 (Mt, 5H, aromatiques); 7,52 (D, J = 8, 1H, -H en 3); 7,55 (S, 1H, -H en 1); 8,67 (T, J = 5,5, 1H, -CONH-); 10,02 (Mf, 1H, $-NH^+$).

Spectre infra-rouge (KBr), bandes caractéristiques en $cm^{-1}$ : 3300, 2960, 2870, 2800 à 2200, 1640, 1595, 1540, 1465, 1415, 1060, 830, 755.

EXEMPLE 58

A une solution 0,05 M d'éthylate de sodium dans 10 cm3 d'éthanol, on ajoute une suspension de 5,4 g de chlorhydrate de cyclopropylméthylamine dispersé dans 25 cm3 d'éthanol contenant 3,43 g de [diméthylamino-1 propyl-2-(2RS)]-10 phénothiazinecarbothioamide-2. Le mélange est porté à 200°C pendant 5 heures puis dilué par 150 cm3 d'acétate d'éthyle, lavé par 2 fois 100 cm3 d'eau distillée, séché sur sulfate de magnésium puis concentré à sec sous pression réduite (30 mm de Hg; 4 kPa) à 50°C. Le solide résiduel est purifié par chromatographie sur une colonne (hauteur : 18 cm; diamètre : 2,5 cm) de gel de silice (0,6-0,02 mm) en éluant avec des mélanges d'acétate d'éthyle et de cyclohexane 30-70 (2,5 litres) et 50-50 (1,1 litre) (en volumes) en recueillant des fractions de 60 cm3. Les fractions 50 à 59 sont réunies et concentrées à sec sous pression réduite (30 mm de Hg; 4 kPa) à 50°C pour donner 0,42 g de N-cyclopropylméthyl [diméthylamino-1 propyl-2-(2RS)]-10 phénothiazinecarboxamide-2 fondant à 90°C.

Spectre de RMN (250 MHz, DMSO, δ en ppm, J en Hz).

0,22 et 0,44 (2Mt, 2H chacun, $-CH_2-CH_2-$ du cyclopropyle); 1,03 (Mt, 1H, $-CH<$ du cyclopropyle); 1,58 (D, J = 7,5, 3H, $-CH_3$); 2,22 (S, 6H, $-N(CH_3)_2$); 2,63 (DD, J = 13 et 7, 1H, 1H du $>N-CH_2-$); 2,85 (DD, J = 13 et 5,5, 1H, 1H du $>N-CH_2-$); 3,14 (T, J = 6,5, 2H, $-CONH-C\underline{H}_2-$); 4,14 (Mt, J = 7,5 - 7 et 5,5, 1H, $>N-CH<$); 6,90 à 7,3 (Mt, 5H, aromatiques); 7,42 (D, J = 8,5, 1H, -H en 3); 7,52 (S, 1H, -H en 1); 8,51 (T, J = 6,5, 1H, -CONH-).

Spectre infra-rouge (KBr), bandes caractéristiques en $cm^{-1}$ : 3230, 2970, 2940, 2860, 2820, 2765, 1630, 1590, 1535, 1460, 1420, 885, 830, 750.

Le [diméthylamino-1 propyl-2-(2RS)]-10 phénothiazinecarbothioamide-2 peut être obtenu de la manière suivante :

Un mélange de 30,9 g de [diméthylamino-1 propyl-2-(2RS)]-10 phénothiazinecarbonitrile-2 et de 1,42 cm3 de triéthylamine dans 310 cm3 de pyridine anhydre est saturé par bullage d'acide sulfhydrique pendant 5 heures à 25°C. La solution limpide obtenue est maintenue sous agitation pendant 12 heures à 25°C, puis le mélange est dégazé par bullage d'azote pendant 90 minutes. Le mélange réactionnel est alors dilué par 500 cm3 d'acétate d'éthyle et lavé par 8 fois 200 cm3 d'eau distillée. La phase organique est séchée sur sulfate de magnésium, filtrée et le filtrat est concentré à sec à 50°C sous pression réduite (30 mm de Hg; 4 kPa). Le résidu

est purifié par chromatographie sur colonne (hauteur : 31 cm; diamètre : 4,5 cm) de gel de silice (0,02-0,6 mm) en éluant par un mélange de cyclohexane et d'acétate d'éthyle 40-60 (en volumes) (7 litres) et en recueillant des fractions de 500 cm3. Les fractions 8 à 10 sont réunies et concentrées à sec à 50°C sous pression réduite (30 mm de Hg; 4 kPa) pour donner 25,4 g de [diméthylamino-1 propyl-2-(2RS)]-10 phénothiazinecarbothioamide-2.

Le [diméthylamino-1 propyl-2-(2RS)]-10 phénothiazinecarbonitrile-2 peut être obtenu de la manière suivante :

Dans un erlenmeyer, on charge une solution de 342 g de chlorhydrate de chloro-2 diméthylamino-1 propane-(2RS) dans 700 cm3 d'eau distillée. On ajoute 270 cm3 d'une solution de soude 10N et le mélange est extrait par 1,3 litre de toluène. La phase organique est séchée sur sulfate de magnésium, filtrée et concentrée à 50°C sous pression réduite (30 mm de Hg; 4 kPa) jusqu'à obtenir un volume résiduel de 800 cm3.

A une suspension de 242 g de cyano-2 phénothiazine dans 2,2 litres de méthyléthylcétone on ajoute 96,7 g de potasse pulvérisée. La température du mélange monte spontanément à 24°C et on porte le mélange à 60°C pendant une demi-heure en agitant. On ajoute en 30 minutes la solution toluénique de chloro-2 diméthylamino-1 propane préparée précédemment. Le mélange est chauffé au reflux pendant 12 heures. Après refroidissement, le mélange est transféré dans une ampoule à décanter et est lavé par 2 fois 2 litres d'eau distillée. La phase organique est séchée sur sulfate de magnésium, filtrée puis concentrée à sec sous pression réduite (30 mm de Hg; 4 kPa) à 50°C pour donner une huile qui est diluée dans 2 litres d'éther éthylique. La solution obtenue est refroidie et amorcée par grattage. Le précipité jaune cristallisé est éliminé par filtration. Les liqueurs-mères sont concentrées à sec sous pression réduite (100 mm de Hg; 13,3 kPa) à 30°C pour donner une huile marron qui est purifiée par chromatographie sur colonne (hauteur : 80 cm; diamètre : 8,5 cm) de silice (0,2-0,06 mm) en éluant par un mélange de cyclohexane et d'acétate d'éthyle 80-20 (en volumes) et en recueillant des fractions de 1 litre. Les fractions 7 à 16 sont réunies et concentrées à sec à 50°C sous pression réduite (30 mm de Hg ; 4 kPa) pour donner 54,8 g de [diméthylamino-1 pr_pyl-2-(2RS)]-10 phénothiazinecarbonitrile-2 sous la forme d'une huile marron.

EXEMPLE 59

On opère comme décrit à l'exemple 56, mais à partir de 1,49 g de N-allyl [(dihydro-2,5 pyrrolyl-1)-1 propyl-2]-10 phénothiazinecarbothioamide-2, série L, dans 25 cm3 d'acide acétique, et d'une solution de 1,16 g d'acétate mercurique dans 25 cm3 d'acide acétique. Le résidu meringué jaune (1,27 g) est purifié par chromatographie sur colonne (hauteur: 23 cm; diamètre: 2,6 cm) de gel de silice (0,04-0,063 mm) sous légère surpression d'azote (40kPa) en éluant avec 1,5 litre d'acétate d'éthyle et en recueillant des fractions de 25 cm3. Les fractions 10 à 58 sont réunies et concentrées à sec sous pression réduite (30 mm de Hg; 4 kPa) à 40°C pour donner 0,45 g d'une laque jaune. 0,3 g de ce produit est dissous dans 1,5 cm3 de propanol-2 à une température voisine de 50°C, et on ajoute sous agitation une solution de 0,09 g d'acide fumarique dans 1,8 cm3 de propanol-2 au reflux. Après refroidissement les cristaux formés sont filtrés, lavés par 10 cm3 d'oxyde de diéthyle et séchés sous pression réduite (5 mm de Hg; 0,67 kPa) à 40°C. On obtient ainsi 0,24 g de fumarate de N-allyl [(dihydro-2,5 pyrrolyl-1)-1 propyl-2]-10 phénothiazinecarboxamide-2, série L, sous forme d'un solide blanc fondant à 164°C.

$[\alpha]_D^{20}$ = + 20,4 ± 0,5° (1 % ; méthanol)

RMN du proton (250 MHz, DMSO D6, δ en ppm, J en Hz).

1,62 (D, J = 7, 3H, $-CH_3$) ; 3,08 (DD, J = 12,5 et 7,5, 1H, 1 H du $>N-CH_2-$) ; 3,26 (DD, J = 12,5 et 6,5, 1H, 1 H du $>N-CH_2-$) ; 3,54 (AB, 4H, $>N-CH_2-$ de la pyrroline) ; 3,91 (DD, J = 6,5 et 5,5, 2H, $-CONHC\underline{H_2}-$) ; 4,19 (Mt, J = 7,5, 7 et 6,5, 1H, $>N-CH<$) ; 5,14 (Mt, 2H, $-CH=C\underline{H_2}$) ; 5,8 (Mf, 2H, -CH=CH- de la pyrroline) ; 5,90 (Mt, 1H, $-C\underline{H}=CH_2$) ; 6,64 (S, 2 H, -CH=CH- du fumarate) ; 6,90 à 7,3 (Mt, 5H, aromatiques) ; 7,47 (DD, J = 8 et 1, 1H, -H en 3) ; 7,57 (D, J = 1, 1H, -H en 1) ; 8,67 (T, J = 5,5, 1H, -CONH-).

Spectre infra-rouge (KBr), bandes caractéristiques en $cm^{-1}$ : 2980, 2930, 2720, 2100, 2100, 1800, 1705, 1640, 1590, 1560, 1460, 1535, 985, 915, 830, 760, 645.

EXEMPLE 60

A une solution de 3,43 g de [diméthylamino-1 propyl-2-(2RS)]-10 phénothiazinecarbothioamide-2 dans 35 cm3 d'éthanol absolu, on ajoute 5 cm3 de benzylamine. Le mélange est porté à 200°C pendant 5 heures, puis à 150°C pendant 48 heures. Il est ensuite dilué par 100 cm3 d'acétate d'éthyle puis lavé par 3 fois 100 cm3 d'eau distillée. La phase organique est séchée sur sulfate de magnésium, filtrée puis concentrée à sec sous pression réduite (30 mm de Hg; 4 kPa) à 50°C. Le résidu est purifié par chromatographie sur une colonne

(hauteur : 50 cm; diamètre : 2,4 cm) de gel de silice (0,06-0,2 mm) en éluant avec un mélange d'acétate d'éthyle et de cyclohexane 50-50 (en volumes) (1,8 litre) et en recueillant des fractions de 60 cm3. Les fractions 21 à 28 sont réunies et concentrées à sec sous pression réduite (30 mm de Hg; 4 kPa) à 50°C pour donner 1,5 g d'une meringue orangée. Une partie aliquote de ce produit (1,4 g) est dissoute dans 30 cm3 d'acétate d'éthyle puis additionnée d'une solution de 0,29 g d'acide oxalique dans 10 cm3 d'acétate d'éthyle. La suspension obtenue est agitée pendant 1 heure, filtrée et le solide est séché à 50°C sous pression réduite (5 mm de Hg; 0,7 kPa) pour donner 1,79 g d'oxalate de N-benzyl [diméthylamino-1 propyl-2-(2RS)]-10 phénothiazinecarboxamide-2 fondant à 105°C.

Spectre de RMN (250 MHz, DMSO, δ en ppm, J en Hz).

1,74 (D, J = 7,5, 3H, $-CH_3$); 2,66 (S, 6H, $-N(CH_3)_2$); 3,34 (DD, J = 14 et 5, 1H, 1H du >N-$CH_2$-); 3,53 (DD, J = 14 et 7, 1H, 1H du >N$CH_2$-); 4,50 (D, J = 6, 2H, -CONH-C$\underline{H}_2$-); 4,58 (Mt, J = 7,5 - 7 et 5,5, 1H, >N-CH<); 7,00 à 7,6 (Mt, 12H, aromatiques); 9,17 (T, J = 6, 1H, -CONH-).

Spectre infra-rouge (KBr), bandes caractéristiques en $cm^{-1}$ : 3300, 2980, 2920, 2660, 1730, 1640, 1595, 1540, 1460, 1415, 870, 830, 750, 700.

## EXEMPLE 61

A une solution de 3,0 g de [diméthylamino-1 propyl-2-(2RS)]-10 phénothiazinecarbothioamide-2 dans 30 cm3 d'éthanol et 3 cm3 d'eau distillée, on ajoute 5,0 cm3 de fluoro-2-benzylamine. Le mélange est porté à 200°C pendant 10 heures. Après refroidissement, le mélange réactionnel est dilué par 150 cm3 d'acétate d'éthyle puis lavé par 3 fois 100 cm3 d'eau distillée. La phase organique est séchée sur sulfate de magnésium, filtrée puis concentrée à sec sous pression réduite (30 mm de Hg; 4 kPa) à 50°C. Le résidu est purifié par chromatographie sur une colonne (hauteur : 25,0 cm; diamètre : 3,5 cm) de gel de silice (0,04-0,06 mm) avec une légère surpression d'azote (40 kPa) en éluant avec des mélanges de cyclohexane et d'acétate d'éthyle 80-20 (2,0 litres) puis 50-50 (3 litres) (en volumes) puis par de l'acétate d'éthyle pur (2,5 litres) en recueillant des fractions de 100 cm3. Les fractions 62 à 71 sont réunies et concentrées à sec sous pression réduite (30 mm de Hg; 4 kPa) à 50°C pour donner 0,7 g d'une meringue orangée. Ce produit est dissous dans 20 cm3 d'oxyde d'isopropyle bouillant et la suspension est portée au reflux pendant 2 heures. Le solide est filtré à chaud, lavé par 2 fois 10 cm3 d'oxyde d'isopropyle puis séché à l'air pour donner 0,5 g de N-(fluoro-2 benzyl) [diméthylamino-1 propyl-2-(2RS)]-10 phénothiazinecarboxamide-2 fondant à 130°C.

Spectre de RMN (250 MHz, DMSO, δ en ppm, J en Hz).

1,59 (D, J = 7,5, 3H, $-CH_3$); 2,2 (S, 6H, $-N(CH_3)_2$); 2,65 (DD, J = 13 et 7, 1H, 1H du >N$CH_2$-); 2,88 (DD, J = 13 et 6, 1H, >N-$CH_2$-); 4,17 (Mt, J = 7,5 - 7 et 6, 1H, >N-CH<); 4,53 (D, J = 5,5, 2H, -CONH-C$\underline{H}_2$-); 6,90 à 7,45 (Mt, 9H, aromatiques); 7,50 (D large, J = 8, 1H, -H en 3); 7,58 (S large, 1H, -H en 1); 9 (T, J = 5,5, 1H, -CONH-).

Spectre infra-rouge (KBr), bandes caractéristiques en $cm^{-1}$ : 3320, 2970, 2940, 2860, 2820, 2765, 1640, 1535, 1590, 1560, 1485, 1460, 1410, 835, 750.

## EXEMPLE 62

A une solution de 6,00 g de [(pyrrolidinyl-1)-1 propyl-2-(2RS)]-10 phénothiazinecarbothioamide-2 dans 60 cm3 d'éthanol absolu, on ajoute 9,2 cm3 de fluoro-2 benzÜlamine. Le mélange est porté à 160°C pendant 30 heures. Après refroidissement, le mélange est concentré à sec sous pression réduite (30 mm de Hg; 4 kPa) à 50°C et le résidu obtenu est ensuite dilué par 200 cm3 d'acétate d'éthyle puis lavé par 2 fois 200 cm3 d'eau distillée. La phase organique est séchée sur sulfate de magnésium, filtrée puis concentrée à sec sous pression réduite (30 mm de Hg; 4 kPa) à 50°C. Le résidu est purifié par chromatographie sur une colonne (hauteur : 22 cm; diamètre : 4,2 cm) de gel de silice (0,04-0,06 mm) avec une légère surpression d'azote (40 kPa) en éluant avec un mélange de cyclohexane et d'acétate d'éthyle 70-30 (6 litres) (en volumes) et en recueillant des fractions de 100 cm3. Les fractions 40 à 53 sont réunies et concentrées à sec à 50°C sous pression réduite (30 mm de Hg; 4 kPa) pour donner une meringue orangée. Ce produit est dissous dans 10 cm3 d'acétate d'éthyle et on verse dans 100 cm3 d'éther de pétrole maintenu sous vive agitation. Le solide est filtré sur verre fritté, lavé avec 2 fois 25 cm3 d'éther de pétrole puis séché à 50°C sous pression réduite (5 mm de Hg; 0,7 kPa) à 50°C pour donner 0,80 g de N-(fluoro-2 benzyl)[(pyrrolidinyl-1)-1 propyl-2-(2RS)]-10 phénothiazinecarboxamide-2 fondant à 60°C.

RMN du proton (250 MHz, DMSO, δ en ppm, J en Hz).

1,6 (D, J = 7, 3H, $-CH_3$); 1,67 (Mt, 4H, $-CH_2$-$CH_2$- de la pyrrolidine); environ 2,52 (Mt masqué, $-CH_2$-N-$CH_2$- de la pyrrolidine); 2,9 (DD, J = 13 et 6,5, 1H, 1H du >N-$CH_2$-); 3,03 (DD, J = 13 et 6, 1H, 1H du >N-$CH_2$-); 4,2 (Mt, J = 7 - 6,5 et 6, 1H, N-CH ); 4,54 (D, J = 5,5, 2H, -CONH-C$\underline{H}_2$-); 6,90 à 7,45 (Mt, 9H, aromatiques); 7,5

(D, J = 8, 1H, -H en 3); 7,61 (S, 1H, -H en 1); 9 (T, J = 5,5, 1H, -CONH-).

Spectre infra-rouge (KBr), bandes caractéristiques en $cm^{-1}$ : 3460, 2975, 2800, 1660, 1595, 1560, 1520, 1490, 1460, 1415, 870, 835.

EXEMPLE 63

A une solution de 3,00 g de [diméthylamino-1 propyl-2-(2RS)]-10 phénothiazinecarbothioamide-2 dans 30 cm3 d'éthanol absolu, on ajoute 5 cm3 de fluoro-4 benzylamine. Le mélange est porté à 170°C pendant 10 heures. Après refroidissement, le mélange est concentré à sec sous pression réduite (30 mm de Hg; 4 kPa) à 50°C et le résidu obtenu est ensuite dilué par 100 cm3 d'acétate d'éthyle puis lavé par 6 fois 100 cm3 d'eau distillée. La phase organique est séchée sur sulfate de magnésium, filtrée puis concentrée à sec sous pression réduite (30 mm de Hg; 4 kPa) à 50°C. Le résidu est purifié par passage sur une colonne (hauteur : 22 cm; diamètre : 4,2 cm) de gel de silice (0,04-0,06 mm) avec une légère surpression d'azote (40 kPa) en éluant avec des mélanges de cyclohexane et d'acétate d'éthyle 60-40 (3 litres), 20-80 (2 litres), puis par de l'acétate d'éthyle pur (3 litres) enfin par un mélange d'acétate d'éthyle et de méthanol 95-5 (2 litres) (en volumes) et en recueillant des fractions de 60 cm3. Les fractions 55 à 80 sont réunies et concentrées à sec sous pression réduite (30 mm de Hg; 4 kPa) pour donner 1,61 g d'une laque jaune. Ce produit est dissous dans 70 cm3 d'éther éthylique et additionné d'une solution d'éther chlorhydrique 2,2 N. La suspension obtenue est agitée à 25°C pendant 2 heures. Le solide est filtré sur verre fritté, lavé avec 2 fois 4 cm3 d'éther éthylique puis séché à 50°C sous pression réduite (5 mm de Hg; 0,7 kPa) pour donner 0,75 g de chlorhydrate de N-(fluoro-4 benzyl) [diméthylamino-1 propyl-2-(2RS)]-10 phénothiazinecarboxamide-2 fondant à 140°C.

Spectre de RMN du proton (250 MHz, DMSO, δ en ppm, J en Hz).

1,77 (D, J = 7, 3H, $-CH_3$); 2,8 (S, 6H, $-N(CH_3)_2$); 3,53 et 3,75 (2Mt, de 1H chacun, $N-CH_2-$); 4,45 (D, J = 6, 2H, $-CONH-C\underline{H_2}-$); 4,72 (Mt, 1H, >N-CH<); 7 à 7,45 (Mt, 9H, aromatiques); 7,6 (Mt, 2H, -H en 1 et -H en 3); 9,3 (T, J = 6, -CONH-); 10,44 (Mf, 1H, $NH^+$).

Spectre infra-rouge (KBr), bandes caractéristiques en $cm^{-1}$ : 3280, 2960, 2930, 2600, 2500, 2460, 1645, 1590, 1530, 1510, 1460, 1415, 875, 835, 755.

EXEMPLE 64

A une solution de 3,00 g de [diméthylamino-1 propyl-2-(2RS)]-10 phénothiazinecarbothioamide-2 dans 30 cm3 d'éthanol contenant 3 cm3 d'eau distillée, on ajoute 3,85 cm3 de furfurylamine. Le mélange est porté à 160°C pendant 30 heures. Après refroidissement, le mélange est concentré à sec sous pression réduite (30 mm de Hg; 4 kPa) à 50°C et le résidu obtenu est ensuite dilué par 100 cm3 d'acétate d'éthyle puis lavé par 2 fois 200 cm3 d'eau distillée. La phase organique est séchée sur sulfate de magnésium, filtrée puis concentrée à sec sous pression réduite (30 mm de Hg; 4 kPa) à 50°C. Le résidu est purifié par chromatographie sur une colonne (hauteur : 22 cm; diamètre : 4,2 cm) de gel de silice (0,04-0,06 mm) avec une légère surpression d'azote (40 kPa) en éluant avec un mélange de cyclohexane et d'acétate d'éthyle 50-50 (2 litres) puis par de l'acétate d'éthyle pur (2,5 litres) enfin par des mélanges d'acétate d'éthyle et de méthanol 98-2 (3,5 litres), 95-5 (1 litre) et 90-10 (1 litre) (en volumes) et en recueillant des fractions de 60 cm3. Après avoir éliminé les cinq premiers litres, les fractions 56 à 85 sont réunies et concentrées à sec à 50°C sous pression réduite (30 mm de Hg; 4 kPa) pour donner 0,7 g d'une laque jaune. Ce produit est dissous dans 60 cm3 d'éther éthylique et additionné d'une solution d'éther chlorhydrique 2,2N. La suspension obtenue est agitée à 25°C pendant 2 heures. Le solide est filtré sur verre fritté, lavé avec 2 fois 20 cm3 d'éther éthylique puis séché à 50°C sous pression réduite (5 mm de Hg; 0,7 kPa) pour donner 0,52 g de chlorhydrate de N-furfuryle [diméthylamino-1 propyl-2-(2RS)]-10 phénothiazine- carboxamide-2 fondant à 134°C.

Spectre de RMN (250 MHz, DMSO, δ en ppm, J en Hz).

1,8 (D, J = 7, 3H, $-CH_3$); 2,77 (S, 6H, $-N(CH_3)_2$); 3,51 (DD large, J = 14 et 4, 1H, 1H du $>NCH_2-$); 3,72 (DD large, J = 14 et 7,5, 1H, 1H du $>NCH_2-$); 4,47 (D, J = 6, 2H, $-CONH-C\underline{H_2}-$); 4,74 (Mt, J = 7,5 - 7 et 4, 1H, >N-CH<); 6,29 (D, J = 3, 1H, =CH-); 6,4 (DD, J = 2,0 et 3, 1H, -O-C=CH- du furyle); 7 à 7,35 (Mt, 5H, aromatiques); 7,58 (Mt, 3H, -H en 1, -H en 3 et -O-CH=); 9,18 (T, J = 6, -CONH-); 10,75 (Mf étalé, 1H, $-NH^+$).

Spectre infra-rouge (KBr), bandes caractéristiques en $cm^{-1}$ : 3270, 2970, 2600, 2500, 2460, 1650, 1590, 1535, 1460, 1415, 870, 830, 755.

EXEMPLE 65

A une solution de 3,0 g de [diméthylamino-1 propyl-2-(2RS)]-10 phénothiazinecarbothioamide-2 dans 30 cm3 d'éthanol et 3 cm3 d'eau distillée, on ajoute 4,5 cm3 de thiényl-2 méthylamine. Le mélange est porté à

200°C pendant 5,5 heures. Le mélange réactionnel est dilué par 100 cm3 d'acétate d'éthyle puis lavé par 3 fois 100 cm3 d'eau distillée. La phase organique est séchée sur sulfate de magnésium, filtrée puis concentrée à sec sous pression réduite (30 mm de Hg; 4 kPa) à 50°C. Le résidu est purifié par chromatographie sur une colonne (hauteur : 25,0 cm; diamètre : 3,5 cm) de gel de silice (0,04-0,06 mm) avec une légère surpression d'azote (40 kPa) en éluant avec des mélanges de cyclohexane et d'acétate d'éthyle 50-50 (2,5 litres), 30-70 (0,5 litre) (en volumes) puis par de l'acétate d'éthyle pur (1,5 litre) en recueillant des fractions de 60 cm3. Les fractions 59 à 79 sont réunies et concentrées à sec à 50°C sous pression réduite (30 mm de Hg; 4 kPa) pour donner 2,0 g d'une meringue orangée. Ce produit est dissous dans 40 cm3 d'oxyde d'isopropyle bouillant et la suspension est portée au reflux pendant 2 heures. Le solide est filtré à chaud, lavé par 2 fois 10 cm3 d'oxyde d'isopropyle puis séché à l'air. Il est ensuite repris dans 30 cm3 de toluène bouillant, filtré à chaud. La cristallisation est amorcée par grattage. Le mélange est laissé sous agitation pendant 24 heures à 5°C puis les cristaux sont filtrés, séchés à 50°C sous pression réduite (5 mm de Hg; 0,7 kPa) pour donner 0,56 g de [diméthylamino-1 propyl-2-(2RS)]-10 N-(thiényl-2 méthyl) phénothi- azinecarboxamide-2 fondant à 135°C.

Spectre de RMN (250 MHz, DMSO, δ en ppm, J en Hz).

1,6 (D, J = 7, 3H, $-CH_3$); 2,20 (S, 6H, $-N(CH_3)_2$); 2,65 (DD, J = 13 et 6,5, 1H, 1H du $>N-CH_2-$); 2,9 (DD, J = 13 et 6, 1H, 1H du$>N-CH_2-$); 4,18 (Mt, J = 7 - 6,5 et 6, 1H, $>CH-N<$); 4,65 (D, J = 5,5, 2H, $-CONHC\underline{H_2}-$); 6,95 à 7,25 (Mt, 7H, aromatiques); 7,4 (DD, J = 5 et 0,5, 1H, =CH-S- du thiophène); 7,45 (DD, J = 8 et 1, 1H, -H en 3); 7,57 (D, J = 1, 1H, -H en 1); 9,14 (T, J = 5,5, 1H, -CONH-).

Spectre infra-rouge (KBr), bandes caractéristiques en $cm^{-1}$ : 3310, 2970, 2930, 2860, 2820, 2760, 1625, 1590, 1545, 1460, 1420, 840, 820, 750, 705.

EXEMPLES 66 A 77

En opérant comme décrit à l'exemple 62 à partir du thioamide de formule générale (II) [X=S] correspondant on obtient l'amide de formule générale (I) [X=O] dont les caractéristiques sont données ci-après :

| Exemple | Produit (I) | Point de fusion |
|---|---|---|
| 66 | 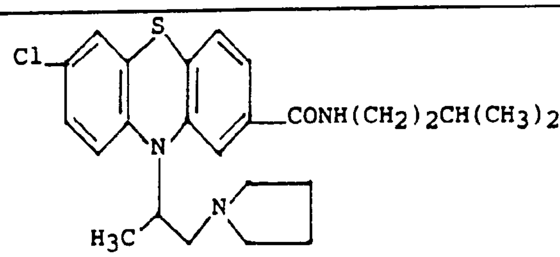 | 246-8°C (chlorhydrate) |
| 67 | 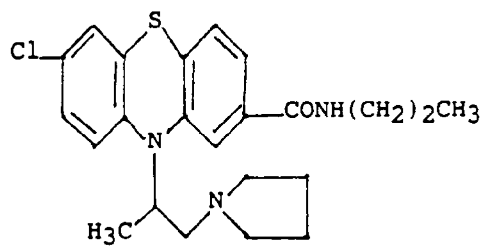 | 90°C (chlorhydrate) |
| 68 | 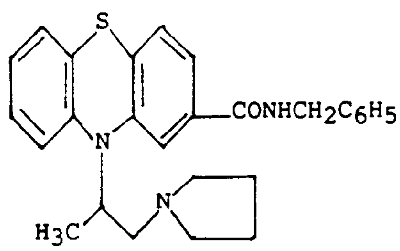 | 196°C (chlorhydrate) |
| 69 | 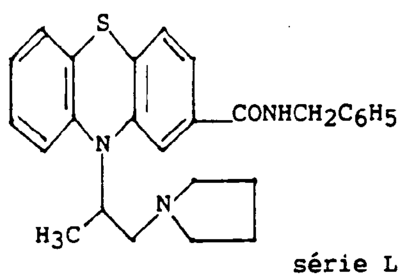 série L | 218°C (chlorhydrate) $[\alpha]_D^{20}$ = +15,8 (méthanol; c = 1,034%) |
| 70 | 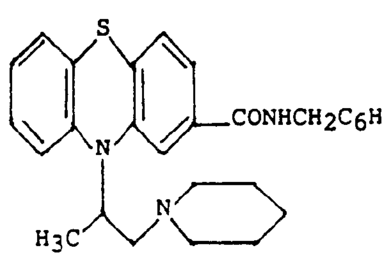 | 220°C (chlorhydrate) |

| Exemple | Produit (I) | Point de fusion |
|---|---|---|
| 71 | 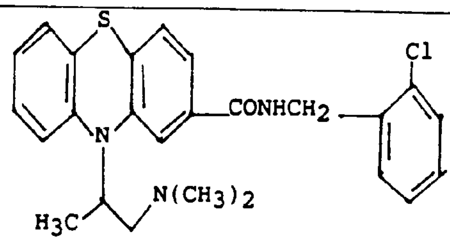 | 158°C (chlorhydrate) |
| 72 | 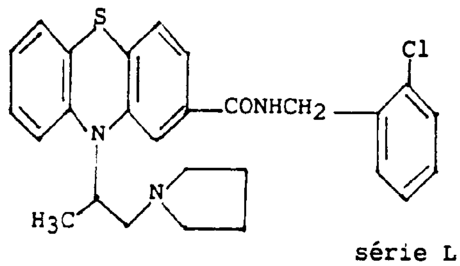 série L | 225°C (chlorhydrate) $[\alpha]_D^{20}$ = + 11,6 (base) (chloroforme; c = 1,002%) |
| 73 | 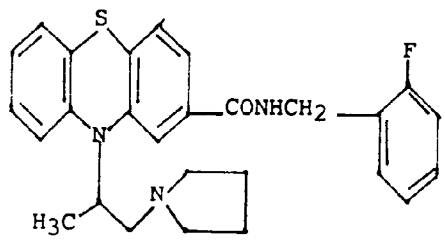 série L | 203°C (chlorhydrate) $[\alpha]_D^{20}$ = + 9,4 (base) (chloroforme; c = 1,001%) |

125

| | | |
|---|---|---|
| 74 | 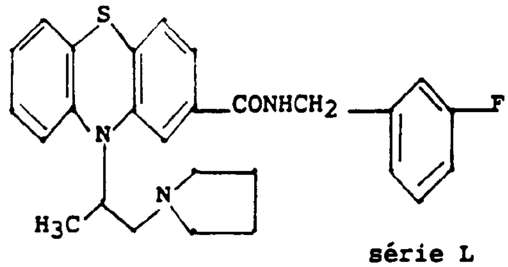<br>série L | environ 70°C |
| 75 | 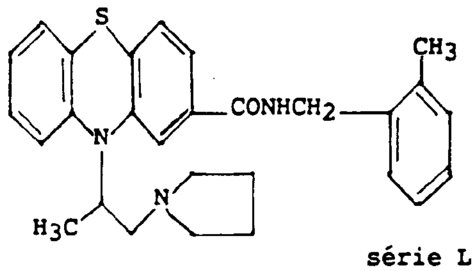<br>série L | environ 125°C (chlorhydrate)<br>$[\alpha]_D^{20} = + 4,8$<br>(DMF; c = 1,017%) |
| 76 | 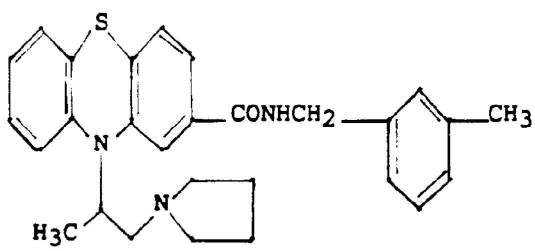 | environ 120°C (fumarate neutre) |
| 77 | 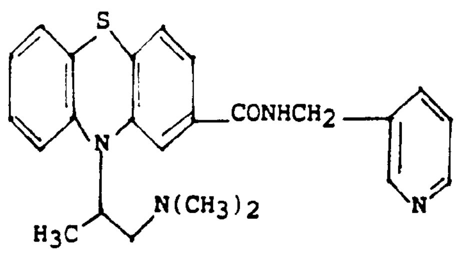 | 50°C |

EXEMPLE 78

A une solution de 4,29 g d'{[hydroxyméthyl-2-(2R) pyrrolidinyl-1]-1 propyl-2}-10 N-(méthyl-3 butyl) phénothiazinecarbothioamide-2, série L, dans 60 cm3 d'acide acétique, on ajoute en 20 minutes 2,91 g d'acétate mercurique en solution dans 60 cm3 d'acide acétique glacial et on agite pendant 45 minutes à une température voisine de 20°C. La suspension noire obtenue est filtrée sur verre fritté colmaté par de la célite et le filtrat jaune est concentré sous pression réduite (30 mm de Hg; 4 kPa) à 40°C. Le résidu est repris par 250 cm3 d'acétate d'éthyle et 50 cm3 d'eau distillée. La phase organique est lavée successivement par 2 fois 100 cm3 de soude normale, 3 fois 100 cm3 d'eau distillée et par 100 cm3 d'une solution aqueuse saturée de chlorure de sodium et séchée sur sulfate de magnésium. Après filtration, le filtrat jaune est concentré sous pression réduite (30 mm de Hg; 4 kPa) à 40°C et on obtient ainsi 4,6 g d'huile jaune brute. Ce résidu est purifié par chromatographie

sur une colonne (hauteur : 25 cm; diamètre : 3,0 cm) de gel de silice (0,04-0,06 mm) en éluant avec de l'acétate d'éthyle pur (3,6 litres) avec une légère surpression d'azote (40 kPa) et en recueillant des fractions de 150 cm3. Les fractions 12 à 24 sont réunies et concentrées à sec sous pression réduite (30 mm de Hg; 4 kPa) à 40°C pour donner 0,89 g d'{[hydroxyméthyl-2-(2R) pyrrolidinyl-1]-1 propyl-2}-10 N-(méthyl-3 butyl) phénothiazinecarboxamide-2, série L, sous forme d'un solide blanc cassé fondant à 140°C.

RMN du proton (250 MHz, DMSO, $\delta$ en ppm, J en Hz).

0,93 (D, J = 6,5, 6H, $-CH(C\underline{H}_3)_2$); 1,45 (Mt, 2H, $-C\underline{H}_2CH(CH_3)_2$); 1,6 (D, J = 7, 3H, $-CH_3$); 1,5 à 1,85 (Mt, 5H, $-CH_2-CH_2-$ de la pyrrolidine et $-C\underline{H}(CH_3)_2$); 2,22 (Mt, 1H, 1H du $>NCH_2-$ de la pyrrolidine); 2,55 (Mt, 1H, $>N-CH<$ de la pyrrolidine); 2,92 (DD, J = 13 et 5,5, 1H, 1H du $>NCH_2-$); 3,12 (Mt, 1H, 1H du $>NCH_2-$ de la pyrrolidine); 3,20 à 3,5 (Mt, 5H, 1H du $>NCH_2-$, $-CH_2O-$et $-CONH-C\underline{H}_2-$); 4,18 (Mt, 1H, $>N-CH<$); 4,46 (Mt, 1H, -OH); 6,9 à 7,3 (Mt, 5H, aromatiques); 7,43 (D, J = 8, 1H, -H en 3); 7,6 (S, 1H, -H en 1); 8,4 (T, J = 6, 1H, -CONH-).

Spectre infra-rouge ($CHCl_3$), bandes caractéristiques en $cm^{-1}$ : 3460, 2960, 2880, 2820, 1650, 1595, 1560, 1520, 1460, 1415, 1040.

EXEMPLE 79

On opère comme à l'exemple 78 mais en recueillant les fractions 25 à 48 de la chromatographie. Ces fractions sont réunies et concentrées à sec sous pression réduite (30 mm de Hg; 4 kPa) à 40°C pour donner 3,17 g d'[(acétoxyméthyl-2-(2R) pyrrolidinyl-1)-1 propyl-2]-10 N-(méthyl-3 butyl) phénothiazinecarboxamide-2, série L, sous forme d'une huile orangée.

A une solution de 2,40 g d'[(acétoxyméthyl-2-(2R) pyrrolidinyl-1)-1 propyl-2]-10 N-(méthyl-3 butyl) phénothiazinecarboxamide-2, série L, dans 14 cm3 d'acétonitrile, on ajoute 2,02 cm3 d'une solution d'éther chlorhydrique 2,2 N. La solution obtenue est versée goutte à goutte dans 400 cm3 d'éther éthylique agité vivement; un précipité se développe et la suspension est agitée pendant 12 heures à 25°C. Le précipité est filtré sur verre fritté, lavé par 3 fois 20 cm3 d'éther éthylique et séché à 40°C sous pression réduite (5 mm de Hg; 0,068 kPa) pour donner 2,17 g de chlorhydrate d'[(acétoxyméthyl-2-(2R) pyrrolidinyl-1)-1 propyl-2]-10 N-(méthyl-3 butyl) phénothiazinecarboxamide-2, série L, fondant à 140°C.

RMN du proton (250 MHz, DMSO, $\delta$ en ppm, J en Hz).

0,91 (D, J = 7, 6H, $-CH(C\underline{H}_3)_2$); 1,43 (Mt, 2H, $-C\underline{H}_2-$ $-CH(CH_3)_2$); 1,6 (Mt, 1H, $-C\underline{H}(CH_3)_2$); 1,85 (D, J = 7, 3H, $-CH_3$); 1,6 à 2,10 (Mt, 4H, $-CH_2-CH_2-$ de la pyrrolidine); 2,06 (S, 3H, $-OCOCH_3$); 3,03 (Mt, 1H, 1H du $>N-CH_2-$ de la pyrrolidine); 3,27 (Mt, 2H, $-CONH-C\underline{H}_2-$); 3,65 à 4 (Mt, 4H, 1H du $>N-CH_2-$ de la pyrrolidine, $>N-CH<$ de la pyrrolidine et $>N-CH_2-$); 4,4 (AB limite, 2H, $-CH_2OCO-$); 4,94 (Mt, 1H, $>N-CH<$); 7 à 7,35 (Mt, 5H, aromatiques); 7,54 (S, 1H, -H en 1); 7,57 (D, J = 8, 1H, -H en 3); 8,7 (T large, J = 6, -CONH-).

Spectre infra-rouge (KBr), bandes caractéristiques en $cm^{-1}$ : 3300, 2960, 2870, 2580, 2500, 1750, 1645, 1595, 1535, 1460, 1230, 1045, 835, 755.

EXEMPLE 80

A une solution de 4,36 g de N-cyclobutylméthyl [(pyrrolidinyl-1)-1 propyl-2]-10 phénothiazinecarbothioamide-2, série L, dans 20 cm3 d'acideçacétique, on ajoute une solution tiède de 3,19 g d'acétate mercurique dans 40 cm3 d'acide acétique. Après agitation à une température voisine de 25°C pendant 2 heures, on filtre le mélange réactionnel et le filtrat est concentré à sec sous pression réduite (30 mm de Hg; 4 kPa) à 40°C. Le résidu est repris par 100 cm3 d'acétate d'éthyle, lavé successivement par 2 fois 50 cm3 d'une solution aqueuse 4N de soude puis par 3 fois 50 cm3 d'eau distillée. La phase organique est séchée sur sulfate de magnésium, filtrée et concentrée à sec sous pression réduite (30 mm de Hg ; 4 kPa) à 40°C pour donner un miel jaune clair (2,65 g)qui est purifié par chromatographie sur une colonne (hauteur: 38 cm; diamètre: 2 cm) de gel de silice (0,06-0,2 mm) en éluant par 900 cm3 d'acétate d'éthyle et en recueillant des fractions de 60 cm3. Les fractions 3 à 12 sont réunies et concentrées à sec sous pression réduite ( 30 mm de Hg ; 4 kPa) pour donner 1,89 g de N-cyclobutylméthyl [(pyrrolidinyl -1)-1 propyl-2]-10 phénothiazinecarboxamide-2, série L.

$[\alpha]_D^{20} = + 22 \pm 0,6°$ (c = 1 % ; Méthanol)

On laisse refroidir une solution bouillante de 1,7 g de N-cyclobutylméthyl [(pyrrolidinyl-1)-1 propyl-2]-10 phénothiazinecarboxamide-2, série L, dans 17 cm3 de propanol-2 contenant 0,47 g d'acide fumarique. Le mélange est concentré sous pression réduite (30 mm de Hg; 4 kPa) à 40°C pour donner un résidu qui est repris par 100 cm3 d'oxyde d'isopropyle. Le mélange est agité pendant une heure à 20°C. La suspension obtenue est filtrée et le solide est lavé par 2 fois 5 cm3 d'oxyde d'isopropyle et séché sous pression réduite (5 mm de Hg; 0,67 kPa) à 50°C pour donner une poudre crème qui est dissoute dans 25 cm3 d'acétone au reflux. La cristallisation se développe. Le mélange est agité pendant 16 heures à 20°C et les cristaux sont essorés et

séchés sous pression réduite ( 5 mm de Hg; 0,67kPa) à 50°C. On obtient ainsi 0,64 g de fumarate de N-cyclobutylméthyl [(pyrrolidinyl-1)-1 propyl-2]-10 phénothiazinecarboxamide-2, série L, sous la forme de cristaux crème fondant à 126-128°C.

$[\alpha]_D^{20}$ = + 12,4 (c = 0,8 %; Méthanol)

RMN du proton (200 MHz, DMSO D6, δ en ppm, J en Hz).

1,68 (D, J = 7, 3H, $-CH_3$) ; 1,77 (Mt, 4H, $-CH_2-$ de la pyrrolidine) ; 1,6 à 2,05 (Mt, 6H, $-CH_2-$ du cyclobutylméthyle) ; 2,5 (Mt, en partie masqué, >CH-du cyclobutylméthyle) ; 2,85 et 3 (2 Mt, respectivement 2H chacun, >N-$CH_2$- de la pyrrolidine) ; 3,20 à 3,25 (Mt, 4H, >N-$CH_2$- et -CONHC$\underline{H_2}$-) ; 4,46 (Mt, 1H, >N-CH< ) ; 6,62 (S, 2 H, -CH=CH- du fumarate) ; 6,95 à 7,3 (Mt, 5H, aromatiques) ; 7,47 (D, J = 8, 1H, -H en 3) ; 7,51 (S, 1H, -H en 1) ; 8,5 (T, J = 5,5 , 1 H, -CONH-).

Spectre infra-rouge (KBr), bandes caractéristiques $cm^{-1}$ : 3320, 2975, 2930, 2860, 2750, 2000, 2000, 1800, 1710, 1640, 1595, 1465, 1560, 980, 835, 750, 640.

## EXEMPLE 81

A une solution de 1,5 g de fumarate neutre de N-benzyl [N'-éthyl N'-(hydroxy-2 éthyl)amino-1 propyl-2-(2RS)]-10 phénothiazinecarbothioamide-2 dans 25 cm3 d'acide acétique, on ajoute en 20 minutes, 0,9 g d'acétate mercurique et on agite le mélange obtenu pendant 12 heures à une température voisine de 20°C. La suspension noire obtenue est filtrée sur verre fritté colmaté par de la célite et le filtrat jaune est concentré sous pression réduite (30 mm de Hg; 4 kPa) à 40°C. Le résidu est repris par 100 cm3 d'acétate d'éthyle et la phase organique est lavée successivement par 2 fois 20 cm3 de soude N, 2 fois 20 cm 3 d'eau distillée et par 20 cm3 d'une solution aqueuse saturée de chlorure de sodium et séchée sur sulfate de magnésium. Après filtration, le filtrat jaune est concentré à sec sous pression réduite (30 mm de Hg; 4 kPa) à 40°C et on obtient ainsi 1,0 g d'une huile jaune qui est purifiée par chromatographie sur colonne (hauteur : 30 cm; diamètre: 1,5 cm) de silice (0,06-0,2 mm) en éluant par de l'acétate d'éthyle pur (500 cm3) et en recueillant des fractions de 40 cm3. Les fractions 5 à 8 sont réunies et concentrées à sec sous pression réduite (30 mm de Hg ; 4 kPa) à 40°C. On obtient 0,8 g d'une huile jaune qui est dissoute dans 3 cm3 d'éthanol bouillant; on ajoute 0,2 g d'acide fumarique et la solution est concentrée à sec sous pression réduite (30 mm de Hg; 4 kPa) à 40°C pour donner 1,0 g de fumarate d'acétate de [(benzylcarbamoyl-2 phénothiazinyl-10)-2 N-éthyl propylamino-(2RS)]-2 éthyle sous la forme d'une poudre jaune fondant à environ 70°C.

RMN du proton (250 MHz, DMSO, δ en ppm, J en Hz).

0,85 (T, J = 7, 3H, $-NCH_2C\underline{H_3}$); 1,65 (D, J = 7, 3H, $-CH_3$); 1,90 (S, 3H, $-OCOCH_3$); 2,52 (Mt, >N-$C\underline{H_2}CH_3$); 2,66 (T, J = 6, 2H, >N-$C\underline{H_2}$-$CH_2OCOCH_3$); 2,78 (DD, J = 13 et 6, 1H, 1H du >N-$CH_2$-); 3,09 (DD, J = 13 et 7,5, 1H, 1H du N-$CH_2$-); 3,93 (T, J = 6, 2H, -$C\underline{H_2}$OCO-); 4,15 (Mt, J = 7,5 - 7 et 6, 1H, >N-CH<); 4,50 (D, J = 6, 2H, -CSNH-$C\underline{H_2}$-); 6,64 (S, 2H, -CH=CH- du fumarate); 6,9 à 7,4 (Mt, 10H, aromatiques); 7,48 (D large, J = 8, 1H, -H en 3); 7,58 (S large, 1H, -H en 1); 9,05 (T, J = 6, 1H, -CSNH-).

Spectre infra-rouge (KBr), bandes caractéristiques en $cm^{-1}$ : 2980, 2700 à 2100, 1740, 1710, 1640, 1590, 1460, 1235, 980, 825, 750, 700, 635.

## EXEMPLE 82

A une solution de 1,30 g de fumarate neutre de N-(fluoro-3 benzyl) [(pyrrolidinyl-1)-1 propyl-2-(2RS)]-10 phénothiazinecarbothioamide-2 dans 20 cm3 d'acide acétique, on ajoute 0,80 g d'acétate mercurique et on agite le mélange obtenu pendant 20 heures à une température voisine de 20°C. La suspension noire obtenue est filtrée sur verre fritté colmaté par de la célite et le filtrat jaune est concentré sous pression réduite (30 mm de Hg; 4 kPa) à 40°C. Le résidu est repris par 100 cm3 d'acétate d'éthyle et la phase organique est lavée successivement par 2 fois 20 cm3 de soude normale, 2 fois 20 cm3 d'eau distillée et par 20 cm3 d'une solution aqueuse de chlorure de sodium et séchée sur sulfate de magnésium. Après filtration, le filtrat jaune est concentré sous pression réduite (30 mm de Hg; 4 kPa) à 40°C et on obtient ainsi 0,90 g d'une huile jaune brute. Ce résidu est dissous dans 3 cm3 d'éthanol chaud et additionné de 0,25 g d'acide fumarique en solution dans 2 cm3 d'éthanol. Après 4 heures de refroidissement à 4°C, le solide cristallisé est séparé par filtration, lavé par 2 fois 5 cm3 d'éthanol froid puis séché à 50°C sous pression réduite (5 mm de Hg; 0,7 kPa) pour donner 0,7 g de fumarate neutre de N-(fluoro-3 benzyl) [(pyrrolidinyl-1)-1 propyl-2-(2RS)]-10 phénothiazinecarboxamide-2 fondant à 165°C.

RMN du proton (250 MHz, DMSO, δ en ppm, J en Hz).

1,62 (D, J = 7,5, 3H, $-CH_3$); 1,7 (Mt, 4H, $-CH_2-CH_2-$ de la pyrrolidine); 2,62 (Mt, 4H, $-CH_2-\overset{N}{|}-CH_2-$ de la pyrro-

lidine); 3,01 (DD, J = 13,5 et 7, 1H, 1H du >N-$CH_2$-); 3,12 (DD, J = 13,5 et 6, 1H, 1H du>N-$CH_2$-); 4,27 (Mt, J = 7,5 - 7 et 6, 1H, >N-CH<); 4,51 (D, J = 5,5, 2H, -CONH-$CH_2$-); 6,57 (S, 1H, -CH=CH- de l'hémifumarate); 6,90 à 7,45 (Mt, 9H, aromatiques); 7,5 (D large, J = 8, 1H, -H en 3); 7,6 (S large, 1H, -H en 1); 8,09 (T, J = 5,5, 1H, -CONH-).

Spectre infra-rouge (KBr), bandes caractéristiques en $cm^{-1}$ : 3240, 2970, 2915, 2500 à 2000, 1655, 1610, 1590, 1540, 1485, 1460, 820, 785, 760, 750, 670.

EXEMPLE 83

A une solution de 2,0 g de fumarate acide de N-(méthyl-2 benzyl) [(pyrrolidinyl-1)-1 propyl-2-(2RS)]-10 phénothiazinecarbothioamide- 2 dans 28 cm3 d'acide acétique, on ajoute 1,1 g d'acétate mercurique et on agite le mélange obtenu pendant 20 heures à une température voisine de 20°C. La suspension blanche obtenue est filtrée sur verre fritté colmaté par de la célite et le filtrat jaune est concentré sous pression réduite (30 mm de Hg; 4 kPa) à 40°C. Le résidu est repris par 100 cm3 d'acétate d'éthyle et la phase organique est lavée successivement par 2 fois 20 cm3 de soude normale, 2 fois 20 cm3 d'eau distillée et par 20 cm3 d'une solution aqueuse de chlorure de sodium et séchée sur sulfate de magnésium. Après filtration, le filtrat jaune est concentré sous pression réduite (30 mm de Hg; 4 kPa) à 40°C et on obtient ainsi 1,0 g d'une huile jaune. Ce produit est purifié par chromatographie sur une colonne (hauteur : 22 cm; diamètre : 4,2 cm) de gel de silice (0,04-0,06 mm) avec une légère surpression d'azote (40 kPa) en éluant par un mélange de cyclohexane et d'acétate d'éthyle 20-80 (1000 cm3) et en recueillant des fractions de 50 cm3. Les fractions 8 à 20 sont réunies et concentrées sous pression réduite (30 mm de Hg; 4 kPa) à 40°C pour donner 0,6 g de N-(méthyl-2 benzyl) [(pyrrolidinyl-1)-1 propyl-2-(2RS)]-10 phénothiazinecarboxamide-2 sous le forme d'une huile jaune. Ce produit est dissous dans 20 cm3 de chlorure de méthylène et on ajoute 1 cm3 d'acide chlorhydrique en solution 3,4 N dans l'oxyde d'isopropyle. Le mélange est concentré à sec sous pression réduite (30 mm de Hg; 4 kPa) à 40°C et le résidu est repris par 20 cm3 d'éther éthylique. La suspension est agitée 2 heures à 25°C et le solide est séparé par filtration, lavé par 2 fois 5 cm3 d'éther puis séché à 30°C sous pression réduite (5 mm de Hg; 0,7 kP) pour donner 0,5 g de chlorhydrate de N-(méthyl-2 benzyl) [(pyrrolidinyl-1)-1 propyl-2-(2RS)]-10 phénothiazinecarboxamide-2 fondant à 90°C.

RMN du proton (250 MHz, DMSO, δ en ppm, J en Hz).

1,8 (D, J = 7, 3H, -$CH_3$); 1,7 à 2 (Mt, 4H, -$CH_2$-$CH_2$- de la pyrrolidine); 2,35 (S, 3H, -$CH_3$ du méthyl-2 benzyle); 2,85 -3,12 - 3,60 et 3,80 (4Mf, de 1H chacun, -$CH_2$-N-$CH_2$- de la pyrrolidine); 3,7 (DD large, J = 13 et 5, 1H, 1H du >$NCH_2$-); 3,85 (DD large, J = 13 et 7,5, 1H, 1H du >N-$CH_2$-); 4,48 (AB limite, 2H, -CONH-$CH_2$-); 4,75 (Mt, J = 7,5 - 7 et 5, 1H, >N-CH<); 7 à 7,3 (Mt, 8H, aromatiques); 7,35 (D, J = 8, 1H, -H en 4); 7,58 (S large, 1H, -H en 1); 7,63 (D large, J = 8, 1H, -H en 3); 9,15 (T, J = 5,5, 1H, -CONH-); 10,6 (Mf, 1H, -$NH^+$).

Spectre infra-rouge (KBr), bandes caractéristiques en $cm^{-1}$ : 3270, 2975, 2580, 2480, 1650, 1595, 1535, 1460, 1415, 870, 830, 750.

EXEMPLE 84

A 35 cm3 d'une solution éthanolique de 2,8 g de [diméthylamino-1 propyl-2-(2RS)]-10 phénothiazinecarbothioamide-2, on ajoute 9,3 g de diamino-1,2 cyclohexane. Le mélange est chauffé en autoclave à 160 °C pendant 4,5 heures. Le mélange réactionnel est dilué par 100 cm3 d'acétate d'éthyle et lavé par 3 fois 100 cm3 d'eau distillée. La phase organique est décantée, séchée sur sulfate de magnésium, filtrée et évaporée à sec sous pression réduite (30 mm de mercure; 4 kPa) à 40°C. Le résidu est purifié par chromatographie sur une colonne (hauteur : 22 cm; diamètre : 4 cm) de gel de silice (0,04-0,06 mm) avec une légère surpression d'azote (40 kPa) en éluant par du dichloro-1,2 éthane (1 litre) des mélanges de dichloro-1,2 éthane et de méthanol : 90-10 (3 litres), 80-20 (2 litres), 70-30 (2 litres), 50-50 (1 litre) et par du méthanol pur (1 litre) (en volumes) et en recueillant des fractions de 125 cm3. Les fractions 63 à 80 contenant le produit pur sont réunies et concentrées à sec sous pression réduite (30 mm de mercure; 4 kPa) à 40°C pour donner 1,2 g de [diméthylamino-1 propyl-2-(2RS)]-10 (hexahydro-3a,4,5,6,7,7a 1H-benzimidazolyl-2)-2 phénothiazine sous la forme d'une meringue jaune.

Le produit obtenu est repris dans 80 cm3 d'éther éthylique bouillant, et additionné goutte à goutte d'une solution d'éther chlorhydrique 1,4N sous forte agitation jusqu'à fin de précipitation.

La suspension obtenue est agitée pendant une heure. Le précipité est filtré et le gâteau est lavé par 2 fois 20 cm3 d'oxyde d'éthyle, puis séché à 40°C sous pression réduite (10 mm de mercure; 1,3 kPa). On obtient ainsi 1,05 g de dichlorhydrate de [diméthylamino-1 propyl-2-(2RS)]-10 (hexahydro-3a,4,5,6,7,7a 1H-benzimidazolyl-2)-2 phénothiazine fondant à 175°C.

RMN du proton (250 MHz, DMSO, δ en ppm, J en Hz).

1,45 et 1,85 (2Mt, 8H, $-CH_2-$ de l'hexahydrobenzimidazole); 1,82 (D, J = 7, 3H, $-CH_3$); 2,89 (2D, 6H, $-N(CH_3)_2$); 3,85 (AB limite large, 2H, $>N-CH_2-$); 4,32 (AB limite, 2H, -CH de l'hexahydrobenzimidazole); 4,78 (Mt, 1H, >N-CH<); 7,10 à 7,45 (Mt, 4H, aromatiques); 7,54 (D, J = 8, 1H, -H en 4); 7,77 (Mt, 2H, -H en 1 et -H en 3); 10,70 (Mt, 1H, $-NH^+$).

Spectre infra-rouge (KBr), bandes caractéristiques en $cm^{-1}$ : 3060, 2940, 2670, 1605, 1575, 1465, 870, 830, 755.

EXEMPLE 85

A 32 cm3 d'une solution éthanolique de 2,96 g de [(pyrrolidinyl)-1 propyl-2-(2RS)]-10 phénothiazinecarbothioamide-2, on ajoute 9,12 g de diamino-1,2 cyclohexane. Le mélange est chauffé en autoclave à 150°C pendant 10 heures. Le mélange réactionnel est dilué par 150 cm3 d'acétate d'éthyle et lavé par 4 fois 100 cm3 d'eau distillée. La phase organique est décantée, séchée sur sulfate de magnésium, filtrée et concentrée à sec sous pression réduite (30 mm de mercure; 4 kPa) à 40°C. Le résidu est purifié par chromatographie sur une colonne (hauteur : 23 cm; diamètre : 2,5 cm) d'alumine neutre FLUKA type 507 C en éluant par des mélanges de cyclohexane et d'acétate d'éthyle : 50 - 50 (en volumes) (1 litre), et en recueillant des fractions de 50 cm3. Les fractions 2 à 15 contenant le produit pur sont réunies et concentrées à sec sous pression réduite (30 mm de mercure; 4 kPa) à 40°C pour donner 1,07 g de (hexahydro-3a,4,5,6,7,7a 1H-benzimidazolyl-2)-2 [(pyrrolidinyl)-1 propyl-2-(2RS)]-10 phénothiazine sous la forme d'une meringue marron.

Le produit est dissous dans 50 cm3 d'hexane au reflux; la cristallisation est amorcée par grattage. Le mélange est mis sous agitation à 0-5°C pendant 12 heures, puis les cristaux sont filtrés et séchés sous pression réduite (10 mm de mercure; 1,4 kPa) à 40°C pendant 4 heures. On obtient ainsi 0,62 g de (hexahydro-3a,4,5,6,7,7a 1H-benzimidazolyl-2)-2 [(pyrrolidinyl)-1 propyl-2-(2RS)]-10 phénothiazine à l'état de cristaux crème fondant à 109°C.

Spectre de RMN (250 MHz, DMSO, δ en ppm, J en Hz).

1,2 à 1,8 (Mt, 12H, $-CH_2-$ de l'hexahydrobenzimidazole et $-C\underline{H}_2-C\underline{H}_2-$ de la pyrrolidine); 1,59 (D, J = 7, 3H, $-CH_3$); 2,50 (Mt masqué, 4H, $>N-CH_2-$ de la pyrrolidine); 2,87 (DD, J = 13 et 6,5, 1H, 1H du $>NCH_2-$); 2,98 (DD, J = 13 et 6, 1H, 1H du $>NCH_2-$); 3,7 (Mf, 2H, -CH< de l'hexahydrobenzimidazole); 4,15 (Mt, J = 7 - 6,5 et 6, 1H, >CH-N<); 6,9 à 7,25 (Mt, 5H, aromatiques); 7,35 (DD, J = 8 et 1, 1H, -H en 3); 7,6 (D, J = 1, 1H, -H en 1).

Spectre infra-rouge ($CHCl_3$), bandes caractéristiques en $cm^{-1}$ : 3400, 2940, 2860, 2810, 1615, 1590, 1560, 1470, 1445, 830.

EXEMPLE 86

A 32 cm3 d'une solution éthanolique de 2,96 g de [(pyrrolidinyl-1)-1 propyl-2]-10 phénothiazinethiocarboxamide-2, série D, on ajoute 9,12 g de diamino-1,2-cyclohexane. Le mélange est chauffé en autoclave à 140 °C pendant 18 heures, puis dilué par 150 cm3 d'acétate d'éthyle et lavé par 4 fois 70 cm3 d'eau distillée. La phase organique est décantée, séchée sur sulfate de magnésium, filtrée et concentrée à sec sous pression réduite (30 mm de mercure; 4 kPa) à 40°C. Le résidu est purifié par chromatographie sur une colonne (hauteur : 23,5 cm; diamètre : 2,5 cm) d'alumine neutre en éluant par des mélanges de cyclohexane et d'acétate d'éthyle : 75 - 25 (250 cm3), 50-50 (1250 cm3) (en volumes) et en recueillant des fractions de 50 cm3. Les fractions 4 à 20 cont_nant le produit pur sont réunies et concentrées à sec sous pression réduite (30 mm de mercure; 4 kPa) à 40°C pour donner 0,90 g d'un produit contenant le produit attendu. Le lot obtenu est dissous dans 25 cm3 d'oxyde d'isopropyle bouillant; la solution est filtrée et on ajoute 25 cm3 d'hexane à la solution chaude. La cristallisation est amorcée par grattage et les cristaux sont filtrés sur verre fritté pour donner 0,48 g de (hexahydro-3a,4,5,6,7,7a 1H-benzimidazolyl-2)-2 [(pyrrolidinyl-1)-1 propyl-2]-10 phénothiazine, série D, sous la forme de cristaux blancs fondant à 108-110°C, et dont le spectre de RMN est identique à celui du produit obtenu à l'exemple 85.

$[\alpha]_D^{20}$ = +7,5 (chloroforme; c = 0,868%).

Spectre infra-rouge (KBr), bandes caractéristiques en $cm^{-1}$ : 3170, 2925, 2850, 2795, 1600, 1575, 1550, 1450, 875, 825, 750.

EXEMPLE 87

A 32 cm3 d'une solution éthanolique de 2,96 g de [(pyrrolidinyl-1)-1 propyl-2]-10 phénothiazinecarbothioamide-2, série L, on ajoute 7,98 g de diamino-1,2 cyclohexane. Le mélange est chauffé en autoclave à 140 °C

pendant 18 heures puis dilué par 150 cm3 d'acétate d'éthyle et lavé par 3 fois 50 cm3 d'eau distillée. La phase organique est décantée, séchée sur sulfate de magnésium, filtrée et concentrée à sec sous pression réduite (30 mm de mercure; 4 kPa) à 40°C. Le résidu est purifié par chromatographie sur une colonne (hauteur : 24 cm; diamètre : 2,5 cm) d'alumine neutre en éluant par des mélanges de cyclohexane et d'acétate d'éthyle : 75 - 25 (500 cm3), 50 - 50 (750 cm3) et 25 - 75 (750 cm3) (en volumes) et en recueillant des fractions de 50 cm3. Les fractions 10 à 29 contenant le produit pur sont réunies et concentrées à sec sous pression réduite (30 mm de mercure ; 4 kPa) à 40°C pour donner 0,38 g d'(hexahydro-3a,4,5,6,7,7a 1H-benzimidazolyl-2)-2 [(pyrrolidinyl-1)-1 propyl-2]-10 phénothiazine, série L, sous la forme d'une meringue marron.

A une solution de 0,34 g d'(hexahydro-3a,4,5,6,7a 1H-benzimidazolyl-2)-2 [(pyrrolidinyl-1)-1 propyl-2]-10 phénothiazine, série L, dans 34 cm3 d'éther éthylique bouillant, on ajoute goutte à goutte 1,13 cm3 d'éther chlorhydrique 1,4N sous forte agitation. La suspension obtenue est agitée pendant une heure. Le précipité est filtré et le gâteau est lavé par 2 fois 10 cm3 d'éther éthylique, puis séché à 40°C sous pression réduite (10 mm de mercure; 1,3 kPa). On obtient ainsi 0,18 g de dichlorhydrate d'(hexahydro-3a,4,5,6,7,7a 1H-benzimidazolyl-2)-2 [(pyrrolidinyl-1)-1 propyl-2]-10 phénothiazine, série L, fondant à 198-9°C, dont les caractéristiques RMN sont identiques à celles du produit obtenu à l'exemple 85.

Spectre infra-rouge (KBr), bandes caractéristiques en $cm^{-1}$ : 2940, 2680, 1605, 1575, 1540, 1465, 865, 825, 755.

EXEMPLE 88

A 70 cm3 d'une solution éthanolique de 5,20 g de chloro-7 [(pyrrolidinyl-1)-1 propyl-2-(2RS)]-10 phénothiazinecarbothioamide-2, on ajoute 15,78 cm3 de diamino-1,2 cyclohexane. Le mélange est chauffé en autoclave à 150°C pendant 17 heures. Après refroidissement, le mélange réactionnel est dilué par 250 cm3 d'acétate d'éthyle et lavé par 5 fois 250 cm3 d'eau distillée. La phase organique est extraite par 300 cm3 d'une solution normale d'acide chlorhydrique. Après décantation de la phase organique, la phase aqueuse acide est alcalinisée avec de la lessive de soude concentrée, extraite par 200 cm3 d'acétate d'éthyle. La phase organique est décantée, séchée sur sulfate de magnésium, filtrée et évaporée sous pression réduite (30 mm de mercure; 4 kPa) à 40°C. Le résidu est chromatographié sur une colonne (hauteur: 52 cm; diamètre : 2,5 cm) de gel de silice (0,2 - 0,06 mm) en éluant par de l'acétate d'éthyle pur (2 litres) et en recueillant des fractions de 120 cm3. Le premier litre est éliminé et les fractions 4 à 7 contenant le produit pur sont réunies et concentrées à sec sous pression réduite (30 mm de mercure; 4 kPa) à 40°C pour donner 0,72 g de chloro-7 (hexahydro-3a,4,5,6,7,7a 1H-benzimidazolyl-2)-2 [(pyrrolidinyl-1)-1 propyl-2-(2RS)]-10 phénothiazine sous la forme d'un solide jaune fondant à 120°C.

RMN du proton (250 MHz, DMSO, δ en ppm, J en Hz).

1,25 à 1,9 (Mt, 12H, $-CH_2-$ de l'hexahydrobenzimidazole et de la pyrrolidine); 1,62 (D, J = 7,5, 3H, $-CH_3$); 2,82 (DD, J = 13 et 6, 1H, 1H du $>N-CH_2-$); 3 (DD, J = 13 et 7, 1H, 1H du $N-CH_2-$); 4 (Mt, 2H, $>CH-CH<$ de l'hexahydrobenzimidazole); 4,19 (Mt, J = 7,5, 7 et 6, 1H,$>N-CH<$); 7,10 à 7,35 (Mt, 4H, aromatiques); 7,45 (DD, J = 8 et 1, 1H, -H en 3); 7,55 (D, J = 1, 1H, -H en 1).

Spectre infra-rouge (KBr), bandes caractéristiques en $cm^{-1}$ : 2920, 2860, 2790, 1605, 1535, 1460, 865, 805.

Le chloro-7 [(pyrrolidinyl-1)-1 propyl-2-(2RS)]-10 phénothiazinecarbothioamide-2 peut être obtenu de la manière suivante :

Un mélange de 31,07 g de chloro-7 [(pyrrolidinyl-1)-1 propyl-2-(2RS)]-10 phénothiazinecarbonitrile-2 et de 11,8 cm3 de triéthylamine dans 300 cm3 de pyridine anhydre est saturé d'acide sulfhydrique pendant 4 heures à 25°C. La solution limpide obtenue est maintenue sous agitation pendant 12 heures à 25°C. Le mélange est dégazé par bullage d'azote pendant 2 heures, puis dilué par 250 cm3 d'acétate d'éthyle et lavé par 5 fois 200 cm3 d'eau distillée. La phase organique est séchée sur sulfate de magnésium, filtrée et le filtrat est concentré à sec sous pression réduite (30 mm de Hg; 4 kPa). Le résidu est purifié par chromatographie sur colonne (hauteur : 38 cm; diamètre : 6,5 cm) de gel de silice (0,06-0,2 mm) en éluant par un mélange de cyclohexane et d'acétate d'éthyle 70-30 (6 litres), 50-50 (4 litres), 30-70 (4 litres) (en volumes) puis par de l'acétate d'éthyle pur (2 litres) et enfin par un mélange d'acétate d'éthyle et de méthanol 90-10 (6 litres) en recueillant des fractions de 50 cm3. Les 13,3 premiers litres sont éliminéss et les fractions 6 à 94 sont réunies et concentrées à sec sous pression réduite (30 mm de Hg; 4 kPa) à 50°C pour donner 21,43 g de chloro-7 [(pyrrolidinyl-1)-1 propyl-2-(2RS)]-10 phénothiazinethiocarboxamide-2.

Le chloro-7 [(pyrrolidinyl-1)-1 propyl-2-(2RS)]-10 phénothiazinecarbonitrile-2 peut être obtenu de la manière suivante :

A une suspension de 72,1 g de méthanesulfonate de (chloro-7 cyano-2 phénothiazinyl-10)-2 propyle-(2RS) dans 166 cm3 de toluène, on ajoute 21,1 cm3 de pyrrolidine. Le mélange est porté à 120°C pendant 21

heures, puis à 150°C pendant 12 heures. Après refroidissement, le mélange est lavé par 5 fois 250 cm3 d'eau distillée. La phase organique est séchée sur sulfate de magnésium, filtrée et concentrée à sec sous pression réduite (30 mm de Hg; 4 kPa) pour donner 19,2 g de chloro-7 [(pyrrolidinyl-1)-1 propyl-2-(2RS)]-10 phénothiazinecarbonitrile-2.

Le méthanesulfonate de (chloro-7 cyano-2 phénothiazinyl-10)-2-propyle-(2RS) peut être obtenu de la manière suivante :

A une solution de 40 g de chloro-7 (hydroxy-1 propyl-2-(2RS))-10 phénothiazinecarbonitrile-2 dans 400 cm3 de chlorure de méthylène, on ajoute 30 cm3 de triéthylamine. Le mélange est refroidi à 0°C puis additionné goutte à goutte en 1 heure de 16,6 cm3 de méthanesulfochlorure. On ajoute alors 1 litre d'eau distillée puis le mélange est décanté et la phase organique est lavée avec 500 cm3 d'eau distillée. Elle est alors séchée sur sulfate de magnésium, filtrée et concentrée sous pression réduite (30 mm de Hg; 4 kPa) jusqu'à un volume résiduel de 100 cm3. On verse alors lentement 150 cm3 d'oxyde d'isopropyle et on laisse agiter pendant 30 minutes. On ajoute ensuite lentement 300 cm3 d'oxyde d'isopropyle et la suspension est agitée pendant 48 heures. Le précipité est filtré, lavé par 2 fois 50 cm3 d'oxyde d'isopropyle et séché à 40°C sous pression réduite (5 mm de Hg; 0,67 kPa) pour donner 34,53 g de méthanesulfonate de (chloro-7 cyano-2 phénothiazinyl-10)-2-propyle-(2RS) fondant à 132°C.

Le chloro-7 [hydroxy-1 propyl-2-(2RS)]-10 phénothiazinecarbonitrile-2 peut être obtenu de la manière suivante :

A une suspension de 25,8 g de tétrahydroborure de sodium dans 700 cm3 de tétrahydrofuranne anhydre contenant 57 cm3 d'éthanedithiol, on ajoute une solution de 157,6 g de (chloro-7 cyano-2 phénothiazinyl-10)-2 propionate d'éthyle-(2RS) dans 720 cm3 de tétrahydrofuranne sec. Le mélange est porté au reflux pendant 20 heures puis il est refroidi à 5°C et on ajoute lentement 1 litre d'une solution de soude 4N. On dilue le mélange réactionnel par 2 litres de chlorure de méthylène et on agite pendant 15 minutes. Le mélange est décanté et la phase organique est lavée par 4 fois 1,5 litre d'eau distillée, séchée sur sulfate de magnésium, filtrée et concentrée à sec sous pression réduite (30 mm de Hg; 4 kPa) à 50°C. Le résidu est purifié par chromatographie sur colonne (hauteur : 80 cm; diamètre : 8 cm) de silice (0,02 - 0,6 mm) en éluant avec des mélanges d'acétate d'éthyle et de cyclohexane 15-85 (15 litres) et 35-65 (10 litres) en recueillant des fractions de 600 cm3. Les huit premiers litres sont jetés et les fractions 21 à 28 sont réunies et concentrées à sec sous pression réduite (30 mm de Hg; 4 kPa) à 50°C pour donner 80 g de chloro-7 [hydroxy-1 propyl-2-(2RS)]-10 phénothiazinecarbonitrile-2.

Le (chloro-7 cyano-2 phénothiazinyl-10)-2 propionate d'éthyle-(2RS) peut être obtenu de la manière suivante :

A une solution de 10 g de chloro-7 phénothiazinecarbonitrile-2 dans 250 cm3 de N,N-diméthylformamide, on ajoute lentement en 10 minutes, 1,85 g d'hydrure de sodium à 50% en dispersion dans la vaseline. Le mélange est ensuite porté à 120°C et additionné en 30 minutes d'une solution de 18,5 cm3 de chloro-2 propionate d'éthyle-(2RS) dans 100 cm3 de N,N-diméthylformamide. Le chauffage est poursuivi pendant 2 heures. Après refroidissement, le mélange est dilué dans 1 litre d'acétate d'éthyle et lavé par 1,5 litre d'eau distillée. La phase organique est séchée sur sulfate de magnésium, traitée au noir 3S, filtrée et concentrée à sec sous pression réduite (30 mm de Hg; 4 kPa) à 50°C pour donner un résidu qui est purifié par chromatographie sur colonne (hauteur : 25 cm; diamètre : 4,0 cm) de silice (0,2-0,06 mm) en éluant par un mélange de cyclohexane et d'acétate d'éthyle 90-10 (1,5 litre) (en volumes) et en recueillant des fractions de 100 cm3. Les fractions 6 à 11 sont réunies et concentrées à sec sous pression réduite (30 mm de Hg; 4 kPa) pour donner 5,3 g de (chloro-7 cyano-2 phénothiazinyl-10)-2 propionate d'éthyle-(2RS) sous la forme d'une meringue jaune pâle.

Le chloro-7 phénothiazinecarbonitrile-2 peut être obtenu de la manière suivante :

A une solution de 100 g de phénothiazinecarbonitrile-2 dans 3000 cm3 de dichloro-1,2 éthane chauffée à 50°C, on ajoute 0,5 g de chlorure d'aluminium et 39,7 cm3 de chlorure de sulfuryle en 4 heures. Le mélange réactionnel est porté au reflux pendant 2 heures, puis est agité 48 heures à 25°C. Le précipité formé est filtré, lavé par 2 fois 200 cm3 de dichloro-1,2 éthane puis par 200 cm3 d'éther éthylique et séché à 40°C sous pression réduite (30 mm de Hg; 4 kPa) pour donner 69,3 g de chloro-7 phénothiazinecarbonitrile-2 sous la forme d'un solide jaune vert fondant à 235°C.

## EXEMPLE 89

A 40 cm3 d'une solution éthanolique de 3,65 g d'{[hydroxyméthyl-2-(2R) pyrrolidinyl-1]-1 propyl-2}-10 phénothiazinecarbothioamide-2, série D, saturée en acide sulfhydrique, on ajoute 10,57 g de diamino-1,2 cyclohexane. Le mélange est chauffé en autoclave à 150°C pendant 11 heures, puis dilué par 250 cm3 d'acétate d'éthyle et lavé par 4 fois 200 cm3 d'eau distillée. La phase organique est décantée, séchée sur sulfate de magnésium, filtrée et concentrée à sec sous pression réduite (30 mm de mercure; 4 kPa) à 40°C. Le résidu

est dilué dans 200 cm3 d'éthanol absolu bouillant et un léger insoluble est filtré. Le filtrat est agité à 25°C pendant 2 heures puis refroidi à 5°C pendant 12 heures. Le précipité formé est filtré, lavé avec 3 fois 10 cm3 d'éthanol froid et séché sous pression réduite (5 mm de Hg; 0,67 kPa) à 40°C pour donner 1,1 g d'(hexahydro-3a,4,5,6,7,7a 1H-benzimidazolyl-2)-2 {[hydroxyméthyl-2-(2R) pyrrolidinyl]-1 propyl-2}-10 phénothiazine, série D, sous la forme d'un solide de couleur crème.

$[\alpha]_D^{20}$ = 0,7 (chloroforme; c = 1,144%).

RMN du proton (250 MHz, DMSO, δ en ppm, J en Hz).

1,2 à 1,9 (Mt, 12H, $-CH_2-$ de l'hexahydrobenzimidazole et de la pyrrolidine); 1,6 (D, J = 7, 3H, $-CH_3$); 2,20 (Mt, 1H, 1H du $NCH_2-$ de la pyrrolidine); 2,48 (Mt, 1H, NCH- de la pyrrolidine); 2,65 (DD, J = 13 et 5, 1H, 1H du $N-CH_2-$); 3,12 (D, J = 5, 2H, $-CH_2O-$); 3,15 (Mt, 1H, 1H du $NCH_2-$ de la pyrrolidine); 3,5 (DD, J = 13 et 6,5, 1H, 1H du $N-CH_2-$); 3,75 (Mt, 2H, CH-CH de l'hexahydrobenzimidazole); 4,15 (Mt, J = 7 - 6,5 et 5, 1 H, N-CH ); 6,90 à 7,25 (Mf, 5H, aromatiques); 7,35 (DD, J = 8 et 1, 1H, -H en 3); 7,8 (D, J = 1, 1H, -H en 1).

Spectre infra-rouge ($CHCl_3$), bandes caractéristiques en $cm^{-1}$ : 3400, 2940, 2860, 2800, 1610, 1580, 1550, 1445, 1040.

L'{[hydroxyméthyl-2-(2R) pyrrolidinyl]-1 propyl-2}-10 phénothiazinecarbothioamide-2, série D, peut être préparé de la manière suivante :

A une solution de 3,65 g d'{[hydroxyméthyl-2-(2R) pyrrolidinyl-1]-1 propyl-2}-10 phénothiazinecarbonitrile-2, série D, dans 40 cm3 de pyridine anhydre, on ajoute 1,01 cm3 de triéthylamine. Le mélange est alors saturé en hydrogène sulfuré par bullage pendant 6 heures, puis est agité à 25°C pendant 12 heures. Le mélange réactionnel est alors dilué par 600 cm3 d'acétate d'éthyle et est lavé par 3 fois 200 cm3 d'eau distillée. La phase organique est décantée, séchée sur sulfate de magnésium, filtrée et concentrée à sec sous pression réduite (30 mm de mercure; 4 kPa) pour donner un résidu qui est chromatographié sur une colonne (hauteur : 46 cm; diamètre : 5 cm) de gel de silice (0,2 - 0,06 mm) en éluant par de l'acétate d'éthyle (3 litres) et en recueillant des fractions de 250 cm3. Les fractions 1 à 15 contenant le produit pur sont réunies et concentrées à sec sous pression réduite (30 mm de mercure; 4 kPa) à 40°C pour donner 3,7 g d'{[hydroxyméthyl-2-(2R) pyrrolidinyl]-1 propyl-2}-10 phénothiazinecarbothioamide-2, série D, sous la forme d'une meringue orangée.

L'{[hydroxyméthyl-2-(2R) pyrrolidinyl-1]-1 propyl-2}-10 phénothiazinecarbonitrile-2, série D, peut être préparé de la manière suivante :

A une suspension de 89,1 g de méthanesulfonate de (cyano-2 phénothiazinyl-10)-2-propyle-(2RS) dans 2000 cm3 d'éthanol absolu, on ajoute 50 g de (R)-(-)-pyrrolidinyl-2 méthanol. Le mélange est porté au reflux pendant 23 heures. Après refroidissement, le mélange est concentré à sec sous pression réduite (30 mm de Hg; 4 kPa) à 50°C. Le résidu est dilué par 350 cm3 d'acétate d'éthyle, filtré et le filtrat est lavé par 150 cm3 d'eau distillée et 2 fois 100 cm3 de solution saturée de chlorure de sodium. Après décantation, la phase organique est extraite par 350 cm3 d'acide chlorhydrique en solution 3N. Les jus aqueux acide sont extraits par 200 cm3 d'acétate d'éthyle puis alcalinisés à la soude concentrée et réextraits par 3 fois 200 cm3 d'acétate d'éthyle. La phase organique est lavée par 2 fois 200 cm3 d'eau distillée séchée sur sulfate de magnésium, filtrée et concentrée à sec sous pression réduite (30 mm de Hg; 4 kPa) à 50°C pour donner un résidu qui est purifié par chromatographie sur colonne (hauteur: 46 cm; diamètre : 8,5 cm) de gel de silice (0,06-0,2 mm) en éluant par des mélanges de cyclohexane et d'acétate d'éthyle 40-10 (12 litres), 70-30 (30 litres) (en volumes) en recueillant des fractions de 250 cm3. Les fractions 1 à 44 sont réunies et évaporées sous pression réduite (30 mm de Hg; 4 kPa) à 50°C pour donne 15,7 g d'une meringue jaune contenant le produit attendu. Ce résidu est dissous dans 15 cm3 d'oxyde d'isopropyle et la cristallisation est amorcée par grattage. La suspension est agitée 12 heures à 25°C et les cristaux sont filtrés sur verre fritté pour donner 14,4 g d'{[hydroxyméthyl-2-(2R) pyrrolidinyl-1]-1 propyl-2}-10 phénothiazinecarbonitrile-2, série D.

$[\alpha]_D^{20}$ = -4,6° (c = 1,166%; chloroforme).

## EXEMPLE 90

A 30 cm3 d'une solution éthanolique de 2,79 g d'{[hydroxyméthyl-2-(2R) pyrrolidinyl-1]-1 propyl-2}-10 phénothiazinecarbothioamide-2, série L, saturée en acide sulfhydrique on ajoute 7,98 g de diamino-1,2 cyclohexane. Le mélange est chauffé en autoclave à 150°C pendant 17 heures, puis dilué par 250 cm3 d'acétate d'éthyle et lavé par 4 fois 200 cm3 d'eau distillée. La phase organique est décantée, séchée sur sulfate de magnésium, filtrée et concentrée à sec sous pression réduite (30 mm de mercure; 4 kPa) à 40°C. Le résidu est agité avec 6 fois 30 cm3 d'acétonitrile bouillant et l'huile obtenue est concrétée par agitation avec 100 cm3 d'oxyde d'isopropyle. Le résidu est chromatographié sur une colonne (hauteur : 52 cm; diamètre : 2,5 cm) de gel de silice (0,2 - 0,06 mm) en éluant par un mélange de dichloro-1,2 éthane et de méthanol : 60 - 40 (2 litres) (en volumes) et en recueillant des fractions de 120 cm3. Le premier litre est éliminé et les fractions 4 à 7 contenant le produit

pur sont réunies et concentrées à sec sous pression réduite (30 mm de mercure; 4 kPa) à 40°C pour donner 1,8 g d'(hexahydro-3a,4,5,6,7,7a 1H-benzimidazolyl-2)-2 {[hydroxyméthyl-2-(2R) pyrrolidinyl-1]-1 propyl-2}-10 phénothiazine, série L, sous la forme d'un solide jaune.

A une solution de 1,8 g d'(hexahydro-3a,4,5,6,7,7a 1H-benzimidazolyl-2)-2 {[hydroxyméthyl-2-(2R) pyrrolidinyl-1]-1 propyl-2}-10 phénothiazine, série L,, dans 14 cm3 d'éthanol absolu, on ajoute 2,9 cm3 d'éther chlorhydrique 2,4 N. La solution ainsi obtenue est ajoutée goutte à goutte à 500 cm3 d'éther éthylique sous vive agitation. L'addition terminée, la suspension est agitée pendant 15 minutes à 25°C et le mélange est filtré, lavé par 3 fois 25 m3 d'éther éthylique puis le gâteau est séché à 40°C sous pression réduite (10 mm de mercure; 1,3 kPa). On obtient ainsi 1,48 g de dichlorhydrate d'(hexahydro-3a,4,5,6,7,7a 1H-benzimidazolyl-2)-2 {[hydroxyméthyl-2-(2R) pyrrolidinyl-1]-1 propyl-2}-10 phénothiazine, série L, sous la forme d'un solide jaune clair amorphe fondant à 240°C.

RMN du proton (250 MHz, DMSO, δ en ppm, J en Hz).

1,3 à 2,10 (Mt, 12H, $-CH_2-$ de l'héxahydrobenzimidazole et de la pyrrolidine); 1,87 (D, J = 7, 3H, $-CH_3$); 3,04 (Mt, 1H, 1H du $N-CH_2-$ de la pyrrolidine); 3,50 à 4,05 (Mt, 6H, 1H, du $N-CH_2-$ de la pyrrolidine, N-CH de la pyrrolidine, $-CH_2O-$ et $N-CH_2-$); 4,32 (Mf, 2H, CH-CH de l'héxahydrobenzimidazole); 4,9 (Mt, 1H, N-CH); 7 à 7,55 (Mt, 5H, aromatiques); 7,6 (S large, 1H, -H en 1); 7,78 (D large, J = 8, 1H, -H en 3); 10,18 et 11,35 (2Mf, 3H en totalité, $-NH^+$ et $NH_2^+$).

Spectre infra-rouge (KBr), bandes caractéristiques en $cm^{-1}$ : 3060, 2940, 2870, 2740, 2660, 1605, 1575, 1460, 1420, 1060, 1040, 870, 820, 755.

L' {[hydroxyméthyl-2-(2R) pyrrolidinyl-1]-1 propyl-2}-10 phénothiazinecarbothioamide-2, série L, peut être préparé de la manière suivante :

Un mélange de 1,3 g d'{[hydroxyméthyl-2-(2R) pyrrolidinyl-1]-1 propyl-2}-10 phénothiazinecarbonitrile-2, série L, et de 0,5 cm3 de triéthylamine dans 20 cm3 de pyridine anhydre est saturé par bullage d'acide sulfhydrique pendant 6 heures à 25°C. La solution limpide obtenue est maintenue sous agitation pendant 12 heures à 25°C. Le mélange est dégazé par bullage d'azote pendant 2 heures, puis dilué par 150 cm3 d'acétate d'éthyle et lavé par 6 fois 100 cm3 d'eau distillée, et par 3 fois 120 cm3 de solution saturée de chlorure de sodium. La phase organique est séchée sur sulfate de magnésium, filtrée et le filtrat est concentré à sec sous pression réduite (30 mm de Hg; 4 kPa) à 40°C. Le résidu est purifié par chromatographie sur colonne (hauteur : 25 cm; diamètre : 4 cm) de gel de silice (0,06-0,2 mm) en éluant par de l'acétate d'éthyle (2 litres) (en volumes) et en recueillant des fractions de 100 cm3. Les fractions 1 à 20 sont réunies et concentrées à sec sous pression réduite (30 mm de Hg; 4 kPa) à 40°C pour donner 0,96 g d'{[hydroxyméthyl-2-(2R) pyrrolidinyl-1]-1 propyl-2}-10 phénothiazinecarbothioamide-2, série L, sous la forme d'une meringue orangée.

L'{[hydroxyméthyl-2-(2R) pyrrolidinyl-1]-1 propyl-2}-10 phénothiazinecarbonitrile-2, série L, peut être préparé de la manière suivante :

A une suspension de 89,1 g de méthanesulfonate de (cyano-2 phénothiazinyl-10)-2-propyle-(2RS) dans 2000 cm3 d'éthanol absolu, on ajoute 50 g de (R)-(-)-pyrrolidinyl-2 méthanol. Le mélange est porté au reflux pendant 23 heures. Après refroidissement, le mélange est concentré à sec sous pression réduite (30 mm de Hg; 4 kPa). Le résidu est dilué par 350 cm3 d'acétate d'éthyle, filtré et le filtrat est lavé par 150 cm3 d'eau distillée et 2 fois 100 cm3 de solution saturée de chlorure de sodium. Après décantation, la phase organique est extraite par 350 cm3 d'acide chlorhydrique en solution 3N. Les extraits aqueux acide sont extraits par 200 cm3 d'acétate d'éthyle puis alcalinisés à la soude concentrée et réextraits par 3 fois 200 cm3 d'acétate d'éthyle. La phase organique est lavée par 2 fois 200 cm3 d'eau distillée puis séchée sur sulfate de magnésium, filtrée et concentrée à sec sous pression réduite (30 mm de Hg; 4 kPa) à 50°C pour donner un résidu qui est purifié par chromatographie sur colonne (hauteur : 46 cm; diamètre : 8,5 cm) de gel de silice (0,2-0,06 mm) en éluant par des mélanges de cyclohexane et d'acétate d'éthyle 80-20 (12 litres) et 70-30 (30 litres) (en volumes) en recueillant des fractions de 250 cm3. Les 12 premiers litres sont éliminés et les fractions 61 à 118 sont réunies et évaporées sous pression réduite (30 mm de Hg; 4 kPa) à 40°C pour donner 11,0 g d'{[hydroxyméthyl-2-(2R) pyrrolidinyl-1]-1 propyl-2}-10 phénothiazinecarbonitrile-2, série L, sous la forme d'une meringue jaune.

## EXEMPLE 91

Une solution de 0,93 g de chlorhydrate de $N^2$-cyano [(pyrrolidinyl-1)-1 propyl-2-(2RS)]-10 phénothiazinecarboxamidine-2 et de 1,86 cm3 de propylamine dans 25 cm3 d'éthanol est porté à 100°C pendant 16 heures. Le mélange réactionnel est concentré à sec à 50°C sous pression réduite (30 mm de Hg; 4 kPa) pour donner un résidu qui est purifié par chromatographie sur colonne (hauteur : 13,6 cm; diamètre : 2,4 cm) de silice (O,O4-O,06) avec une légère surpression d'azote (40 kPa) en éluant avec un mélange de cyclohexane et d'acétate d'éthyle 20-80 (en volumes) (750 cm3) puis par de l'acétate d'éthyle pur (750 cm3) et enfin avec un mélange d'acétate d'éthyle et de méthanol 90-10 en (volumes) (750 cm3) tout en recueillant des fractions de 50 cm3.

Les 1000 premiers cm3 sont éliminés et les fractions 5 à 10 sont réunies et concentrées à sec à 50°C sous pression réduite (30 mm de Hg; 4 kPa) pour donner un solide qui est dissous à chaud dans 4 cm3 de toluène. La cristallisation est amorcée et après refroidissement les cristaux sont séparés par filtration sur verre fritté, séchés sous pression réduite (1 mm de Hg; 0,13 kPa) à 40°C pour donner 0,15 g de $N^2$-cyano $N^1$-propyl [(pyrrolidinyl-1)-1 propyl-2-(2RS)]-10 phénothiazinecarboxamidine-2 sous la forme de cristaux jaunes fondant à 154°C.

RMN du proton (250 MHz, DMSO, δ en ppm, J en Hz).

0,93 (T, J = 7,5, 3H, $-(CH_2)_2C\underline{H}_3$); 1,6 (D, J = 7, 3H, $-CH_3$); 1,63 (Mt, 2H, $-CH_2C\underline{H}_2CH_3$); 1,69 (Mt, 4H, $-CH_2-CH_2-$ de la pyrrolidine), 2,54 (Mt en partie masqué, 4H, $-CH_2-\overset{|}{N}-CH_2-$ de la pyrrolidine); 2,92 (DD, J = 13 et 6,5, 1H, 1H du $<NCH_2-$); 3,06 (DD, J = 13 et 6, 1H, 1H du $>NCH_2-$); 3,30 (Mt en partie masqué, 2H, $-NHC\underline{H}_2-CH_2CH_3$); 4,23 (Mt, J = 7 - 6,5 et 6, 1H, $>N-CH<$); 6,95 à 7,35 (Mt, 6H, aromatiques); 7,42 (D, J = 1, 1H, -H en 1); 9,10 (T, J = 5,5, 1H, -NH-).

Spectre infra-rouge (KBr), bandes caractéristiques en $cm^{-1}$ : 3250, 2970, 2930, 2875, 2780, 2175, 1570, 1545, 1465, 880, 815, 750.

Le $N^2$-cyano [(pyrrolidinyl-1)-1 propyl-2-(2RS)]-10 phénothiazinecarboxamidine-2 peut être obtenu de la manière suivante :

A une solution de 38,7 g de dichlorhydrate de [(pyrrolidi nyl-1)-1 propyl-2-(2RS)]-10 phénothiazinecarboximidate-2 d'éthyle dans 1500 cm3 de dioxanne sec, on ajoute 21,1 cm3 de triéthylamine. Le mélange est agité pendant 10 minutes puis filtré sur verre fritté. On ajoute 3,15 g de cyanamide dans le filtrat. Le mélange est ensuite porté au reflux pendant 16 heures. Le mélange est concentré à sec à 50°C sous pression réduite (30 mm de Hg; 4 kPa) pour donner une meringue jaune qui est purifiée par chromatographie sur colonne (hauteur : 31 cm; diamètre : 6 cm) de silice (0,04-0,06 mm) avec une légère surpression d'azote (40 kPa) en éluant par de l'acétate d'éthyle pur (4 litres) puis avec un mélange d'acétate d'éthyle et de méthanol 95-5 (en volumes) (9 litres) et en recueillant des fractions de 125 cm3. Les 4,5 premiers litres sont éliminés et les fractions 47 à 68 sont réunies et concentrées à sec à 50°C sous pression réduite (30 mm de Hg; 4 kPa) pour donner 3,05 g de $N^2$-cyano [(pyrrolidinyl-1)-1 propyl-2-(2RS)]-10 phénothiazinecarboxamidine-2 sous la forme d'une meringue jaune.

On dissous 2,6 g de $N^2$-cyano [(pyrrolidinyl-1)-1 propyl-2-(2RS)]-10 phénothiazinecarboxamidine-2 dans 85 cm3 d'éthanol bouillant. Après filtration, on ajoute 1,5 cm3 d'éthanol chlorhydrique 5N. La cristallisation est amorcée et le mélange est maintenu sous agitation pendant 12 heures. Les cristaux sont filtrés sur verre fritté, lavés par 3 fois 15 cm3 d'éther éthylique puis séchés à 40°C sous pression réduite (1 mm de Hg; 0,13 kPa) pour donner 1,61 g de chlorhydrate de $N^2$-cyano [(pyrrolidinyl-1)-1 propyl-2-(2RS)]-10 phénothiazinecarboxamidine-2 sous la forme de cristaux jaunes fondant à 184°C.

Le dichlorhydrate de [(pyrrolidinyl-1)-1 propyl-2-(2RS)-10 phénothiazinecarboximidate-2 d'éthyle peut être préparé de la manière suivante :

A une solution de 25,16 g de [(pyrrolidinyl-1)-1 propyl-2-(2RS)]-10 phénothiazinecarbonitrile-2 dans 150 cm3 d'éthanol à 40°C, on fait barbotter de l'acide chlorhydrique gazeux pendant 5 heures. La solution est concentrée à sec à 50°C sous pression réduite (30 mm de Hg; 4 kPa) pour donner 38,7 g de dichlorhydrate de [pyrrolidinyl-1)-1 propyl-2-(2RS)]-10 phénothiazinecarboximidate-2 d'éthyle sous la forme d'une meringue marron.

EXEMPLE 92

A une suspension de 1,6 g de chlorhydrate de l'acide [(pyrrolidinyl-1)-1 propyl-2-(2RS)]-10 phénothiazinecarboxylique-2 dans 50 cm3 de chlorure de méthylène refroidie à 5°C, on ajoute 2 cm3 de chlorure de thionyle. La suspension est agitée 3 heures à 25°C puis concentrée à sec à 30°C sous pression réduite (30 mm de Hg; 4 kPa) pour donner un résidu jaune qui est repris dans 50 cm3 de chlorure de méthylène à 5°C. On ajoute 1,1 g de cyclopentylméthylamine et le mélange est agité 12 heures à 25°C, puis on ajoute 150 cm3 d'une solution saturée de bicarbonate de sodium. Le mélange est extrait par 2 fois 100 cm3 d'acétate d'éthyle. La phase organique est lavée successivement par 100 cm3 d'eau distillée et par 100 cm3 de saumure, séchée sur sulfate de magnésium, filtrée et concentrée à sec à 40°C sous pression réduite (30 mm de Hg; 4 kPa). Le solide jaune obtenu (1,6 g) est dissous dans 30 cm3 d'acétonitrile bouillant et la solution est filtrée. Le filtrat est amorcé par grattage puis refroidi sous agitation pendant 1 heure. Le solide est séparé par filtration, lavé par 2 fois 2 cm3 d'acétonitrile et séché à 40°C sous pression réduite (1 mm de Hg; 0,13 kPa). On obtient 1,3 g de N-cyclopentylméthyl [(pyrrolidinyl-1)-1 propyl-2-(2RS)]-10 phénothiazinecarboxamide-2 sous la forme de cristaux blancs fondant à 124°C.

RMN du proton (250 MHz, $CDCl_3$, δ en ppm, J en Hz).

1,28 - 1,64 - 1,83 (3Mt, 2H - 4H et 2H respectivement, $-CH_2-$ du cyclopentyle); 1,7 (D, J = 7, 3H, $-CH_3$); 1,84 (Mt, 4H, $-CH_2-CH_2-$ de la pyrrolidine); 2,18 (Mt, 1H, -CH- du cyclopentyle); 2,74 (Mt, 4H, $-CH_2-N-CH_2-$de la pyrrolidine); 3,02 (DD, J = 13,5 et 6, 1H, 1H du >N-$CH_2$-); 3,23 (DD, J = 13,5 et 6,5, 1H, 1H du >N-$CH_2$-); 3,4 (DD, J = 7 et 5,5, 2H, -CONH-$CH_2$-); 4,4 (Mt, J = 7 -6,5 et 6, 1H, >N-CH<); 6,3 (T, J = 5,5, 1H, -CONH-); 6,9 à 7,20 (Mt, 5H, aromatiques); 7,29 (DD, J = 8 et 1, 1H, -H en 3); 7,65 (D, J = 1, 1H, -H en 1).

Spectre infra-rouge (KBr), bandes caractéristiques en $cm^{-1}$ : 3310, 2950, 2860, 2790, 1630, 1595, 1545, 1460, 870, 825.

EXEMPLE 93

En opérant d'une manière analogue à celle décrite ci-après l'exemple 103 mais à partir de 3,5 g de chlorhydrate de chlorure de [(pyrrolidinyl-1)-1 propyl-2-(2RS)]-10 phénothiazinecarbonyle-2 dans 100 cm3 de dichlorométhane et de 3,8 g de cyclopropylméthylamine, on obtient 3 g de N-(cyclopropylméthyl) [(pyrrolidinyl-1)-1 propyl-2-(2RS)]-10 phénothiazinecarboxamide-2 sous la forme d'une meringue crème qui est dissoute dans 20 cm3 d'acétonitrile. Puis on ajoute 8 cm3 d'une solution 2,4 N d'acide chlorhydrique dans l'éther diéthylique. Les cristaux sont essorés, séchés sous pression réduite (5mm de Hg; 0,67 kPa) à 40°C. On obtient 2,6 g de chlorhydrate de N-(cyclopropylméthyl) [(pyrrolidinyl-1)-1 propyl-2-(2RS)]-10 phénothiazinecarboxamide-2 sous la forme de cristaux blancs fondant à 220°C.

RMN du proton (250 MHz, DMSO D6, δ en ppm, J en Hz).

0,23 et 0,42 (2 Mt, 2H chacun, $-CH_2-$ du cyclopropyle) ; 1,04 (Mt, 1H, >CH- du cyclopropyle) ; 1,77 (D, J = 7, 3H, $-CH_3$) ; 1,7 à 2 (Mt, 4H, $-CH_2-$ de la pyrrolidine) ; 2,83, 3,08, 3,55 et 3,73 (4 Mf, 1H chacun respectivement, >N-$CH_2$- de la pyrrolidine) ; 3,14 (DD, J = 7,5 et 5,5, 2H, -CONHC$\underline{H_2}$-) ; 3,73 (AB limite, 2H, N-$CH_2$-); 4,75 (Mt, 1H, >N-CH<) ; 7 à 7,35 (Mt, 5H, aromatiques) ; 7,53 (S large, 1H, -H en 1) ; 7,54 (D large, J = 8, 1H, -H en 3) ; 8,75 (T, J = 5,5, 1H, -CONH-).

Spectre infra-rouge (KBr), bandes caractéristiques en $cm^{-1}$ : 3270, 2780 à 2000, 1655, 1595, 1575, 1560, 1465, 1535, 850, 820, 750.

EXEMPLE 94

En opérant d'une manière analogue à celle décrite ci-après à l'exemple 103 mais à partir de 3,4 g de chlorhydrate de chlorure de [(pyrrolidinyl-1)-1 propyl-2-(2RS)]-10 phénothiazinecarbonyle-2 dans 100 cm3 de dichlorométhane et de 4,5 g de cyclobutylméthylamine, on obtient, après recristallisation dans l'oxyde d'isopropyle, 1,7 g de N-(cyclobutylméthyl) [(pyrrolidinyl-1)-1 propyl-2-(2RS)]-10 phénothi- azinecarboxamide-2 sous la forme de cristaux blancs fondant à 133°C.

RMN du proton (250 MHz, $CDCl_3$ D, δ en ppm, J en Hz).

1,68 (D, J = 7, 3H, $-CH_3$) ; 1,82 (Mt, 4H, $-CH_2-$ de la pyrrolidine) ; 1,7 à 2,2 (Mt, 6 H, $-CH_2-$ du cyclobutylméthyle) ; 2,59 (Mt, 1 H, >CH- du cyclobutylméthyle) ; 2,65 (Mt, 4 H, N-$CH_2$ de la pyrrolidine) ; 2,96 (DD, J = 12,5 et 6,5, 1 H, 1 H du >N-$CH_2$-) ;3,17 (DD, J = 12,5 et 6, 1H, 1 H du >N-$CH_2$-) ; 3,49 (DD, J= 7,5 et 5,5, 2H, -CONHC$\underline{H_2}$-) ; 4,28 (Mt, J = 7, 6,5 et 6, 1H, >N-CH<) ; 6,15 (T, J = 5,5, 1H, -CONH-) ; 6,90 à 7,25 (Mt, 5H, aromatiques) ; 7,27 (DD, J = 8 et 1, 1H, -H en 3) ; 7,67 (D, J = 1, 1H, -H en 1).

Spectre infra-rouge (KBr), bandes caractéristiques en $cm^{-1}$ : 3270, 2970, 2935, 2865, 2790, 1630, 1595, 1560, 1465, 1540, 870, 830, 745.

EXEMPLE 95

3,12 g de chlorhydrate d'acide [(pyrrolidinyl-1)-1 propyl-2-(2RS)]-10 phénothiazinecarboxylique-2 dans 100 cm3 de chlorure de méthylène sont refroidis à une température voisine de 5°C et additionné, sous agitation, 4 cm3 de chlorure de thionyle. Après 10 minutes, on porte la température à 20°C et on poursuit l'agitation pendant 1 heure. La solution orangée obtenue est concentrée à sec sous pression réduite (30 mm de Hg; 4 kPa) à 30°C. La meringue jaune résiduaire est dissoute dans 100 cm3 de chlorure de méthylène et additionnée, goutte à goutte, sous agitation et à une température voisine de 5°C, de 1,5 cm3 d'alkylamine puis on poursuit l'agitation pendant 2 heures. Le mélange réactionnel est alors dilué avec 200 cm3 d'acétate d'éthyle, lavé successivement avec 200 cm3 d'une solution aqueuse saturée d'hydrogénocarbonate de sodium et avec 200 cm3 d'une solution aqueuse saturée de chlorure de sodium, séché sur sulfate de magnésium, filtré et concentré à sec sous pression réduite (30 mm de Hg; 4 kPa) à 40°C pour donner 3,2 g d'une huile jaune. Ce produit est dissous dans 35 cm3 d'acétate d'éthyle de additionné sous agitation de 2,7 cm3 d'une solution 3,3N d'acide

chlorhydrique dans l'oxyde d'isopropyle, à une température voisine de 5°C. Le précipité formé est essoré, lavé avec 10 cm3 d'oxyde d'isopropyle et séché sous pression réduite (5 mm de Hg; 0,68 kPa) à 40°C. On obtient ainsi 2,6 g de chlorhydrate de N-allyl [(pyrrolidinyl-1)-1 propyl-2-(2RS)]-10 phénothiazinecarboxamide-2 sous forme d'un solide beige fondant à 208°C.

RMN du proton (250 MHz, DMSO, δ en ppm, J en Hz).

En solution dans le DMSO, on observe deux formes dues à la salification de l'azote.

1,8 (D, J = 7, 3H, $-CH_3$); 1,9 (Mt, 4H, $-CH_2-$ de la pyrrolidine); 2,84 - 3,10 - 3,58 et 3,74 (4Mf, 1H chacun, $-CH_2-\overset{N}{|}-CH_2-$ de la pyrrolidine); 3,77 (Mt, 2H, >N-$CH_2$-); 3,92 (Mt, 2H, -CONH-C$\underline{H_2}$-); 4,78 (Mt, 1H, >N-CH<); 5,10 et 5,19 (2DD, 1H chacun, =$CH_2$); 5,92 (Mt, 1H, -CH=); 7 à 7,35 (Mt, 5H, aromatiques); 7,55 (S large, 1H, -H en 1); 7,58 (D large, J = 8, 1H, -H en 3); 8,90 (T, J = 5,5, 1H, -CONH-); 10,88 (Mf, 1H, $-NH^+$).

Spectre infra-rouge (KBr), bandes caractéristiques en $cm^{-1}$ : 3260, 2975, 2680, 2610, 2480, 1655, 1590, 1555, 1530, 1460, 995, 910, 825, 750.

## EXEMPLE 96

Dans une suspension de 6 g de chlorhydrate d'acide [(pyrrolidinyl-1)-1 propyl-2]-10 phénothiazinecarboxylique-2, série L, dans 190 cm3 de chlorure de méthylène, on verse en 5 minutes, sous agitation, 7,7 cm3 de chlorure de thionyle en maintenant la température aux environs de 5°C. L'agitation est poursuivie pendant 4 heures en chauffant à une température voisine de 20°C et la solution jaune obtenue est concentrée à sec sous pression réduite (30 mm de Hg; 4 kPa) à 40°C. Le résidu est dissous dans 110 cm3 de chlorure de méthylène et additionné d'une solution de 2,9 cm3 d'allylamine dans 30 cm3 de chlorure de méthylène sous agitation, en maintenant la température à 5°C puis on poursuit l'agitation pendant 16 heures à 20°C. Le mélange réactionnel est dilué par 250 cm3 d'acétate d'éthyle, filtré, lavé successivement par 50 cm3 d'une solution aqueuse saturée d'hydrogénocarbonate de sodium et par 50 cm3 d'une solution aqueuse saturée de chlorure de sodium, séché sur sulfate de magnésium, filtré et concentré à sec sous pression réduite (30 mm de Hg; 4 kPa) à 49°C. Le résidu huileux jaune (4,7 g) est purifié par chromatographie sur une colonne (hauteur: 35 cm; diamètre: 4 cm) de gel de silice (0,04-0,06 mm) avec une légère surpression d'azote (40,5 kPa) en éluant par 2 litres d'un mélange d'acétate d'éthyle et d'éthanol (90-10 en volumes) et en recueillant des fractions de 60 cm3. Les fractions 6 à 28 sont réunies et concentrées à sec sous pression réduite ( 30 mm de Hg 4 kPa) à 40°C pour donner 3,98 g d'une huile jaune. On dissous cette huile dans 50 cm3 d'oxyde de diéthyle et on ajoute 3,4 cm3 d'une solution 0,3 N d'acide chlorhydrique dans l'oxyde d'isopropyle sous agitation à 6°C. Après une heure d'agitation à 6°C, le solide formé est filtré, lavé par 5 cm3 d'oxyde de diéthyle et séché sous pression réduite (5 mm de Hg; 0,67 kPa) à 40°C pour donner 3,8 g de chlorhydrate de N-allyl [(pyrrolidinyl-1)-1 propyl-2]-10 phénothiazinecarboxamide-2, forme L, sous forme d'un solide jaune fondant vers 170°C.

$[\alpha]_D^{20}$ = + 17,7 ± 0,5° (c = 1 % ; méthanol).

RMN du proton (250 MHz, DMSO D6, δ en ppm, J en Hz).

1,8 (D, J = 7, 3H, $-CH_3$) ; 1,7 à 2 (Mt, 4H, $-CH_2-$ de la pyrrolidine) ; 2,83, 3,09, 3,59 et 3,77 (4 Mf, respectivement 1H chacun, >N$CH_2$- de la pyrrolidine) ; 3,77 (Mt, 2H >N-$CH_2$-) ; 3,92 (DD, J = 7,5 et 5,5, 2H, -CONHC$\underline{H_2}$-) ; 4,76 (Mt, 1H, >N-CH<) ; 5,15 (Mt, 2 H, -CH=$CH_2$) ; 5,92 (Mt, 1H, -C$\underline{H}$=$CH_2$) ; 7 à 7,4 (Mt, 5H, aromatiques) ; 7,55 (S large, 1H, -H en 1) ; 7,57 (D large, J = 8, 1H, -H en 3) ; 8,85 (T, J = 5,5, 1H, -CONH-).

Spectre infra-rouge (KBr), bandes caractéristiques en $cm^{-1}$ : 3260, 2975, 2680, 2580, 2480, 1650, 1640, 1590, 1555, 1460, 1530, 870, 830, 755.

Le chlorhydrate de l'acide [(pyrrolidinyl-1)-1 propyl-2]-10 phénothiazinecarboxylique-2, série L, peut être obtenu de la manière suivante :

A une solution de 1,75 g de potasse dans 30 $cm^3$ de glycol, on ajoute 5 g de chlorhydrate de [(pyrrolidinyl-1)-1C propyl-2]-10 phénothiazinecarboxylique-2, série L, et on agite pendant 4 heures au reflux. Après refroidissement, la solution jaune obtenue est diluée avec 75 $cm^3$ d'acétone et 5 $cm^3$ d'une solution 3 N d'acide chlorhy- drique dans l'éther éthylique, filtrée et diluée à nouveau avec 75 $cm^3$ d'acétone et 5 $cm^3$ d'une solution 3N d'acide chlorhydrique dans l'éther éthylique. Après amorçage, on laisse cristalliser pendant 15 heures à une température voisine de 5°C. Le solide obtenu est essoré, lavé avec 10 $cm^3$ d'éther éthylique et séché sous pression réduite (5 mm de Hg ; 0,7 kPa) à 40°C. On obtient ainsi 2,9 g de chlorhydrate d'acide [(pyrrolidinyl-1)-1 propyl-2]-10 phénothiazinecarboxylique-2, série L, sous forme d'un solide jaune clair fondant à 200-210°C (fusion pâteuse).

EXEMPLE 97

Dans une suspension de 1 g de chlorhydrate d'acide [(pyrrolidinyl-1)-1 propyl-2-(2RS)]-10 phénothiazinecarboxylique-2 dans 30 cm3 de chlorure de méthylène, on verse pendant 5 minutes et sous agitation 0,5 cm3 de chlorure de thionyle en maintenant la température aux environs de 5°C. L'agitation est poursuivie pendant 40 minutes en chauffant à une température voisine de 30°C et la solution jaune obtenue est concentrée à sec sous pression réduite (30 mm de Hg; 4 kPa) à 40°C. Le résidu est dissous dans 30 cm3 de chlorure de méthylène et on ajoute 0,73 g (méthyl-3 butène-2 yl)amine et de 0,84 cm3 de triéthylamine. L'agitation est maintenue pendant 30 minutes à 20°C. Le mélange réactionnel est dilué par 100 cm3 d'acétate d'éthyle, lavé successivement par 50 cm3 d'une solution aqueuse N de soude puis par 2 fois 50 cm3 d'une solution aqueuse saturée de chlorure de sodium, séché sur sulfate de magnésium, filtré et concentré à sec sous pression réduite (30 mm de Hg; 4 kPa) à 40°C. Le résidu huileux orangé (0,9 g) est dissous dans 35 cm3 d'un mélange d'acétate d'éthyle et d'oxyde d'isopropyle (30-70 en volumes) et on ajoute 5,5 cm3 d'une solution 0,3 N d'acide chlorhydrique dans l'oxyde d'isopropyle sous agitation à 6°C. Après une heure d'agitation à 6°C, le solide formé est filtré lavé par 5 cm3 d'oxyde d'isopropyle et séché sous pression réduite (5 mm de Hg; 0,67 kPa) à 40°C pour donner 0,52 g de chlorhydrate de N-(méthyl-3 butène-2 yl) [(pyrrolidinyl-1)-1 propyl-2-(2RS)]-10 phénothiazinecarboxamide-2 sous forme d'un solide crème fondant vers 150°C.

RMN du proton (250 MHz, DMSO D6 + quelques gouttes de $CD_3COOD$, δ en ppm, J en Hz).

1,69 (S, 6H, $-CH=C(C\underline{H}_3)_2$), 1,79 (D, J = 7, 3H, $-CH_3$) ; 1,87 (Mt, 4H, $-CH_2-$ de la pyrrolidine) ; 3,19 et 3,43 (2 Mt, 2H chacun, >N-$CH_2$- de la pyrrolidine) ; 3,66 (DD, J = 12,5 et 4,5, 1 H, 1 H du >N-$CH_2$-) ; 3,8 (DD, J = 12,5 et 8,1 H, 1H du >N-$CH_2$-); 3,88 (T, J = 5,5, 2H, $-CONHC\underline{H}_2-$) ; 4,68 (Mt, J = 8, 7 et 4,5, 1H, >N-CH<) ; 5,23 (T large, J = 5,5, 1H, =CH-) ; 7 à 7,35 (Mt, 5H, aromatiques) ; 7,53 (S, 1H, -H en 1) ; 7,54 (D, J = 8, 1H, -H en 3).

Spectre infra-rouge (KBr), bandes caractéristiques en $cm^{-1}$ : 3270, 2965, 2925, 2680, 2610, 2480, 1655, 1595, 1465, 1535, 855, 825, 755.

EXEMPLE 98

A une solution de 1 cm3 de chlorure de thionyle dans 25 cm3 de chlorure de méthylène, on ajoute 0,78 g de chlorhydrate d'acide [(pyrrolidinyl-1)-1 propyl-2-(2RS)]-10 phénothiazinecarboxylique-2, par portions, sous agitation et à une température voisine de 5°C puis on poursuit l'agitation pendant 3 heures à une température voisine de 20°C. Le mélange réactionnel est alors concentré à sec sous pression réduite (30 mm de Hg; 4 kPa) à 40°C et le résidu est repris par 25 cm3 de chlorure de méthylène. A la solution obtenue, agitée et refroidie à une température voisine de 5°C, on ajoute goutte à goutte 0,75 cm3 de propargylamine. Après 1 heure, le mélange réactionnel est dilué avec 100 cm3 d'acétate d'éthyle et lavé avec 2 fois 50 cm3 d'une solution aqueuse saturée d'hydrogénocarbonate de sodium puis avec 50 cm3 d'une solution aqueuse saturée de chlorure de sodium. La phase organique séparée est séchée sur sulfate de magnésium, filtrée et concentrée à sec sous pression réduite (30 mm de Hg; 4 kPa) à 40°C. On obtient 0,94 g d'un résidu gommeux jaune. Ce produit est purifié par chromatographie sur colonne (hauteur : 40 cm; diamètre : 3 cm) de gel de silice (0,063-0,04 mm) sous légère surpression d'azote (40 kPa) en éluant avec 1,5 litre d'un mélange de chlorure de méthylène et de méthanol (97,5-2,5 en volumes) et en recueillant des fractions de 50 cm3. Les fractions 10 à 25 sont réunies et concentrées à sec sous pression réduite (30 mm de Hg;, 4 kPa) à 40°C. Le résidu gommeux jaune obtenu (0,59 g) est dissous dans 30 cm3 d'éther éthylique et additionné de 7 cm3 d'une solution 0,23N d'acide chlorhydrique dans l'éther éthylique. Le précipité formé est essoré, lavé par 2 fois 10 cm3 d'éther éthylique et séché sous pression réduite (5 mm de Hg; 0,68 kPa) à 40°C. On obtient ainsi 0,6 g de chlorhydrate de N-propargyl [(pyrrolidinyl-1)-1 propyl-2-(2RS)]-10 phénothiazinecarbOxamide-2 sous forme d'un solide jaune claire fondant à 178-180°C (fusion pâteuse).

RMN du proton (250 MHz, DMSO, δ en ppm, J en Hz).

En solution dans le DMSO, on observe deux formes dues à la salification de l'azote.

1,82 (D, J = 7, 3H, $-CH_3$); 1,88 (Mt, 4H, $-CH_2-CH_2-$ de la pyrrolidine); 2,82 - 3,10 - 3,58 et 3,78 (4Mf, 1H chacun, $-CH_2-\overset{N}{|}-CH_2-$ de la pyrrolidine); 3,15 (T, J = 2, 1H, ≡CH); 3,6 à 3,9 (Mt, 2H,>N-$CH_2$-); 4,07 (Mt, 2H, $-CONH-C\underline{H}_2-$); 4,72 (Mt, 1H, >N-CH<); 7 à 7,4 (Mt, 5H, aromatiques); 7,53 (S large, 1 H, -H en 1); 7,56 (D large, J = 8, 1H, -H en 3); 9,11 (T, J = 5,5, 1H, -CONH-); 10,5 (Mf, 1H, $-NH^+$).

Spectre infra-rouge (KBr), bandes caractéristiques en $cm^{-1}$ : 3240, 2680, 2610, 2480, 2120, 1655, 1595, 1560, 1530, 1460, 875, 825, 750.

Le chlorhydrate d'acide [(pyrrolidinyl-1)-1 propyl-2-(2RS)]-10 phénothiazinecarboxylique-2 peut être préparé de la manière suivante :

A une solution de 1,7 g de potasse dans 20 cm3 de glycol, on ajoute 3,72 g de chlorhydrate de [(pyrrolidinyl-1)-1 propyl-2-(2RS)]-10 phénothiazinecarbonitrile-2 et on agite pendant 5 heures au reflux. Après refroidissement, la solution jaune obtenue est additionnée de 10 cm3 d'une solution 3N d'éther chlorhydrique et diluée avec 100 cm3 d'acétone et 100 cm3 d'éther éthylique puis filtrée. Le filtrat jaune, après amorçage, laisse cristalliser un produit que l'on essore, lave avec 2 fois 10 cm3 d'éther éthylique et sèche sous pression réduite (5 mm de Hg; 0,68 kPa) à 40°C. On obtient ainsi 3,5 g de chlorhydrate d'acide [(pyrrolidinyl-1)-1 propyl-2-(2RS)]-10 phénothiazinecarboxylique-2 sous forme de cristaux jaunes clairs fondant à 215-217°C.

EXEMPLE 99

Dans une suspension de 6 g de chlorhydrate d'acide [(pyrrolidinyl-1)-1 propyl-2]-10 phénothiazinecarboxylique-2, série L, dans 190 cm3 de chlorure de méthylène, on verse en 5 minutes, sous agitation, 7,7 cm3 de chlorure de thionyle en maintenant la température aux environs de 5°C. L'agitation est poursuivie pendant 16 heures à une température voisine de 20°C et la solution jaune obtenue est concentrée à sec sous pression réduite (30 mm de Hg; 4 kPa) à 40°C. Le résidu est dissous dans 130 cm3 de chlorure de méthylène et additionné d'une solution de 2,45 cm3 de propargylamine dans 25 cm3 de chlorure de méthylène sous agitation et en maintenant la température à 5°C puis on poursuit l'agitation pendant 4 heures à 20°C. Le mélange réactionnel est dilué par 200 cm3 d'acétate d'éthyle, filtré, lavé successivement par 50 cm3 d'une solution aqueuse saturée d'hydrogéno- carbonate de sodium et par 50 cm3 d'une solution aqueuse saturée de chlorure de sodium, séché sur sulfate de magnésium, filtré et concentré à sec sous pression réduite (30 mm de Hg; 4 kPa) à 49°C. Le résidu huileux brun (5,32 g) est dissous dans 70 cm3 d'oxyde de diéthyle au reflux. Après refroidissement, le solide formé est filtré, lavé avec 10 cm3 d'oxyde de diéthyle et repris par 40 cm3 d'acétate d'éthyle. La solution obtenue est lavée successivement par 50 cm3 d'eau distillée puis par 50 cm3 d'une solution aqueuse saturée de chlorure de sodium, séchée sur sulfate de magnésium, filtrée et concentrée à sec sous pression réduite ( 30 mm de Hg 4 kPa) à 40°C pour donner 2,8 g d'une meringue brune. On reprend cette meringue par 50 cm3 d'oxyde de diéthyle et la suspension obtenue est agité pendant 2 heures à 20°C. Le solide est filtre, lavé par 2 fois 5 cm3 d'oxyde de diéthyle et séché sous pression réduite ( 5 mm de Hg; 0,67 kPa) à 40°C pour donner 1,42 g de N-propargyl [(pyrrolidinyl-1)-1 propyl-2]-10 phénothiazinecarboxamide-2, série L, sous forme d'un solide ocre fondant à 130°C.

$[\alpha]_D^{20}$ = + 24,8 ± 0,6° (c = 1 % ; Méthanol)

RMN du proton (250 MHz, DMSO D6, δ en ppm, J en Hz).

1,62 (D, J = 7, 3H, $-CH_3$) ; 1,7 (Mt, 4H, $-CH_2-$ de la pyrrolidine) ; 3,90 (DD, J = 12,5 et 6,5, 1H, 1 H du $>N-CH_2-$) ; 3,02 (DD, J = 12,5 et 6, 1H, 1 H du $>N-CH_2-$) ; 3,11 (T, J = 2, 1H, $-C\equiv CH$); 4,08 (DD, J = 5,5 et 2, 2H, $-CONHC\underline{H_2}-$) ; 4,2 (Mt, J = 7, 6,5 et 6, 1H, $>N-CH<$) ; 6,90 à 7,3 (Mt, 5H, aromatiques) ; 7,44 (DD, J = 8 et 1, 1H, -H en 3) ; 7,58 (D, J = 1, 1H, -H en 1) ; 8,85 (T, J = 5,5, 1H, -CONH-).

Spectre infra-rouge (KBr), bandes caractéristiques en $cm^{-1}$ : 3360, 3300, 3240, 2965, 2880, 2790, 2120, 1635, 1590, 1560, 1460, 1530, 880, 830, 745.

EXEMPLE 100

A une suspension de 2,7 g de chlorhydrate de l'acide [(pyrrolidinyl-1)-1 propyl-2-(2RS)]-10 phénothiazinecarboxylique-2 dans 70 cm3 de chlorure de méthylène refroidie à 5°C, on ajoute 3,6 cm3 de chlorure de thionyle. La suspension est agitée 3 heures à 25°C puis concentrée à sec à 30°C sous pression réduite (30 mm de Hg; 4 kPa) pour donner un résidu jaune qui est repris dans 50 cm3 de chlorure de méthylène à 5°C. On ajoute 6,9 g de chloro-3 benzylamine et le mélange est agité 12 heures à 25°C, puis on ajoute 150 cm3 d'une solution saturée de bicarbonate de sodium. Le mélange est extrait par 2 fois 100 cm3 d'acétate d'éthyle. La phase organique est lavée successivement par 100 cm3 d'eau distillée et par 100 cm3 de saumure, séchée sur sulfate de magnésium, filtrée et concentrée à sec à 40°C sous pression réduite (30 mm de Hg; 4 kPa) pour donner une huile brune qui est purifiée par chromatographie sur colonne (hauteur : 30 cm; diamètre : 2,5 cm) de silice (0,06-0,2 mm) en éluant par du chlorure de méthylène (1 litre) et en recueillant des fractions de 40 cm3. Les fractions 12 à 24 sont réunies et concentrées à sec sous pression réduite (30 mm de Hg; 4 _Pa) à 40°C pour donner un solide crème qui est repris dans 20 cm3 d'oxyde d'isopropyle. Le solide est séparé par filtration, lavé par 2 fois 5 cm3 d'oxyde d'isopropyle et séché à 40°C sous pression réduite (1 mm de Hg; 0,13 kPa). On obtient 2 g de N-(chloro-3 benzyl) [(pyrrolidinyl-1)-1 propyl-2-(2RS)]-10 phénothiazinecarboxamide-2 sous la forme d'un solide jaune fondant à environ 80°C.

RMN du proton (250 MHz, $CDCl_3$, δ en ppm, J en Hz).

1,68 (D, J = 7, 3H, $-CH_3$); 1,75 (Mf, 4H, $-CH_2-CH_2-$ de la pyrrolidine); 2,62 (Mt, 4H,$-CH_2-N-CH_2-$ de la pyrro-

lidine); 2,95 (DD, J = 12,5 et 6,5, 1H, 1H du >N-$CH_2$-); 3,17 (DD, J = 12,5 et 6, 1H, 1H du >N-$CH_2$-); 4,26 (Mt, J = 7 - 6,5 et 6, 1H, >N-CH<); 4,75 (D, J = 5,5, 2H, -CONH-C$\underline{H}_2$-); 6,68 (T, J = 5,5, 1H, -CONH-); 6,9 à 7,5 (Mt, 10H, aromatiques); 7,71 (D, J = 1, 1H, -H en 1).

Spectre infra-rouge (KBr), bandes caractéristiques en $cm^{-1}$ : 3320, 2960, 2930, 2870, 2780, 1640, 1590, 1570, 1530, 1460, 1440, 875, 820, 750.

EXEMPLE 101

Dans une suspension de 3,42 g d'acide [(pyrrolidinyl-1)-1 propyl-2-(2RS)]-10 phénothiazinecarboxylique-2 dans 100 cm3 de chlorure de méthylène, on verse sous agitation, pendant 5 minutes, 4,44 cm3 de chlorure de thionyle en maintenant la température aux environs de 5°C. L'agitation est poursuivie pendant 3 heures en chauffant à une température voisine de 20°C et la solution jaune obtenue est concentrée à sec sous pression réduite (30 mm de Hg; 4 kPa) à 30°C. Le résidu est dissous dans 80 cm3 de chlorure de méthylène et on ajoute une solution de 2g de dichloro-2,3 benzylamine dans 20 cm3 de chlorure de méthylène. L'agitation est maintenue pendant 60 minutes à 20°C. Le mélange réactionnel est concentré à sec sous pression réduite (30 mm de Hg; 4 kPa) à 30°C. Le résidu est dilué par 150 cm3 d'acétate d'éthyle, lavé successivement par 150 cm3 d'une solution aqueuse N de soude puis par 2 fois 150 cm3 d'une solution aqueuse saturée de chlorure de sodium, séché sur sulfate de magnésium, filtré et concentré à sec sous pression réduite (30 mm de Hg; 4 kPa) à 40°C. Le résidu huileux orangé est purifié par chromatographie sur une colonne (hauteur:25 cm; diamètre:2,5cm) de gel de silice (0,04-0,06mm) sous une légère surpression d'azote (40,5 kPa) en éluant par un litre de chlorure de méthylène et en recueillant des fractions de 100 cm3. Les fractions 5 à 8 sont réunies et concentrées à sec sous pression réduite (30 mm de Hg; 4 kPa) à 30°C. Le résidu (4,18 g) est repris dans 10 cm3 d'éthanol, filtré et les liqueur-mères sont additionnées de 5 cm3 d'une solution 3,3 N d'acide chlorhydrique dans l'oxyde d'isopropyle. Après trente minutes à 5°C, le solide formé est filtré, lavé par 2 fois 3 cm3 d'oxyde d'isopropyle, essoré et séché sous pression réduite (5 mm de Hg; 0,67 kPa) à 40°C pour donner 0,6 g de chlorhydrate de N-(dichloro-2,3 benzyl) [(pyrrolidinyl-1)-1 propyl-2 -(2RS)]-10 phénothiazinecarboxamide-2 sous forme d'un solide crème fondant au dessus de 260°C.

RMN du proton (250 MHz, DMSO D6 + quelques gouttes de $CD_3COOD$ à une température de 413°C, δ en ppm, J en Hz).

1,75 (D, J = 7, 3H, -$CH_3$) ; 1,9 (Mf, 4H, -$CH_2$- de la pyrrolidine) ; 3,24 et 3,38 (2 Mt, respectivement 2H chacun, >N-$CH_2$- de la pyrrolidine) ; 3,54 (DD, J = 13 et 6, 1H, 1 H du >N-$CH_2$-) ; 3,76 (DD, J = 13 et 8, 1H, 1H du >N-$CH_2$-) ; 4,63 (S, 2H -CONHC$\underline{H}_2$-); 4,63 (Mt, J = 8, 7 et 6, 1H, >N-CH<) ; 7 à 7,55 (Mt, 8H aromatiques) ; 7,57 (D, J = 8, 1H, -H en 3) ; 7,59 (S, 1H, -H en 1).

Spectre infra-rouge (KBr), bandes caractéristiques en $cm^{-1}$ : 3280, 2970, 2930, 2565, 2475, 1655, 1590, 1560, 1465, 1535, 820, 750.

EXEMPLE 102

A une suspension de 2,0 g de chlorhydrate de l'acide [(pyrrolidinyl-1)-1 propyl-2-(2RS)]-10 phénothiazinecarboxylique-2 dans 60 cm3 de dichloro-1,2 éthane refroidie à 5°C, on ajoute 2,6 cm3 de chlorure de thionyle. La suspension est agitée 3 heures à 25°C puis concentrée à sec à 30°C sous pression réduite (30 mm de Hg; 4 kPa) pour donner un résidu jaune qui est repris dans 50 cm3 de dichloro-1,2 éthane à 5°C. On ajoute 4,4 g de dichloro-2,6 benzylamine et le mélange est agité 12 heures à 25°C, puis on ajoute 150 cm3 d'une solution saturée de bicarbonate de sodium. Le mélange est extrait par 2 fois 100 cm3 d'acétate d'éthyle. La phase organique est lavée successivement par 100 cm3 d'eau distillée et par 100 cm3 de saumure, séchée sur sulfate de magnésium, filtrée et concentrée à sec à 40°C sous pression réduite (30 mm de Hg; 4 kPa) pour donner 5,8 g d'une huile jaune qui est purifiée par chromatographie sur colonne (hauteur : 35 cm; diamètre : 4 cm) de silice (0,06-0,2 mm) en éluant par un mélange de chlorure de méthylène et de méthanol 95-5 (en volumes) (1,2 litre) et en recueillant des fractions de 40 cm3. Les fractions 13 à 25 sont réunies et concentrées à sec sous pression réduite (30 mm de Hg; 4 kPa) à 40°C pour donner une huile jaune qui est dissoute dans 12 cm3 d'acétonitrile bouillant. La cristallisation est amorcée par grattage. Après refroidissement, le solide est séparé par filtration, lavé par 2 fois 3 cm3 d'acétonitrile et séché à 40°C sous pression réduite (1 mm de Hg; 0,13 kPa). On obtient 1,6 g de N-(dichloRo-2,6 benzyl) [(pyrrolidinyl-1)-1 propyl-2-(2RS)]-10 phénothiazinecarboxamide-2 sous la forme de cristaux crème fondant à 138°C.

RMN du proton (250 MHz, DMSO, δ en ppm, J en Hz).

1,57 (D, J = 7, 3H, -$CH_3$); 1,67 (Mf, 4H, -$CH_2$-$CH_2$- de la pyrrolidine); 2,52 (Mt masqué, 4H, -$CH_2$-N-$CH_2$- de la pyrrolidine); 2,87 (DD, J = 12,5 et 6,5, 1H, 1H du >N-$CH_2$-); 3 (DD, J = 12,5 et 6, 1H, 1H du >N-$CH_2$-);

4,18 (Mt, J = 7 - 6,5 et 6, 1H, >N-CH<); 4,68 (D, J = 4,5, 2H, -C<u>H</u>$_2$-NHCO-); 6,9 à 7,6 (Mt, 10H, aromatiques); 8,68 (D, J = 4,5, 1H, -CONH-).

Spectre infra-rouge (KBr), bandes caractéristiques en $cm^{-1}$ : 3440, 3280, 2960, 2870, 2790, 1630, 1590, 1580, 1560, 1530, 1460, 1435, 865, 830, 780, 765, 750.

## EXEMPLE 103

Dans une suspension de 9,77 g de chlorhydrate d'acide [(pyrrolidinyl-1)-1 propyl-2-(2RS)]-10 phénothiazinecarboxylique-2 dans 250 cm3 de chlorure de méthylène, on ajoute en cinq minutes sous agitaition et à une température de 4°C, 12,7 cm3 de chlorure de thionyle et on poursuit l'agitation pendant 90 minutes à une température de 20°C. La solution jaune limpide obtenue est concentrée à sec sous pression réduite (30 mm de Hg; 4 kPa) à 40°C et séchée à poids constant sous pression réduite (5 mm de Hg; 0,67 kPa) à 50°C pour donner 10,2 g de chlorhydrate de chlorure de [(pyrrolidinyl-1)-1 propyl-2-(2RS)]-10 phénothiazinecarbonyle-2 sous la forme d'une pâte jaune.

Dans une solution de 13,2 g de chlorydrate de chlorure de [(pyrrolidinyl-1)-1 propyl-2-(2RS)]-10 phénothiazinecarbonyle-2 dans 125 cm3 de chlorure de méthylène, on ajoute goutte à goutte à une température de 10°C et sous agitation 26,1 cm3 de méthoxy-2 benzylamine. L'agitation est poursuivie pendant 15 minutes à 10°C et 16 heures à 20°C. Le mélange réactionnel est lavé par 3 fois 100 cm3 d'eau distillée et la phase organique est séché sur sulfate de magnésium, filtrée et concentrée à sec sous pression réduite (30 mm de Hg; 4 kPa) à 40°C. Le résidu est purifié par chromatographie sur colonne (hauteur: 64cm; diamètre: 3 cm) de gel de silice (0,06-0,2mm) en éluant par 2 litres d'acétate d'éthyle et en recueillant des fractions de 200 cm3. Les fractions 3 à 10 sont réunies et concentrées à sec sous pression réduite (30 mm de Hg; 4 kPa) à 40°C pour donner 9,3 g de N-(méthoxy-2 benzyl) [(pyrrolidinyl-1)-1 propyl-2-(2RS)]-10 phénothiazinecarboxamide-2 sous la forme d'un solide cristallin blanc fondant à 127°C.

On dissous 2 g de N-(méthoxy-2 benzyl) [(pyrrolidinyl-1)-1 propyl-2-(2RS)]-10 phénothiazinecarboxamide-2 dans 80 cm3 d'un mélange d'acétate d'éthyle et d'oxyde d'isopropyle (50-50 en volumes) au reflux. Un léger insoluble est éliminé par filtration à chaud et après refroidissement la cristallisation se développe. Après 16 heures à 20°C, le solide formé est filtré, lavé par 10 cm3 d'oxyde d'isopropyle et séché sous pression réduite (30 mm de Hg; 4 kPa) à 50°C pour donner 1,49 g de N-(méthoxy-2 benzyl) [(pyrrolidinyl-1)-1 propyl-2-(2RS)]-10 phénothiazinecarboxamide-2 sous forme de cristaux blancs à reflets jaunes fondant à 131°C.

RMN du proton (250 MHz, $CDCl_3$ D, δ en ppm, J en Hz).

1,68 (D, J = 7, 3H, $-CH_3$) ; 1.75 (Mt, 4H, $-CH_2$- de la pyrrolidine) ; 2,61 (Mt, 4H, >N-$CH_2$- de la pyrrolidine) ; 2,96 (DD, J = 12,5 et 6,5, 1H, 1 H du >N-$CH_2$-) ; 3,14 (DD, J = 12,5 et 6, 1H H, 1H du >N-$CH_2$-) ; 3,9 (S, 3H, $-OCH_3$) ; 4,26 (Mt, J = 7, 6,5 et 6, 1H, >N-CH<) ; 4,65 (D, J = 5,5, 2H, -CONHC<u>H</u>$_2$-) ; 6,7 (T, J = 5,5, 1H, -CONH-) ; 6,85 à 7,4 (Mt, 9H, aromatiques) ; 7,26 (DD, J = 8 et 1, 1H, -H en 3) ; 7,69 (D, J = 1, 1H, -H en 1).

Spectre infra-rouge ($CHCl_3$), bandes caractéristiques en $cm^{-1}$ :3460, 2970, 2880, 2840, 2810, 1655, 1600, 1560, 1460, 1520, 1495, 1245, 1035, 820.

## EXEMPLE 104

Dans une solution de 4,74 g de N-(méthoxy-2 benzyl) [(pyrrolidinyl-1)-1 propyl-2-(2RS)]-10 phénothiazinecarboxamide-2 dans 100 cm3 de chlorure de méthylène refroidie à -50°C, on ajoute sous agitation goutte à goutte pendant 15 minutes une solution de 5,01 g de tribromure de bore dans 100 cm3 de chlorure de méthylène. L'agitation est maintenue à -50°C pendant 45 minutes puis à 20°C pendant 48 heures. On ajoute alors avec précaution 120 cm3 d'une solution aqueuse saturée d'hydrogénocarbonate de sodium. Après agitation jusqu'à fin de dégagement d'anhydride carbonique, la phase organique est séparée, lavée par 3 fois 100 cm3 d'eau distillée, séchée sur sulfate de magnésium, filtrée et concentrée à sec sous pression réduite (30 mm de Hg; 4 kPa). Le résidu meringué jaune est purifié par chromatographie sur colonne (hauteur: 48cm; diamètre: 2,4 cm) de gel de silice (0,06-0,2mm) en éluant par 2 litres d'acétate d'éthyle et en recueillant des fractions de 100 cm3. Les fractions 4 à 15 sont réunies et concentrées à sec sous pression réduite (30 mm de Hg; 4 kPa) à 40°C. Le résidu meringué jaune (2,84 g) est purifié par chromatographie sur colonne (hauteur: 33 cm; diamètre: 2,4 cm) de gel de silice (0,06-0,2mm) en éluant sucessivement par 250 cm3 d'un mélange de cyclohexane et d'acétate d'éthyle (50-50 en volumes) puis par 500 cm3 d'un mélange de cyclohexane et d'acétate d'éthyle (25-75 en volumes) puis par 500 cm3 d'acétate d'éthyle et en recueillant des fractions de 60 cm3. Les fractions 7 à 18 sont réunies et concentées à sec sous pression réduite (30 mm de Hg; 4 kPa) à 40°C pour donner 1,9 g de N-(hydroxy-2 benzyl) [(pyrrolidinyl-1)-1 propyl-2-(2RS)]-10 phénothiazinecarboxamide-2 sous forme d'une meringue jaune.

On dissous 1,86 g de N-(hydroxy-2 benzyl) [(pyrrolidinyl-1) -1 propyl-2-(2RS)]-10 phénothiazinecarboxamide-2 dans 22 cm3 de propanol-2 bouillant. A cette solution, on ajoute une solution de 0,47 g d'acide fumarique dans 18 cm3 de propanol-2 au reflux. Après refroidissement, les cristaux formés sont lavés avec 2 fois 10 cm3 d'éther éthylique, filtrés et séchés sous pression réduite (5 mm de Hg; 0,67kPa) à 50°C. On obtient ainsi 1,09 g de fumarate de N-(hydroxy-2 benzyl) [(pyrrolidinyl-1)-1 propyl-2-(2RS)]-10 phénothiazinecarboxamide-2 sous la forme de cristaux jaunes fondant vers 80°C.

RMN du proton (250 MHz, DMSO D6, δ en ppm, J en Hz).

1,64 (D, J = 7, 3H, $-CH_3$) ; 1,71 (Mf, 4H, $-CH_2$- de la pyrrolidine) ; 2,6 à 2,85 (Mt, 4H, >N-$CH_2$- de la pyrrolidine) ; 3,09 (DD, J = 12 et 7,5, 1H, 1 H du >N-$CH_2$-) ; 3,18 (DD, J = 12 et 6, 1H, 1 H du >N-$CH_2$-) ; 4,32 (Mt, J = 7,5, 7 et 6, 1H, >N-CH<) ; 4,43 (D, J = 5,5, 2H, -CONHC$\underline{H_2}$-) ; 6,59 (S, 2 H, -CH=CH- du fumarate) ; 6,75 (T, J = 7,5, 1H, -H en 5 de l'hydroxy-2 benzyle) ; 6,82 (D, J = 7,5, 1H, -H en 3 de l'hydroxy-2 benzyle) ; 6,95 à 7,30 (Mt, 3H, aromatiques, -H en 4 et 6 de l'hydroxy-2 benzyle) ; 7,52 (D, J = 8, 1H, -H en 3) ; 7,60 (S, 1H, -H en 1) ; 9 (T, J = 5,5, 1H -CONH-).

Spectre infra-rouge (KBr), bandes caractéristiques en $cm^{-1}$ : 3260, 2965, 2880, 3200, 2800, 2800, 2000, 1700, 1630, 1590, 1555, 1485, 1460, 980, 870, 815, 755.

EXEMPLE 105

En opérant d'une manière analogue à celle décrite à l'exemple 103 mais à partir de 1,8 g de chlorhydrate de chlorure de [(pyrrolidinyl-1)-1 propyl-2-(2RS)]-10 phénothiazinecarboxamide-2 dans 50 cm3 de dichlorométhane et de 1,8 g de trifluorométhyl-2 benzylamine, on obtient, après recristallisation dans l'oxyde d'isopropyle, 1,2 g de N-(trifluoro-3 benzyl) [(pyrrolidinyl-1)-1 propyl-2-(2RS)]-10 phénothiazinecarboxamide-2 sous la forme de cristaux blancs fondant à 112°C.

RMN du proton (250 MHz, $CDCl_3$ D, δ en ppm, J en Hz).

1,68 (D, J = 7, 3H, $-CH_3$) ; 1,75 (Mt, 4H, $-CH_2$- de la pyrrolidine) ; 2,63 (Mt, 4H, >N-$CH_2$- de la pyrrolidine) ; 2,95 (DD, J = 12,5 et 6, 1H, 1 H du >N-$CH_2$-) ; 3,18 (DD, J = 12,5 et 5,5, 1 H, 1 H du >N-$CH_2$-) ; 4,28 (Mt, J = 7, 6 et 5,5, 1 H >N-CH<) ; 4,83 (D, J = 6, 2H, -CONHC$\underline{H_2}$-) ; 6,61 (T, J = 6, 1H, -CONH-) ; 6,90 à 7,80 (Mt, 9H, aromatiques) ; 7,30 (DD, J = 8 et 1, 1H, -H en 3) ; 7,72 (D, J = 1, 1H, -H en 1).

Spectre infra-rouge (KBr), bandes caractéristiques en $cm^{-1}$ : 3310, 2970, 2875, 2795, 1640, 1610, 1595, 1465, 1540, 1315, 1165, 1120, 875, 830, 765, 750.

EXEMPLE 106

En opérant d'une manière analogue à celle décrite à l'exemple 103 mais à partir de 2,5 g de chlorhydrate de chlorure de [(pyrrolidinyl-1)-1 propyl-2-(2RS)]-10 phénothiazinecarbonyle-2 dans 50 cm3 de dichlorométhane et de 2,1 g de nitro-2 benzylamine, on obtient, après recristallisation dans l'oxyde d'isopropyle, 1,1 g de N-(nitro-2 benzyl) [(pyrrolidinyl-1)-1 propyl-2-(2RS)]-10 phénothi- azinecarboxamide-2 sous la forme de cristaux jaunes fondant à 95°C.

RMN du proton (250 MHz, DMSO D6, δ en ppm, J en Hz).

1,60 (D, J = 7, 3H, $-CH_3$) ; 1,67 (Mt, 4H, $-CH_2$- de la pyrrolidine) ; 2,52 (Mt, en partie masqué, <N$CH_2$- de la pyrrolidine); 2,90 (DD, J = 12,5 et 6,5, 1H, 1 H du >N$CH_2$-) ; 3,02 (DD, J = 12,5 6, 1H, 1 H du >N$CH_2$) ; 4,21 (Mt, J = 7, 6,5 et 6, 1H >N-CH<) ; 4,76 (D, J = 6, 2H, -CONHC$\underline{H_2}$-) ; 6,9 à 7,3 (Mt, 5H, aromatiques) ; 7,45 à 7,65 (Mt, 4H, -H en 1 et -H en 3 de la phénothiazine, et 2H du nitro-2 benzyle) ; 8,07 (D large, J = 8, 1H nitro-2 benzyle) ; 9,10 (T, J = 6, 1H, -CONH-).

Spectre infra-rouge (KBr), bandes caractéristiques en $cm^{-1}$ : 3310, 2960, 2875, 2790, 1640, 1610, 1590, 1580, 1460, 1520, 1340, 875, 820, 855, 790, 750.

EXEMPLE 107

A une suspension de 3,9 g de chlorhydrate de l'acide [(pyrrolidinyl-1)-1 propyl-2-(2RS)]-10 phénothiazinecarboxylique-2 dans 120 cm3 de chlorure de méthylène refroidie à 5°C, on ajoute 5,2 cm3 de chlorure de thionyle. La suspension est agitée 3 heures à 25°C puis concentrée à sec à 30°C sous pression réduite (30 mm de Hg; 4 kPa) pour donner un résidu jaune qui est repris dans 50 cm3 de chlorure de méthylène à 5°C. On ajoute 5 cm3 de cyclopentylamine et le mélange est agité 12 heures à 25°C, puis on ajoute 150 cm3 d'une solution saturée de bicarbonate de sodium. Le mélange est extrait par 2 fois 100 cm3 d'acétate d'éthyle. La phase organique est lavée successivement par 100 cm3 d'eau distillée et par 100 cm3 de saumure, séchée sur sulfate de magnésium, filtrée et concentrée à sec à 40°C sous pression réduite (30 mm de Hg; 4 kPa) pour donner une huile jaune qui est dissoute dans 70 cm3 d'acétonitrile bouillant. La cristallisation est amorcée par grattage. Après refroidissement, le solide est séparé par filtration, lavé par 2 fois 3 cm3 d'acétonitrile et séché

à 40°C sous pression réduite (1 mm de Hg; 0,13 kPa). On obtient 2,1 g de N-cyclopentyl [(pyrrolidinyl-1)-1 propyl-2-(2RS)]-10 phénothiazinecarboxamide-2 sous la forme de cristaux crème fondant à 132°C.

RMN du proton (250 MHz, $CDCl_3$, δ en ppm, J en Hz).

1, 51 - 1,72 et 2,10 (3Mt, de 2H chacun, $-CH_2-$ du cyclopentyle); 1,67 (D, J = 7, 3H, $-CH_3$); 1,82 (Mt, 4H, $-CH_2-CH_2-$de la pyrrolidine); 2,68 (Mt, 4H, $-CH_2-N-CH_2-$ de la pyrrolidine); 2,98 (DD, J = 12,5 et 6,5, 1H, 1H du >N-$CH_2$-); 3,19 (DD, J = 12,5 et 6, 1H, 1H du >N-$CH_2$-); 4,34 (Mt, J = 7 - 6,5 et 6, 1H, 1H du >N-$CH_2$-); 4,40 (Mt, 1H, -CONH-C<u>H</u><); 6,14 (D, J = 7, 1H, -CONH-); 6,9 à 7,25 (Mt, 5H, aromatiques); 7,26 (DD, J = 8 et 1, 1H, -H en 3); 7,62 (D, J = 1, 1H, -H en 1).

Spectre infra-rouge (KBr), bandes caractéristiques en $cm^{-1}$ : 3300, 2960, 2870, 2790, 1625, 1590, 1540, 1460, 880, 830, 750.

EXEMPLE 108

A une suspension de 1,6 g de chlorhydrate de l'acide [(pyrrolidinyl-1)-1 propyl-2-(2RS)]-10 phénothiazinecarboxylique-2 dans 50 cm3 de chlorure de méthylène, refroidie à 5°C, on ajoute 2 cm3 de chlorure de thionyle. La suspension est agitée 3 heures à 25°C puis concentrée à sec à 30°C sous pression réduite (30 mm de Hg; 4 kPa) pour donner un résidu jaune qui est repris dans 50 cm3 de chlorure de méthylène à 5°C. On ajoute 1,1 g de cyclohexylméthylamine et le mélange est agité 12 heures à 25°C, puis on ajoute 150 cm3 d'une solution saturée de bicarbonate de sodium. Le mélange est extrait par 2 fois 100 cm3 d'acétate d'éthyle. La phase organique est lavée successivement par 100 cm3 d'eau distillée et par 100 cm3 de saumure, séchée sur sulfate de magnésium, filtrée et concentrée à sec à 40°C sous pression réduite (30 mm de Hg; 4 kPa) pour donner 1,7 g d'un solide jaune contenant le produit attendu. Le solide obtenu est dissous dans 80 cm3 d'oxyde d'isopropyle bouillant puis filtré. La solution est amorcée par grattage puis refroidie sous agitation pendant 1 heure. Le solide est séparé par filtration, lavé par 2 fois 4 cm3 d'oxyde d'isopropyle et séché à 40°C sous pression réduite (1 mm de Hg; 0,13 kPa). On obtient 1,1 g de N-(cyclohexylméthyl) [(pyrrolidinyl-1)-1 propyl-2-(2RS)]-10 phénothiazinecarboxamide-2 sous la forme de cristaux blancs fondant à 120°C.

RMN du proton (250 MHz, $CDCl_3$, δ en ppm, J en Hz).

1 - 1,22 et de 1,5 à 1,8 (3Mt, 2H-3H et 6H respectivement, $-C_6H_{11}$); 1,7 (D, J = 7, 3H, $-CH_3$); 1,85 (Mt, 4H, $-CH_2-CH_2-$ de la pyrrolidine); 2,75 (Mt, 4H, $-CH_2-$ N-$CH_2$- de la pyrrolidine); 3,03 (DD, J = 13 et 6,5, 1H, 1H du >N-$CH_2$-); 3,25 (DD, J = 13 et 6, 1H, 1H du >N-$CH_2$-); 3,30 (T, J = 6, 2H, -CONH-C<u>H</u><u>2</u>-); 4,42 (Mt, J = 7 - 6,5 et 6, 1H, >N-CH<); 6,38 (T, J = 6, 1H, -CONH-); 6,9 à 7,25 (Mt, 5H, aromatiques); 7,3 (DD, J = 8 et 1, 1H, -H en 3); 7,67 (D, J = 1, 1H, -H en 1).

Spectre infra-rouge (KBr), bandes caractéristiques en $cm^{-1}$ : 3300, 2920, 2850, 2790, 1630, 1595, 1545, 1460, 865, 820 750.

EXEMPLE 109

A une suspension de 2 g de chlorhydrate de l'acide [(pyrrolidinyl-1)-1 propyl-2-(2RS)]-10 phénothiazinecarboxylique-2 dans 60 cm3 de chlorure de méthylène refroidie à 5°C, on ajoute 2,6 cm3 de chlorure de thionyle. La suspension est agitée 3 heures à 25°C puis concentrée à sec à 30°C sous pression réduite (30 mm de Hg; 4 kPa) pour donner un résidu jaune qui est repris dans 50 cm3 de chlorure de méthylène à 5°C. On ajoute 4 cm3 d'aniline et le mélange est agité 12 heures à 25°C, puis on ajoute 150 cm3 d'une solution saturée de bicarbonate de sodium. Le mélange est extrait par 2 fois 100 cm3 d'acétate d'éthyle. La phase organique est lavée successivement par 100 cm3 d'eau distillée et par 100 cm3 de saumure, séchée sur sulfate de magnésium, filtrée et concentrée à sec à 40°C sous pression réduite (30 mm de Hg; 4 kPa) pour donner une huile brune qui est purifiée par chromatographie sur colonne (hauteur : 30 cm, diamètre : 2,5 cm) de silice (0,06-0,2 mm) en éluant par du chlorure de méthylène (1,8 litre) et en recueillant des fractions de 40 cm3. Les fractions 12 à 36 sont réunies et concentrées à sec sous pression réduite (30 mm de Hg; 4 kPa) à 40°C pour donner 1,1 g d'un solide crème qui est dissous dans 10 cm3 d'acétonitrile bouillant. La solution est amorcée par grattage puis refroidie sous agitation pendant 1 heure. Le solide est séparé par filtration, lavé par 2 fois 1 cm3 d'acétonitrile et séché à 40°C sous pression réduite (1 mm de Hg; 0,13 kPa). On obtient 0,75 g de N-phényl [(pyrrolidinyl-1)-1 propyl-2-(2RS)]-10 phénothiazinecarboxamide-2 sous la forme de cristaux jaunes fondant à 163°C.

RMN du proton (250 MHz, DMSO, δ en ppm, J en Hz).

1,61 (D, J = 7, 3H, $-CH_3$); 1,68 (Mf, 4H, $-CH_2-CH_2-$ de la pyrrolidine); 2,54 (Mt masqué, $-CH_2-N-CH_2-$ de la pyrrolidine); 2,91 (DD, J = 13 et 6,5, 1H, 1H du >N-$CH_2$-); 3,06 (DD, J = 13 et 6, 1H, 1H du >N-$CH_2$-); 4,25

(Mt, 1H, >N-CH<); 6,95 à 7,45 (Mt, 8H, aromatiques); 7,55 (D large, J = 8, 1H, -H en 3); 7,68 (S large, 1H, -H en 1); 7,77 (D large, J = 8,5, 2H, aromatiques du phényle en ortho du -CONH-); 10,22 (S, 1H, -CONH-).

Spectre infra-rouge (KBr), bandes caractéristiques en $cm^{-1}$ : 3370, 2970, 2880, 2790, 1650, 1600, 1570, 1560, 1535, 1500, 1470, 825, 750, 690.

EXEMPLE 110

On ajoute, en deux minutes, sous agitation et à une température de 20°C, 0,75 cm3 d'eau distillée dans une suspension de 4,7 g de tertiobutylate de potassium dans 45 cm3 de tertiobutanol. Dans la solution obtenue, on ajoute sous agitation et à une température de 20°c une solution de 2,59 g de [(N-cyclopropylméthyl méthylamino)-1 propyl-2]-10 phénothiazinecarbonitrile-2, série L, dans 15 cm3 de tertiobutanol. La suspension jaune obtenue est alors portée au reflux pendant 30 minutes. Après refroidissement à une température de 30°C, on ajoute sous agitation 4,1 cm3 d'iodo-1 propane et on porte au reflux pendant 1 heure. Après refroidissement, on concentre à sec sous pression réduite (30 mm de Hg; 4 kPa) à 40°C. Le résidu est repris avec 100 cm3 d'acétate d'éthyle et la phase organique est lavée avec 3 fois 100 cm3 d'eau distillée, séchée sur sulfate de magnésium, filtrée et concentrée à sec sous pression réduite (30 mm de Hg; 4kPa) à 40°C. Le résidu huileux jaune (3 g) est purifié par chromatographie sur colonne (hauteur: 30 cm; diamètre: 3,6 cm) de gel de silice (0,04-0,063 mm) sous légère surpression d'azote (40kPa) en éluant successivement avec 2,5 litres d'un mélange de cyclohexane et d'acétate d'éthyle (80/20 en volumes) puis avec 2 litres d'un mélange de cyclohexane et d'acétate d'éthyle (60/40 en volumes) et enfin 5 litres d'acétate d'éthyle et en recueillant des fractions de 125 cm3. Les fractions 37 à 72 sont réunies et concentrées à sec sous pression réduite ( 30 mm de Hg; 4 kPa) à 40°C pour donner 2,52 g d'un résidu huileux jaune. 1,62 g de ce résidu est dissous dans 125 cm3 d'éther éthylique et additionné de 1,3 cm3 d'une solution 3,6 N d'acide chlorhydrique dans l'éther éthylique. Après 16 heures d'agitation à 20°C, le précipité formé est filtré, lavé avec 2 fois 50 cm3 d'oxyde d'isopropyle et séché sous pression réduite (5 mm de Hg; 0,67 kPa) à 40°C pour donner 1,2 g de chlorhydrate de [(N-cyclopropylméthyl méthylamino)-1 propyl-2]-10 N-propyl phénothiazine-carboxamide-2, série L, sous forme d'un solide jaune fondant vers 160°C.

$[\alpha]_D^{20}$ = + 19,4 ± 0,9° (0,5 % ; méthanol)

RMN du proton (250 MHz, DMSO D6 + quelques gouttes de $CD_3COOD$, δ en ppm, J en Hz).

0,27 et 0,53 (2 Mt, respectivement 2H chacun, $-CH_2-$ du cyclopropylméthyle) ; 0,9 (T, J = 7, 3H, $-(CH_2)_2C\underline{H_3}$) ; 0,9 à 1,15 (Mf, 1H, >CH- du cyclopropyleméthyle) ; 1,06 (T, J = 6,5, 3H, $-CH_3$ de l'éthylamino) ; 1,55 (Mt, 2H, $-CH_2C\underline{H_2}CH_3$) ; 1,85 (D, J = 7, 3H, $-CH_3$) ; 3,03 (D, J = 7,5, 2H, $>NCH_2-$ du cyclopropylméthyle) ; 3,25 (Mt, 4H, $>N-CH_2-$ de l'éthylamino et $-CONHC\underline{H_2}-$) ; 3,45 (D large, J = 14, 1H, 1 H du $>NCH_2$) ; 3,83 (DD, J = 14 et 7,5, 1 H, 1 H du $>N\ CH_2-$) ; 4,78 (Mt, 1H, >N-CH<) ; 7 à 7,35 (Mt, 5H, aromatiques) ; 7,54 (D, J = 8, 1H, -H en3) ; 7,54 (S, 1H, -H en 1).

Spectre infra-rouge (KBr), bandes caractéristiques en $cm^{-1}$ : 3270, 2960, 2930, 2870, 2580, 2500, 1645, 1590, 1555, 1460, 1535, 875, 830, 755.

Le [(N-cyclopropylméthyl méthylamino)-1 propyl-2]-10 phéno- thiazinecarbonitrile-2, série L peut être préparé de la manière suivante :

Un mélange de 3,1 g d'(éthylamino-1 propyl-2)-10 phénothiazinecarbonitrile-2, série L, de 1,19 g de carbonate de sodium et de 2,7 g de bromométhylcyclopropane dans 32 cm3 de diméthylformamide est chauffé à 150°C pendant 9 heures. Après refroidissement le mélange réactionnel est versé dans 300 cm3 d'eau distillée et extrait par 3 fois 150 cm3 d'acétate d'éthyle. Les phases organiques réunies sont lavées par 4 fois 100 cm3 d'eau distillée, séchées sur sulfate de magnésium, filtrées et concentrées à sec sous pression réduite (30 mm de Hg; 4kPa) à 50°C. Le résidu huileux brun (3,38 g) est purifié par chromatographie sur colonne (hAuteur: 30 cm; diamètre: 2,5 cm) de gel de silice (0,06-0,20 mm) en éluant par 2 litres d'un mélange de chlorure de méthylène et d'acétate d'éthyle (80/20 en volumes) et en recueillant des fractions de 60 cm3. Les fractions 4 à 12 sont réunies et concentrées à sec sous pression réduite (30 mm de Hg; 4kPa) à 40°C. On obtient ainsi 1,83 g de [(N-cyclopropylméthyl méthylamino)-1 propyl-2]-10 phénothiazinecarbonitrile-2, série L, sous forme d'une huile jaune.

$[\alpha]_D^{20}$ = - 1,5 ± 0,3° (1 % ; chloroforme)

EXEMPLE 111

A une solution refroidie à 0-5°C de 3,49 g de dichlorhydrate de [diéthylamino-1 propyl-2-(2RS)]-10 phénothiazinecarboximidate-2 de méthyle dans 21 cm3 de méthanol, on ajoute goutte à goutte une solution de 1,95 cm3 de propylamine dans 5 cm3 de méthanol en 5 minutes. Le mélange est agité pendant 2 heures à

5°C puis concentré à sec sous pression réduite (30 mm de Hg; 4 kPa) à 50°C pour donner un résidu qui est repris par 50 cm3 d'eau distillée. La solution est lavée par 50 cm3 d'eau distillée alcalinisée à pH 9 par de la soude N. Les deux phases sont décantées et la phase organique est lavée par 50 cm3 d'une solution à demi saturée de chlorure de sodium, séchée sur sulfate de magnésium, filtrée puis concentrée à sec sous pression réduite (30 mm de Hg; 4 kPa) à 50°C. Le résidu est purifié par chromatographie sur alumine neutre (hauteur : 20 cm ; diamètre : 2,1 cm) en éluant par de l'acétate d'éthyle pur (375 cm3) enfin par des mélanges d'acétate d'éthyle et de méthanol 99-1 (375 cm3), 98-2 (500 cm3), 95-5 (500 cm3), 90-10 (500 cm3) et 80-20 (500 cm3) (en volumes) et en recueillant des fractions de 25 cm3. Les fractions 46 à 70 sont réunies, concentrées à sec sous pression réduite (30 mm de Hg; 4 kPa) à 50°C pour donner 1,49 g de [diéthylamino-1 propyl-2-(2RS)]-10 N-propyl phénothiazinecarboxamidine-2 sous la forme d'une meringue jaune.

A 7 cm3 d'une solution acétonique de 0,74 g de [diéthylamino-1 propyl-2-(2RS)]-10 N-propyl phénothiazinecarboxamidine-2, on ajoute goutte à goutte 1,42 cm3 d'une solution acétonique d'acide chlorhydrique 3,5 N. Le mélange est agité pendant 3 heures à 25°C puis concentré à sec sous pression réduite (30 mm de Hg; 4 kPa) à 50°C. Le résidu est repris dans 50 cm3 d'éther éthylique et la suspension est maintenue sous agitation pendant 3 heures à 25°C. Le solide est filtré sur verre fritté, lavé par 2 fois 5 cm3 d'éther éthylique et séché pendant 12 heures sous pression réduite (5 mm de Hg; 0,7 kPa) pour donner 1,44 g de dichlorhydrate de [diéthylamino-1 propyl-2-(2RS)]-10 N-propyl phénothiazinecarboxamidine-2 fondant à 216-218°C.

RMN du proton (250 MHz, DMSO, δ en ppm, J en Hz).

0,96 (T, J = 7,5, 3H, $-(CH_2)_2C\underline{H}_3$); 1,05 à 1,25 (Mt, 6H, $-N(CH_2C\underline{H}_3)_2$); 1,68 (Mt, 2H, $-CH_2-CH_2CH_3$); 1,87 (D, J = 7,5, 3H, $-CH_3$); 3,21 (Mt, 4H, $-N(C\underline{H}_2-CH_3)_2$); 3,40 (Mt, 2H, $-NH-C\underline{H}_2-$); 3,45 (Mt, 1H, 1H du $>NCH_2-$); 3,8 (Mt, 1H, autre H du $>NCH_2-$); 5,10 (Mt, 1H, $>N-CH<$); 7 à 75 (Mt, 7H, aromatiques); 9,2 et 9,8 (2S, 1H chacun, $C=NH_2^+$); 10,23 (T, J = 5,5, 1H, -NH-); 10,67 (Mf, 1H, $-NH^+$).

Spectre infra-rouge (KBr), bandes caractéristiques en $cm^{-1}$ : 3300 à 2700, 2650, 2480, 1670, 1620, 1590, 1560, 1460, 1420, 870, 810, 755.

EXEMPLE 112

A une solution refroidie à 0-5°C de 3,49 g de dichlorhydrate de [diéthylamino-1 propyl-2-(2RS)]-10 phénothiazinecarboximidate-2 de méthyle dans 21 cm3 de méthanol, on ajoute goutte à goutte une solution de 3,18 cm3 de méthyl-2-(2RS) butylamine dans 5 cm3 de méthanol en 5 minutes. Le mélange est agité pendant 2 heures à 5°C puis concentré à sec sous pression réduite (30 mm de Hg; 4 kPa) à 50°C pour donner un résidu qui est repris par 50 cm3 d'acétate d'éthyle. La solution est lavée par 50 cm3 d'eau distillée alcalinisée à pH 9 par de la soude N. Les deux phases sont décantées et la phase organique est lavée par 50 cm3 d' une solution saturée au demi de chlorure de sodium, séchée sur sulfate de magnésium, filtrée puis concentrée à sec sous pression réduite (30 mm de Hg; 4 kPa) à 50°C. Le résidu est purifié par chromatographie sur une colonne d'alumine neutre (hauteur : 23 cm; diamètre : 2,1 cm) en éluant par des mélanges d'acétate d'éthyle et de cyclohexane 20-80 (850 cm3), 10-90 (500 cm3) puis par de l'acétate d'éthyle pur (500 cm3) enfin par des mélanges d'acétate d'éthyle et de méthanol 95-5 (500 cm3), 90-10 (500 cm3) et 80-20 (500 cm3) (en volumes) et en recueillant des fractions de 50 cm3. Le premier litre est éliminé, puis les fractions 40 à 56 sont réunies, concentrées à sec sous pression réduite (30 mm de Hg; 4 kPa) à 50°C pour donner 1,33 g de [diéthylamino-1 propyl-2-(2RS)]-10 N-[méthyl-2-(2RS) butyl] phénothiazinecarboxamidine-2 sous la forme d'une meringue jaune.

A 11 cm3 d'une solution acétonique de 1,11 g de [diéthylamino-1 propyl-2-(2RS)]-10 N-[méthyl-2-(2RS) butyl] phénothiazinecarboxamidine-2, on ajoute goutte à goutte 2,24 cm3 d'une solution acétonique d'acide chlorhydrique 3,5 N. Le mélange est agité pendant 3 heures à 25°C puis est concentré à sec sous pression réduite (30 mm de Hg; 4 kPa) à 50°C. Le résidu est repris dans 50 cm3 d'éther éthylique et la suspension est maintenue sous agitation pendant 3 heures à 25°C. Le solide est filtré sur verre fritté, lavé par 2 fois 5 cm3 d'éther éthylique et séché pendant 12 heures sous pression réduite (5 mm de Hg; 0,7 kPa) pour donner 1,07 g de dichlorhydrate de [diéthylamino-1 propyl-2-(2RS)]-10 N-[méthyl-2-(2RS) butyl] phénothiazinecarboxamidine-2 fondant à 212-214°C.

RMN du proton (250 MHz, DMSO, δ en ppm, J en Hz).

0,92 (Mt, 6H, $-CH(C\underline{H}_3)CH_2C\underline{H}_3$); 1,08 et 1,18 (2T, 6H, $-N(CH_2C\underline{H}_3)_2$); environ 1,20 et 1,5 (2Mt, 1H chacun, $-CH(CH_3)C\underline{H}_2CH_3$); 1,85 (D, J = 7,5, 3H, $-CH_3$); 1,9 (Mt, 1H, $-CH_2-C\underline{H}(CH_3)-$); 3,25 (Mt, 6H, $-N(C\underline{H}_2CH_3)_2$ et $-NH-CH_2-$); 3,45 et 3,80 (2Mt, 2H, $>NCH_2-$); 5,12 (Mf, 1H, $>N-CH<$); 7 à 7,5 (Mt, 7H, aromatiques); 9,25 et 9,85 (2S, 1H chacun, $C=NH_2^+$); 10,23 (T, J = 5, 1H, -NH-); 10,75 (Mf, 1H, $-NH^+$).

Spectre infra-rouge (KBr), bandes caractéristiques en $cm^{-1}$ :
3300 à 2700, 2660, 2490, 1670, 1620, 1595, 1560, 1460, 1420, 870, 810, 755.

EXEMPLE 113

A une solution refroidie à 0-5°C de 3,49 g de dichlorhydrate de [diéthylamino-1 propyl-2-(2RS)]-10 phénothiazinecarboximidate-2 de méthyle dans 21 cm3 de méthanol, on ajoute goutte à goutte une solution de 3,18 cm3 de benzylamine dans 5 cm3 de méthanol en 5 minutes. Le mélange est agité pendant 2 heures à 5°C puis est concentré à sec à 50°C sous pression réduite (30 mm de Hg; 4 kPa) pour donner un résidu qui est repris par 50 cm3 d'eau distillée. La solution est lavée par 50 cm3 d'eau distillée alcalinisée à pH voisin de 9 par de la soude N. Les deux phases sont décantées et la phase organique est lavée par 50 cm3 d'une solution à demi saturée de chlorure de sodium, séchée sur sulfate de magnésium, filtrée puis concentrée à sec sous pression réduite (30 mm de Hg; 4 kPa) à 50°C. Le résidu est purifié par chromatographie sur une colonne d'alumine neutre (hauteur : 24 cm; diamètre : 2,1 cm) en éluant successivement par des mélanges de cyclohexane et d'acétate d'éthyle 50-50 (500 cm3), 25-75 (500 cm3) puis par de l'acétate d'éthyle pur (500 cm3) enfin par des mélanges d'acétate d'éthyle et de méthanol 95-5 (500 cm3) et 90-10 (500 cm3) et en recueillant des fractions de 50 cm3. Les fractions 35 à 53 sont réunies, concentrées à sec sous pression réduite (30 mm de Hg; 4 kPa) à 50°C pour donner 0,96 g de N-benzyl [diéthylamino-1 propyl-2-(2RS)]-10 phénothiazinecarboxamidine-2 sous la forme d'une meringue jaune.

A 11 cm3 d'une solution acétonique de 0,74 g de N-benzyl [diéthylamino-1 propyl-2-(2RS)]-10 phénothiazinecarboxamidine-2, on ajoute goutte à goutte 1,42 cm3 d'une solution acétonique d'acide chlorhydrique 3,5 N. Le mélange est agité pendant 3 heures à 25°C puis concentré à sec sous pression réduite (30 mm de Hg; 4 kPa) à 50°C. Le résidu est repris dans 50 cm3 d'éther éthylique et la suspension est maintenue sous agitation pendant 3 heures à 25°C. Le solide est filtré sur verre fritté, lavé par 2 fois 4 cm3 d'éther éthylique et séché pendant 12 heures sous pression réduite (5 mm de Hg; 0,7 kPa) pour donner 0,76 g de dichlorhydrate de N-benzyl [diéthylamino-1 propyl-2-(2RS)]-10 phénothiazinecarboxamidine-2 fondant à 206-207°C.

RMN du proton (250 MHz, DMSO, $\delta$ en ppm, J en Hz).

1,07 et 1,18 (2T, 6H, $-N(CH_2C\underline{H_3})_2$); 1,9 (D, J = 7, 3H, $-CH_3$); 3,22 (Mt, 4H, $-N(C\underline{H_2}CH_3)_2$); 3,5 et 3,85 (2Mt, 2H, $>N-CH_2-$); 4,75 (D, J = 6, 2H, $-NH-C\underline{H_2}-$); 5,06 (Mt, 1H, $>N-CH<$); 7,05 à 7,55 (Mt, 12H, aromatiques); 9,4 et 9,95 (2S, 1H chacun, $>C=NH_2^+$); 10,43 (Mf, 1H, $-NH^+$); 10,75 (T, J = 6, -NH-).

Le dichlorhydrate de [diéthylamino-1 propyl-2-(2RS)]-10 phénothiazinecarboximidate-2 de méthyle peut être obtenu de la manière suivante :

A 80 cm3 d'une solution méthanolique de 8,0 g de [diéthylamino-1 propyl-2-(2RS)]-10 phénothiazinecarbonitrile-2 refroidie à 0-5°C et maintenue sous agitation, on fait barbotter pendant 4 heures de l'acide chlorhydrique et on laisse le mélange agiter pendant 12 heures à 25°C. Le méthanol est distillé sous pression réduite (30 mm de Hg; 4 kPa) à 30°C et le résidu est repris par 50 cm3 de méthanol anhydre. Le solvant est à nouveau évaporé sous pression réduite (30 mm de Hg; 4 kPa) et le résidu est dilué à nouveau par 50 cm3 de méthanol anhydre. Cette opération est répétée une fois encore pour donner finalement 50 cm3 d'une solution méthanolique de dichlorhydrate de [diéthylamino-1 propyl-2-(2RS)]-10 phénothiazinecarboximidate-2 de méthyle.

EXEMPLE 114

A une solution de 1,76 g de chlorhydrate de [(pyrrolidinyl-1)-1 propyl-2-(2RS)]-10 phénothiazinecarboxamidine-2 dans 17 cm3 d'éthanol sec, on ajoute 0,44 cm3 de chloracétone et 1 g de tamis moléculaire 4 Å. La suspension est maintenue au reflux pendant 20 heures. Après refroidissement, la suspension est filtrée, concentrée à sec à 40°C sous pression réduite (30 mm de Hg; 4 kPa) pour donner un résidu qui est dilué dans 200 cm3 de dichloro-1,2 éthane. La phase organique est lavée par 3 fois 100 cm3 d'eau distillée, puis par 100 cm3 de solution saturée de chlorure de sodium, séchée sur sulfate de magnésium, filtrée et concentrée à sec à 40°C sous pression réduite (30 mm de Hg; 4 kPa) pour donner un résidu qui est purifié par chromatographie sur une colonne (hauteur : 15 cm; diamètre : 2,4 cm) de silice (0,04-0,06 mm) en éluant par un mélange de dichloro-1,2 éthane et de méthanol 90-10 en volumes (800 cm3) et en recueillant des fractions de 50 cm3. Les 300 premiers cm3 d'éluat sont éliminés et les fractions 4 à 10 sont concentrées à sec à 40°C sous pression réduite (30 mm de Hg; 4 kPa) pour donner 0,33 g de (méthyl-4 imidazolyl-2)-2 [(pyrrolidinyl-1)-1 propyl-2-(2RS)]-10 phénothiazine sous la forme d'un solide jaune d'or.

A une solution de 0,30 g de (méthyl-4 imidazolyl-2)-2 [(pyrrolidinyl-1)-1 propyl-2-(2RS)]-10 phénothiazine dans 3 cm3 d'éthanol anhydre, on ajoute 0,64 cm3 d'éther chlorhydrique 1,2N. Le mélange est agité pendant 5 minutes puis il est concentré à sec à 40°C sous pression réduite (30 mm de Hg; 4 kPa) pour donner un résidu qui est repris dans 70 cm3 d'éther éthylique. Le solide obtenu est filtré sur verre fritté pour donner 0,35 g de chlorhydrate de (méthyl-4 imidazolyl-2)-2 [(pyrrolidinyl-1)-1 propyl-2-(2RS)]-10 phénothiazine fondant à 210°C sous la forme d'une poudre marron.

RMN du proton (250 MHz, DMSO, $\delta$ en ppm, J en Hz).

En solution dans le DMSO, on observe deux formes dues à la salification de l'azote.

1,88 (D, J = 7, 3H, $-CH_3$); 1,75 à 2,1 (Mt, 4H, $-CH_2-CH_2-$); 2,4 (S, 3H, $-CH_3$ du méthyl-4 imidazolyle-2); 2,98 -3,16 - 3,64 et 3,78 (4Mf, 1H chacun, $-CH_2-N-CH_2-$ de la pyrrolidine); 3,95 (Mf, 2H, >N-$CH_2$-); 4,77 (Mt, 1H, >N-CH<); 7,05 à 7,55 (Mt, 5H, aromatiques); 7,51 (S, 1H, -CH= du méthyl-4 imidazolyle-2); 7,73 (S large, 1H, -H en 1); 7,78 (D large, J = 8, 1H, -H en 3); 10,58 (Mf, 1H, -NH- du méthyl-4 imidazolyle-2); 15,1 et 15,3 (2Mf, 1H en totalité, $-NH^+$).

Spectre infra-rouge (KBr), bandes caractéristiques en $cm^{-1}$ : 2720, 2630, 1640, 1595, 1575, 1460, 860, 820, 755.

Le chlorhydrate de [(pyrrolidinyl-1)-1 propyl-2-(2RS)]-10 phénothiazinecarboxamide-2 peut être préparé de la manière suivante :

A une solution de 13,63 g de dichlorhydrate de [(pyrrolidinyl-1)-1 propyl-2-(2RS)]-10 phénothiazinecarboximidate-2 d'éthyle dans 150 cm3 d'éthanol absolu, on ajoute 8,4 cm3 de triéthylamine et on dilue le mélange réactionnel par 100 cm3 d'éthanol sec. On fait barbotter pendant 4 heures de l'ammoniac gazeux dans le mélange réactionnel qui est ensuite agité pendant 12 heures à 25°C. La suspension obtenue est filtrée sur verre fritté et le filtrat est concentré à sec à 25°C sous pression réduite (30 mm de Hg; 4 kPa) pour donner une meringue jaune qui est purifié par chromatographie sur colonne d'alumine (hauteur : 23 cm; diamètre : 4 cm) en éluant par des mélanges de chlorure de méthylène et de méthanol 95-5 (750 cm3), 50-50 (1500 cm3) et en recueillant des fractions de 125 cm3. Les 900 premiers cm3 d'éluat sont éliminés et les fractions 8 à 11 sont réunies et concentrées à sec à 25°C sous pression réduite (30 mm de Hg; 4 kPa) pour donner 4,5 g de chlorhydrate de [(pyrrolidinyl-1)-1 propyl-2-(2RS)]-10 phénothiazinecarboxamidine-2 sous la forme d'une meringue jaune.

On dissous 4,3 g de chlorhydrate de [(pyrrolidinyl-1)-1 propyl-2-(2RS)]-10 phénothiazinecarboxamidine-2 dans 150 cm3 d'acétone au reflux. La cristallisation est amorcée par grattage. La suspension est maintenue sous agitation lors du refroidissement. Après filtration sur verre fritté, on obtient 3,35 g de chlorhydrate de [(pyrrolidinyl-1)-1 propyl-2-(2RS)]-10 phénothiazinecarboxamidine-2 sous la forme de cristaux jaunes fondant à 228-30°C.

EXEMPLE 115

A une solution de 3,89 g de chlorhydrate de [(pyrrolidinyl -1)-1 propyl-2-(2RS)]-10 phénothiazinecarboxamidine-2 dans 30 cm3 d'éthanol, on ajoute sous agitation 1,68 g d'hydrogénocarbonate de sodium puis 2 g de tamis moléculaire 3 Å et on porte à reflux pendant 1 heure. On verse alors sous agitation et à une température voisine de 70°C, une solution de 1,84 g de bromo-1 butanone-2 dans 25 cm3 d'éthanol goutte à goutte pendant 6 heures et on poursuit l'agitation au reflux pendant 1 heure. Après refroidissement, la suspension obtenue est filtrée et l'insoluble est lavé avec 2 fois 10 cm3 d'éthanol. Les solutions éthanoliques sont réunies et concentrées à sec sous pression réduite (30 mm de Hg; 4 kPa) à 40°C. Le résidu huileux est repris par 100 cm3 de chlorure de méthylène et l'insoluble est éliminé par filtration. La solution chlorométhylénique est concentrée à sec sous pression réduite (30 mm de Hg; 4 kPa) à 20°C. Le résidu est repris par 125 cm3 d'oxyde d'isopropyle et la suspension obtenue est agitée pendant 1 heure. Le solide formé est séparé par filtration, lavé par 3 fois 15 cm3 d'oxyde d'isopropyle, essoré et séché sous pression réduite (1mm de Hg; 0,13 kPa) pour donner 3 g d'un solide jaune qui est purifié par chromatographie sur colonne (hauteur: 47 cm; diamètre: 4 cm) de gel de silice (0,04-0,063 mm) sous légère surpression d'azote (40kPa) en éluant avec 1 litre d'un mélange de chlorure de méthylène et de méthanol (80-20 en volumes) et en recueillant des fractions de 100 cm3. Les fractions 3 à 7 sont réunies et concentrées à sec sous pression réduite (30 mm de Hg; 4 kPa) à 40°C pour donner 1 g d'une meringue jaune. Cette meringue est dissoute dans 50 cm3 d'eau distillée additionnée de 20 cm3 d'une solution aqueuse d'acide chlorhydrique N/10 et la solution est lavée par 3 fois 20 cm3 d'acétate d'éthyle. La phase aqueuse est alcalinisée par de la lessive de soude et extraite par 3 fois 50 cm3 d'acétate d'éthyle. Les phases organiques sont réunies, lavées successivement par 50 cm3 d'eau distillée puis par 50 cm3 d'une solution aqueuse demi-saturée de chlorure de sodium, séchées sur sulfate de magnésium, filtrées et concentrées à un volume de 10 cm3 sous pression réduite (30 mm de Hg; 4 kPa) à 40°C. Après 16 heures de repos à une température voisine de 20°C, le solide formé est essoré, lavé par 2 cm3 d'acétate d'éthyle et séché sous pression réduite (5 mm de Hg;0,67 kPa) à 40°C. On obtient ainsi 0,6 g de [(pyrrolidinyl-1)-1 propyl-2-(2RS)]-10 (éthyl-4 imidazolyl-2)-2 phénothiazine sous forme d'un solide blanc fondant à 197°C.

RMN du proton (250 MHz, DMSO D6, δ en ppm, J en Hz).

1,24 (T, J = 7, 3H, $-CH_2C\underline{H}_3$) ; 1,66 (D, J = 7, 3 H, $-CH_3$) ; 1,7 (Mt, 4 H, $-CH_2-$ de la pyrrolidine) ; 2,55 (Mt, en partie masqué, >N-$CH_2$- de la pyrrolidine et $-C\underline{H}_2CH_3$) ; 2,98 (AB limite, 2H, >$NCH_2$-) ; 4,20 (Mt, 1H, >NCH<) ; 6,73 (S, 1H, -CH= de l'imidazole) ; 6,90 à 7,25 (Mt, 5H, aromatiques) ; 7,46 (D large, J = 8, 1H, -H en 3) ; 7,66

(S large, 1 H, -H en 1) ; 12,2 (Mf, 1H, -NH-).

Spectre infra-rouge (KBr), bandes caractéristiques en $cm^{-1}$ : 3175, 2150, 2965, 2935, 2875, 2790, 1580,1570,1460, 875, 815, 750.

La présente invention concerne également les compositions pharmaceutiques constituées par un produit de formule générale (I), sous forme libre ou sous forme de sel d'addition avec un acide pharmaceutiquement acceptable, à l'état pur ou sous forme d'une association avec tout autre produit pharmaceutiquement compatible, pouvant être inerte ou physiologiquement actif. Les médicaments selon l'invention peuvent être utilisés par voie parentérale, orale, rectale ou topique.

Les compositions stériles pour administration parentérale qui peuvent être notamment utilisées sous forme de perfusions sont de préférence des solutions aqueuses ou non aqueuses, des suspensions ou des émulsions. Comme solvant ou véhicule, ou peut employer l'eau, le propylèneglycol, un polyéthylèneglycol, des huiles végétales, en particulier l'huile d'olive, des esters organiques injectables, par exemple d'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration topique peuvent être par exemple des crèmes, des pommades, ou des lotions.

En thérapeutique humaine, les produits selon l'invention sont particulièrement utiles dans le traitement des douleurs d'origine traumatique, post-chirurgicale, homotopique, menstruelle, céphaliques..., ainsi que dans les traitements diurétiques. Les doses dépendent de l'effet recherché et de la durée du traitement. Pour un adulte, elles sont généralement comprises entre 0,25 et 1500 mg par jour en prises échelonnées.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

EXEMPLE A

On prépare, selon la technique habituelle, des comprimés dosés à 25 mg de produit actif (base) ayant la composition suivante :

- chlorhydrate de N-(chloro-2 benzyl) [(pyrrolidinyl-1)-1 propyl-2]-10 phénothiazinecarboxamide-2, série L............26,9 mg
- amidon................................................83 mg
- silice................................................30 mg
- stéarate de magnésium................................ 3 mg

EXEMPLE B

On prépare selon la technique habituelle une solution pour administration intra-veineuse dosée à 25 mg/cm3 de produit actif (base) :

| | |
|---|---|
| - chlorhydrate de N-(chloro-2 benzyl) [(pyrrolidinyl-1)-1 propyl-2]-10 phénothiazinecarboxamide-2, série L.. | 2,69 g |
| - acide ascorbique................................ | 0,100 g |
| - sulfite neutre de sodium.......................... | 0,050 g |
| - soude 1N (q.s.p. pH 4)......................environ | 0,08 cm3 |
| - NaCl (q.s.p. isotonie)......................environ | 0,650 g |
| - eau pour préparation injectable............q.s.p.. | 100 cm3 |

## Revendications

### Revendications pour les Etats contractants suivants:AT, BE, CH, DE, FR, GB, IT, LI, LU, NL, SE

**1.** Un dérivé de la phénothiazine, caractérisé en ce qu'il répond à la formule générale :

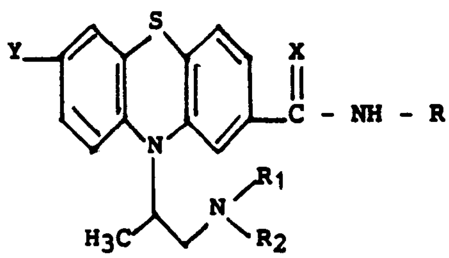

dans laquelle :
le symbole Y est un atome d'hydrogène ou d'halogène, les symboles $R_1$ et $R_2$ identiques ou différents représentent des radicaux alcoyle, cycloalcoylalcoyle, hydroxyalcoyle ou acétyloxyalcoyle ou forment ensemble avec l'atome d'azote auquel ils sont rattachés, un hétérocycle saturé ou partiellement insaturé contenant 4 à 7 chaînons et éventuellement substitué par 1 ou 2 radicaux alcoyle, hydroxyalcoyle ou acétyloxyalcoyle et

- soit le symbole X représente un atome d'oxygène ou de soufre ou un radical N-$R_4$, et le symbole R représente un radical cycloalcoyle contenant 4 à 6 chaînons ou phényle ou représente un radical -$CH_2R_3$ dans lequel $R_3$ est un atome d'hydrogène, un radical alcoyle contenant 1 à 5 atomes de carbone, un radical ou alcynyle contenant 2 à 4 atomes de carbone, un radical cycloalcoyle contenant 3 à 6 chaînons, ou un radical phényle éventuellement substitué (par 1 ou 2 atome d'halogène ou par un radical hydroxy, alcoyle, alcoyloxy, trifluro- méthyle ou nitro), ou un radical hétérocyclyle choisi parmi furyle, thiényle ou pyridyle, et le symbole $R_4$ est un atome d'hydrogène ou un radical cyano, étant entendu que le symbole X ne peut pas représenter l'atome d'oxygène lorsque simultanément $R_3$ est un atome d'hydrogène ou un radical alcoyle, $R_1$ et $R_2$ sont des radicaux alcoyle ou forment ensemble un hétérocycle azoté non substitué et Y est un atome d'hydrogène,
- soit le symbole X est un radical N-$R_4$ et le symbole R forme avec $R_4$ et les atomes auquels il sont rattachés, un radical imidazolinyle ou imidazolyle éventuellement substitués par 1 ou 2 radicaux alcoyle, ou un radical hexahydrobenzimidazolyle, étant entendu que (sauf mention spéciale) les radicaux alcoyle sont droits ou ramifiés et contiennent 1 à 4 atomes de carbone, sous ses formes isomères et leurs mélanges,ainsi que ses sels d'addition avec les acides.

**2.** Un dérivé de la phénothiazine selon la revendication 1 caractérisé en ce que :
le symbole Y est un atome d'hydrogène ou d'halogène,
les symboles $R_1$ et $R_2$ identiques ou différents représentent des radicaux alcoyle contenant 1 à 3 atomes de carbone en chaîne droite ou ramifiée, alcoyle contenant 1 ou 2 atomes de carbone substitué par cycloalcoyle, hydroxy ou acétyloxy ou forment ensemble avec l'atome d'azote auquel ils sont rattachés, un

hétérocycle saturé ou partiellement insaturé contenant 4 à 7 chaînons et éventuellement substitué par 1 ou 2 radicaux méthyle, ou alcoyle contenant 1 ou 2 atomes de carbone substitué par hydroxy ou acétyloxy,

- soit le symbole X représente un atome d'oxygène ou de soufre ou un radical N-$R_4$, et le symbole R représente un radicale cycloalcoyle contenant 4 à 6 chaînons ou phényle ou représente un radical -$CH_2R_3$ dans lequel $R_3$ est un atome d'hydrogène, un radical alcoyle droit ou ramifié contenant 1 à 5 atomes de carbone, un radical alcényle ou alcynyle contenant 2 à 4 atomes de carbone, un radical cycloalcoyle contenant 3 à 6 chaînons, ou un radical phényle éventuellement substitué (par 1 ou 2 atomes d'halogène ou par un radical hydroxy, méthyle, méthoxy, trifluorométhyle ou nitro), ou un radical hétérocyclyle choisi parmi furyle, thiényle ou pyridyle, et le symbole $R_4$ est un atome d'hydrogène ou un radical cyano, étant entendu que le symbole X ne peut pas représenter l'atome d'oxygène lorsque simultanément $R_3$ est un atome d'hydrogène ou un radical alcoyle, $R_1$ et $R_2$ sont des radicaux alcoyle ou forment ensemble un hétérocycle azoté non substitué et Y est un atome d'hydrogène,
- soit le symbole X est un radical N-$R_4$ et le symbole R forme avec $R_4$ et les atomes auxquels ils sont rattaché, un radical imidazolyle éventuellement substitué par 1 ou 2 radicaux alcoyle, contenant 1 ou 2 atomes de carbone ou un radical hexahydrobenzimidazolyle, sous ses formes isomères et leurs mélanges, ainsi que ses sels d'addition avec les acides.

3. Un dérivé de la phénothiazine selon la revendication 1 caractérisé en ce que :
le symbole Y est un atome d'hydrogène ou d'halogène,
les symboles $R_1$ et $R_2$ identiques ou différents représentent des radicaux alcoyle contenant 1 ou 2 atomes de carbone à l'exception de représenter simultanément des radicaux méthyle, acétyloxyalcoyle dont la partie alcoyle contient 1 ou 2 atomes de carbone ou forment ensemble avec l'atome d'azote auquel ils sont rattachés, un hétérocycle saturé ou partiellement insaturé contenant 4 à 7 chaînons et éventuellement substitué par 1 ou 2 radicaux méthyle, hydroxyméthyle ou acétyloxyméthyle et,
   - soit le symbole X représente un atome d'oxygène ou de soufre ou un radical N-$R_4$, et le symbole R représente un radical cycloalcoyle contenant 4 à 6 chaînons ou représente un radical -$CH_2R_3$ dans lequel $R_3$ est un un radical alcoyle droit ou ramifié contenant 1 à 5 atomes de carbone, un radical alcényle ou alcynyle contenant 2 à 4 atomes de carbone, un radical cycloalcoyle contenant 3 à 6 chaînons, ou un radical phényle éventuellement substitué (par 1 ou 2 atomes d'halogène ou par un radical hydroxy, méthyle, méthoxy, trifluorométhyle ou nitro), ou un radical hétérocyclyle choisi parmi furyle, thiényle ou pyridyle, et le symbole $R_4$ est un atome d'hydrogène ou un radical cyano, étant entendu que le symbole X ne peut pas représenter l'atome d'oxygène lorsque simultanément $R_3$ est un atome d'hydrogène ou un radical alcoyle, $R_1$ et $R_2$ sont des radicaux alcoyle ou forment ensemble un hétérocycle azoté non substitué et Y est un atome d'hydrogène,
   - soit le symbole X est un radical N-$R_4$ et le symbole R forme avec $R_4$ et les atomes auxquels ils sont rattachés, un radical imidazolyle éventuellement substitué par 1 radical alcoyle, contenant 1 ou 2 atomes de carbone ou un radical hexahydrobenzimidazolyle, sous forme L ou sous forme d'un mélange d'isomères, ainsi que ses sels d'addition avec les acides.

4. Le N-cyclobutylméthyl [(pyrrolidinyl-1)-1 propyl-2] -10 phénothiazinecarboxamide-2, sous forme L sous forme d'un mélange d'isomères, ainsi que ses sels d'addition avec les acides.

5. Le N-(chloro-3 benzyl) [(pyrrolidinyl-1)-1 propyl-2]-10 phénothiazine carboxamide-2, sous forme L sous forme d'un mélange d'isomères, ainsi que ses sels d'addition avec les acides.

6. Le N-(chloro-2 benzyl) [(pyrrolidinyl-1)-1 propyl-2]-10 phénothiazine carboxamide-2, sous forme L sous forme d'un mélange d'isomères, ainsi que ses sels d'addition avec les acides.

7. Le N-(fluoro-2 benzyl) [(pyrrolidinyl-1)-1 propyl-2]-10 phénothiazine carboxamide-2, sous forme L sous forme d'un mélange d'isomères, ainsi que ses sels d'addition avec les acides.

8. Le N-benzyl [(pyrrolidinyl-1)-1 propyl-2]-10 phénothiazine carboxamide, sous forme L sous forme d'un mélange d'isomères, ainsi que ses sels d'addition avec les acides.

9. Procédé de préparation d'un dérivé de la phénothiazine selon la revendication 1, pour lequel X est défini comme dans la revendication 1 à l'exception de représenter un radical N-$R_4$ dans lequel $R_4$ forme avec R et les atomes voisins un radical imidazolyle, caractérisé en ce que l'on fait agir une amine de formule générale :

$$R - NH_2$$

dans laquelle R est défini comme dans la revendication 1, ou une diamine (si X représente un radical NH et si l'on veut obtenir un dérivé de la phénothiazine dans lequel R et $R_4$ sont liés ensemble pour former avec les atomes d'azote et de carbone auquels ils sont rattachés, un radical imidazolinyle éventuellement substitué ou hexahydrobenzimidazolyle), sur un dérivé de la phénothiazine de formule générale :

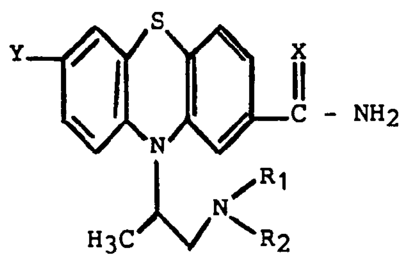

dans laquelle Y, $R_1$, $R_2$ sont définis comme dans la revendication 1 et X est un atome de soufre ou un radical N-$R_4$ dans lequel $R_4$ est défini selon la revendication 1, puis le cas échéant, lorsque l'on veut obtenir l'amide pour lequel X est un atome d'oxygène, si l'on a isolé intermédiairement le thioamide substitué pour lequel X est un atome de soufre, on oxyde ce thioamide en l'amide correspondant par toute méthode connue qui n'altère pas le reste de la molécule, puis transforme éventuellement le produit obtenu en un sel d'addition avec un acide.

**10.** Procédé de préparation d'un dérivé de la phénothiazine selon la revendication 1 pour lequel X est un atome d'oxygène et Y, $R_1$, $R_2$ et R sont définis comme dans la revendication 1, caractérisé en ce que l'on transforme un acide de formule générale :

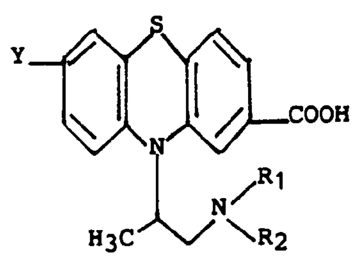

dans laquelle Y, $R_1$ et $R_2$ sont définis comme dans la revendication 1, ou un dérivé réactif de cet acide, par action d'une amine de formule générale :

$$R - NH_2$$

dans laquelle R est défini comme dans la revendication 1, puis transforme éventuellement le produit obtenu en un sel d'addition avec un acide.

**11.** Procédé de préparation d'un dérivé de la phénothiazine selon la revendication 1, pour lequel X est un atome d'oxygène et Y, $R_1$, $R_2$ et R sont définis comme dans la revendication 1, caractérisé en ce que l'on transforme un nitrile de formule générale :

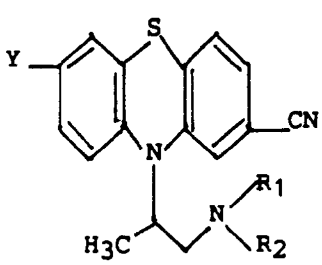

dans laquelle Y, $R_1$ et $R_2$ sont définis comme dans la revendication 1, par toute méthode connue pour

obtenir un amide substitué à partir d'un nitrile, sans toucher au reste de la molécule, puis transforme éventuellement le produit obtenu en un sel d'addition avec un acide.

**12.** Procédé de préparation d'un dérivé de la phénothiazine selon la revendication 1, pour lequel X est un radical N-$R_4$ pour lequel $R_4$ est un atome d'hydrogène et R est défini comme dans la revendication 1, ou $R_4$ forme avec R et les atomes auquels ils sont rattachés un radical imidazolinyle éventuellement substitué ou un radical hexahydrobenzimidazolyle et les autres radicaux sont définis comme dans la revendication 1, caractérisé en ce que l'on fait agir une amine de formule générale :

$$R - NH_2$$

dans laquelle R est défini comme dans la revendication 1, ou une diamine (lorsque l'on veut obtenir un produit selon la revendication 1 pour lequel R et $R_4$ forment avec les atomes voisins un radical dérivé de l'imidazole), sur un iminoéther de formule formule :

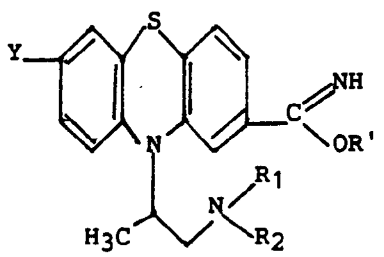

dans laquelle Y, $R_1$ et $R_2$ sont définis comme dans la revendication 1 et R' est un radical alcoyle (contenant 1 à 10 atomes de carbone), ou sur le sel de ce produit, puis transforme éventuellement le produit obtenu en un sel d'addition avec un acide.

**13.** Procédé de préparation d'un dérivé de la phénothiazine selon la revendication 1, pour lequel X est un radical N-$R_4$ dans lequel $R_4$ forme avec R un radical imidazolyle éventuellement substitué par un radical alcoyle, caractérisé en ce que l'on fait agir une α-halogénocétone ou un α-halogénoaldéhyde de formule générale :

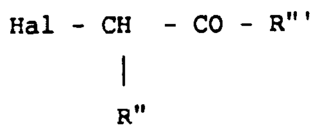

dans laquelle Hal est un atome d'halogène et R'' et R''' identiques ou différents représentent un atome d'hydrogène ou un radical alcoyle, sur un dérivé de la phénothiazine de formule générale :

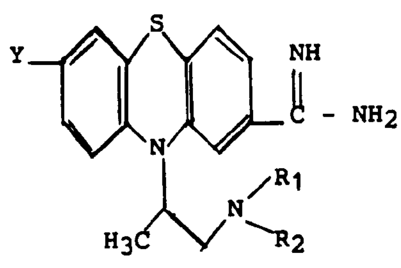

dans laquelle Y, $R_1$ et $R_2$ sont définis comme dans la revendication 1, ou sur son sel, puis transforme éventuellement le produit obtenu en un sel d'addition avec un acide.

**14.** Composition pharmaceutique caractérisée en ce qu'elle contient un produit selon la revendication 1 à l'état pur ou sous forme d'association avec un ou plusieurs diluants ou adjuvants compatibles et pharceutiquement acceptables.

**Revendications pour les Etats contractants suivants: ES, GR**

1. Procédé de préparation d'un dérivé de la phénothiazine, de formule générale :

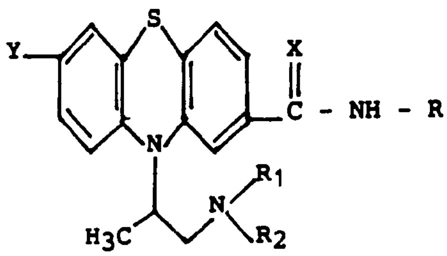

dans laquelle :
le symbole Y est un atome d'hydrogène ou d'halogène, les symboles $R_1$ et $R_2$ identiques ou différents représentent des radicaux alcoyle, cycloalcoylalcoyle, hydroxyalcoyle ou acétyloxyalcoyle ou forment ensemble avec l'atome d'azote auquel ils sont rattachés, un hétérocycle saturé ou partiellement insaturé contenant 4 à 7 chaînons et éventuellement substitué par 1 ou 2 radicaux alcoyle, hydroxyalcoyle ou acétyloxyalcoyle et
   - soit le symbole X représente un atome d'oxygène ou de soufre ou un radical $N-R_4$, et le symbole R représente un radical cycloalcoyle contenant 4 à 6 chaînons ou phényle ou représente un radical $-CH_2R_3$ dans lequel $R_3$ est un atome d'hydrogène, un radical alcoyle contenant 1 à 5 atomes de carbone, un radical alcényle ou alcynyle contenant 2 à 4 atomes de carbone, un radical cycloalcoyle contenant 3 à 6 chaînons, ou un radical phényle éventuellement substitué (par 1 ou 2 atomes d'halogène ou par un radical hydroxy, alcoyle, alcoyloxy, trifluro- méthyle ou nitro), ou un radical hétérocyclyle choisi parmi furyle, thiényle ou pyridyle, et le symbole $R_4$ est un atome d'hydrogène ou un radical cyano, étant entendu que le symbole X ne peut pas représenter l'atome d'oxygène lorsque simultanément $R_3$ est un atome d'hydrogène ou un radical alcoyle, $R_1$ et $R_2$ sont des radicaux alcoyle ou forment ensemble un hétérocyle azoté non substitué et Y est un atome d'hydrogène,
   - soit le symbole X est un radical $N-R_4$ et le symbole R forme avec $R_4$ et les atomes auquels ils sont rattachés, un radical imidazolinyle éventuellement substitués par 1 ou 2 radicaux alcoyle, ou un radical hexahydrobenzimidazolyle, les radicaux alcoyle étant, sauf mention spéciale, droits ou ramifiés et contenant 1 à 4 atomes de carbone, sous ses formes isomères et leurs mélanges, caractérisé en ce que l'on fait agir une amine de formule générale :

$$R-NH_2$$

dans laquelle R est défini comme ci-dessus, ou une diamine (si X représente un radical NH et si l'on veut obtenir un dérivé de la phénothiazine dans lequel R et $R_4$ sont liés ensemble pour former avec les atomes d'azote et de carbone auquels ils sont rattachés, un radical imidazolinyle éventuellement substitué ou hexahydrobenzimidazolyle), sur un dérivé de la phénothiazine de formule générale :

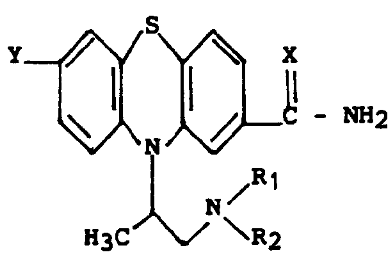

dans laquelle Y, $R_1$, $R_2$ sont définis comme ci-dessus et X est un atome de soufre ou un radical $N-R_4$ dans lequel $R_4$ est défini comme ci-dessus, puis le cas échéant, lorsque l'on veut obtenir l'amide pour lequel X est un atome d'oxygène, si l'on a isolé intermédiairement le thioamide substitué pour lequel X est un atome de soufre, on oxyde ce thioamide en l'amide correspondant par toute méthode connue qui n'altère pas le reste de la molécule, puis transforme éventuellement le produit obtenu en un sel d'addition avec un acide.

2. Procédé de préparation d'un dérivé de la phénothiazine de formule générale :

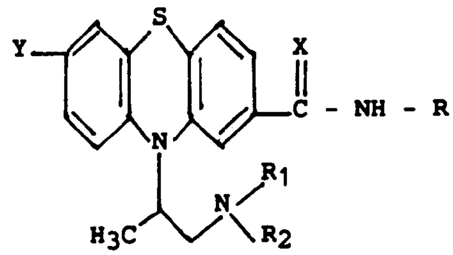

dans laquelle :
le symbole Y est un atome d'hydrogène ou d'halogène, les symboles $R_1$ et $R_2$ identiques ou différents représentent des radicaux alcoyle, cycloalcoylalcoyle, hydroxyalcoyle ou acétyloxyalcoyle ou forment ensemble avec l'atome d'azote auquel ils sont rattachés, un hétérocycle saturé ou partiellement insaturé contenant 4 à 7 chaînons et éventuellement substitué par 1 ou 2 radicaux alcoyle, hydroxyalcoyle ou acétyloxyalcoyle et
-soit le symbole X représente un atome d'oxygène et le symbole R représente un radical cycloalcoyle contenant 4 à 6 chaînons ou phényle ou représente un radical $-CH_2R_3$ dans lequel $R_3$ est un atome d'hydrogène, un radical alcoyle contenant 1 à 5 atomes de carbone, un radical alcényle ou alcynyle contenant 2 à 4 atomes de carbone, un radical cycloalcoyle contenant 3 à 6 chaînons, ou un radical phényle éventuellement substitué (par 1 ou 2 atomes d'halogène ou par un radical hydroxy, alcoyle, alcoyloxy, trifluorométhyle ou nitro), ou un radical hétérocyclyle choisi parmi furyle, thiényle ou pyridyle, étant entendu que $R_3$ ne peut pas représenter hydrogène ou alcoyle si $R_1$ et $R_2$ sont alcoyle ou forment un hétérocycle azoté non substitué et Y est un atome d'hydrogène, les radicaux alcoyle étant, sauf mention spéciale, droits ou ramifiés et contenant 1 à 4 atomes de carbone, sous ses formes isomères et leurs mélanges caractérisé en ce que l'on transforme un acide de formule générale :

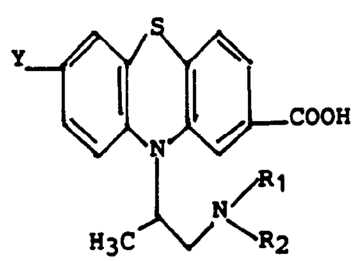

dans laquelle Y, $R_1$ et $R_2$ sont définis comme ci-dessus, ou un dérivé réactif de cet acide, par action d'une amine de formule générale :

$$R - NH_2$$

dans laquelle R est défini comme ci-dessus, puis transforme éventuellement le produit obtenu en un sel d'addition avec un acide.

3. Procédé de préparation d'un dérivé de la phénothiazine, pour lequel X, Y, $R_1$, $R_2$ et R sont définis comme dans la revendication 2, caractérisé en ce que transforme un nitrile de formule générale :

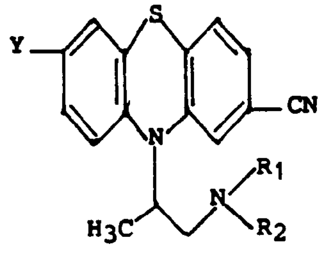

dans laquelle Y, $R_1$ et $R_2$ sont définis comme ci-dessus, par toute méthode connue pour obtenir un amide substitué à partir d'un nitrile, sans toucher au reste de la molécule, puis transforme éventuellement le produit obtenu en un sel d'addition avec un acide.

4. Procédé de préparation d'un dérivé de la phénothiazine de formule générale :

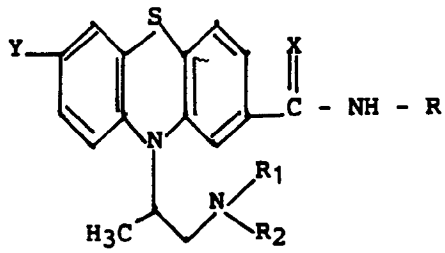

dans laquelle :
le symbole Y est un atome d'hydrogène ou d'halogène, les symboles $R_1$ et $R_2$ identiques ou différents représentent des radicaux alcoyle, cycloalcoylalcoyle, hydroxyalcoyle ou acétyloxyalcoyle ou forment ensemble avec l'atome d'azote auquel ils sont rattachés, un hétérocycle saturé ou partiellement insaturé contenant 4 à 7 chaînons et éventuellement substitué par 1 ou 2 radicaux alcoyle, hydroxyalcoyle ou acétyloxyalcoyle, X est un radical N-$R_4$ pour lequel $R_4$ est un atome d'hydrogène et R représente un radical cycloalcoyle contenant 4 à 6 chaînons ou phényle ou représente un radical -$CH_2R_3$ dans lequel $R_3$ est un atome d'hydrogène, un radical alcoyle contenant 1 à 5 atomes de carbone, un radical alcényle ou alcynyle contenant 2 à 4 atomes de carbone, un radical cycloalcoyle contenant 3 à 6 chaînons, ou un radical phényle éventuellement substitué (par 1 ou 2 atomes d'halogène ou par un radical hydroxy, alcoyle, alcoyloxy, trifluoro- méthyle ou nitro), ou un radical hétérocyclyle choisi parmi furyle, thiényle ou pyridyle, ou $R_4$ forme avec R est les atomes auxquels ils sont rattachés un radical imidazolinyle éventuellement substitué par 1 ou 2 radicaux alcoyle, ou un radical hexahydrobenzimidazolyle, les radicaux alcoyles étant, sauf mention spéciale, droits ou ramifiés et contenant 1 à 4 atomes de carbone, sous ses formes isomères et leurs mélanges, caractérisé en ce que l'on fait agir une amine de formule générale :

$$R - NH_2$$

dans laquelle R est défini comme ci-dessus, ou une diamine (lorsque l'on veut obtenir un produit pour lequel R et $R_4$ forment avec les atomes voisins un radical dérivé de l'imidazole), sur un iminoéther de formule :

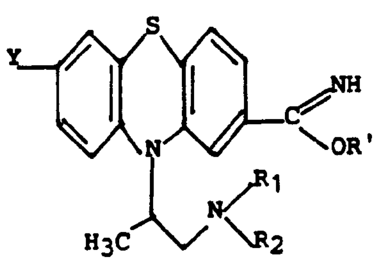

dans laquelle Y, $R_1$ et $R_2$ sont définis comme précédemment et R' est un radical alcoyle (contenant 1 à 10 atomes de carbone), ou sur le sel de ce produit, puis transforme éventuellement le produit obtenu en un sel d'addition avec un acide.

5. Procédé de préparation d'un dérivé de la phénothiazine de formule générale :

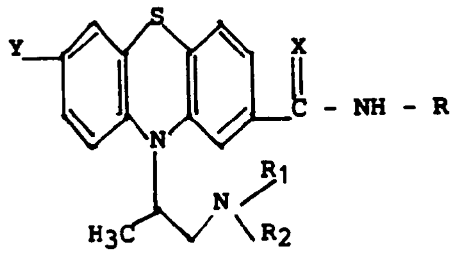

dans laquelle :
le symbole Y est un atome d'hydrogène ou d'halogène, les symboles $R_1$ et $R_2$ identiques ou différents représentent des radicaux alcoyle, cycloalcoylalcoyle, hydroxyalcoyle ou acétyloxyalcoyle ou forment ensemble avec l'atome d'azote auquel ils sont rattachés, un hétérocycle saturé ou partiellement insaturé contenant 4 à 7 chaînons et éventuellement substitué par 1 ou 2 radicaux alcoyle, hydroxyalcoyle ou acétyloxyalcoyle et X est un radical N-$R_4$ dans lequel $R_4$ forme avec R un radical imidazolyle éventuellement substitué par un radical alcoyle, les radicaux alcoyles étant, sauf mention spéciale, droits ou ramifiés et contenant 1 à 4 atomes de carbone, sous ses formes isomères et leurs mélanges, caractérisé en ce que l'on fait agir une α-halogénocétone ou un α-halogénoaldéhyde de formule générale :

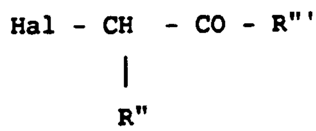

dans laquelle Hal est un atome d'halogène et R" et R‴ identiques ou différents représentent un atome d'hydrogène ou un radical alcoyle, sur un dérivé de la phénothiazine de formule générale :

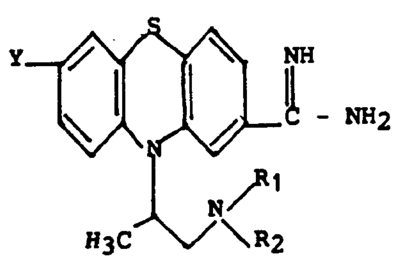

dans laquelle Y, $R_1$ et $R_2$ sont définis comme précédemment, ou sur son sel, puis transforme éventuellement le produit obtenu en un sel d'addition avec un acide.

## Patentansprüche

### Patentansprüche für folgende Vertragsstaaten: AT, BE, CH, DE, FR, GB, IT, LI, LU, NL, SE

**1.** Phenothiazinderivat, dadurch gekennzeichnet, daß es der allgemeinen Formel

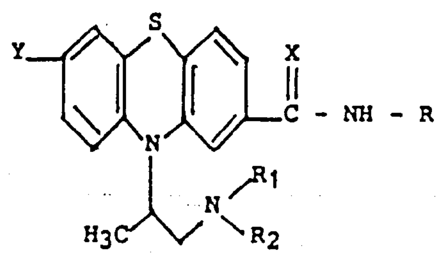

entspricht, worin:
das Symbol Y ein Wasserstoff- oder Halogenatom ist, die Symbole $R_1$ und $R_2$, identisch oder voneinander verschieden, Alkyl-, Cycloalkalkyl-, Hydroxyalkyl- oder Acetoxyalkylreste wiedergeben oder gemeinsam mit dem Stickstoffatom, an das sie gebunden sind, einen gesättigten oder teilweise ungesättigten Heterocyclus mit 4 bis 7 Kettengliedern, der gegebenenfalls durch 1 oder 2 Alkyl-, Hydroxyalkyl- oder Acetoxyalkylreste substituiert ist, bilden, und

- entweder das Symbol X ein Sauerstoff- oder Schwefelatom oder einen Rest N-$R_4$ bedeutet, und das Symbol R einen Cycloalkylrest mit 4 bis 6 Kettengliedern oder einen Phenylrest wiedergibt oder einen Rest -$CH_2R_3$ bedeutet, worin $R_3$ ein Wasserstoffatom, ein Alkylrest mit 1 bis 5 Kohlenstoffatomen, ein Alkenyl- oder Alkinylrest mit 2 bis 4 Kohlenstoffatomen, ein Cycloalkylrest mit 3 bis 6 Kettengliedern oder ein Phenylrest, der gegebenenfalls substituiert ist (durch 1 oder 2 Halogenatome oder durch eine Hydroxy-, Alkyl-, Alkoxy-, Trifluormethyl- oder Nitrogruppe) oder ein heterocyclischer Rest, ausgewählt unter Furyl, Thienyl oder Pyridyl, ist, und das Symbol $R_4$ ein Wasserstoffatom oder eine Cyanogruppe bedeutet, mit der Maßgabe, daß das Symbol X kein Sauerstoffatom bedeuten kann, wenn gleichzeitig $R_3$ ein Wasserstoffatom oder eine Alkylgruppe ist, $R_1$ und $R_2$ Alkylreste sind oder gemeinsam einen unsubstituierten, stickstoffhaltigen Heterocyclus bilden, und Y ein Wasserstoffatom bedeutet,
- oder das Symbol X ein Rest N-$R_4$ ist, und das Symbol R mit $R_4$ und den Atomen, an die sie gebunden sind, einen gegebenenfalls durch 1 oder 2 Alkylreste substituierten Imidazolinyl- oder Imidazolylrest oder einen Hexahydrobenzimidazolylrest bilden, mit der Maßgabe, daß (sofern nicht anders angegeben) die Alkylreste geradkettig oder verzweigt sind und 1 bis 4 Kohlenstoffatome enthalten,

in seinem isomeren Formen und deren Gemische sowie ihre Additionssalze mit Säuren.

2. Phenothiazinderivat gemäß Anspruch 1, dadurch gekennzeichnet, daß:
das Symbol Y ein Wasserstoff- oder Halogenatom ist, die Symbole $R_1$ und $R_2$, identisch oder voneinander verschieden, Alkylreste mit 1 bis 3 Kohlenstoffatomen in gerader oder verzweigter Kette durch Cycloalkyl, Hydroxy oder Acetoxy substituiertes Alkyl mit 1 oder 2 Kohlenstoffatomen bedeuten oder gemeinsam mit dem Stickstoffatom, an das sie gebunden sind, einen gesättigten oder teilweise ungesättigten Heterocyclus mit 4 bis 7 Kettengliedern, der gegebenenfalls substituiert ist durch 1 oder 2 Methylreste oder Alkylreste mit 1 oder 2 Kohlenstoffatomen, substituiert durch Hydroxy oder Acetoxy, bilden,

- oder das Symbol X ein Sauerstoff- oder Schwefelatom oder einen Rest N-$R_4$ bedeutet, und das Symbol R einen Cycloalkylrest mit 4 bis 6 Kettengliedern oder Phenyl bedeutet, oder einen Rest -$CH_2R_3$ bedeutet, worin $R_3$ ein Wasserstoffatom, einen geradkettigen oder verzweigten Alkylrest mit 1 bis 5 Kohlenstoffatomen, einen Alkenyl- oder Alkinylrest mit 2 bis 4 Kohlenstoffatomen, einen Cycloalkylrest mit 3 bis 6 Kettengliedern oder einen gegebenenfalls substituierten Phenylrest (durch 1 oder 2 Halogenatome oder durch eine Hydroxy-, Methyl-, Methoxy-, Trifluormethyl- oder Nitrogruppe) oder einen heterocyclischen Rest, ausgewählt unter Furyl, Thienyl oder Pyridyl, bedeutet, und das Symbol $R_4$ ein Wasserstoffatom oder eine Cyanogruppe ist, mit der Maßgabe, daß das Symbol X kein Sauerstoffatom bedeuten kann, wenn gleichzeitig $R_3$ für ein Wasserstoffatom oder einen Alkylrest steht, $R_1$ und $R_2$ Alkylreste sind oder gemeinsam einen unsubstituierten, stickstoffhaltigen Heterocyclus bilden, und Y ein Wasserstoffatom bedeutet,
- oder das Symbol X ein Rest N-$R_4$ ist, und das Symbol R mit $R_4$ und den Atomen, an die sie gebunden sind, einen gegebenenfalls durch 1 oder 2 Alkylreste mit 1 oder 2 Kohlenstoffatomen substituierten Imidazolylrest oder einen Hexahydrobenzimidazolylrest bilden, in seinenisomeren Formen und deren Gemische sowie deren Additionssalze mit Säuren.

3. Phenothiazinderivat gemäß Anspruch 1, dadurch gekennzeichnet, daß:
das Symbol Y ein Wasserstoff- oder Halogenatom ist, die Symbole $R_1$ und $R_2$, identisch oder voneinander

verschieden, Alkylreste mit 1 oder 2 Kohlenstoffatomen, mit der Ausnahme, daß sie gleichzeitig Methylreste bedeuten, Acetoxyalkylreste, deren Alkylteil 1 oder 2 Kohlenstoffatome enthält, bedeuten oder gemeinsam mit dem Stickstoffatom, an das sie gebunden sind, einen gesättigten oder teilweise ungesättigten Heterocyclus mit 4 bis 7 Kettengliedern, der gegebenenfalls durch 1 oder 2 Methyl-, Hydroxymethyl- oder Acetoxymethylreste substituiert ist, bilden, und

- entweder das Symbol X ein Sauerstoff- oder Schwefelatom oder einen Rest $N-R_4$ bedeutet, und das Symbol R einen Cycloalkylrest mit 4 bis 6 Kettengliedern bedeutet oder einen Rest $-CH_2R_3$ wiedergibt, worin $R_3$ ein geradkettiger oder verzweigter Alkylrest mit 1 bis 5 Kohlenstoffatomen, ein Alkenyl- oder Alkinylrest mit 2 bis 4 Kohlenstoffatomen, ein Cycloalkylrest mit 3 bis 6 Kettengliedern oder ein gegebenenfalls (durch 1 oder 2 Halogenatome oder durch eine Hydroxy-, Methyl-, Methoxy-, Trifluormethyl- oder Nitrogruppe) substituierter Phenylrest oder ein heterocyclischer Rest, ausgewählt unter Furyl, Thienyl oder Pyridyl, ist, und das Symbol $R_4$ ein Wasserstoffatom oder eine Cyanogruppe bedeutet, mit der Maßgabe, daß das Symbol X kein Sauerstoffatom bedeuten kann, wenn gleichzeitig $R_3$ ein Wasserstoffatom oder ein Alkylrest ist, $R_1$ und $R_2$ Alkylreste sind oder gemeinsam einen unsubstituierten, stickstoffhaltigen Heterocyclus bilden, und Y ein Wasserstoffatom bedeutet,
- oder das Symbol X ein Rest $N-R_4$ ist, und das Symbol R mit $R_4$ und den Atomen, an die sie gebunden sind, einen gegebenenfalls durch einen Alkylrest mit 1 oder 2 Kohlenstoffatomen substituierten Imidazolylrest oder einen Hexahydrobenzimidazolylrest bilden, in der L-Form oder in Form eines Gemisches der Isomeren sowie deren Additionssalze mit Säuren.

**4.** N-Cyclobutylmethyl-10-[1-(pyrolidin-1-yl)-prop-2-yl]-phenothiazin-2-carboxamid in der L-Form in Form eines Gemisches der Isomeren sowie dessen Additionssalze mit Säuren.

**5.** N-3-Chlorbenzyl-10-[1-(pyrolidin-1-yl)-prop-2-yl]-phenothiazin-2-carboxamid in der L-Form in Form eines Gemisches der Isomeren sowie dessen Additionssalze mit Säuren.

**6.** N-2-Chlorbenzyl-10-[1-(pyrolidin-1-yl)-prop-2-yl]-phenothiazin-2-carboxamid in der L-Form in Form eines Gemisches der Isomeren sowie dessen Additionssalze mit Säuren.

**7.** N-2-Fluorbenzyl-10-[1-(pyrolidin-1-yl)-prop-2-yl]-phenothiazin-2-carboxamid in der L-Form in Form eines Gemisches der Isomeren sowie dessen Additionssalze mit Säuren.

**8.** N-Benzyl-10-[1-(pyrolidin-1-yl)-prop-2-yl]-phenothiazincarboxamid in der L-Form in Form eines Gemisches der Isomeren sowie dessen Additionssalze mit Säuren.

**9.** Verfahren zur Herstellung eines Phenothiazinderivats gemäß Anspruch 1, worin X wie in Anspruch 1 definiert ist, mit Ausnahme der Bedeutung eines Restes $N-R_4$, worin $R_4$ mit R und den benachbarten Atomen einen Imidazolylrest bildet, dadurch gekennzeichnet, daß man ein Amin der allgemeinen Formel

$$R - NH_2$$

worin R wie in Anspruch 1 definiert ist, oder ein Diamin (wenn X einen Rest NH wiedergibt, und wenn man ein Phenothiazinderivat erhalten möchte, in dem R und $R_4$ miteinander verbunden sind, um mit den Stickstoff- und Kohlenstoffatomen, an die sie gebunden sind, einen gegebenenfalls substituierten Imidazolinylrest oder einen Hexahydrobenzimidazolylrest zu bilden) mit einem Phenothiazinderivat der allgemeinen Formel

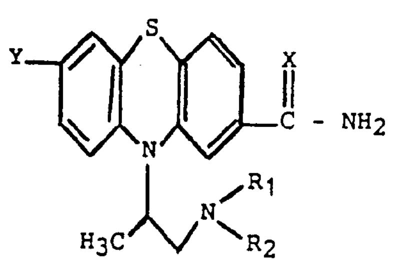

umsetzt, worin Y, $R_1$ und $R_2$ wie in Anspruch 1 definiert sind, und X für ein Schwefelatom oder einen Rest $N-R_4$ steht, worin $R_4$ wie in Anspruch 1 definiert ist, wonach man gegebenenfalls, wenn man das Amid erhalten möchte, worin X ein Sauerstoffatom ist, wenn man intermediär das substituierte Thioamid, worin X für ein Schwefelatom steht, isoliert hat, dieses Thioamid zu dem entsprechenden Amid nach jeder bekannten Methode, die den Rest des Moleküls nicht beeinträchtigt, oxidiert, wonach man gegebenenfalls das erhaltene Produkt in ein Additionssalz mit einer Säure überführt.

**10.** Verfahren zur Herstellung eines Phenothiazinderivats gemäß Anspruch 1, worin X ein Sauerstoffatom bedeutet, und Y, $R_1$, $R_2$ und R wie in Anspruch 1 definiert sind, dadurch gekennzeichnet, daß man eine Säure der allgemeinen Formel

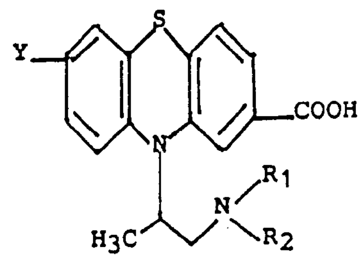

worin Y, $R_1$ und $R_2$ wie in Anspruch 1 definiert sind, oder ein reaktives Derivat dieser Säure durch Umsetzung mit einem Amin der allgemeinen Formel

$$R - NH_2$$

worin R wie in Anspruch 1 definiert ist, umwandelt, wonach man gegebenenfalls das erhaltene Produkt in ein Additionssalz mit einer Säure überführt.

**11.** Verfahren zur Herstellung eines Phenothiazinderivats gemäß Anspruch 1, worin X ein Sauerstoffatom bedeutet, und Y, $R_1$, $R_2$ und R wie in Anspruch 1 definiert sind, dadurch gekennzeichnet, daß man ein Nitril der allgemeinen Formel

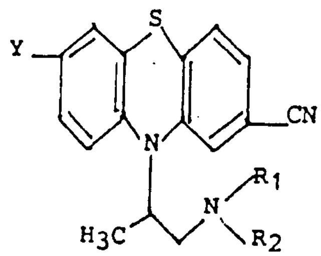

worin Y, $R_1$ und $R_2$ wie in Anspruch 1 definiert sind, nach jeder für die Erzielung eines substituierten Amids aus einem Nitril ohne Beeinträchtigung des Rests des Moleküls bekannten Methode umwandelt, wonach man gegebenenfalls das erhaltene Produkt in ein Additionssalz mit einer Säure überführt.

**12.** Verfahren zur Herstellung eines Phenothiazinderivats gemäß Anspruch 1, worin X ein Rest N-$R_4$ ist, worin $R_4$ für ein Wasserstoffatom steht und R wie in Anspruch 1 definiert ist, oder $R_4$ mit R und den Atomen, an die sie gebunden sind, einen gegebenenfalls substituierten Imidazolinylrest oder einen Hexahydrobenzimidazolylrest bildet, und die anderen Reste wie in Anspruch 1 definiert sind, dadurch gekennzeichnet, daß man ein Amin der allgemeinen Formel

$$R - NH_2$$

worin R wie in Anspruch 1 definiert ist, oder ein Diamin (wenn man ein Produkt gemäß Anspruch 1 erhalten möchte, worin R und $R_4$ mit den benachbarten Atomen einen sich von dem Imidazol ableitenden Rest bilden) mit einem Iminoether der Formel

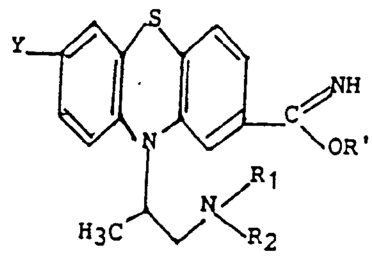

worin Y, $R_1$ und $R_2$ wie in Anspruch 1 definiert sind, und R' einen Alkylrest (mit 1 bis 10 Kohlenstoffatomen) bedeutet, oder mit dem Salz dieses Produktes umsetzt, und anschließend gegebenenfalls das erhaltene Produkt in ein Additionssalz mit einer Säure überführt.

13. Verfahren zur Herstellung eines Phenothiazinderivats gemäß Anspruch 1, worin X ein Rest N-$R_4$ ist, worin $R_4$ mit R einen gegebenenfalls durch einen Alkylrest substituierten Imidazolylrest bildet, dadurch gekennzeichnet, daß man ein α-Halogenketon oder einen α-Halgenaldehyd der allgemeinen Formel

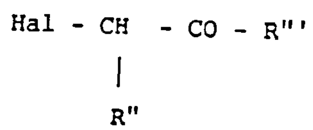

worin Hal für ein Halogenatom steht, und R" und R‴, identisch oder voneinander verschieden, ein Wasserstoffatom oder einen Alkylrest bedeuten, mit einem Phenothiazinderivat der allgemeinen Formel

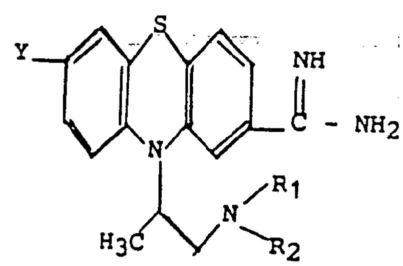

worin Y, $R_1$ und $R_2$ wie in Anspruch 1 definiert sind, oder mit seinem Salz umsetzt und danach gegebenenfalls das erhaltene Produkt in ein Additionssalz mit einer Säure überführt.

14. Pharmazeutische Zusammensetzung, dadurch gekennzeichnet, daß sie ein Produkt gemäß Anspruch 1 in reiner Form oder in Form einer Assoziation mit einem oder mehreren verträglichen und pharmazeutisch annehmbaren Verdünnungsmitteln oder Adjuvantien enthält.

**Patentansprüche für folgende Vertragsstaaten: ES, GR**

1. Verfahren zur Herstellung eines Phenothiazinderivats der allgemeinen Formel

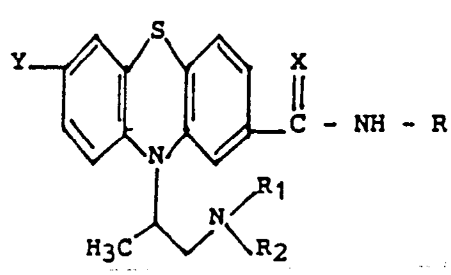

worin:

das Symbol Y ein Wasserstoff- oder Halogenatom ist, die Symbole $R_1$ und $R_2$, identisch oder voneinander verschieden, Alkyl-, Cycloalkalkyl-, Hydroxyalkyl- oder Acetoxyalkylreste bedeuten oder gemeinsam mit dem Stickstoffatom, an das sie gebunden sind, einen gesättigten oder teilweise ungesättigten Heterocyclus mit 4 bis 7 Kettengliedern, der gegebenenfalls durch 1 oder 2 Alkyl-, Hydroxyalkyl- oder Acetoxyalkylreste substituiert ist, bilden, und

- entweder das Symbol X ein Sauerstoff- oder Schwefelatom oder einen Rest N-$R_4$ bedeutet, und das Symbol R einen Cycloalkylrest mit 4 bis 6 Kettengliedern oder Phenyl bedeutet oder einen Rest -$CH_2R_3$ bedeutet, worin $R_3$ ein Wasserstoffatom, ein Alkylrest mit 1 bis 5 Kohlenstoffatomen, ein Alkenyl- oder Alkinylrest mit 2 bis 4 Kohlenstoffatomen, ein Cycloalkylrest mit 3 bis 6 Kettengliedern oder ein gegebenenfalls (durch 1 oder 2 Halogenatome oder durch eine Hydroxy-, Alkyl-, Alkoxy-, Trifluormethyl- oder Nitrogruppe) substituierter Phenylrest oder ein heterocyclischer Rest, ausgewählt unter Furyl, Thienyl oder Pyridyl, ist, und das Symbol $R_4$ ein Wasserstoffatom oder eine Cyanogruppe bedeutet, mit der Maßgabe, daß das Symbol X kein Sauerstoffatom bedeuten kann, wenn gleichzeitig $R_3$ ein Wasserstoffatom oder ein Alkylrest ist, $R_1$ und $R_2$ Alkylreste sind oder gemeinsam einen unsubstituierten, stickstoffhaltigen Heterocyclus bilden, und Y ein Wasserstoffatom bedeutet,
- oder das Symbol X ein Rest N-$R_4$ ist, und das Symbol R mit $R_4$ und den Atomen, an die sie gebunden sind, einen gegebenenfalls durch 1 oder 2 Alkylreste substituierten Imidizolinylrest oder einen Hexahydrobenzimidazolylrest bildet, wobei die Alkylreste, sofern nicht anders angegeben, geradkettig oder verzweigt sind und 1 bis 4 Kohlenstoffatome enthalten, in seinenisomeren Formen und von deren Gemischen, dadurch gekennzeichnet, daß man ein Amin der allgemeinen Formel

$$R - NH_2$$

worin R wie vorstehend definiert ist, oder ein Diamin (wenn X einen Rest NH bedeutet, und wenn man ein Phenothiazinderivat erhalten möchte, in dem R und $R_4$ miteinander verbunden sind, um mit den Stickstoff- und Kohlenstoffatomen, an die sie gebunden sind, einen gegebenenfalls substituierten Imidazolinylrest oder einen Hexahydrobenzimidazolylrest zu bilden) mit einem Phenothiazinderivat der allgemeinen Formel

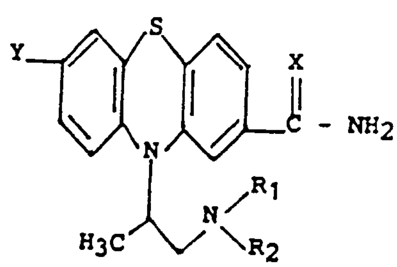

worin Y, $R_1$, $R_2$ wie vorstehend definiert sind, und X ein Schwefelatom oder einen Rest N-$R_4$ bedeutet, worin $R_4$ wie vorstehend definiert ist, umsetzt, danach gegebenenfalls, wenn man das Amid erhalten möchte, worin X ein Sauerstoffatom bedeutet, wenn man intermediär das substituierte Thioamid isoliert hat, worin X ein Schwefelatom bedeutet, dieses Thioamid zu dem entsprechenden Amid nach jeder bekannten Methode, die nicht den Rest des Moleküls verändert, oxidiert, wonach man gegebenenfalls das erhaltene Produkt in ein Additionssalz mit einer Säure überführt.

2. Verfahren zur Herstellung eines Phenothiazinderivats der allgemeinen Formel

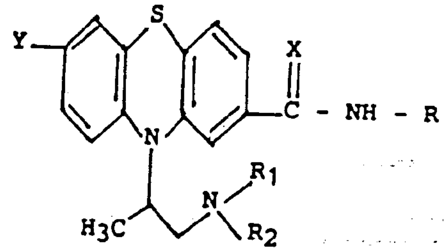

worin:

das Symbol Y ein Wasserstoff- oder Halogenatom ist, die Symbole $R_1$ und $R_2$, identisch oder voneinander verschieden, Alkyl-, Cycloalkalkyl-, Hydroxyalkyl- oder Acetoxyalkylreste bedeuten oder gemeinsam mit dem Stickstoffatom, an das sie gebunden sind, einen gesättigten oder teilweise ungesättigten Heterocyclus mit 4 bis 7 Kettengliedern, der gegebenenfalls durch 1 oder 2 Alkyl-, Hydroxyalkyl- oder Acetoxyalkylreste substituiert ist, bilden, und

-entweder das Symbol X ein Sauerstoffatom bedeutet, und das Symbol R einen Cycloalkylrest mit 4 bis 6 Kettengliedern oder Phenyl wiedergibt oder einen Rest $-CH_2R_3$ bedeutet, worin $R_3$ ein Wasserstoffatom, einen Alkylrest mit 1 bis 5 Kohlenstoffatomen, einen Alkenyl- oder Alkinylrest mit 2 bis 4 Kohlenstoffatomen, einen Cycloalkylrest mit 3 bis 6 Kettengliedern oder ein gegebenenfalls (durch 1 oder 2 Halogenatome oder durch eine Hydroxy-, Alkyl-, Alkoxy-, Trifluormethyl- oder Nitrogruppe) substituierter Phenylrest oder ein Heterocyclus, ausgewählt unter Furyl, Thienyl oder Pyridyl, ist, mit der Maßgabe, daß $R_3$ nicht Wasserstoff oder Alkyl bedeuten kann, wenn $R_1$ und $R_2$ Alkyl bedeuten, oder einen unsubstituierten, stickstoffhaltigen Heterocyclus bilden, und Y ein Wasserstoffatom ist, wobei die Alkylreste, sofern nicht anders angegeben, geradkettig oder verzweigt sind und 1 bis 4 Kohlenstoffatome enthalten, in Form seiner Isomeren und von deren Gemischen, dadurch gekennzeichnet, daß man eine Säure der allgemeinen Formel

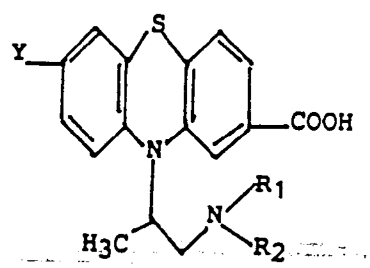

worin Y, $R_1$ und $R_2$ wie vorstehend definiert sind, oder ein reaktives Derivat dieser Säure durch Umsetzung eines Amins der allgemeinen Formel

$$R - NH_2$$

worin R wie vorstehend definiert ist, umwandelt und danach gegebenenfalls das erhaltene Produkt in ein Additionssalz mit einer Säure überführt.

3. Verfahren zur Herstellung eines Phenothiazinderivats, worin X, Y, $R_1$, $R_2$ und R wie in Anspruch 2 definiert sind, dadurch gekennzeichnet, daß man ein Nitril der allgemeinen Formel

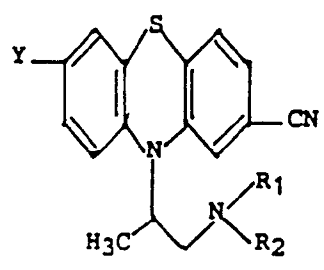

worin Y, $R_1$ und $R_2$ wie vorstehend definiert sind, nach jeder bekannten Methode für die Erzielung eines substituierten Amids aus einem Nitril ohne Beeinträchtigung des Rests des Moleküls umwandelt, wonach man gegebenenfalls das erhaltene Produkt in ein Additionssalz mit einer Säure überführt.

4. Verfahren zur Herstellung eines Phenothiazinderivats der allgemeinen Formel

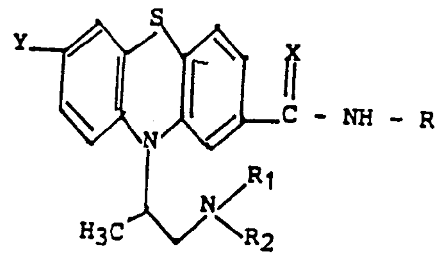

worin:

das Symbol Y ein Wasserstoff- oder Halogenatom ist, die Symbole $R_1$ und $R_2$, identisch oder voneinander verschieden, Alkyl-, Cycloalkalkyl-, Hydroxyalkyl- oder Acetoxyalkylreste bedeuten oder gemeinsam mit dem Stickstoffatom, an das sie gebunden sind, einen gesättigten oder teilweise ungesättigten Heterocyclus mit 4 bis 7 Kettengliedern, der gegebenenfalls durch 1 oder 2 Alkyl-, Hydroxyalkyl- oder Acetoxyalkylreste substituiert ist, bilden, X ein Rest N-$R_4$ ist, worin $R_4$ für ein Wasserstoffatom steht, und R einen Cycloalkylrest mit 4 bis 6 Kettengliedern oder Phenyl bedeutet oder einen Rest -$CH_2R_3$ wiedergibt, worin $R_3$ ein Wasserstoffatom, ein Alkylrest mit 1 bis 5 Kohlenstoffatomen, ein Alkenyl- oder Alkinylrest mit 2 bis 4 Kohlenstoffatomen, ein Cycloalkylrest mit 3 bis 6 Kettengliedern oder ein gegebenenfalls (durch 1 oder 2 Halogenatome oder durch eine Hydroxy-, Alkyl-, Alkoxy-, Trifluormethyl- oder Nitrogruppe) substituierter Phenylrest oder ein heterocyclischer Rest, ausgewählt unter Furyl, Thienyl oder Pyridyl, ist, oder $R_4$ mit R und den Atomen, an die sie gebunden sind, einen gegebenenfalls durch 1 oder 2 Alkylreste substituierten Imidazolinylrest oder einen Hexahydrobenzimidazolylrest bilden, wobei die Alkylreste, sofern nicht anders angegeben, geradkettig oder verzweigt sind und 1 bis 4 Kohlenstoffatome enthalten, in seinen isomeren Formen und von deren Gemischen, dadurch gekennzeichnet, daß man ein Amin der allgemeinen Formel

$$R - NH_2$$

worin R wie vorstehend definiert ist, oder ein Diamin (wenn man ein Produkt erhalten möchte, worin R und $R_4$ mit den benachbarten Atomen einen sich von dem Imidazol ableitenden Rest bilden) mit einem Iminoether der allgemeinen Formel

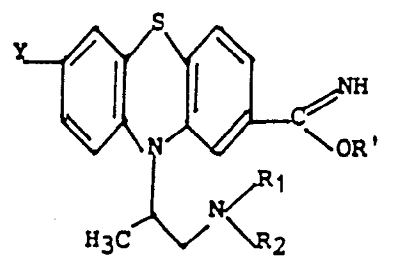

worin Y, $R_1$ und $R_2$ wie vorstehend definiert sind, und R' ein Alkylrest (mit 1 bis 10 Kohlenstoffatomen) ist oder mit dem Salz dieses Produkts umsetzt, wonach man gegebenenfalls das erhaltene Produkt in ein Additionssalz mit einer Säure überführt.

5. Verfahren zur Herstellung eines Phenothiazinderivats der allgemeinen Formel

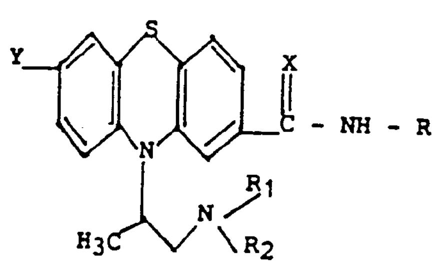

worin:

das Symbol Y ein Wasserstoff- oder Halogenatom ist, die Symbole $R_1$ und $R_2$, identisch oder voneinander verschieden, Alkyl-, Cycloalkalkyl-, Hydroxyalkyl- oder Acetoxyalkylreste bedeuten oder gemeinsam mit dem Stickstoffatom, an das sie gebunden sind, einen gesättigten oder teilweise ungesättigten Heterocyclus mit 4 bis 7 Kettengliedern, der gegebenenfalls durch 1 oder 2 Alkyl-, Hydroxyalkyl- oder Acetoxyalkylreste substituiert ist, bilden, und X ein Rest N-$R_4$ ist, worin $R_4$ mit R einen gegebenenfalls durch einen Alkylrest substituierten Imidazolylrest bildet, wobei die Alkylreste, sofern nicht anders angegeben, geradkettig oder verzweigt sind und 1 bis 4 Kohlenstoffatome enthalten, in seinen isomeren Formen und von deren Gemischen, dadurch gekennzeichnet, daß man ein α-Halogenketon oder einen α-Halogenaldehyd der allgemeinen Formel

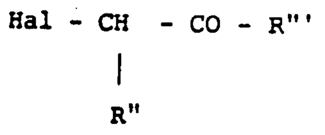

worin Hal für ein Halogenatom steht, und R" und R‴, identisch oder voneinander verschieden, ein Wasserstoffatom oder einen Alkylrest bedeuten, mit einem Phenothiazinderivat der allgemeinen Formel

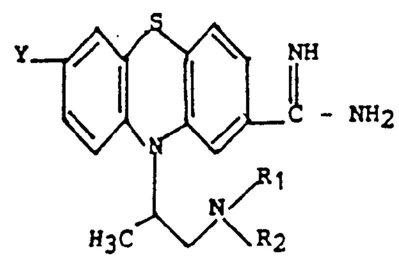

worin Y, $R_1$ und $R_2$ wie vorstehend definiert sind, oder mit seinem Salz umsetzt, wonach man gegebenenfalls das erhaltene Produkt in ein Additionssalz mit einer Säure überführt.

## Claims

### Claims for the following Contracting States: AT, BE, CH, DE, FR, GB, IT, LI, LU, NL, SE

**1.** A phenothiazine derivative, characterised in that it corresponds to the general formula:

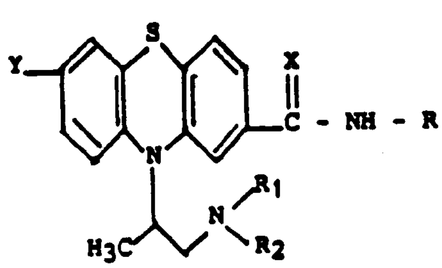

in which:
the symbol Y is a hydrogen or halogen atom, the symbols $R_1$ and $R_2$, which may be identical or different, represent alkyl, cycloalkylalkyl, hydroxyalkyl or acetyloxyalkyl radicals or, together with the nitrogen atom to which they are attached, form a saturated or partially unsaturated 4- to 7-membered heterocycle optionally substituted with 1 or 2 alkyl, hydroxyalkyl or acetyloxyalkyl radicals, and

- either the symbol X represents an oxygen or sulphur atom or a radical N-$R_4$, and the symbol R represents a 4-to 6-membered cycloalkyl radical or a phenyl radical or represents a radical -$CH_2R_3$ in which $R_3$ is a hydrogen atom, an alkyl radical containing 1 to 5 carbon atoms, an alkenyl or alkynyl radical containing 2 to 4 carbon atoms, a 3- to 6-membered cycloalkyl radical or an optionally substituted phenyl radical (optionally substituted with 1 or 2 halogen atoms or with a hydroxyl, alkyl, alkyloxy, trifluoromethyl or nitro radical), or a heterocyclic radical chosen from furyl, thienyl or pyridyl, and the symbol $R_4$ is a hydrogen atom or a cyano radical, on the understanding that the symbol X cannot represent an oxygen atom when, simultaneously, $R_3$ is a hydrogen atom or an alkyl radical, $R_1$ and $R_2$ are alkyl radicals or, together, form an unsubstituted nitrogenous heterocycle and Y is a hydrogen atom,

- or the symbol X is a radical N-$R_4$ and the symbol R, with $R_4$ and the atoms to which they are attached, forms an imidazolinyl or imidazolyl radical optionally substituted with 1 or 2 alkyl radicals, or a hexahydrobenzimidazolyl radical, on the understanding that (except where specifically stated) the alkyl radicals are unbranched or branched and contain 1 to 4 carbon atoms,

in its isomeric forms and mixtures thereof, as well as its addition salts with acids.

2. A phenothiazine derivative according to Claim 1, characterised in that:
the symbol Y is a hydrogen or halogen atom,
the symbols $R_1$ and $R_2$, which may be identical or different, represent unbranched- or branched-chain alkyl radicals containing 1 to 3 carbon atoms or alkyl radicals containing 1 or 2 carbon atoms and substituted with cycloalkyl, hydroxy or acetyloxy, or, together with the nitrogen atom to which they are attached, form a saturated or partially unsaturated 4- to 7-membered heterocycle optionally substituted with 1 or 2 methyl radicals or alkyl radicals containing 1 or 2 carbon atoms and substituted with hydroxy or acetyloxy,
   - either the symbol X represents an oxygen or sulphur atom or a radical N-$R_4$, and the symbol R represents a 4- to 6-membered cycloalkyl radical or a phenyl radical or represents a radical -$CH_2R_3$, in which $R_3$ is a hydrogen atom, an unbranched or branched alkyl radical containing 1 to 5 carbon atoms, an alkenyl or alkynyl radical containing 2 to 4 carbon atoms, a 3- to 6-membered cycloalkyl radical, or an optionally substituted phenyl radical (optionally substituted with 1 or 2 halogen atoms or with a hydroxyl, methyl, methoxy, trifluoromethyl or nitro radical), or a heterocyclic radical chosen from furyl, thienyl or pyridyl, and the symbol $R_4$ is a hydrogen atom or a cyano radical, on the understanding that the symbol X cannot represent an oxygen atom when, simultaneously, $R_3$ is a hydrogen atom or an alkyl radical, $R_1$ and $R_2$ are alkyl radicals or, together, form an unsubstituted nitrogenous heterocycle and Y is a hydrogen atom,
   - or the symbol X is a radical N-$R_4$ and the symbol R, with $R_4$ and the atoms to which they are attached, forms an imidazolyl radical optionally substituted with 1 or 2 alkyl radicals containing 1 or 2 carbon atoms, or a hexahydrobenzimidazolyl radical, in its isomeric forms and mixtures thereof, as well as its addition salts with acids.

3. A phenothiazine derivative according to Claim 1, characterised in that:
the symbol Y is a hydrogen or halogen atom, the symbols $R_1$ and $R_2$, which may be identical or different, represent alkyl radicals containing 1 or 2 carbon atoms with the exception of simultaneously representing methyl radicals, or acetyloxyalkyl radicals in which the alkyl portion contains 1 or 2 carbon atoms, or, together with the nitrogen atom to which they are attached, form a saturated or partially unsaturated 4- to 7-membered heterocycle optionally substituted with 1 or 2 methyl, hydroxymethyl or acetyloxymethyl radicals, and
   - either the symbol X represents an oxygen or sulphur atom or a radical N-$R_4$, and the symbol R represents a 4- to 6-membered cycloalkyl radical or represents a radical -$CH_2R_3$, in which $R_3$ is an unbranched or branched alkyl radical containing 1 to 5 carbon atoms, an alkenyl or alkynyl radical containing 2 to 4 carbon atoms, a 3- to 6-membered cycloalkyl radical, or an optionally substituted phenyl radical (optionally substituted with 1 or 2 halogen atoms or with a hydroxyl, methyl, methoxy, trifluoromethyl or nitro radical), or a heterocyclic radical chosen from furyl, thienyl or pyridyl, and the symbol $R_4$ is a hydrogen atom or a cyano radical, on the understanding that the symbol X cannot represent an oxygen atom when, simultaneously, $R_3$ is a hydrogen atom or an alkyl radical, $R_1$ and $R_2$ are alkyl radicals or, together, form an unsubstituted nitrogenous heterocycle and Y is a hydrogen atom,
   - or the symbol X is a radical N-$R_4$ and the symbol R, with $R_4$ and the atoms to which they are attached, forms an imidazolyl radical optionally substituted with 1 alkyl radical containing 1 or 2 carbon atoms, or a hexahydrobenzimidazolyl radical, in L form or in the form of a mixture of isomers, as well as its addition salts with acids.

4. N-Cyclobutylmethyl-10-[1-(1-pyrrolidinyl)-2-propyl]-2-phenothiazinecarboxamide, in L form or in the form of a mixture of isomers, as well as its addition salts with acids.

5. N-(3-Chlorobenzyl)-10-[1-(1-pyrrolidinyl)-2-propyl]-2-phenothiazinecarboxamide, in L form or in the form of a mixture of isomers, as well as its addition salts with acids.

6. N-(2-Chlorobenzyl)-10-[1-(1-pyrrolidinyl)-2-propyl]-2-phenothiazinecarboxamide, in L form or in the form of a mixture of isomers, as well as its addition salts with acids.

7. N-(2-Fluorobenzyl)-10-[1-(1-pyrrolidinyl)-2-propyl]-2-phenothiazinecarboxamide, in L form or in the form of a mixture of isomers, as well as its addition salts with acids.

8. N-Benzyl-10-[1-(1-pyrrolidinyl)-2-propyl]-2-phenothiazinecarboxamide, in L form or in the form of a mixture of isomers, as well as its addition salts with acids.

9. Process for preparing a phenothiazine derivative according to Claim 1, for which X is defined as in Claim 1 with the exception of representing a radical N-$R_4$ in which $R_4$, with R and the neighbouring atoms, forms an imidazolyl radical, characterised in that an amine of general formula:

$$R - NH_2$$

in which R is defined as in Claim 1, or a diamine (if X represents an NH radical and if it is desired to obtain a phenothiazine derivative in which R and $R_4$ are linked together to form, with the nitrogen and carbon atoms to which they are attached, an optionally substituted imidazolinyl radical or a hexahydrobenzimidazolyl radical), is reacted with a phenothiazine derivative of general formula:

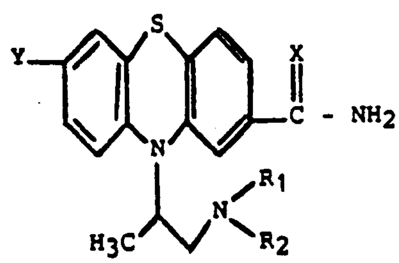

in which Y, $R_1$ and $R_2$ are defined as in Claim 1 and X is a sulphur atom or a radical N-$R_4$ in which $R_4$ is defined according to Claim 1, and then, where appropriate, when it is desired to obtain the amide for which X is an oxygen atom, if the substituted thioamide for which X is a sulphur atom has been isolated as an intermediate, this thioamide is oxidised to the corresponding amide by any known method which does not adversely affect the remainder of the molecule, and the product obtained is then optionally converted to an addition salt with an acid.

10. Process for preparing a phenothiazine derivative according to Claim 1 for which X is an oxygen atom and Y, $R_1$, $R_2$ and R are defined as in Claim 1, characterised in that an acid of general formula:

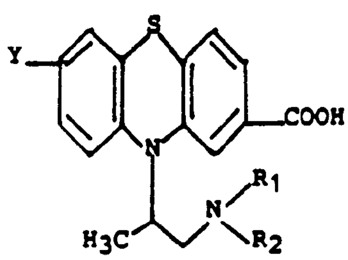

in which Y, $R_1$ and $R_2$ are defined as in Claim 1, or a reactive derivative of this acid, is converted by the action of an amine of general formula:

$$R - NH_2$$

in which R is defined as in Claim 1, and the product obtained is then optionally converted to an addition salt with an acid.

11. Process for preparing a phenothiazine derivative according to Claim 1 for which X is an oxygen atom and Y, $R_1$, $R_2$ and R are defined as in Claim 1, characterised in that a nitrile of general formula:

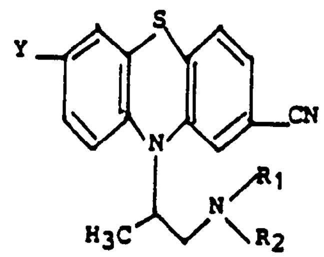

in which Y, $R_1$ and $R_2$ are defined as in Claim 1, is converted by any known method for obtaining a substituted amide from a nitrile without affecting the remainder of the molecule, and the product obtained is then optionally converted to an addition salt with an acid.

12. Process for preparing a phenothiazine derivative according to Claim 1 for which X is a radical N-$R_4$ for which $R_4$ is a hydrogen atom and R is defined as in Claim 1, or $R_4$, with R and the atoms to which they are attached, forms an optionally substituted imidazolinyl radical or a hexahydrobenzimidazolyl radical, and the other radicals are defined as in Claim 1, characterised in that an amine of general formula:

$$R - NH_2$$

in which R is defined as in Claim 1, or a diamine (when it is desired to obtain a product according to Claim 1 for which R and $R_4$, with the neighbouring atoms, form a radical derived from imidazole), is reacted with an imino ether of formula:

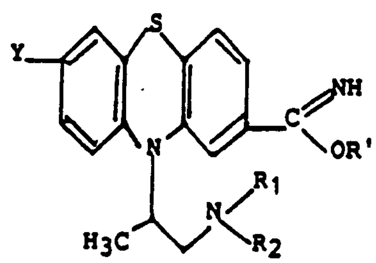

in which Y, $R_1$ and $R_2$ are defined as in Claim 1 and R' is an alkyl radical (containing 1 to 10 carbon atoms), or with the salt of this product, and the product obtained is then optionally converted to an addition salt with an acid.

13. Process for preparing a phenothiazine derivative according to Claim 1 for which X is a radical N-$R_4$ in which $R_4$, with R, forms an imidazolyl radical optionally substituted with an alkyl radical, characterised in that an α-halo ketone or an α-halo aldehyde of general formula:

Hal - CH - CO - R'''
|
R''

in which Hal is a halogen atom and R'' and R''', which may be identical or different, represent a hydrogen atom or an alkyl radical, is reacted with a phenothiazine derivative of general formula:

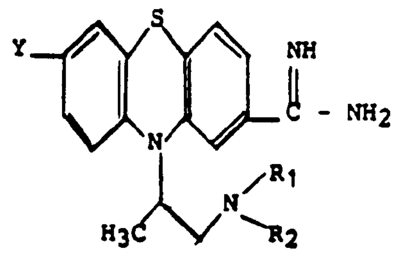

in which Y, $R_1$ and $R_2$ are defined as in Claim 1, or with its salt, and the product obtained is then optionally converted to an addition salt with an acid.

**14.** Pharmaceutical composition, characterised in that it contains a product according to Claim 1, in the pure state or in the form of a combination with one or more compatible and pharmaceutically acceptable diluents or adjuvants.

**Claims for the following Contracting States: ES, GR**

**1.** Process for preparing a phenothiazine derivative of general formula:

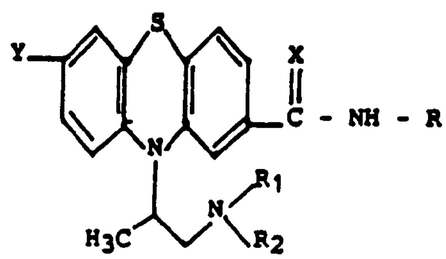

in which:
the symbol Y is a hydrogen or halogen atom, the symbols $R_1$ and $R_2$, which may be identical or different, represent alkyl, cycloalkylalkyl, hydroxyalkyl or acetyloxyalkyl radicals or, together with the nitrogen atom to which they are attached, form a saturated or partially unsaturated 4- to 7-membered heterocycle optionally substituted with 1 or 2 alkyl, hydroxyalkyl or acetyloxyalkyl radicals, and

- either the symbol X represents an oxygen or sulphur atom or a radical N-$R_4$, and the symbol R represents a 4- to 6-membered cycloalkyl radical or a phenyl radical or represents a radical -$CH_2R_3$ in which $R_3$ is a hydrogen atom, an alkyl radical containing 1 to 5 carbon atoms, an alkenyl or alkynyl radical containing 2 to 4 carbon atoms, a 3- to 6-membered cycloalkyl radical or an optionally substituted phenyl radical (optionally substituted with 1 or 2 halogen atoms or with a hydroxyl, alkyl, alkyloxy, trifluoromethyl or nitro radical), or a heterocyclic radical chosen from furyl, thienyl or pyridyl, and the symbol $R_4$ is a hydrogen atom or a cyano radical, on the understanding that the symbol X cannot represent an oxygen atom when, simultaneously, $R_3$ is a hydrogen atom or an alkyl radical, $R_1$ and $R_2$ are alkyl radicals or, together, form an unsubstituted nitrogenous heterocycle and Y is a hydrogen atom,
- or the symbol X is a radical N-$R_4$ and the symbol R, with $R_4$ and the atoms to which they are attached, forms an imidazolinyl radical optionally substituted with 1 or 2 alkyl radicals, or a hexahydrobenzimidazolyl radical, the alkyl radicals being, except where specifically stated, unbranched or branched and containing 1 to 4 carbon atoms, in its isomeric forms and mixtures thereof, characterised in that an amine of general formula:

$$R - NH_2$$

in which R is defined as above, or a diamine (if X represents an NH radical and if it is desired to obtain a phenothiazine derivative in which R and $R_4$ are linked together to form, with the nitrogen and carbon atoms to which they are attached, an optionally substituted imidazolinyl radical or a hexahydrobenzimidazolyl radical), is reacted with a phenothiazine derivative of general formula:

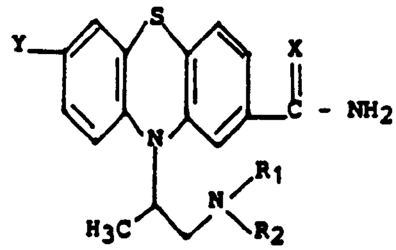

in which Y, $R_1$ and $R_2$ are defined as above and X is a sulphur atom or a radical N-$R_4$ in which $R_4$ is defined as above, and then, where appropriate, when it is desired to obtain the amide for which X is an oxygen atom, if the substituted thioamide for which X is a sulphur atom has been isolated as an intermediate, this thioamide is oxidised to the corresponding amide by any known method which does not adversely affect the remainder of the molecule, and the product obtained is then optionally converted to an addition salt with an acid.

2. Process for preparing a phenothiazine derivative of general formula:

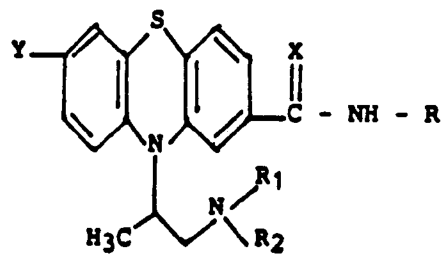

in which:
the symbol Y is a hydrogen or halogen atom, the symbols $R_1$ and $R_2$, which may be identical or different, represent alkyl, cycloalkylalkyl, hydroxyalkyl or acetyloxyalkyl radicals or, together with the nitrogen atom to which they are attached, form a saturated or partially unsaturated 4- to 7-membered heterocycle optionally substituted with 1 or 2 alkyl, hydroxyalkyl or acetyloxyalkyl radicals, and
the symbol X represents an oxygen atom and the symbol R represents a 4- to 6-membered cycloalkyl radical or a phenyl radical or represents a radical -$CH_2R_3$ in which $R_3$ is a hydrogen atom, an alkyl radical containing 1 to 5 carbon atoms, an alkenyl or alkynyl radical containing 2 to 4 carbon atoms, a 3- to 6-membered cycloalkyl radical, or an optionally substituted phenyl radical (optionally substituted with 1 or 2 halogen atoms or with a hydroxyl, alkyl, alkyloxy, trifluoromethyl or nitro radical), or a heterocyclic radical chosen from furyl, thienyl or pyridyl, on the understanding that $R_3$ cannot represent hydrogen or alkyl if $R_1$ and $R_2$ are alkyl or form an unsubstituted nitrogenous heterocycle and Y is a hydrogen atom, the alkyl radicals being, except where specifically stated, unbranched or branched and containing 1 to 4 carbon atoms, in its isomeric forms and mixtures thereof, characterised in that an acid of general formula:

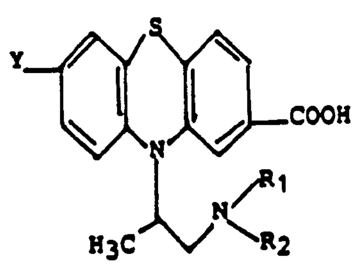

in which Y, $R_1$ and $R_2$ are defined as above, or a reactive derivative of this acid, is converted by the action of an amine of general formula:

$$R - NH_2$$

in which R is defined as above, and the product obtained is then optionally converted to an addition salt

with an acid.

3. Process for preparing a phenothiazine derivative for which X, Y, $R_1$, $R_2$ and R are defined as in Claim 2, characterised in that a nitrile of general formula:

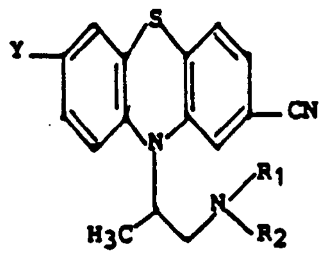

in which Y, $R_1$ and $R_2$ are defined as above, is converted by any known method for obtaining a substituted amide from a nitrile without affecting the remainder of the molecule, and the product obtained is then optionally converted to an addition salt with an acid.

4. Process for preparing a phenothiazine derivative of general formula:

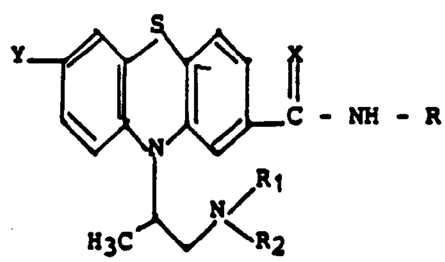

in which:
the symbol Y is a hydrogen or halogen atom, the symbols $R_1$ and $R_2$, which may be identical or different, represent alkyl, cycloalkylalkyl, hydroxyalkyl or acetyloxyalkyl radicals or, together with the nitrogen atom to which they are attached, form a saturated or partially unsaturated 4- to 7-membered heterocycle optionally substituted with 1 or 2 alkyl, hydroxyalkyl or acetyloxyalkyl radicals, X is a radical N-$R_4$ for which $R_4$ is a hydrogen atom and R represents a 4- to 6-membered cycloalkyl radical or a phenyl radical or represents a radical -$CH_2R_3$ in which $R_3$ is a hydrogen atom, an alkyl radical containing 1 to 5 carbon atoms, an alkenyl or alkynyl radical containing 2 to 4 carbon atoms, a 3- to 6-membered cycloalkyl radical, or an optionally substituted phenyl radical (optionally substituted with 1 or 2 halogen atoms or with a hydroxyl, alkyl, alkyloxy, trifluoromethyl or nitro radical), or a heterocyclic radical chosen from furyl, thienyl or pyridyl, or $R_4$, with R and the atoms to which they are attached, forms an imidazolinyl radical optionally substituted with 1 or 2 alkyl radicals, or a hexahydrobenzimidazolyl radical, the alkyl radicals being, except where specifically stated, unbranched or branched and containing 1 to 4 carbon atoms, in its isomeric forms and mixtures thereof, characterised in that an amine of general formula:

$$R - NH_2$$

in which R is defined as above, or a diamine (when it is desired to obtain a product for which R and $R_4$, with the neighbouring atoms, form a radical derived from imidazole), is reacted with an imino ether of general formula:

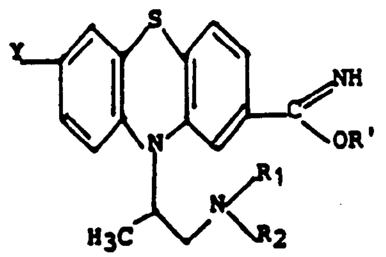

in which Y, $R_1$ and $R_2$ are defined as above and R' is an alkyl radical (containing 1 to 10 carbon atoms), or with the salt of this product, and the product obtained is then optionally converted to an addition salt with an acid.

5. Process for preparing a phenothiazine derivative of general formula:

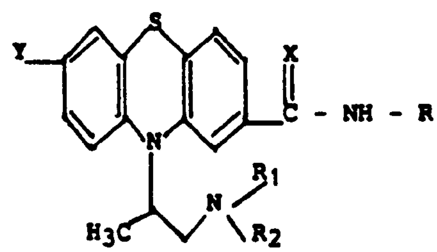

in which:
the symbol Y is a hydrogen or halogen atom, the symbols $R_1$ and $R_2$, which may be identical or different, represent alkyl, cycloalkylalkyl, hydroxyalkyl or acetyloxyalkyl radicals or, together with the nitrogen atom to which they are attached, form a saturated or partially unsaturated 4- to 7-membered heterocycle optionally substituted with 1 or 2 alkyl, hydroxyalkyl or acetyloxyalkyl radicals, and X is a radical N-$R_4$ in which $R_4$, with R, forms an imidazolyl radical optionally substituted with an alkyl radical, the alkyl radicals being, except where specifically stated, unbranched or branched and containing 1 to 4 carbon atoms, in its isomeric forms and mixtures thereof, characterised in that an $\alpha$-halo ketone or an $\alpha$-halo aldehyde of general formula:

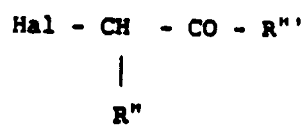

in which Hal is a halogen atom and R" and R''', which may be identical or different, represent a hydrogen atom or an alkyl radical, is reacted with a phenothiazine derivative of general formula:

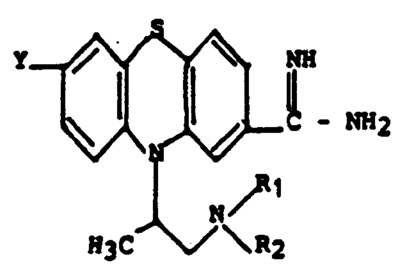

in which Y, $R_1$ and $R_2$ are defined as above, or with its salt, and the product obtained is then optionally converted to an addition salt with an acid.